# EUROPÄISCHE PATENTSCHRIFT

(11) **EP 2 205 590 B1**
(45) Veröffentlichungstag und Bekanntmachung des Hinweises auf die Patenterteilung: **04.06.2014**
(21) Anmeldenummer: 08839851.6
(22) Anmeldetag: 16.10.2008
(51) Int. Cl.: C07D 401/14, C07D 413/14, C07D 471/04, C07D 405/14, C07D 417/14, A61K 31/506, A61K 31/551, A61P 29/00

(54) **CGRP ANTAGONISTEN**
CGRP ANTAGONISTS
ANTAGONISTES DU CGRP

(30) Priorität: 18.10.2007 EP 07118809
(43) Veröffentlichungstag der Anmeldung: 14.07.2010
(73) Patentinhaber: Boehringer Ingelheim International GmbH, 55216 Ingelheim am Rhein (DE)
(72) Erfinder: GOTTSCHLING, Dirk, 55216 Ingelheim Am Rhein (DE); DAHMANN, Georg, 55216 Ingelheim Am Rhein (DE); DOODS, Henri, 55216 Ingelheim Am Rhein (DE); HEIMANN, Annekatrin, 55216 Ingelheim Am Rhein (DE); MUELLER, Stephan Georg, 55216 Ingelheim Am Rhein (DE); RUDOLF, Klaus, 55216 Ingelheim Am Rhein (DE); SCHAENZLE, Gerhard, 55216 Ingelheim Am Rhein (DE); STENKAMP, Dirk, 55216 Ingelheim Am Rhein (DE)
(74) Vertreter: Simon, Elke Anna Maria
(86) Internationale Anmeldenummer: PCT/EP2008/063965
(87) Internationale Veröffentlichungsnummer: WO 2009/050232

(56) Entgegenhaltungen:
- WO-A-2006/100009
- WO-A-2007/045672

## Beschreibung

Gegenstand der vorliegenden Erfindung sind neue CGRP-Antagonisten der allgemeinen Formel **I** 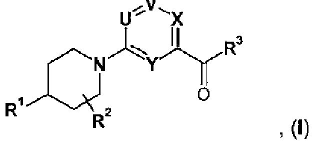 in der **U, V, X, Y, R¹, R²** und **R³** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Die Dokumente WO 2006/100009 and WO 2007/045672 beschreiben bereits Verbindungen mit CGRP-antagonistischen Eigenschaften. Die Verbindungen der vorliegenden Anmeldung unterscheiden sich von denen aus dem Stand der Technik zumindest durch den zentralen (hetero)aromatischen Sechsring.

### DETAILLIERTE BESCHREIBUNG DER ERFINDUNG

In der obigen allgemeinen Formel **I** bedeuten in einer ersten Ausführungsform **R¹** eine Gruppe der allgemeinen Formel 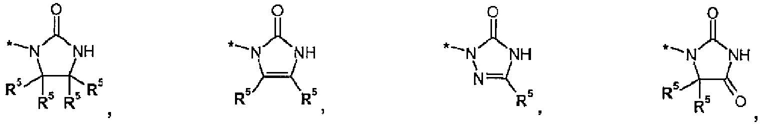 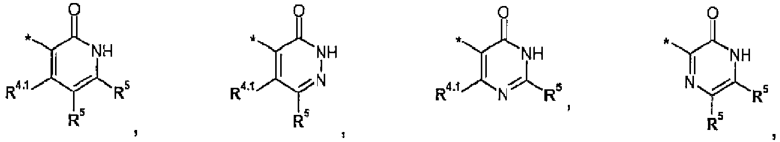 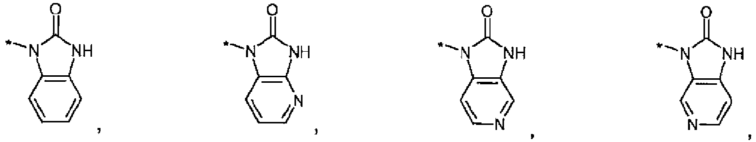 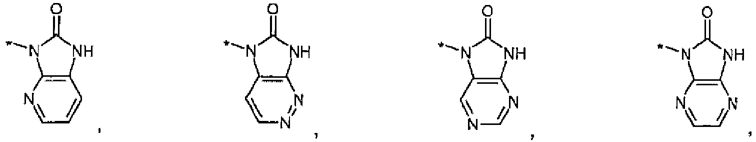 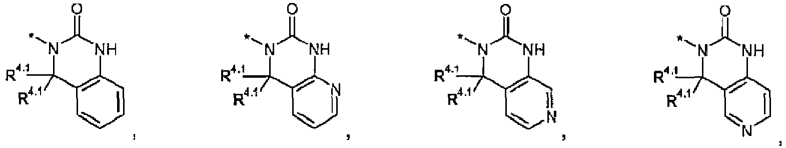 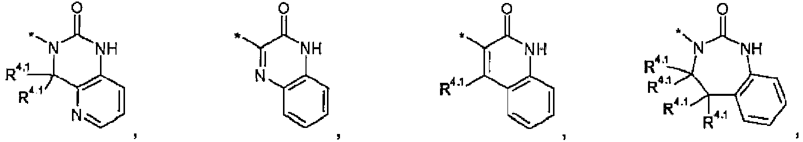 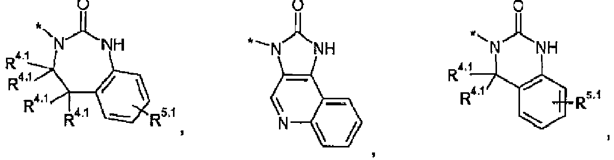
- **R²**: (a) H,
(b) F, -CN, C₁₋₃-Alkyl, -CO₂-R^{2.1} oder
(c) eine C₁₋₃-Alkylgruppe, in der jede Methylengruppe mit bis zu zwei Fluoratomen und jede Methylgruppe mit bis zu drei Fluoratomen substituiert ist,
- **R^{2.1}**: H oder C₁₋₆-Alkyl,
- **R³**: eine mit den Resten **R^{3.1}, R^{3.2}** und **R^{3.3}** substituierte 6- oder 10-gliedrige Arylgruppe oder,
eine mit den Resten **R^{3.1}, R^{3.2}** und **R^{3.3}** substituierte 6-gliedrige Heteroarylgruppe, die über ein Kohlenstoffatom angebunden ist,
- **R^{3.1}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, **R^{3.1.1}** -C₁₋₃-Alkylen, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.1.2}**,
(f) -S(O)₂-**R^{3.1.3}**,
- **R^{3.1.1}**: (a) H,
(b) C₃₋₆-Cycloalkyl, C₅₋₆-Cycloalkenyl,
(c) (**R^{3.1.1.1}**)₂N-,
(d) einen gesättigten, einfach oder zweifach ungesättigten 5- oder 6-gliedrigen Heterocyclus, der an einem Stickstoffatom mit einem Rest **R^{3.1.1.1}** und an einem Kohlenstoffatom mit einem oder zwei Resten **R^{3.1.1.2}** substituiert ist, oder
(e) eine Heteroarylgruppe, die an einem Kohlenstoffatom mit einem Rest **R^{3.1.1.2}** substituiert ist,
- **R^{3.1.1.1}**: unabhängig voneinander
(a) H, C₁₋₄-Alkyl, C₃₋₆-Cycloalkyl,
(b) Heterocyclyl,
(c) Aryl-C₀₋₃-alkylen oder Heteroaryl-C₀₋₃-alkylen,
- **R^{3.1.1.2}**: unabhängig voneinander
(a) H, F, C₁₋₃-Alkyl, -CN, -OH, -O-C₁₋₃-Alkyl, -CO(O)**R^{3.1.1.2.1}**, H₂N-, (C₁₋₄-Alkyl)-NH-, (C₁₋₄-Alkyl)₂N-,
(b) Phenyl oder Phenyl-CH₂-,
(c) eine C₁₋₃-Alkyl- oder -O-C₁₋₃-Alkylgruppe, in der jede Methylengruppe mit bis zu zwei Fluoratomen und jede Methylgruppe mit bis zu drei Fluoratomen substituiert ist, oder
- **R^{3.1.1.2.1}**: H, C₁₋₆-Alkyl, Benzyl,
- **R^{3.1.2}**: -O-C₁₋₃-Alkyl, -OH, -N**R^{3.1.21}R^{3.1.22}**,
- **R^{3.1.2.1}**: H, C₁₋₃-Alkyl,
- **R^{3.1.2.2}**: H, C₁₋₃-Alkyl,
- **R^{3.1.2.1}** und **R^{3.1.2.2}**: zusammen auch einen Ring ausbilden können, der ausgewählt ist aus der Gruppe bestehend aus Azetidinyl, Pyrrolidinyl, Piperidinyl, Piperazinyl und Morpholinyl,
- **R^{3.1.3}**: -O-C₁₋₃-Alkyl, -N**R^{3.1.3.1}R^{3.1.3.2}**,
- **R^{3.1.3.1}**: H, C₁₋₃-Alkyl,
- **R^{3.1.3.2}**: H, C₁₋₃-Alkyl,
- **R^{3.1.3.1}** und **R^{3.1.3.2}**: zusammen auch einen Ring ausbilden können, der ausgewählt ist aus der Gruppe bestehend aus Azetidinyl, Pyrrolidinyl, Piperidinyl, Piperazinyl und Morpholinyl,
- **R^{3.2}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.2.1}**
(f) -S(O)₂-**R^{3.2.2}**,
- **R^{3.2.1}**: -O-C₁₋₃-Alkyl, -OH, -N**R^{3.2.1.1}R^{3.2.1.2}**,
- **R^{3.2.1.1}**: H, C₁₋₃-Alkyl,
- **R^{3.2.1.2}**: H, C₁₋₃-Alkyl,
- **R^{3.2.1.1}** und **R^{3.2.1.2}**: zusammen auch einen Ring ausbilden können, der ausgewählt ist aus der Gruppe bestehend aus Azetidinyl, Pyrrolidinyl, Piperidinyl, Piperazinyl und Morpholinyl,
- **R^{3.2.2}**: **-NR^{3.2.2.1}R^{3.2.2.2},**
- **R^{3.2.2.1}**: H, C₁₋₃-Alkyl,
- **R^{3.2.2.2}**: H, C₁₋₃-Alkyl,
- **R^{3.2.2.1}** und **R^{3.2.2.2}**: zusammen auch einen Ring ausbilden können, der ausgewählt ist aus der Gruppe bestehend aus Azetidinyl, Pyrrolidinyl, Piperidinyl, Piperazinyl und Morpholinyl,
- **R^{3.3}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.3.1}**,
(f) -S(O)₂-**R^{3.3.2}**,
- **R^{3.3.1}**: -O-C₁₋₃-Alkyl, -OH, -N**R^{3.3.1.1}R^{3.3.1.2}**,
- **R^{3.3.1.1}**: H, C₁₋₃-Alkyl,
- **R^{3.3.1.2}**: H, C₁₋₃-Alkyl,
- **R^{3.3.1.1}** und **R^{3.3.1.2}**: zusammen auch einen Ring ausbilden können, der ausgewählt ist aus der Gruppe bestehend aus Azetidinyl, Pyrrolidinyl, Piperidinyl, Piperazinyl und Morpholinyl,
- **R^{3.3.2}**: -O-C₁₋₃-Alkyl, -N**R^{3.3.2.1}R^{3.3.2.2}**,
- **R^{3.3.2.1}**: H, C₁₋₃-Alkyl,
- **R^{3.3.2.2}**: H, C₁₋₃-Alkyl,
- **R^{3.3.2.1}** und **R^{3.3.2.2}**: zusammen auch einen Ring ausbilden können, der ausgewählt ist aus der Gruppe bestehend aus Azetidinyl, Pyrrolidinyl, Piperidinyl, Piperazinyl und Morpholinyl, oder
- **R^{3.2}** und **R^{3.3}**: zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyl oder
(b) C₃₋₆-Cycloalkyl,
- **R^{3.3.4}**: unabhängig voneinander
(a) C₁₋₄-Alkyl oder
(b) C₃₋₆-Cycloalkyl,
(c) Halogen, CN, -O-C₁₋₃-Alkyl, -NH₂,
(d) eine C₁₋₃-Alkyl- oder C₁₋₃-Alkyl-O-gruppe, in der jede Methylengruppe mit bis zu zwei und jede Methylgruppe mit bis zu drei Fluoratomen substituiert ist,
- **R^{4.1}**: (a) H,
(b) C₁₋₃-Alkyl, -OH, -O-C₁₋₃-Alkyl,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R⁵**: (a) H, C₁₋₆Alkyl, C₃₋₆-Cycloalkyl,
(b) eine gegebenenfalls mit 1, 2, oder 3 Substituenten **R^{5.2}** substituierte Phenylgruppe, wobei die Substituenten **R^{5.2}** gleich oder verschieden sein können,
(c) eine gegebenenfalls mit 1, 2 oder 3 Substituenten **R^{5.2}** substituierte Heteroarylgruppe, die ausgewählt ist aus der Gruppe bestehend aus Benzimidazol, Benzothiophen, Furan, Imidazol, Indol, Isoxazol, Oxazol, Pyrazin, Pyrazol, Pyridazin, Pyridin, Pyrimidin, Pyrrol, Thiazol, Thiophen und Triazol, wobei die Substituenten **R^{5.2}** gleich oder verschieden sein können,
(d) einen gegebenenfalls mit 1, 2 oder 3 Substituenten **R^{5.2}** substituierten Heterocyclus, wobei die Substituenten **R^{5.2}** gleich oder verschieden sein können,
- **R^{5.1}**: (a) H, Halogen, C₁₋₆-Alkyl, C₃₋₆-Cycloalkyl,
(b) -O-C₁₋₆-Alkylen-N**R⁷R⁸**, -O-**R⁶**, -CO₂**R⁶**, -C(O)-N**R⁷R⁸**, -SO₂-N**R⁷R⁸**, -N**R⁷**-SO₂-**R⁶**, -S(O)ₘ-**R⁷**, -CN, -N**R⁷R⁸**, -O-C(O)-**R⁶** oder
(c) eine C₁₋₃-Alkyl oder C₁₋₃-Alkyl-O-gruppe, in der jede Methylengruppe mit bis zu zwei Fluoratomen und jede Methylgruppe mit bis zu drei Fluoratomen substituiert ist,
- **R^{5.2}**: (a) Halogen, C₁₋₆-Alkyl, C₃₋₆-Cycloalkyl,
(b) -O-**R⁶**, -O-(CH₂)ₛ-O-**R⁶**, -CO₂**R⁶**, -S(O)ₘ-**R⁶**, -CN, -O-C(O)-**R⁶** oder
(c) eine C₁₋₃-Alkylgruppe, in der jede Methylengruppe mit bis zu zwei Fluoratomen und jede Methylgruppe mit bis zu drei Fluoratomen substituiert ist,
- **R⁶**: (a) H,
(b) C₁₋₆-Alkyl, C₃₋₆-Cycloalkyl, Aryl, Heteroaryl, Benzyl, die mit einem Rest **R^{6.1}** substituiert sein können, oder
(c) eine C₁₋₃-Alkylgruppe, in der jede Methylengruppe mit bis zu zwei Fluoratomen und jede Methylgruppe mit bis zu drei Fluoratomen substituiert ist,
- **R^{6.1}**: HO- oder C₁₋₆-Alkyl-O-,
- **R⁷**: (a) H,
(b) C₁₋₃-Alkyl, Phenyl oder Benzyl, wobei die Reste unsubstituiert oder substituiert sein können mit Halogen, HO- oder H₃C-O-,
- **R⁸**: (a) H,
(b) C₁₋₃-Alkyl, Phenyl oder Benzyl, wobei die Reste unsubstituiert oder substituiert sein können mit Halogen, HO- oder H₃C-O-, oder
**R⁷** und **R⁸** zusammen auch einen Ring ausbilden können, der ausgewählt ist aus der Gruppe bestehend aus Azetidinyl, Pyrrolidinyl, Piperidinyl, Piperazinyl und Morpholinyl, wobei der Ring unsubstituiert oder mit einem Substituenten **R⁶** substituiert sein kann,
- **m**: eine der Ziffern 0, 1 oder 2, und
- **s**: eine der Ziffern 1, 2 oder 3 bedeutet,
- **U**: N, N-Oxid oder C-**R⁹**,
- **V**: N, N-Oxid oder C-**R¹⁰**,
- **X**: N, N-Oxid oder C**R¹¹**,
- **Y**: N oder C-**R¹²**,
wobei höchstens drei der voranstehend genannten Reste **U, V, X** oder **Y** gleichzeitig ein Stickstoffatom darstellen,
- **R⁹**: (a) H,
(b) eine C₁₋₆-Alkyl- oder C₁₋₃-Alkyl-O-gruppe, die jeweils mit einem Rest **R^{9.1}** substituiert ist,
(c) **R^{9.2}R^{9.3}**N-, **R^{9.2}R^{9.3}**N-C₁₋₃-alkylen-,
(d) Halogen, -CN, -OH, -COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{9.1}**: H, OH oder -O-CH₃,
- **R^{9.2}**: H oder C₁₋₃-Alkyl,
- **R^{9.3}**: H oder C₁₋₃-Alkyl, oder
- **R^{9.2}** und **R^{9.3}**: zusammen mit dem Stickstoffatom, an das sie gebunden sind, einen 3- bis 6-gliedrigen Heterocyclus,
- **R¹⁰**: (a) H,
(b) eine C₁₋₆-Alkyl- oder C₁₋₃-Alkyl-O-gruppe, die jeweils mit einem Rest **R^{10.1}** substituiert ist,
(c) -N**R^{10.2}R^{10.3}**, N**R^{10.2}R^{10.3}**-C₁₋₃-Alkylen-,
(d) Halogen, -CN, -OH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine Aryl-C₀₋₃-alkylen-O-gruppe,
(f) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{10.1}**: H, OH oder-D-CH₃,
- **R^{10.2}**: H oder C₁₋₆-Alkyl,
- **R^{10.3}**: H, C₁₋₆-Alkyl oder -SO₂-C₁₋₃-Alkyl, oder
- **R^{10.2}** und **R^{10.3}**: zusammen mit dem Stickstoffatom, an das sie gebunden sind, einen 3- bis 6-gliedrigen Heterocyclus,
- **R¹¹**: (a) H,
(b) eine C₁₋₆-Alkyl- oder C₁₋₃-Alkyl-O-gruppe, die jeweils mit einem Rest **R^{11.1}** substituiert ist,
(c) **R^{11.2}R^{11.3}**N-, **R^{11.2}R^{11.3}**N-C₁₋₃-alkylen-,
(d) Halogen, -CN, -OH, _-COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₂-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{11.1}**: H, OH oder -O-CH₃,
- **R^{11.2}**: H oder C₁₋₃-Alkyl,
- **R^{11.3}**: H oder C₁₋₃-Alkyl, oder
- **R^{11.2}** und **R^{11.3}**: zusammen mit dem Stickstoffatom, an das sie gebunden sind, einen 3- bis 6-gliedrigen Heterocyclus, und
- **R¹²**: H, Halogen oder C₁₋₃-Alkyl,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zweite Ausführungsform der vorliegenden Erfindung besteht in den Verbindungen der obigen allgemeinen Formel **I**, in denen **U, V, X, Y, R²** und **R³** wie voranstehend unter der ersten Ausführungsform definiert sind und
- **R¹**: eine Gruppe ausgewählt aus 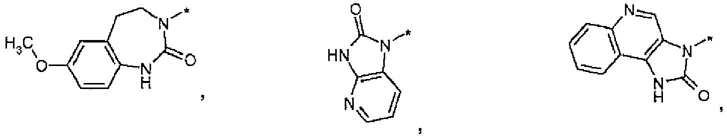 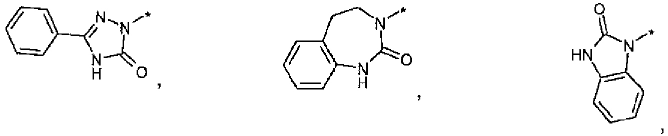 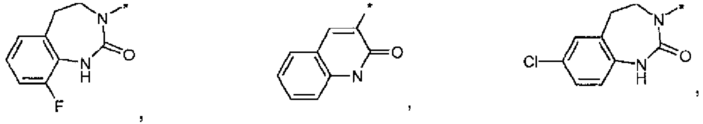
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel **I**, in denen **U, V, X, Y, R¹** und **R³** wie voranstehend unter der ersten oder zweiten Ausführungsform definiert sind und **R²** ein Wasserstoffatom bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierte Ausführungsform der vorliegenden Erfindung besteht in den Verbindungen der obigen allgemeinen Formel **I**, in denen **U,** V, **X,** Y, **R¹** und **R²** wie voranstehend unter der ersten, zweiten oder dritten Ausführungsform definiert sind und
- **R³**: eine Gruppe der allgemeinen Formel **III** 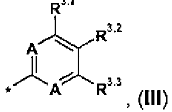
- **A**: unabhängig voneinander CH oder N,
- **R^{3.1}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O)₂-NH-, -CN, -OH, -O-C(O)-NH-C₁₋₃-Alkyl,
(c) C₁₋₄-Alkyl, **R^{3.1.1}**-C₁₋₃-Alkylen, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.1.2}**,
(f) -S(O)₂-**R^{3.1.3}**,
- **R^{3.1.1}**: (a) H,
(b) C₃₋₆-Cycloalkyl, C₅₋₆-Cycloalkenyl,
(c) (**R^{3.1.1.1}**)₂N-,
(d) einen gesättigten, einfach oder zweifach ungesättigten 5- oder 6-gliedrigen Heterocyclus, der an einem Stickstoffatom mit einem Rest **R^{3.1.1.1}** und an einem Kohlenstoffatom mit einem oder zwei Resten **R^{3.1.1.2}** substituiert ist, oder
(e) eine Heteroarylgruppe, die an einem Kohlenstoffatom mit einem Rest **R^{3.1.1.2}** substituiert ist,
- **R^{3.1.1.1}**: unabhängig voneinander
(a) H, C₁₋₄-Alkyl, C₃₋₆-Cycloalkyl,
(b) Heterocyclyl,
(c) Aryl-C₀₋₃-alkylen oder Heteroaryl-C₀₋₃-alkylen,
- **R^{3.1.1.2}**: unabhängig voneinander
(a) H, F, C₁₋₃-Alkyl, -CN, -OH, -O-C₁₋₃-Alkyl, -CO(O)**R^{3.1.1.2.1}**, H₂N-, (C₁₋₄-Alkyl)-NH-, (C₁₋₄-Alkyl)₂N-,
(b) Phenyl oder Phenyl-CH₂-,
(c) eine C₁₋₃-Alkyl- oder -O-C₁₋₃-Alkylgruppe, in der jede Methylengruppe mit bis zu zwei Fluoratomen und jede Methylgruppe mit bis zu drei Fluoratomen substituiert ist, oder
- **R^{3.1.1.2.1}**: H, C₁₋₆-Alkyl, Benzyl,
- **R^{3.1.2}**: -O-C₁₋₃-Alkyl, -OH, -N**R^{3.1.2.1}R^{3.1.2.2}**,
- **R^{3.1.21}**: H, C₁₋₃-Alkyl,
- **R^{3.1.2.2}**: H, C₁₋₃-Alkyl,
- **R^{3.1.3}**: -N**R^{3.1.3.1}R^{3.1.3.2}**,
- **R^{3.1.3.1}**: H, C₁₋₃-Alkyl,
- **R^{3.1.3.2}**: H, C₁₋₃-Alkyl,
- **R^{3.2}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O)₂-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.2.1}**,
(f) -S(O)₂-**R^{3.2.2}**,
- **R^{3.2.1}**: -O-C₁₋₃-Alkyl, -OH, -N**R^{3.2.1.1}R^{3.2.1.2}**,
- **R^{3.2.1.1}**: H, C₁₋₃-Alkyl,
- **R^{3.2.1.2}**: H, C₁₋₃-Alkyl,
- **R^{3.2.2}**: -N**R^{3.2.2.1}R^{3.2.2.2}**,
- **R^{3.2.2.1}**: H, C₁₋₃-Alkyl,
- **R^{3.2.2.2}**: H, C₁₋₃-Alkyl,
- **R^{3.3}**: (a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O)₂-NH-, -CN, -OH, -O-C(O)-NH-C₁₋₃-Alkyl,
(c) C₁₋₄-Alkyl C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.3.1}**,
(f) -S(O)₂-**R^{3.3.2}**,
- **R^{3.3.1}**: -O-C₁₋₃-Alkyl, -OH, -N**R^{3.3.1.1}R^{3.3.1.2}**,
- **R^{3.3.1.1}**: H, C₁₋₃-Alkyl,
- **R^{3.3.1.2}**: H, C₁₋₃-Alkyl,
- **R^{3.3.2}**: -O-C₁₋₃-Alkyl, -N**R^{3.3.2.1}R^{3.3.2.2}**,
- **R^{3.3.2.1}**: H, C₁₋₃-Alkyl,
- **R^{3.3.2.2}**: H, C₁₋₃-Alkyl, oder
**R^{3.2}** und **R^{3.3}** zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}**substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyl oder
(b) C₃₋₆-Cycloalkyl, und
- **R^{3.3.4}**: unabhängig voneinander
(a) C₁₋₄-Alkyl, C₃₋₆-Cycloalkyl,
(b) Halogen, CN, C₁₋₃-Alkyl-O-, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe der allgemeinen Formeln **III** 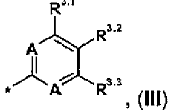
- **A**: unabhängig voneinander CH oder N,
- **R^{3.1}**: (a) H,
(b) Halogen, -NH₂, C₁₋₄-Alkyl-NH-, (C₁₋₄-Alkyl)₂N-, C₁₋₃-Alkyl-C(O)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{3.2}**: (a) H,
(b) Halogen, -NH₂, C₁₋₄-Alkyl-NH-, (C₁₋₄-Alkyl)₂N-, C₁₋₃-Alkyl-C(O)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{3.3}**: (a) H,
(b) Halogen, -NH₂, C₁₋₄-Alkyl-NH-, (C₁₋₄-Alkyl)₂N-, C₁₋₃-Alkyl-C(O)-NH-, -CN, -OH, -O-C(OFNH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
**R^{3.2}**und **R^{3.3}** zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyloder
(b) C₃₋₆-Cycloalkyl, und
- **R^{3.3.4}**: unabhängig voneinander
(a) C₁₋₄-Alkyl, C₃₋₆-Cycloalkyl,
(b) Halogen, CN, C₁₋₃-Alkyl-O-, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine sechste Ausführungsform der vorliegenden Erfindung besteht in den Verbindungen der obigen allgemeinen Formel **I**, in denen U, V, X, **Y, R¹** und **R²** wie voranstehend unter der ersten, zweiten oder dritten Ausführungsform definiert sind und
- **R³**: eine Gruppe der allgemeinen Formeln **III** 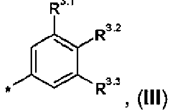
- **R^{3.1}**: (a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{3.2}**: (a) H,
(b) F, Cl, Br, H₂N-, (C₁₋₄-Alkyl)-NH-, (C₁₋₄-Alkyl)₂N-, (C₁₋₃-Alkyl)-C(O)-NH-, -OH,
(c) C₁₋₄-Alkyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{3.3}**: (a) H,
(b) F, Cl, Br, H₂N-, (C₁₋₄-Alkyl)-NH-, (C₁₋₄-Alkyl)₂N-, (C₁₋₃-Alkyl)-C(O)-NH-, -OH,
(c) C₁₋₄-Alkyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}** und **R^{3.3}** zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyl oder
(b) C₃₋₆-Cycloalkyl, und
- **R^{3.3.4}**: unabhängig voneinander
(a) C₁₋₄-Alkyl, C₃₋₆-Cycloalkyl,
(b) Halogen, CN, C₁₋₃-Alkyl-O-, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine siebte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe ausgewählt aus 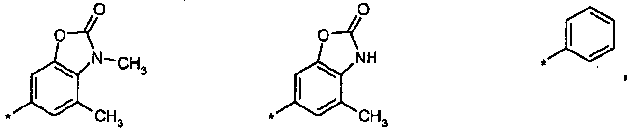 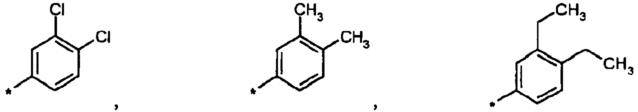 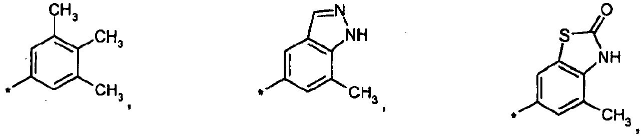 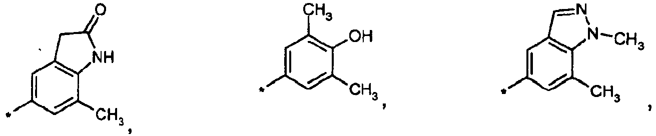 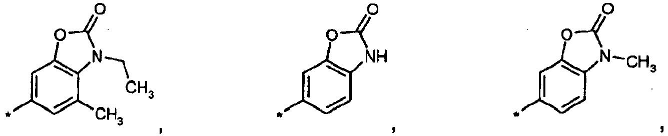 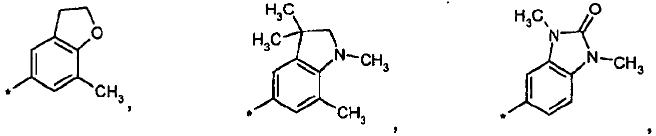 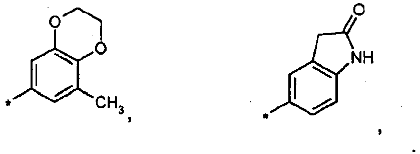
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine achte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe der allgemeinen Formeln **III** 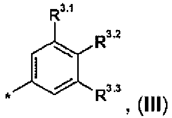
- **R^{3.1}**: (a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}** und **R^{3.3}** zusammen mit den Kohlenstoffatomen, an denen sie gebunden sind, einen einfach ungesättigten 5-gliedrigen Heterocyclus oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyl- oder
(b) C₃₋₆-Cycloalkyl, und
- **R^{3.3.4}**: unabhängig voneinander
(a) C₁₋₄-Alkyl-, C₃₋₆-Cycloalkyl-,
(b) Halogen, -CN, -O-C₁₋₃-Alkyl, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine neunte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe der allgemeinen Formeln **III** 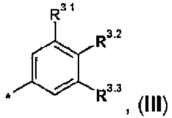
- **R^{3.1}**: (a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}** und **R^{3.3}** zusammen mit den Kohlenstoffatomen, an denen sie gebunden sind, einen einfach ungesättigten 5-gliedrigen Heterocyclus oder eine 5- gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
- **R^{3.3.3}**: unabhängig voneinander
(a) C₁₋₄-Alkyl- oder
(b) C₃₋₆-Cycloalkyl, und
- **R^{3.3.4}**: unabhängig voneinander
(a) C₁₋₄-Alkyl-, C₃₋₆-Cycloalkyl-,
(b) Halogen, -CN, -O-C₁₋₃-Alkyl, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zehnte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe der allgemeinen Formel **IIIa** 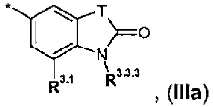
- **T**: O, S, CH₂, NH oder N-**R^{3.3.3}**,
- **R^{3.1}**: (a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, und
- **R^{3.3.3}**: unabhängig voneinander
(a) C₁₋₄-Alkyl- oder
(b) C₃₋₆-Cycloalkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine elfte Ausführungsform der vorliegenden Erfindung besteht in den Verbindungen der obigen allgemeinen Formel **I**, in denen **U, V, X, Y, R¹** und **R²** wie voranstehend unter der ersten, zweiten oder dritten Ausführungsform definiert sind und
- **R³**: eine Gruppe ausgewählt aus 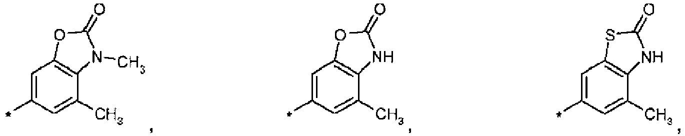 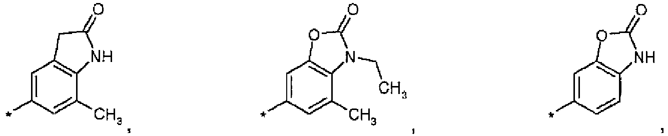 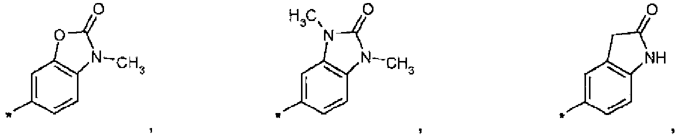 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zwölfte Ausführungsform der vorliegenden Erfindung besteht in den Verbindungen der obigen allgemeinen Formel **I**, in denen Y, **R¹, R²** und **R³** wie voranstehend unter der ersten, zweiten, dritten, vierten, fünften, sechsten, siebten, achten, neunten, zehnten oder elften Ausführungsform definiert sind und
**U-V-X** eine Gruppe ausgewählt aus
-N=N-(C-**R¹¹**)=, -N=(C-**R¹⁰**)-N=, -N=(C-**R¹⁰**)-(C-**R¹¹**)=, -(N-Oxid)=(C-**R¹⁰**)-(C-**R¹¹**)=, -(C**R⁹**)=N-N=, -(C**R⁹**)=N-(C**R¹¹**)=, -(C-**R⁹**)=N(Oxid)-(C-**R¹¹**)=, -(C**R⁹**)=(C-**R¹⁰**)-N=, -(C**R⁹**)=(C-**R¹⁰**)-(N-Oxid)=, -(C**R⁹**)=(C-**R¹⁰**)-(C**R¹¹**)=, und
- **R⁹**: (a) H,
(b) eine C₁₋₆-Alkyl- oder C₁₋₃-Alkyl-O-gruppe, die jeweils mit einem Rest **R^{9.1}** substituiert ist,
(c) **R^{9.2}R^{9.3}**N-, **R^{9.2}R^{9.3}**N-C₁₋₃-alkylen-,
(d) Halogen, -CN, -OH, _-COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{9.1}**: H, OH oder -O-CH₃,
- **R^{9.2}**: H oder C₁₋₃-Alkyl,
- **R^{9.3}**: H oder C₁₋₃-Alkyl, oder
**R^{9.2}** und **R^{9.3}** zusammen mit dem Stickstoffatom, an das sie gebunden sind, einen 3- bis 6-gliedrigen Heterocyclus,
- **R¹⁰**: (a) H,
(b) eine C₁₋₆-Alkyl- oder C₁₋₃-Alkyl-CO-gruppe, die jeweils mit einem Rest **R^{10.1}** substituiert ist,
(c) -N**R^{10.1}R^{10.2}**, N**R^{10.1}R^{10.2}**-C₁₋₃-Alkylen-,
(d) Halogen, -CN, -OH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) Aryl-C₀₋₃-alkylen-O-,
(f) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{10.1}**: H, OH oder -O-CH₃,
- **R^{10.2}**: H oder C₁₋₆-Alkyl,
- **R^{10.3}**: H, C₁₋₆-Alkyl oder -SO₂-C₁₋₃-Alkyl,
- **R¹¹**: (a) H,
(b) eine C₁₋₆-Alkyl- oder C₁₋₃-Alkyl-O-gruppe, die jeweils mit einem Rest **R^{11.1}** substituiert ist,
(c) **R^{11.2}R^{11.3}**N-, **R^{11.2}R^{11.3}**N-C₁₋₃-alkylen-,
(d) Halogen, -CN, -OH, _-COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
- **R^{11.1}**: H, OH oder -O-CH₃,
- **R^{11.2}**: H oder C₁₋₃-Alkyl,
- **R^{11.3}**: H oder C₁₋₃-Alkyl, oder
**R^{11.2}** und **R^{11.3}** zusammen mit dem Stickstoffatom, an das sie gebunden sind, einen 3- bis 6-gliedrigen Heterocyclus, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dreizehnte Ausführungsform der vorliegenden Erfindung besteht in den Verbindungen der obigen allgemeinen Formel **I**, in denen **Y, R¹, R²** und **R³** wie voranstehend unter der ersten, zweiten, dritten, vierten, fünften, sechsten, siebten, achten, neunten, zehnten oder elften Ausführungsform definiert sind und der Ring 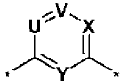 eine Gruppe ausgewählt aus 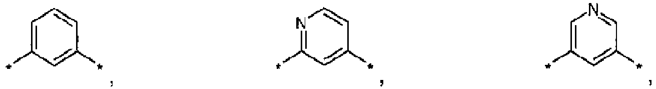 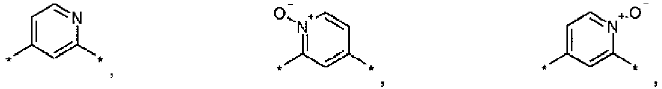 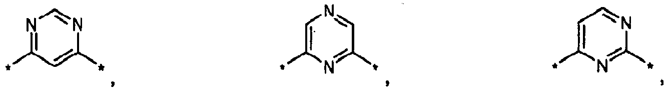 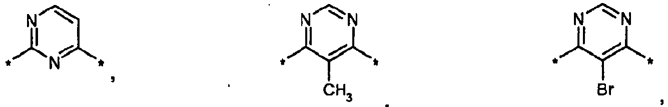 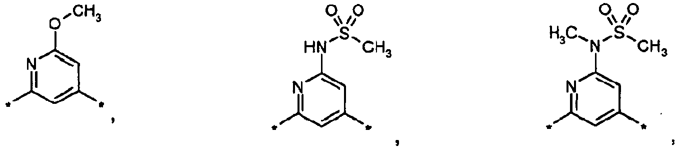 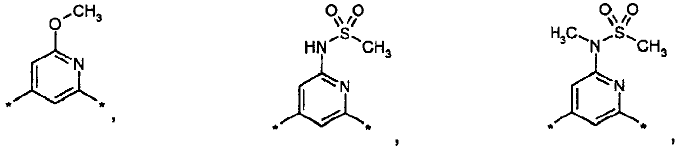 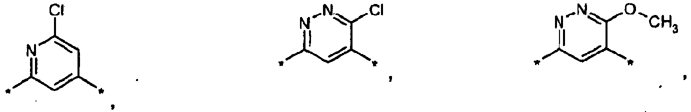 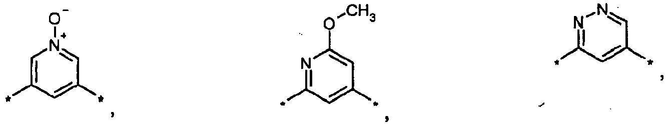 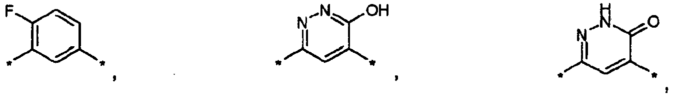 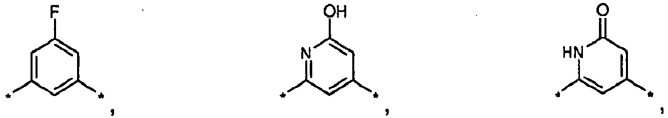 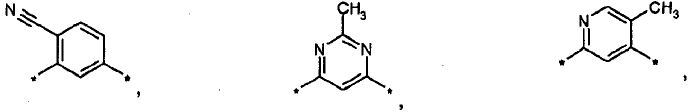 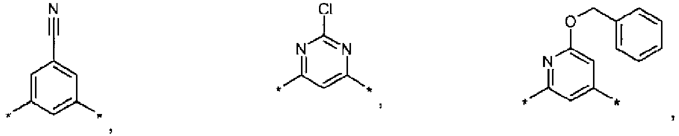 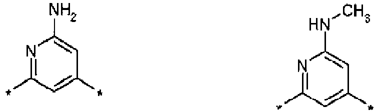 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierzehnte Ausführungsform der vorliegenden Erfindung besteht in den Verbindungen der obigen allgemeinen Formel **I**, in der
- **R¹**: eine Gruppe ausgewählt aus 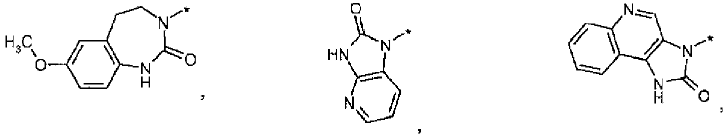 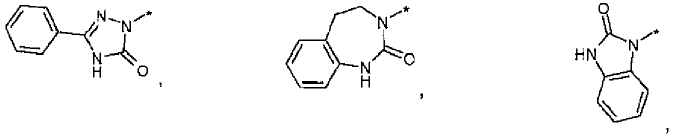 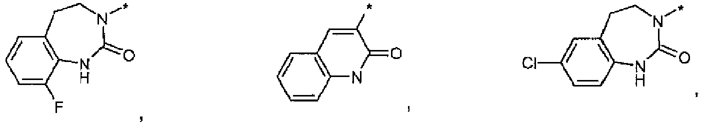
- **R²**: H und
- **R³**: eine Gruppe ausgewählt aus 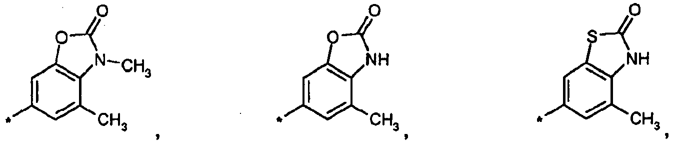 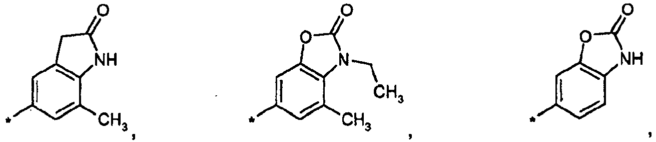 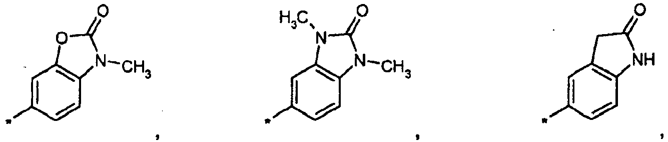 und der Ring 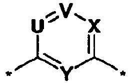 eine Gruppe ausgewählt aus 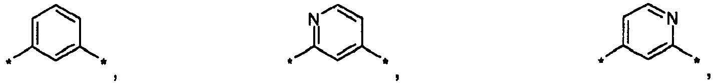 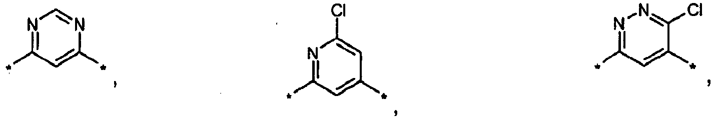 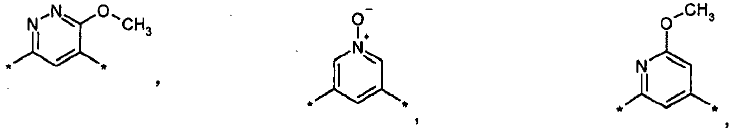 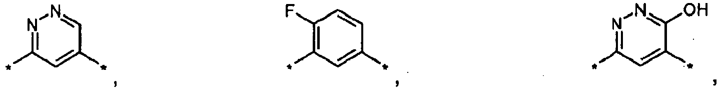 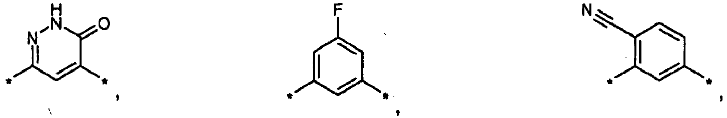 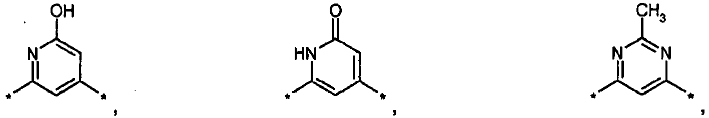 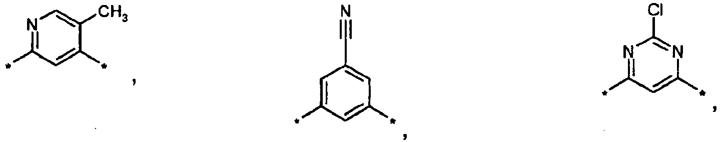 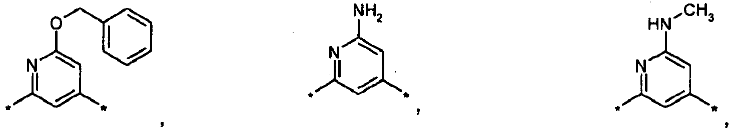
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise folgende Verbindungen genannt:

| **Nr.** | **Struktur** |
|---|---|
| (1) | 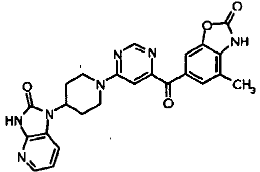 |
| (2) | 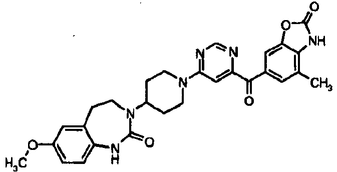 |
| (3) | 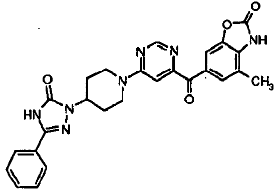 |
| (4) | 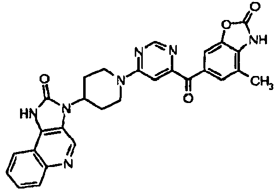 |
| (5) | 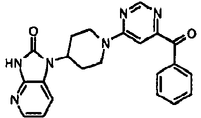 |
| (6) | 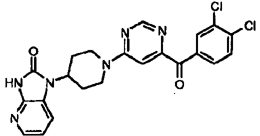 |
| (7) | 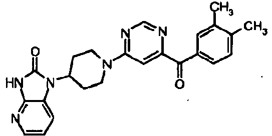 |
| (8) | 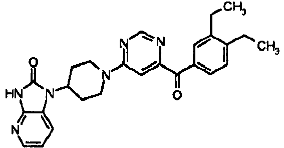 |
| (9) | 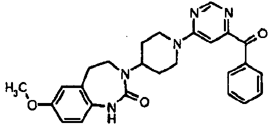 |
| (10) | 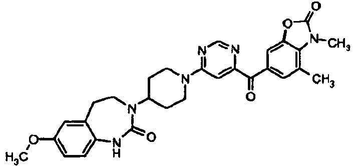 |
| (11) | 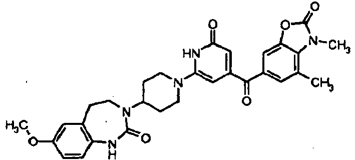 |
| (12) | 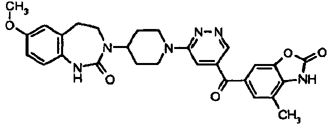 |
| (13) | 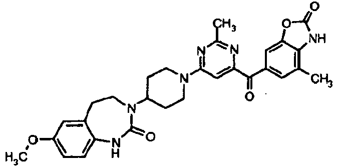 |
| (14) | 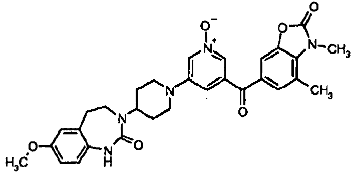 |
| (15) | 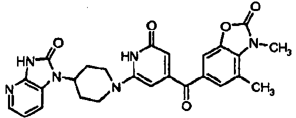 |
| (16) | 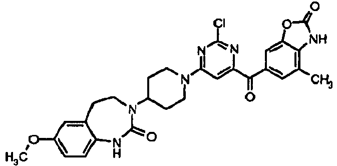 |
| (17) | 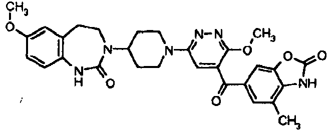 |
| (18) | 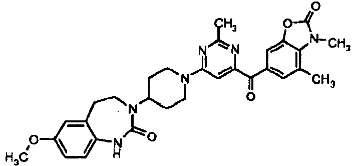 |
| (19) | 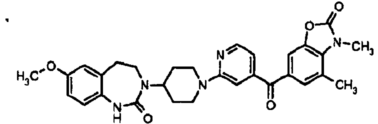 |
| (20) | 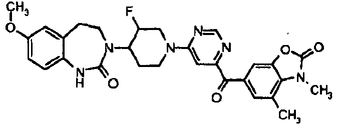 |
| (21) | 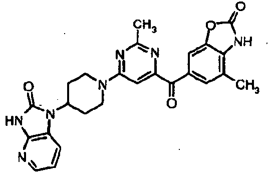 |
| (22) | 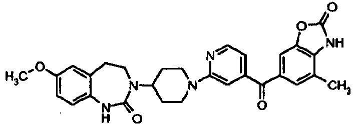 |
| (23) | 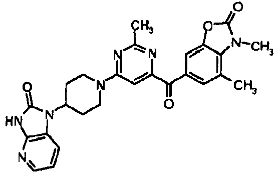 |
| (24) | 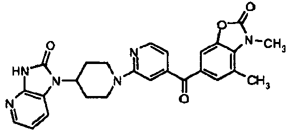 |
| (25) | 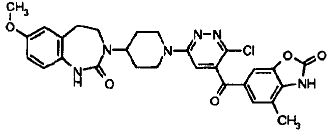 |
| (26) | 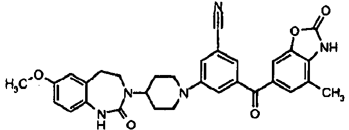 |
| (27) | 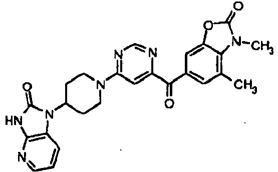 |
| (28) | 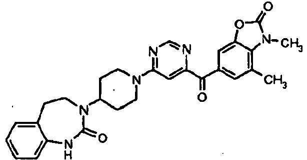 |
| (29) | 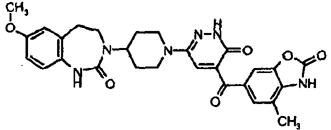 |
| (30) | 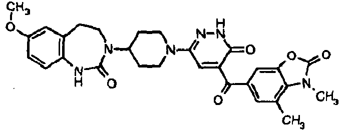 |
| (31) | 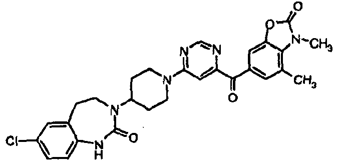 |
| (32) | 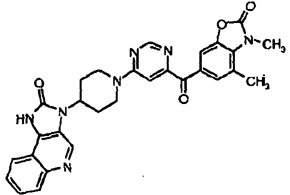 |
| (33) | 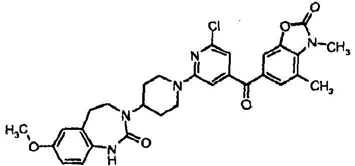 |
| (34) | 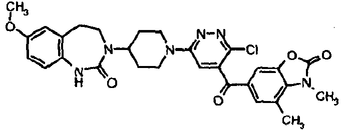 |
| (35) | 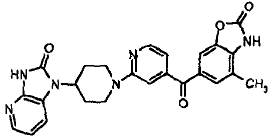 |
| (36) | 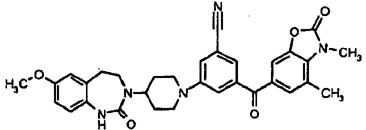 |
| (37) | 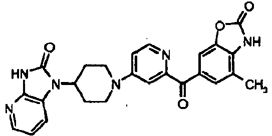 |
| (38) | 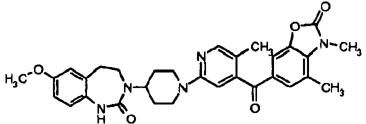 |
| (39) | 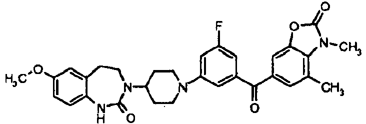 |
| (40) | 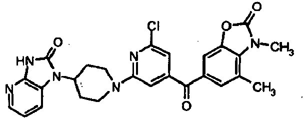 |
| (41) | 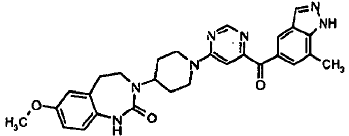 |
| (42) | 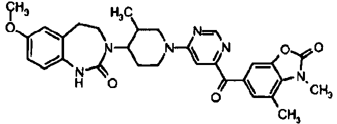 |
| (43) | 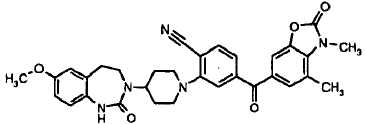 |
| (44) | 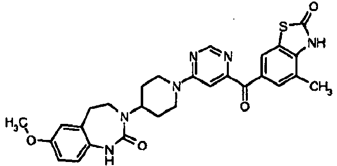 |
| (45) | 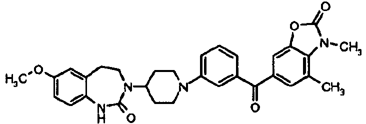 |
| (46) | 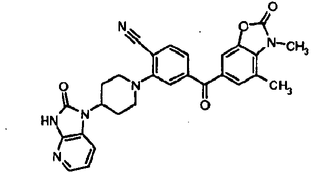 |
| (47) | 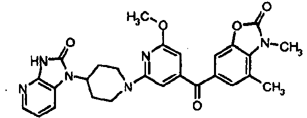 |
| (48) | 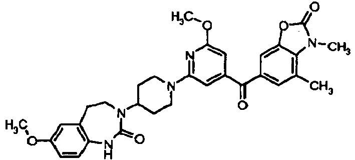 |
| (49) | 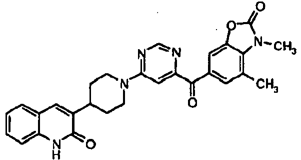 |
| (50) | 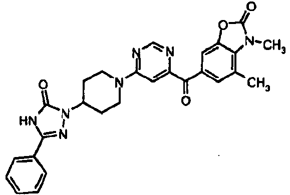 |
| (51) | 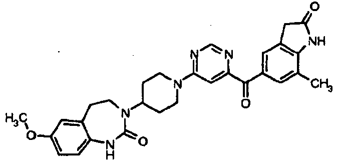 |
| (52) | 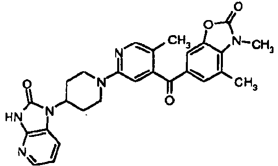 |
| (53) | 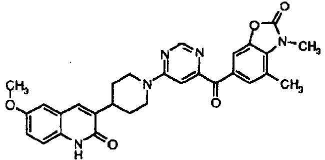 |
| (54) | 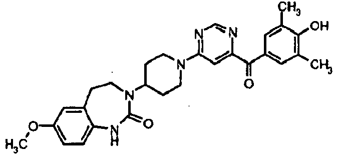 |
| (55) | 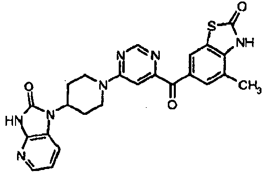 |
| (56) | 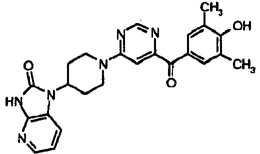 |
| (57) | 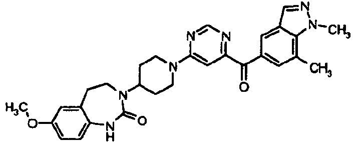 |
| (58) | 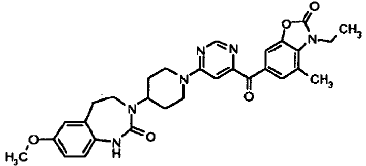 |
| (59) | 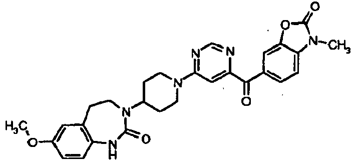 |
| (60) | 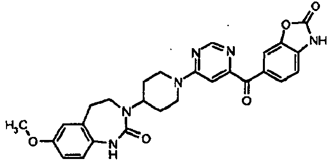 |
| (61) | 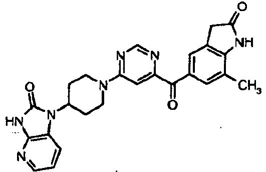 |
| (62) | 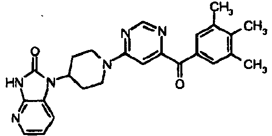 |
| (63) | 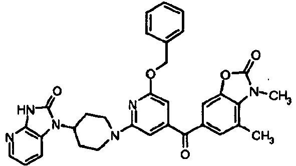 |
| (64) | 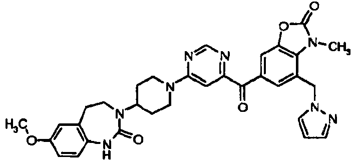 |
| (65) | 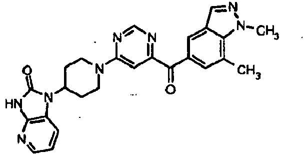 |
| (66) | 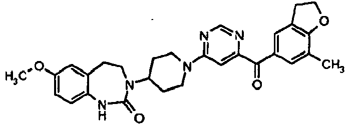 |
| (67) | 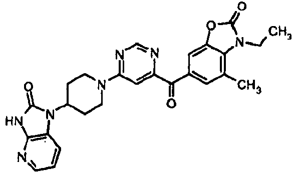 |
| (68) | 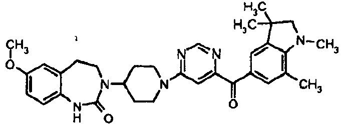 |
| (69) | 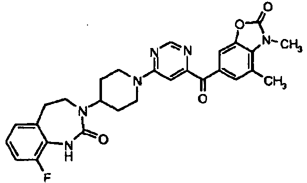 |
| (70) | 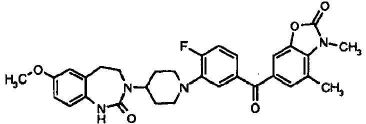 |
| (71) | 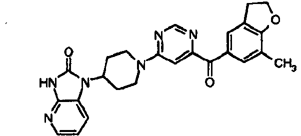 |
| (72) | 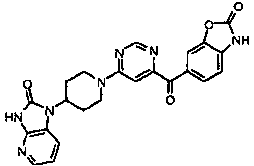 |
| (73) | 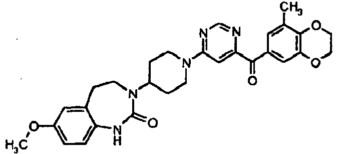 |
| (74) | 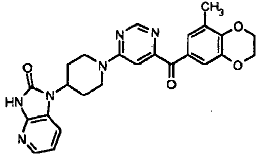 |
| (75) | 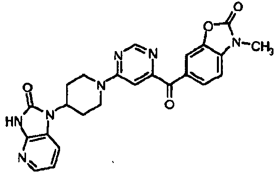 |
| (76) | 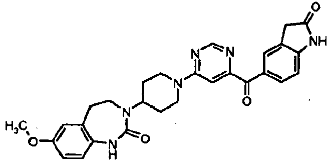 |
| (77) | 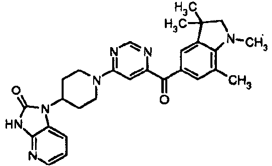 |
| (78) | 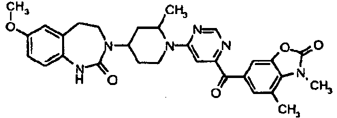 |
| (79) | 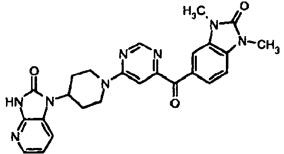 |
| (80) | 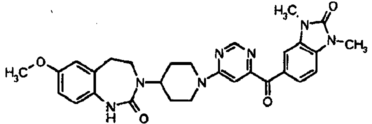 |
| (81) | 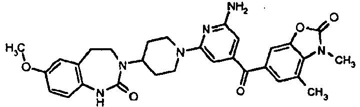 |
| (82) | 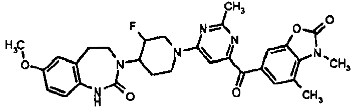 |
| (83) | 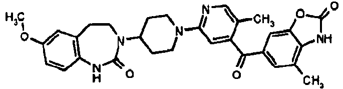 |
| (84) | 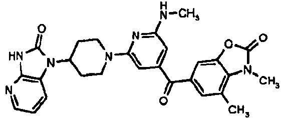 |
| (85) | 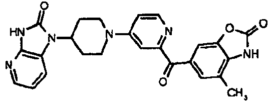 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

Als weiter bevorzugte Verbindungen der obigen allgemeinen Formel I seien beispielsweise folgende Verbindungen genannt:

| **Nr.** | **Struktur** |
|---|---|
| (1) | 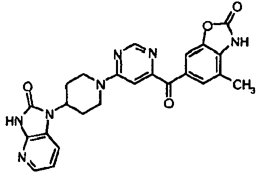 |
| (2) | 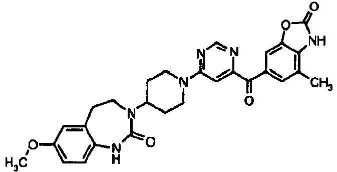 |
| (3) | 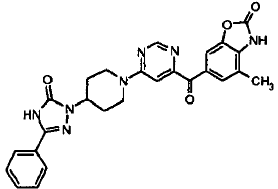 |
| (4) | 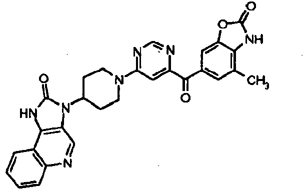 |
| (10) | 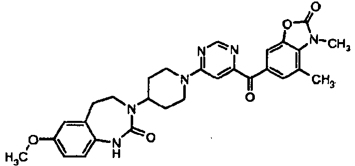 |
| (11) | 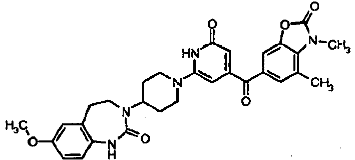 |
| (12) | 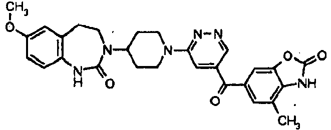 |
| (13) | 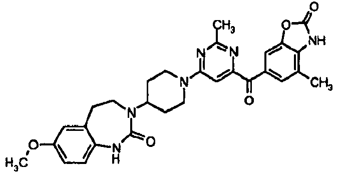 |
| (14) | 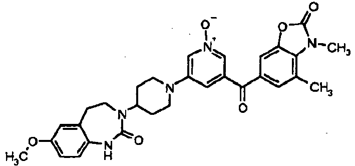 |
| (15) | 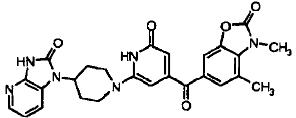 |
| (16) | 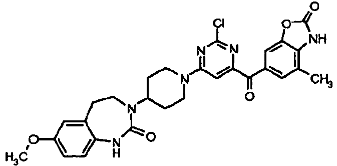 |
| (17) | 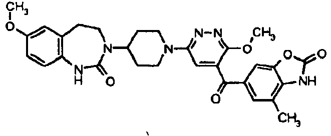 |
| (18) | 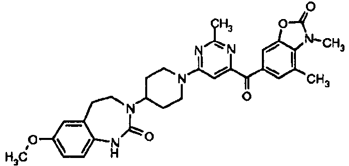 |
| (19) | 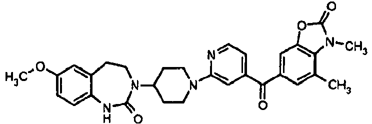 |
| (20) | 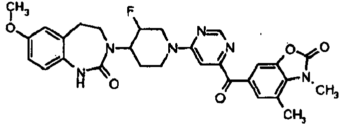 |
| (21) | 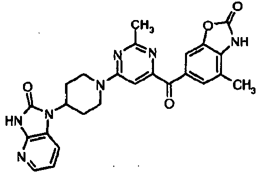 |
| (22) | 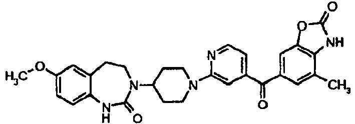 |
| (23) | 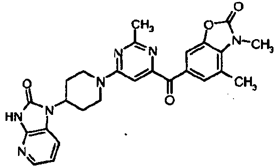 |
| (24) | 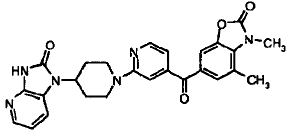 |
| (25) | 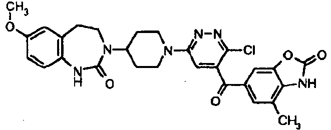 |
| (26) | 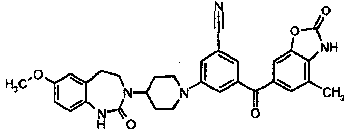 |
| (27) | 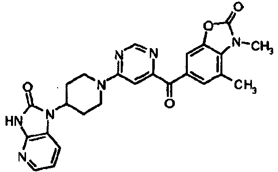 |
| (28) | 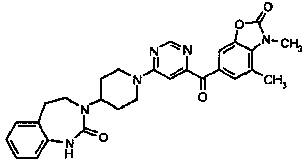 |
| (29) | 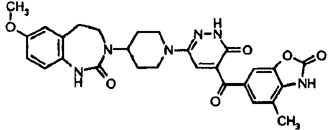 |
| (30) | 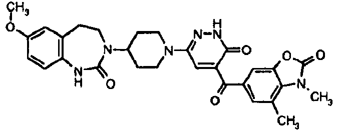 |
| (31) | 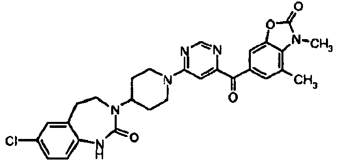 |
| (32) | 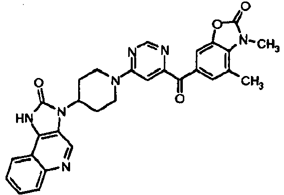 |
| (33) | 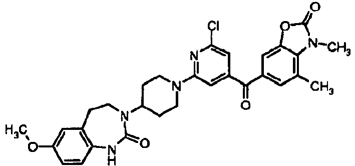 |
| (34) | 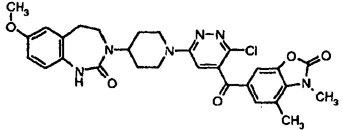 |
| (35) | 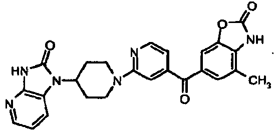 |
| (36) | 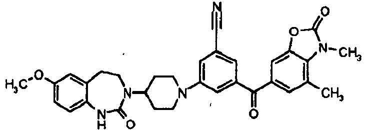 |
| (37) | 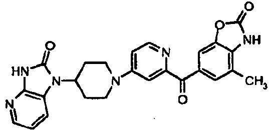 |
| (38) | 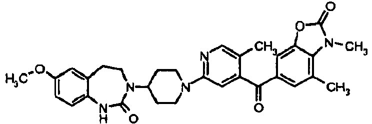 |
| (39) | 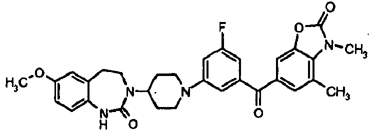 |
| (40) | 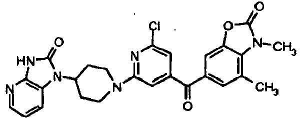 |
| (42) | 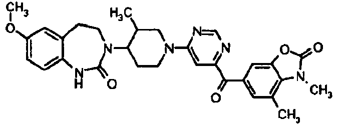 |
| (43) | 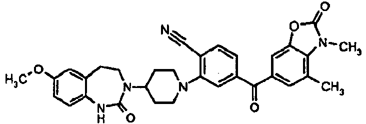 |
| (44) | 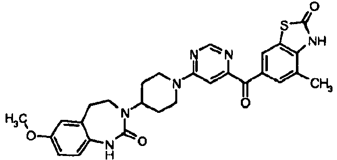 |
| (45) | 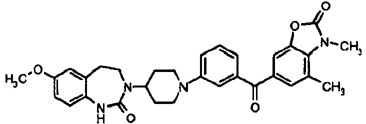 |
| (46) | 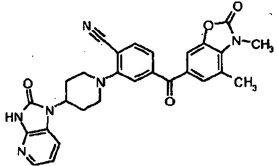 |
| (47) | 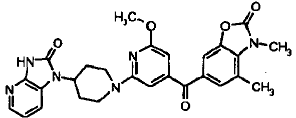 |
| (48) | 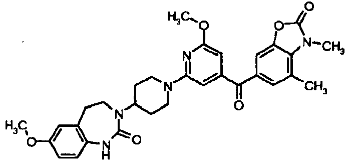 |
| (49) | 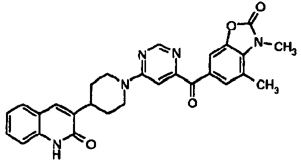 |
| (50) | 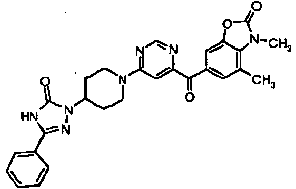 |
| (51) | 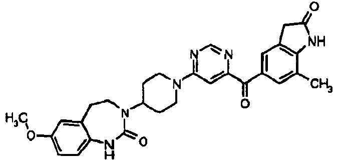 |
| (52) | 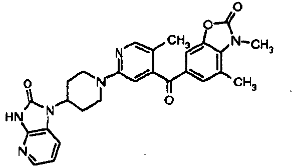 |
| (53) | 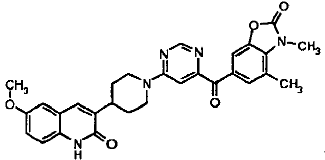 |
| (55) | 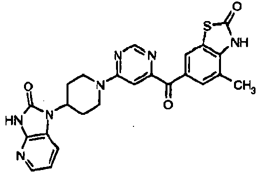 |
| (58) | 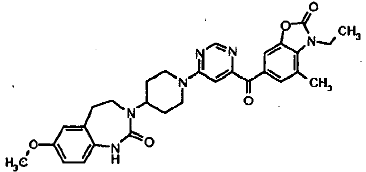 |
| (59) | 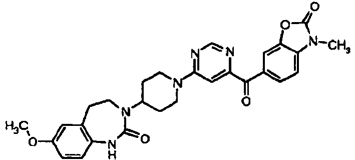 |
| (60) | 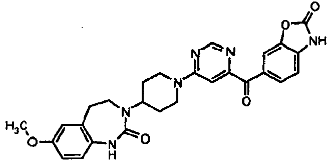 |
| (61) | 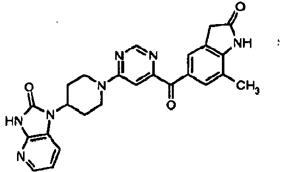 |
| (63) | 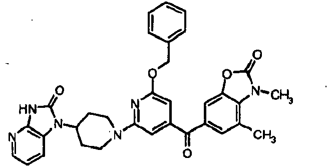 |
| (64) | 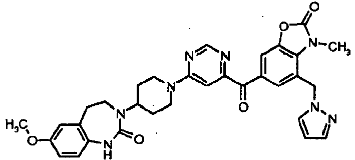 |
| (67) | 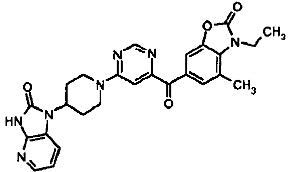 |
| (69) | 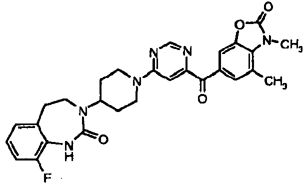 |
| (70) | 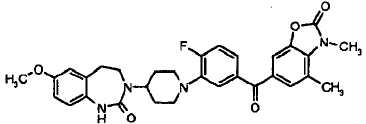 |
| (72) | 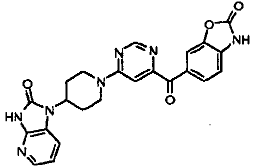 |
| (75) | 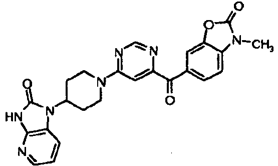 |
| (76) | 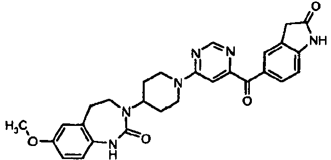 |
| (78) | 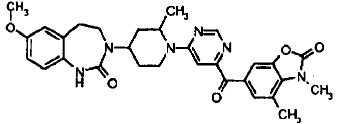 |
| (79) | 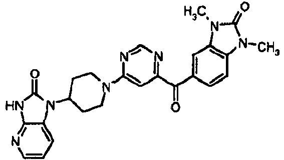 |
| (80) | 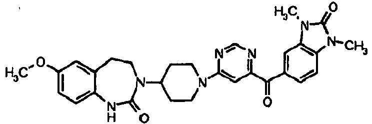 |
| (81) | 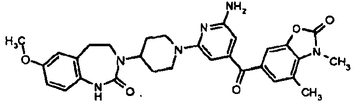 |
| (82) | 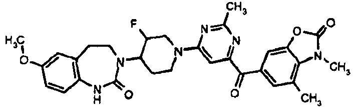 |
| (83) | 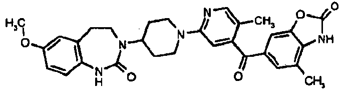 |
| (84) | 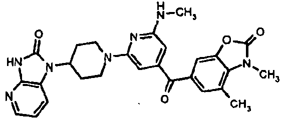 |
| (85) | 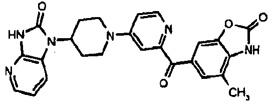 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.
Die Verbindungen, die von dieser Erfindung umfasst sind, sind jene, die auch chemisch stabil sind.
Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

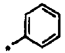

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, Pentyl, Neopentyl oder *n*-Hexyl. Gegebenenfalls werden für vorstehend genannten Gruppen auch die Abkürzungen Me, Et, n-Pr, *i*-Pr, n-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und iso-Propyl, Butyl umfasst *iso*-Bufyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen und unter dem Begriff "C₁₋₃-Alkylen" werden verzweigte und unverzweigte Alkylengruppen mit 1 bis 3 Kohlenstoffatomen verstanden Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Dimethylpropylen oder Hexylen. Sofern nicht anders beschrieben, umfasst die Definition Propylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen. Die Definition für C₀-Alkylen bedeutet eine Bindung.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc..

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc..

Unter dem Begriff "C₃₋₆-Cycloalkyl" (auch soweit sie Bestandteil anderer Reste sind) werden cyclische Alkylgruppen mit 3 bis 6 Kohlenstoffatomen und unter dem Begriff "C₅₋₆-Cycloalkyl" werden cyclische Alkylgruppen mit 5 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₅₋₆-Cycloalkenyl" (auch soweit sie Bestandteil anderer Reste sind) werden cyclische Alkenylgruppen mit 5 oder 6 Kohlenstoffatomen verstanden, die eine ungesättigte Bindung enthalten. Beispielsweise werden hierfür genannt: Cyclopentenyl oder Cyclohexenyl. Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heterocyclyl" oder "Heterocyclus" werden, soweit in den Definitionen nicht anders beschrieben, stabile 5-, 6- oder 7-gliedrige monocyclische oder 8-, 9-, 10-oder 11-gliedrige bicyclische heterocyclische Ringsysteme verstanden, die in mindestens einem Ring kein aromatisches Ringsystem bilden und neben Kohlenstoffatomen ein bis vier Heteroatome tragen können, die ausgewählt sind aus der Gruppe bestehend aus Stickstoff, Sauerstoff und Schwefel. Dabei können sowohl Stickstoffatome als auch Schwefelatome optional oxidiert sein und Stickstoffatome können quaternisiert sein. Der heterocyclische Ring kann eine oder zwei Carbonyl-, Thiocarbonyl- oder Cyaniminogruppen benachbart zu einem Stickstoffatom enthalten. Die voranstehend genannten Heterocyclen können über ein Kohlenstoffatom oder über ein Stickstoffatom mit dem restlichen Molekül verknüpft sein.
Soweit nicht anders beschrieben, können die Heterocyclen substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise-O-Methyl,
(e) -O-C₁₋₃-Alkyl, vorzugsweise-O-Methyl oder -O-Ethyl,
(f) COOH, COO-C₁₋₃-Alkyl, vorzugsweise CO-O-Methyl oder CO-O-Ethyl,
wobei die Reste gleich oder verschieden sein können.
Als Beispiele seien folgende Verbindungen genannt, sollen jedoch nicht auf diese beschränkt sein: Azetidin, Oxetan, Thietan, Thietandioxid, Tetrahydrofuran, Dihydrofuran, Dioxalan, Imidazolidin, Imidazolin, Imidazolidinon, Dihydroimidazolon, Oxazolin, Oxazolidin, Oxazolidinon, Pyrrolidinon, Dihydropyrazol, Pyrrolidin, Pyrrolin, Morpholin, Tetrahydropyridin, Dihydropyran, Tetrahydropyran, Dioxan, Piperazin, Piperidin, Piperazinon, Piperidinon, Pyran, Thiomorpholin-S-oxid, Thiomorpholin-S-dioxid, Thiomorpholin, Dihydrooxazin, Morpholindion, Morpholinthion, Perhydrothiazindioxid, e-Caprolactam, Oxazepanon, Diazepanon, Thiazepanon, Perhydroazepin, Dihydrochinazolinon, Dihydroindol, Dihydroisoindol, Benzoxazolon, Benzimidazolon, Chromanon, Tetrahydrochinolin, Tetrahydrobenzoxazol, Tetrahydrobenzisoxazol, Tetrahydrobenzthiophen, Tetrahydrothieno-pyridin, Tetrahydrobenzofuran, Tetrahydrooxazolopyridin, Tetrahydro-isoxazolopyridin.

Bevorzugt im Sinne dieser Erfindung seien folgende Heterocyclen:

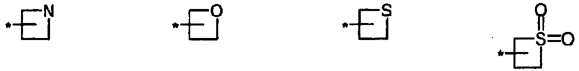

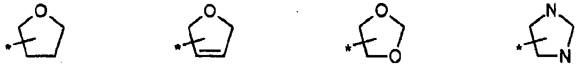

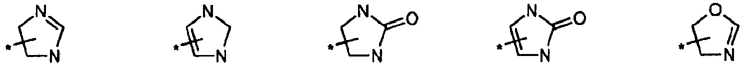

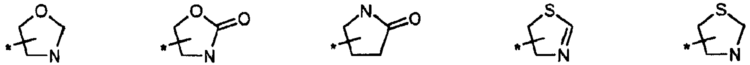

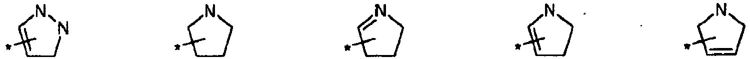

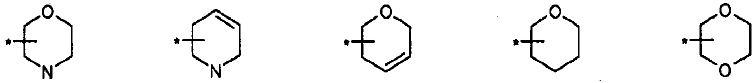

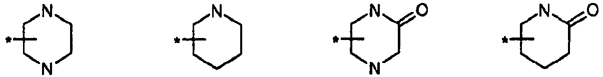

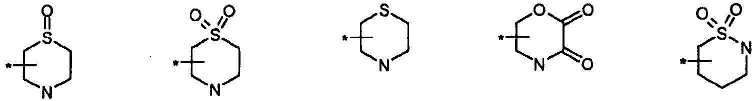

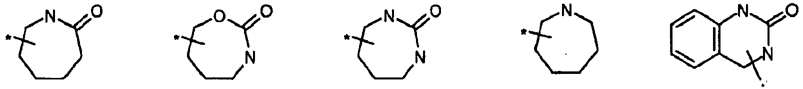

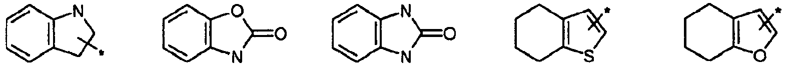

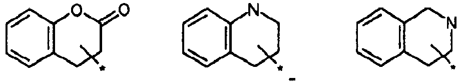

Unter dem Begriff "Aryl" (auch soweit sie Bestandteil anderer Reste sind) werden monocyclische aromatische Ringsysteme mit 6 Kohlenstoffatomen oder bicyclische aromatische Ringsysteme mit 10 Kohlenstoffatomen verstanden. Beispielsweise werden hierfür Phenyl, 1-Naphthyl oder 2-Naphthyl genannt; bevorzugter Arylrest ist Phenyl. Soweit nicht anders beschrieben, können die Aromaten substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆-Alkyl, vorzugsweise C₁₋₃-Alkyl, besonders bevorzugt Ethyl, Methyl, iso-Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Unter dem Begriff "Heteroaryl" werden stabile fünf- oder sechsgliedrige heterocyclische Aromaten oder 8- bis 10-gliedrige bicyclische Heteroarylringe verstanden, die in jedem Ring ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Als Beispiele für fünf- oder sechsgliedrige heterocyclische Aromaten werden genannt, sollen aber nicht auf diese beschränkt sein:
Furan, Pyrrol, Thiophen, Pyrazol, Imidazol, Oxazol, Thiazol, Isothiazol, Isoxazol, Oxadiazol, Triazol, Tetrazol, Furazan, Thiadiazol, Pyridin, Pyrimidin, Pyrazin, Pyridazin, Triazin.

Bevorzugt im Sinne dieser Erfindung seien folgende fünfgliedrige heterocyclische Aromaten:

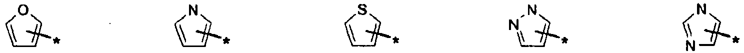

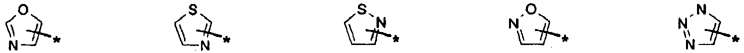

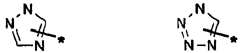

Bevorzugt im Sinne dieser Erfindung seien folgende sechsgliedrige heterocyclische Aromaten:

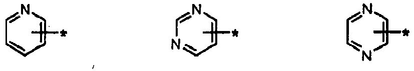

Als Beispiele für 9- oder 10-gliedrige bicyclische Heteroarylringe werden genannt, sollen aber nicht auf diese beschränkt sein:
Indol, Isoindol, Indazol, Indolizin, Benzofuran, Benzthiophen, Benzimldazol, Benzoxazol, Benzthiazol, Benztriazol, Benzisoxazol, Benzisothiazol, Chinolin, Isochinolin, Cinnolin, Phtalazin, Chinoxalin, Chinazolin, Pyridopyrimidin, Pyridopyrazin, Pyridopyridazin, Pyrimidopyrimidin, Pteridin, Purin, Chinolizin, Benzoxazolcarbonitril, Chinolin, Isochinolin, Chinolizin, Pteridin, Purin, Chinolizin, Benzoxazol-carbonitril.

Bevorzugt im Sinne dieser Erfindung seien folgende bicyclische Heteroarylringe:

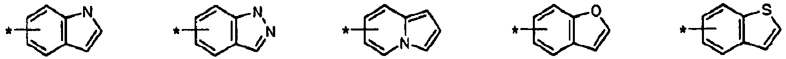

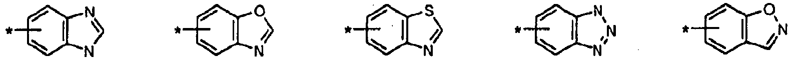

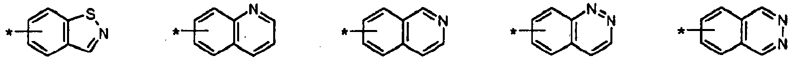

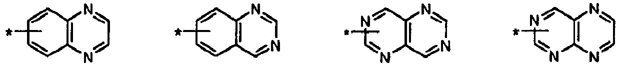

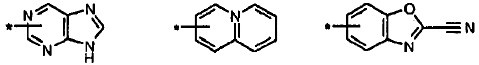

Soweit nicht anders beschrieben, können die voranstehend genannten Heteroaryle substituiert sein mit einem oder mehreren Resten ausgewählt aus der Gruppe bestehend aus:
(a) OH, NO₂, CN, OCF₃, OCHF₂, OCH₂F, NH₂,
(b) Halogen, vorzugsweise Fluor oder Chlor,
(c) C₁₋₆Alkyl, vorzugsweise C₁₋₃-Alkyl, besonders bevorzugt Ethyl, Methyl, *iso-*Propyl oder *tert*-Butyl,
(d) -SO₂-O-C₁₋₃-Alkyl, vorzugsweise -O-Methyl,
(e) -O-C₁₋₃-Alkyl, vorzugsweise -O-Methyl oder -O-Ethyl,
(f) COOH, CO-O-C₁₋₃-Alkyl, vorzugsweise CO-O-Methyl oder CO-O-Ethyl, wobei die Reste gleich oder verschieden sein können.

Bicyclische Heteroarylringe können dabei vorzugsweise im Phenylrest substituiert sein.

Unter dem Begriff "Halogen" werden Fluor-, Chlor-, Brom- oder Iodatome verstanden.

Verbindungen der allgemeinen Formel **I** können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, p-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (*R*)- oder (S)-Form gewonnen werden.

Verbindungen mit einer Kohlenstoff-Doppelbindung können sowohl in der E- als auch Z-Form vorliegen.

Die folgenden stickstoffhaltigen Heteroarylen können in unterscheidlichen tautomeren Formen vorliegen:

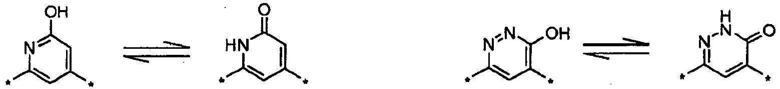

Das bedeutet, dass die die jeweils dargestellte Verbindung nicht auf eine tautomere Form beschränkt ist, sondern alle tautomeren Formen umfasst.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen.

### HERSTELLVERFAHREN

Gegenstand der Erfindung ist weiterhin ein Verfahren zur Herstellung der Verbindungen der allgemeinen Formel **I,** wobei die Substituenten die oben genannte Bedeutung haben.

Einige Methoden zur Herstellung der erfindungsgemäßen Verbindungen der allgemeinen Formel **I** 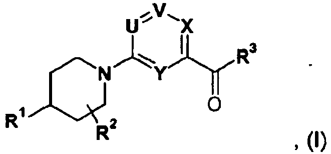 in der **U, V, X,** Y, **R¹, R²** und **R³** wie voranstehend erwähnt definiert sind, sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung vollständig verständlich zu machen. Die Beispiele sollen der Anschaulichkeit der Erfindung dienen und sollen die Erfindung in keinster Weise einschränken.

In einigen Fällen kann das Endprodukt weiter derivatisiert werden, z.B. durch Manipulation der Substituenten. Diese Manipulationen können dem Fachmann allgemein bekannt sein wie Oxidation, Reduktion, Alkylierung, Acylierung und Hydrolyse, müssen allerdings nicht auf diese beschränkt sein.

Die erfindungsgemäßen Verbindungen können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.

Beispielsweise kommt
- als Schutzrest für eine Hydroxygruppe die Methoxy-, Benzyloxy-, Trimethylsilyl-, Acetyl-, Benzoyl-, tert.-Butyl-, Trityl-, Benzyl- oder Tetrahydropyranylgruppe,
- als Schutzreste für eine Carboxylgruppe die Trimethylsilyl-, Methyl-,Ethyl-, tert.-Butyl-, Benzyl- oder Tetrahydropyranylgruppe, und
- als Schutzreste für eine Amidgruppe die N-Methoxymethyl- (MOM), N-Benzyloxymethyl- (BOM), N-(Trimethylsilyl)ethoxymethyl (SEM), N-*tert-*Butyldimethylsiloxymethyl, N-tert-Butyldimethylsilyl- (TBDMS), N-Triisopropylsilyl-(TIPS), N-Benzyl-, N-4-Methoxybenzyl- (PMB), N-Triphenylmethyl- (Trt), N-tert-Butoxycarbonyl- (BOC), N-Benzyloxycarbonyl- (Cbz), N-Trimethylsilylethylsulfonyl-(SES)
- als Schutzrest für eine Amino-, Alkylamino- oder Iminogruppe die Acetyl-, Trifluoracetyl-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht.

Weitere Schutzgruppen und deren Abspaltung sind in T.W. Greene, P.G.M. Wuts, "Protective Groups in Organic Synthesis", Wiley, 2006 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35 und -25°C.

Die Abspaltung eines 2,4-Dimethoxybenzylrestes erfolgt vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n*-Butylamin in einem Lösungsmittel wie Methanol, Ethanol, Isopropanol, Toluol/Wasser oder Dioxan bei Temperaturen zwischen 20°C und 50°C.

Die Abspaltung eines Methoxymethyl-Rests kann in Gegenwart einer Säure wie konzentrierter Salzsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines *N*-(Trimethylsilyl)ethoxymethyl-Rests kann in Gegenwart von TBAF und 1,3-Dimethyl-3,4,5,6-tetrahydro-2(1*H*)-Pyrimidon durchgeführt werden. Alternativ kann die SEM-Schutzgruppe auch mit einer Säure wie Chlorwasserstoff in einem organischen Lösungsmittel wie Dioxan oder Ethanol durchgeführt werden.

Die Abspaltung eines Allyloxycarbonylrestes erfolgt durch Behandlung mit einer katalytischen Menge Tetrakis-(triphenyl-phosphin)-palladium(0) vorzugsweise in einem Lösungsmittel wie Tetrahydrofuran und vorzugsweise in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo-[2.2.2]octan bei Temperaturen zwischen 20°C und 70°C.

Die folgenden Verfahren zur Herstellung der erfindungsgemäßen Verbindungen der allgemeinen Formel **I** und ihrer Vorstufen haben sich besonders bewährt:

Die Herstellung einer Endverbindung der allgemeinen Formel **I,** in der U, **V, X, Y, R¹, R²** und **R³** wie voranstehend erwähnt definiert sind, kann durch Reaktion einer Verbindung der allgemeinen Formel (1-1) mit einer elektronenarmen Verbindung der allgemeinen Formel (1-2), die eine Austrittsgruppe LG besitzt, erfolgen. Als Austrittsgruppe LG können Halogenide, bevorzugt Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden.

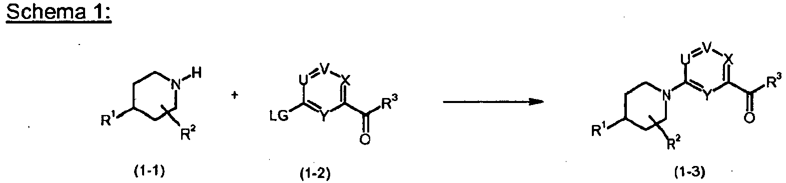

Die Reaktion kann über eine nucleophile aromatische Substitution in einem inerten Lösungsmittel unter Verwendung einer Hilfsbase in einem Temperaturbereich von 0°C bis zum Rückfluss des Lösungsmittels erfolgen. Die Reaktion wird in einem geeigneten, inerten Lösungsmittel, wie Tetrahydrofuran, Toluol, Xylol, einem Dialkylformamid (besonders bevorzugt Dimethylformamid), cyclische Amide (besonders bevorzugt N-Methyl-pyrrolidon), 1,4-Dioxan, Acetonitril oder in inerten Lösungsmittelgemischen durchgeführt. Als geeignete Hilfsbasen können tertiäre Amine wie Triethylamin oder Ethyldiisopropylamin, Alkalimetal Carbonate wie Kaliumcarbonat oder Natriumcarbonat, Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Alternativ können die in Schema 1 dargestellten Strukturen der allgemeinen Form (1-3), in der **U, V, X, Y, R¹, R²** und **R³** wie voranstehend erwähnt definiert sind, über Übergangsmetall-katalysierte Reaktionen synthetisiert werden. Dabei kann eine Verbindung der allgemeinen Formel (1-1) mit einer Verbindung der allgemeinen Formel (1-2), die eine Austrittsgruppe LG besitzt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe LG können Chloride, Bromide, Iodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, 1,4-Dioxan, Acetonitril oder Lösungsmittelgemische verwendet werden. Bevorzugtes Lösungsmittel ist Xylol. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Natriumcarbonat oder Kaliumphosphat. Bevorzugte Reaktionstemperaturen sind von RT bis Rückfluss des Lösungsmittel bei Normaldruck. Typische Katalysatoren sind z.B. Übergangsmetall-Katalysatoren, wie z.B. Palladium-Katalysatoren der Art Tris(dibenzylidenaceton)-cipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Triphenylarsen, 2,2'-Bis(diphenylphosphino)-1,1'-binaphthy) (BINAP), 2-Di-tert-butylphosphino-2',4',6'-triisopropylbiphenyl (XPhos), 4,5-Bis-diphenylphosphanyl-9,9-dimethyl-9H-xanthen (XantPhos) oder 2-(Di-*tert-*butylphosphino)biphenyl.

Verbindungen der allgemeinen Formel (2-4), in der **U, V, X, Y, R¹, R²** und **R³** wie voranstehend erwähnt definiert sind, können wie in Schema 2 gezeigt, dargestellt werden.

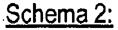

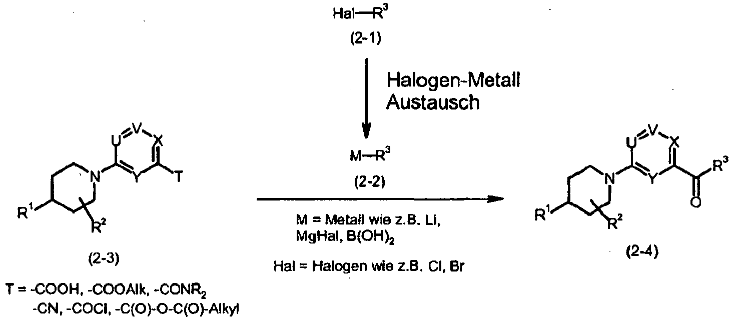

Die Reaktion geht von einer Verbindung der allgemeinen Form (2-1) aus, bei der Hal für ein Halogenatom, bevorzugt Chlor, Brom oder Iod, steht. Die Grignard oder lithiierte Verbindung der allgemeinen Formel (2-2) kann aus der entsprechend halogenierten Verbindung der allgemeinen Form (2-1) entweder durch einen sogenannten Halogen-Metall Austausch oder durch Insertion des Metalls in eine Halogen-Kohlenstoff-Bindung erfolgen. Um die entsprechende lithiierte Verbindung der allgemeinen Formel (2-2) zu synthetisieren, kann der Halogen-Metall-Austausch z.B. mit einer Organo-Lithium Verbindung wie z.B. n-, sec- oder tert.-Butyllithium erfolgen. Die entsprechenden Magnesium-Verbindungen (Grignard-Verbindungen) können ebenfalls duch einen Halogen-Metall-Austausch mit einem entsprechenden Grignard-Reagenz wie z.B. Isopropyl- oder sec-Butyl-Magnesium-Bromid oder Chlorid oder Diisopropyl- oder Di-sec-Butylmagnesium mit oder in Gegenwart eines Salzes wie z.B. Lithiumchlorid (das den Metallierungs-Prozess beschleunigen kann) erhalten werden. Die entsprechende transmetallierende Organo-Magnesium-Verbinung kann ebenso in-situ aus entsprechenden Vorstufen synthetisiert werden. (siehe z.B. Angew. Chem. 2004, 116, 3396-3399 und Angew. Chem. 2006, 118, 165-169 und darin enthaltene Referenzen). Darüber hinaus können auch -ate Komplexe der Organo-Magnesium-Verbindungen verwendet werden, die aus der Kombination von z.B. Butylmagnesiumchlorid oder-Bromid oder Isopropyl-Magnesiumchlorid oder -Bromid und Butyllithium resultieren. (siehe Angew. Chem. 2000, 112, 2594-2596 und Tetrahedron Lett. 2001, 42, 4841-4844 und darin enthaltene Referenzen). Der Halogen-Metall-Austausch wird bevorzugt zwischen-100°C und 40°C durchgeführt, ganz besonders bevorzugt ist ein Temperaturbereich von-80°C und 10°C in einem inerten Lösungsmittel bevorzugt sind Alkylether (ganz besonders bevorzugt Diethylether), cylische Ether (ganz besonders bevorzugt 1,4-Dioxan oder Tetrahydrofuran), Toluol, Hexan oder Lösungsmittelgemische davon. Die so erhaltenen Magnesium- oder Lithium-Organo-Verbindungen können optional transmetalliert werden mit Metallsalzen wie z.B. Certrichlorid, Zinkchlorid oder -Bromid, Indiumchlorid oder-Bromid um alternative Organometall-Verbindungen der allgemeinen Form (2-2) zu synthetisieren, die sich ebenfalls für die beschriebene Reaktion eignen. Alternativ kann die Organo-Metall-Verbindung (2-2) ebenso durch Insertion eines Metalls in eine Kohlenstoff-Halogen-Bindung erfolgen. Lithium oder Magnesium sind geeignete elementare Metalle für diese Transformation. Die Insertions-Reaktion wird bevorzugt zwischen -80°C und 100°C durchgeführt, ganz besonders bevorzugt ist ein Temperaturbereich von -70°C bis 40°C in einem inerten Lösungsmittel, bevorzugt sind Alkylether (ganz besonders bevorzugt ist Diethylether), cylische Ether (ganz besonders bevorzugt ist 1,4-Dioxan oder Tetrahydrofuran), Toluol, Hexan oder Lösungsmittelgemische davon. In Fällen, in denen keine spontane Reaktion stattfindet, ist gegebenenfalls eine Aktivierung des Metalls mit z.B. 1,2-Dibromethan, Iod, Trimethylsilylchlorid, Essigsäure, Chlorwasserstoff oder Ultraschall erforderlich. Die Umsetzung der Organo-Metall-Verbindung der allgemeinen Formel (2-2) mit einer Verbindung (2-3) wird bevorzugt in einem Temperaturbereich von -100°C bis 100°C durchgeführt, besonders bevorzugt ist ein Temperaturbereich von -80°C bis 50°C. Die Reaktion wird in einem inerten Lösungsmittel durchgeführt, wie z.B. bevorzugt sind Alkylether (ganz besonders bevorzugt ist Diethylether oder Dimethoxyethan), cyclische Ether (ganz besonders bevorzugt ist 1,4-Dioxan oder Tetrahydrofuran), aromatische Kohlenwasserstoffe (ganz besonders bevorzugt sind Toluol oder Benzol), Hexan oder Lösungsmittelgemische davon. Alle Reaktionen können an Luft durchgeführt werden, allerdings ist die Durchführung in einer Schutzgas-Atmosphäre wie Argon oder Stickstoff bevorzugt. Es kann sich als vorteilhaft erweisen, wenn funktionelle Gruppe in Verbindung (2-3) temporär geschützt werden.
Die Lithium-substituierte oder Magnesium-substituierte Verbindung der allgemeinen Formel (2-2) kann mit einer Verbindung der allgemeinen Formel (2-3) reagieren, die eine Carboxyl-Gruppe oder Derivate davon wie Ester, Nitrile, Carbonsäurechloride oder Amide, wie z.B. Weinreb-Amide enthalten. Diese Reaktionen können häufig ohne zusätzlichen Übergangsmetall-Katalysator oder Ummetallierung auf ein anderes Metall wie z.B. Cer, Indium oder Zink durchgeführt werden. In einigen Fällen können sich die beiden genannten Abwandlungen aber auch als vorteilhaft erweisen. Aromatische Boronsäuren, davon abgeleitete Ester, Dialkylarylborane oder Aryltrifluorborate können mit Säurechloriden oder Carbonsäuren in Gegenwart eines Übergangsmetalls, wie z.B. Palladium als Katalysator zu den entsprechenden Ketonen umgesetzt werden (V. Polackova, St. Toma, I. Augustinova, Iveta; Tetrahedron; 2006; 62; 50; 11675-11678 und darin zitierte Refrenzen und R. Kakino, H. Narahashi, I. Shimizu, A. Yamamoto, Bull. Chem. Soc. Jpn., 2002, 75, 1333-1345). Die entsprechenden Bor-substituierten Verbindung, wie z.B. Boronsäuren, Dialkylarylborane oder Boronsäureester lassen sich aus der metallierten Spezies durch Reaktion mit einem Bor-Elektrophil wie z.B. Borsäureester oder Derivate davon synthetisieren. Bor-substituierte Verbindungen können darüber hinaus aus den halogenierten bzw. pseudohalogenierten Vorläufer-Molekülen mittels eines Übergangsmetall-Katalysators, bevorzugt Palladium, und einer Bor- bzw Borolan-Verbindung synthetisiert werden. (Tetrahedron Lett. 2003, 4895-4898 und darin zitierte Referenzen).

Die Metallierung und/oder Kupplungsreaktion kann auch in Microreaktoren und/oder im Micromixer durchgeführt werden. Die Reaktionen können ohne weitere Zusätze durchgeführt werden oder im Falle von schlecht reagierenen Reaktionspartnern können auch Promotoren wie z.B. BF₃*OEt₂ zugesetzt werden (siehe M.Schlosser, Organometallics in Synthesis, John Wiley & Sons, Chichester/ New York/ Brisbanel Toronto/ Singapor, 1994)

Die halogenierten Verbindungen der allgemeinen Formel (2-1) sind entweder kommerziell verfügbar oder können nach Verfahren synthetisiert werden, die im Fachgebiet der organischen Chemie bekannt sind oder in der Fachliteratur beschrieben sind (siehe z.B. J. March, Advanced Organic Reactions, Reactions Mechanism, and Structure, 4th Edition, John Wiley & Sons, Chichester/New York/Brisbane/Toronto/Singapore, 1992 und darin zitierte Literatur). Die Verwendung von Übergangsmetallen und Organo-MetallVerbindungen für die Synthese ist detailiert in Monographien beschrieben. (siehe z.B. L. Brandsma, S.F. Vasilevsky, H.D. Verkruijsse, Application of Transition Metals Catalysts in Organic Synthesis, Springer-Verlag, Berlin/Heidelberg, 1999; M. Schlosser, Organometallics in Synthesis, John Wiley & Sons, Chichester/ New York/ Brisbane/ Toronto/ Singapor, 1994, P.J. Stang, F. Diederich, Metal-Catalyzed Cross-Coupling Reactions, Wiley-VCH, Weinheim, 1997 and darin enthaltene Referenzen.)

Eine Synthese für Verbindungen der allgemeinen Formel (3-4), in der **U, V, X, Y und R³** wie voranstehend erwähnt definiert sind, ist in Schema 3 dargestellt.

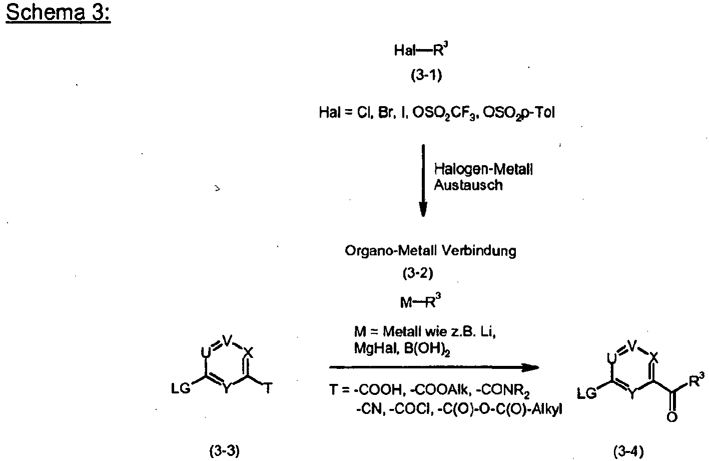

Ausgehend von einer halogenierten Verbindung (besonders bevorzugt sind die Chloride, Bromide und Iodide) der allgemeinen Formel (3-1) kann durch eine Halogen-Metall-Austauschreaktion z.B. mit Butyllithium, Isopropylmagnesium Halogenid oder Diisopropylmagnesium oder auch durch Insertion eines elementaren Metalls in die Halogen-Kohlenstoff-Bindung die entsprechende Lithium- oder Magnesium-substituierte Verbindung synthetisiert werden. Die entsprechenden Bor-substituierten Verbindungen, wie z.B. Boronsäure, Dialkylarylboran oder Boronsäureester lassen sich aus der metallierten Spezies durch Reaktion mit einem Bor-Elektrophil wie z.B. Borsäureester oder Derivate davon synthetisieren. Bor-substituierte Verbindungen können darüber hinaus aus den halogenierten bzw. pseudohalogenierten Vorläufer-Molekülen mittels eines Übergangsmetall-Katalysators, bevorzugt Palladium, und einer Bor- bzw Borolan-Verbindung synthetisiert werden. (Tetrahedron Lett. 2003, 4895-4898 und darin zitierte Referenzen) Die Lithium-substituierte oder Magnesium-substituierte Verbindung der allgemeinen Formel (3-2) kann an eine Verbindung der allgemeinen Formel (3-3) addieren, die eine Carboxyl-Gruppe oder Derivate davon wie Ester, Nitrile, Carbonsäurechloride oder Amide, wie z.B. Weinreb-Amide enthalten. Diese Reaktionen können häufig ohne zusätzlichen Übergangsmetall-Katalysator oder Ummetallierung auf ein anderes Metall wie z.B. Cer, Indium oder Zink durchgeführt werden. In einigen Fällen können sich die beiden genannten Abwandlungen aber auch als vorteilhaft erweisen. Aromatische Boronsäuren, davon abgeleitete Ester, Dialkylarylborane oder Aryltrifluorborate können mit Säurechloriden oder Carbonsäuren in Gegenwart eines Übergangsmetalls, wie z.B. Palladium als Katalysator zu den entsprechenden Ketonen umgesetzt werden (V. Polackova, St. Toma, I. Augustinova, Iveta; Tetrahedron; 2006; 62; 50; 11675-11678 und darin zitierte Refrenzen und R. Kakino, H. Narahashi, I. Shimizu, A. Yamamoto, Bull. Chem. Soc. Jpn., 2002, 75, 1333-1345)

Verbindungen der allgemeinen Formel (4-3), in der **U, V, X, Y** und **R³** wie voranstehend erwähnt definiert sind, können wie in Schema 4 gezeigt, dargestellt werden.

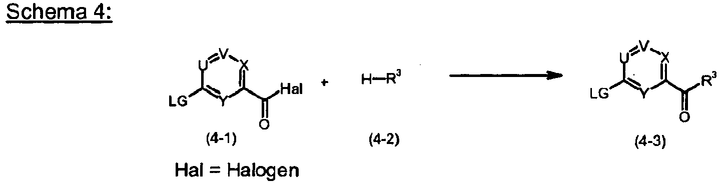

Eine Verbindung der allgemeinen Formel (4-1), die eine Austrittsgruppe LG und eine Säurehalogenid-Gruppe besitzt, kann mit einer aromatischen Verbindung der allgemeinen Formel (4-2) unter Friedel-Crafts Acylierungs Bedingungen bzw. Variationen davon, zur Reaktion gebracht werden. Friedel-Crafts-Reaktionen werden in Gegenwart eines Katalysators durchgeführt, der entweder in katalytischen oder stöchiometrischen Mengen eingesetzt wird. Als Katalysator eignen sich vor allem AlCl₃, FeCl₃, Iod, Eisen, ZnCl₂, Schwefelsäure oder Trifluormethansulfonsäure. Anstelle des Säurehalogenids kann auch die entsprechende Carbonsäure, Anhydrid, Ester oder Nitril verwendet werden. Die Reaktion wird bevorzugt in halogenierten Kohlenwasserstoffen durchgeführt. Besonders bevorzugt ist Dichlormethan und 1,2-Dichlorethan. Friedel-Crafts-Reaktionen werden in einem Temperaturbereich von -30°C bis 120°C, bevorzugt von 30°C bis 100°C. Allerdings können die Reaktionen auch ohne Lösungsmittel durchgeführt werden. Die Reaktionen können auch in der Microwelle durchgeführt werden.

Verbindungen der allgemeinen Formel (5-3), in der **U, V, X, Y, R¹, R²** und **R³** wie voranstehend erwähnt definiert sind, können wie in Schema 5 gezeigt, dargestellt werden.

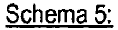

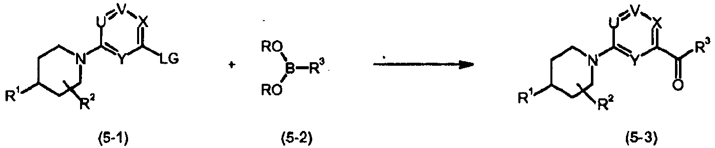

Analog zu einem Verfahren von T. Ishiyama et al. (J. Org. Chem., 1998, 63, 4726) kann eine Verbindung der allgemeinen Formel (5-1), die eine Austrittsgruppe LG besitzt, mit einer Bor-substituierten Verbindung, wie Boronsäure (R=H), Boronsäureester (R= Alkyl) Dialkylarylboran in Anwesenheit eines Katalysator und einer Base in einem inerten Lösungsmittel und einer Kohlenmonoxid-Atmosphäre, bevorzugt in einem Temperaturbereich von Raumtemperatur bis zum Rückfluss des Lösungsmittels, umgesetzt werden. Bevorzugt sind erhöhte Reaktionstemperaturen von 80°C-110°C unter erhöhtem Kohlenmonoxid-Druck. Für den Katalysator kann zusätzlich ein geeigneter Ligand verwendet werden. Es können Alkalimetalliodide wie Natriumjodid oder Kaliumjodid als Additive zugesetzt werden. Als Austrittsgruppe LG können Bromide, lodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, Anisol, 1,4-Dioxan, Acetonitril oder Lösungsmittelgemische verwendet werden. Bevorzugtes Lösungsmittel ist Anisol. Geeignete Basen sind anorganische Basen wie Cäsiumcarbonat, Cäsiumacetat, Kaliumcarbonat, Natriumcarbonat oder Kaliumphosphat. Die Reaktionen werden in einer Kohlenmonoxid-Atmosphäre durchgeführt, wobei der Kohlenmonoxid-Druck 1 bis 50 bar betragen kann. Typische Katalysatoren sind z.B. Palladium-Katalysatoren wie Tris-(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Tricyclohexylphosphin, Tri-*tert*-Butylphosphin, Triphenylarsen, BINAP, XPhos, XantPhos, oder 2-(Di-*tert*-butylphosphino)biphenyl, 1,1'-bis(Diphenylphosphino)ferrocen (Dppf), 1,2-bis(Diphenylphosphino)ethan (dppe), 1,3-bis(Diphenylphosphino)propan (dppp) und 1,4-bis(Diphenylphosphino)butan (dppb). Besonders bevorzugt ist die Verwendung von Pd(PPh₃)₂Cl₂ als Katalysator, Kaliumcarbonat als Base, 1 bar Kohlenmonoxid, Kaliumjodid als Additiv und Anisol als Lösungsmittel. Die entsprechenden Bor-substituierten Verbindungen sind entweder kommerziell erhältlich oder lassen sich aus metallierten Verbindungen durch Reaktion mit einem Bor-Elektrophil wie z.B. Borsäureester oder ein Derivat davon herstellen. Darüber hinaus können die Bor-substituierten Verbindungen aus den entsprechenden halogenierten bzw. pseudohalogenierten Vorläufer-Molekülen in einer Übergangsmetallkatalysierten Reaktion, z.B. mit Palladium und einem Diborolan bzw. Bor-Verbindung hergestellt werden (siehe Tetrahedron Lett. 2003, 4895-4898 und darin zitierte Referenzen).

Eine Synthese für Verbindungen der allgemeinen Formel (6-3), in der **U, V, X, Y** und **R³** wie voranstehend erwähnt definiert sind, ist in Schema 6 dargestellt

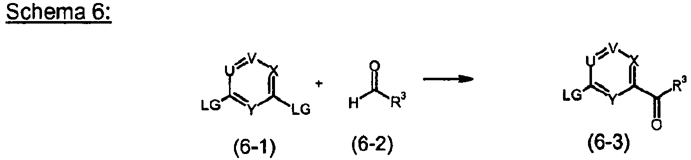

In Analogie zu einem Verfahren von A. Miyashita et al. (Heterocycles, 1997, Vol. 45, No. 11, 2159-2173) lassen sich Verbindungen der allgemeinen Formel (6-1), die eine Austrittsgruppe LG besitzen, mit aromatischen Aldehyden in Gegenwart eines Katalysators und einer Base in inerten Lösungsmitteln zu Verbindungen der allgemeinen Form (6-3) umsetzen. Als Austrittsgruppe LG können Fluoride, Chloride, Bromide, Iodide, Trifluormethansulfonate, Methansulfonate und Toluolsulfonate fungieren, müssen aber nicht auf diese beschränkt sein. Besonders bevorzugt sind Chloride und Bromide. Als inerte Lösungsmittel können cyclische Ether (bevorzugt ist Tetrahydrofuran) und Dialkylformamide (bevorzugt ist Dimethylformamid), verwendet werden. Geeignete Katalysatoren sind Azolium Salze, wie 1,3-Dimethylimidazoliumiodid oder 1,3-Dimethylbenzimidazoliumiodid. Als Base eigen sich Metallhydride. Ganz besonders bevorzugt ist Natriumhydrid. Die Reaktionen werden in einem Temperaturbereich von RT bis Rückfluss des Lösungsmittels durchgeführt. Bevorzugt sind erhöhte Temperaturen.
Die Reaktion kann auch anstelle von Azolium Salze und Base auch mit Natrium p-Tolylsulfinat in Anwesenheit eines Alkalimetallcyanids (bevorzugt Kaliumcyanid) in einem inerten Lösungsmittel bei erhöhten Temperaturen durchgeführt werden. (A. Miyashita et al., Heterocycles, 1998, Vol. 47, No. 1,407-414).

In Analogie zu A. Miyashita et al. (Heterocycles, 1997, Vol. 45, No. 11, 2159-2173) und den darin angegebenen Literaturstellen lassen sich Verbindungen der allgemeinen Formel (7-4), in der **U, V, X, Y** und **R³** wie voranstehend erwähnt definiert sind, wie in Schema 7 dargestellt, herstellen.

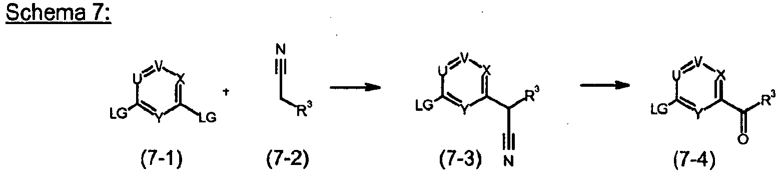

Verbindungen der allgemeinen Formel (7-1), die eine Austrittsgruppe LG besitzen, können mit 2-Arylacetonitril bzw. 2-Heteroarylacetonitril in Gegenwart einer Base in inerten Lösungsmittel zu Verbindungen der allgemeinen Form (7-3) umgesetzt werden. Als Austrittsgruppe LG können Fluoride, Chloride, Bromide, Iodide, Trifluormethansulfonate, Methansulfonate und Toluolsulfonate fungieren, müssen aber nicht auf diese beschränkt sein. Besonders bevorzugt sind Chloride und Bromide. Als inerte Lösungsmittel können Dialkylformamide (bevorzugt ist Dimethylformamid) verwendet werden. Als Base eignen sich Metallhydride. Ganz besonders bevorzugt ist Natriumhydrid. Die Reaktionen werden in einem Temperaturbereich von RT bis Rückfluss des Lösungsmittels durchgeführt. Bevorzugt sind Reaktionen bei erhöhten Temperaturen.
Die Synthese der Verbindungen der allgemeinen Formel (7-4) erfolgt durch oxidative Decyanierung von Verbindungen der allgemeinen Formel (7-3). Oxidative Decyanierungen erfolgen in inerten Lösungsmitteln, durch die in Gegenwart von einer Base Sauerstoff-Gas hindurchgeleitet wird. Als inerte Lösungsmittel können cyclische Ether (bevorzugt ist Tetrahydrofuran) verwendet werden. Als Base eignen sich Metallhydride. Ganz besonders bevorzugt ist Natriumhydrid. Die Reaktionen werden in einem Temperaturbereich von -20°C bis Rückfluss des Lösungsmittels durchgeführt. Bevorzugt sind Reaktionen bei RT.

Die erfindungsgemäßen neuen Verbindungen der allgemeinen Formel **I** können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische. Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.
Die Trennung von unter die allgemeine Formel **I** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (R)-(+)-I-Phenylethylamin, (S)-(-)-I-Phenylethylamin oder (S)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallende, diastereomerer Verbindungen wird auch dadurch erhalten, dass man die oben beschriebenen Synthesen mit jeweils einer geeigneten *(R)-*bzw. (S)-konfigurierten Reaktionskomponente durchführt

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computer-gestützten nichtlinearen Kurvenanpassung.

Die eingangs erwähnten Verbindungen zeigen in dem beschriebenen Test Ki-Werte ≤ 50 µM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei 20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen in *vitro*-Testmodell CGRP-antagonistische Eigenschaften in einem Dosisbereich zwischen 10⁻¹² bis 10⁻⁴ M.

Um zu zeigen, dass die Verbindungen der allgemeinen Formel **I** mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht, um spezifische Verbindungen hervorzuheben:

| **Beispiel** | **Kᵢ [nM]** |
|---|---|
| (1) | 0.4 |
| (3) | 4 |
| (4) | 1 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmem in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel I gemäß der obigen bevorzugten Ausführungsformen.

Besonders bevorzugt ist es, wenn die Verbindungen der Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnussoder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt. Hierzu müssen die Verbindungen der Formel **I** in inhalierbaren Darreichungsformen bereitgestellt werden. Als inhalierbare Darreichungsformen kommen Inhalations-pulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX*™*, 35 bis 70 µm) verwendet.
Die angegebenen HPLC-Daten werden unter nachstehend angeführten Parametern und unter Verwendung der aufgeführten Säulen erhoben:
Verwendete Säulen:

**(Säulentemperatur: 30°C; Injektionsvolumen: 5 µL; Detektion bei 254 nm)**

| | |
|---|---|
| S1 | Zorbax-Säule (Agilent Technologies), SB (Stable Bond) C18; 3.5 µm; 4.6 x 75 mm |
| S2 | Zorbax-Säule (Agilent Technologies), SB (Stable Bond) C18; 1.8 µm; 3.0 x 30 mm |
| S3 | Zorbax-Säule (Agilent Technologies), SB (Stable Bond) C18; 5 µm; 4.6 x 75 mm |
| S4 | Xbridge (Waters) C18; 3.0 x 30 mm, 2.5 µm |
| S5 | Sunfire C18 (Waters); 3.5 µm; 4.6 x 75 mm |
| S6 | Symmetry C18 (Waters); 4.6 x 75 mm, 3.5 µm |

Verwendete Lösungsmittel:
Lösungsmittel A: Wasser (mit 0.1% Ameisensäure), Lösungsmittel B: Acetonitril (mit 0.1% Ameisensäure), Lösungsmittel C: Wasser (mit 0. 1 % Ammoniak), Lösungsmittel D: Acetonitril (mit 0.1% Ammoniak) die prozentualen Angaben beziehen sich auf das Gesamtvolumen

**Gradienten:**

| Gradient | Zeit [min] | %A | %B |
|---|---|---|---|
| **G1** | 0.00 | 95 | 5 |
| (1.6 mL/min) | 0.10 | 95 | 5 |
| | 1.75 | 5 | 95 |
| | 1.90 | 5 | 95 |
| | 1.95 | 95 | 5 |
| | 2.00 | 95 | 5 |
| | | | |
| **G2** (1.6 mL/min) | 0.00 | 95 | 5 |
| | 4.50 | 10 | 90 |
| | 5.00 | 10 | 90 |
| | 5.50 | 95 | 5 |
| | | | |
| **G3** (1.6mL/min) | 0.00 | 95 | 5 |
| | 4.00 | 50 | 50 |
| | 4.50 | 10 | 90 |
| | 5.00 | 10 | 90 |
| | 5.50 | 95 | 5 |
| | | | |
| **G4** (1.6 mL/min) | 0.00 | 95 | 5 |
| | 1.00 | 10 | 90 |
| | 2.50 | 50 | 50 |
| | 2.75 | 95 | 5 |
| | | | |
| **G5** (1.6 mL/min) | 0.00 | 95 | 5 |
| | 2.00 | 10 | 90 |
| | 5.00 | 10 | 90 |
| | 5.50 | 95 | 5 |
| | | | |

| Gradient | Zeit [min] | %C | %D |
|---|---|---|---|
| **G6** (1.4 mL/min) | 0.00 | 95 | 5 |
| | 1.80 | 10 | 90 |
| | 2.00 | 10 | 90 |
| | 2.20 | 95 | 5 |
| | | | |
| **G7** (1.6 mL/min) | 0.00 | 95 | 5 |
| | 2.00 | 50 | 50 |
| | 2.25 | 10 | 90 |
| | 2.50 | 10 | 90 |
| | 2.75 | 95 | 5 |
| | | | |

| Gradient | Zeit [min] | %A | %B |
|---|---|---|---|
| **G8** (1.4 mL/min) | 0.00 | 95 | 5 |
| | 2.00 | 00 | 100 |
| | 3.00 | 00 | 100 |
| | 3.40 | 95 | 5 |

**Methoden:**

| | Säule | Gradient |
|---|---|---|
| Methode A | S1 | G1 |
| Methode B | S2 | G1 |
| Methode C | S1 | G2 |
| Methode D | S1 | G3 |
| Methode E | S2 | G4 |
| Methode F | S1 | G5 |
| Methode G | S4 | G6 |
| Methode H | S2 | G7 |
| Methode I | S5 | G3 |
| Methode K | S5 | G2 |
| Methode L | S6 | G3 |
| Methode M | S6 | G8 |

Bei präparativen HPLC-Reinigungen werden in der Regel die gleichen Gradienten verwendet, die bei der Erhebung der analytischen HPLC-Daten benutzt wurden. Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- 18-Krone-6: Kronenether (1,4,7,10,13,16-Hexaoxacyclooctadecan)
- AcOH: Essigsäure
- AIBN: 2,2'-Azobis(2-methylpropionitril)
- BINAP: 2,2'-Bis(diphenylphosphino)-1,1'-Binaphthyl
- BOC: *tert*.-Butyloxycarbonyl
- Cyc: Cyclohexan
- CDI: 1,1'-Carbonyldiimidazol
- DCM: Dichlormethan
- DIPE: Diisopropylether
- DIPEA: Diisopropylethylamin
- DMF: *N,N*-Dimethylformamid
- d. Th.: der Theorie
- EI: Elektronenstoß-Ionisation (bei MS)
- ESI: Elektrospray-Ionisation (bei MS)
- EtOAc: Essigsäureethylester
- EtOH: Ethanol
- E-Wasser: Entionisiertes Wasser
- FM: Fließmittel
- HCl: Chlorwasserstoff
- HCOOH: Ameisensäure
- HPLC: High Performance Liquid Chromatography
- HPLC-MS: HPLC gekoppelte Massenspektrometrie
- i.vac.: in vacuo (im Vakuum)
- konz.: Konzentriert
- MeOH: Methanol
- MS: Massenspektrometrie
- MW: Molekulargewicht [g/mol]
- NaOH: Natriumhydroxid
- NH₄OH: Ammoniumhydroxid (wässrige Ammoniak-Lösung, 30%)
- NMP: N-Methyl-2-Pyrrolidin
- Pd₂dba₃: Bis(dibenzylidenaceton) palladium (0)
- PE: Petrolether
- R_{f}: Retentionsindex (bei DC)
- RT: Raumtemperatur
- Rₜ: Retentionszeit (bei HPLC)
- TEA: Triethylamin
- TFA: Trifluoressigsäure
- THF: Tetrahydrofuran
- ULTS: Umluft-Trockenschrank

### Herstellung der Ausgangsverbindungen:

### Intermediat 1a

### 1-Piperidin-4-yl-1,3-dihydroimidazo[4,5-b]pyridin-2-on-dihydrochlorid

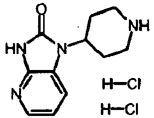

Diese Verbindung und deren Vorstufen wurden wie in der WO 2005/013894 beschrieben synthetisiert.

| | |
|---|---|
| ESI-MS: | m/z = 219 (M+H)⁺ |
| R_{f}: | 0.11 (Kieselgel, DCM/MeOH/NH₄OH = 80:20:2) |

### Intermediat 1b

### 1-Piperidin-4-yl-1,3-dihydroimidazo[4,5-b]pyridin-2-on

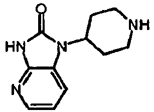

### Stufe 1: 4-(2-Chlor-pyridin-3-yl-amino)-piperidin-1-carbonsäurebenzylester

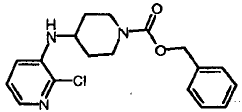

Zu 930 g (3.99 mol) *N*-Benzyloxycarbonyl-4-piperidon und 466 g (3.63 mol) 2-Chlor-3-aminopydridin in 9.5 L Isopropylacetat wurden bei ca. 15°C 560 mL (7.25 mol) TFA zugetropft. 922 g (4.35 mol) Natriumtriacetoxyborhydrid wurden portionsweise zugegeben. Es wurde bis zur Vollständigkeit der Reaktion nachgerührt. Bei RT wurde die Reaktionsmischung mit 860 mL Natronlauge (2 mol/L) versetzt. Die organische Phase wurde abgetrennt, mit 5 L Wasser gewaschen und eingeengt.
Ausbeute: 1250 g (roh 100% d. Th.)
ESI-MS: m/z = 346 (M+H)⁺

### Stufe 2: 4-[1-(2-Chlor-pyridin-3-yl)-ureido]-piperidin-1-carbonsäurebenzylester

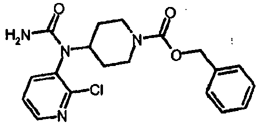

530 mL (6.1 mol) Chlorsulfonylisocyanat wurden in 6 L THF vorgelegt und auf -15°C abgekühlt. Zu diesem Gemisch wurde anschließend eine Lösung von 1.25 kg (3.63 mol) 4-(2-Chlor-pyridin-3-yl-amino)-piperidin-1-carbonsäurebenzylester in 7 L THF innerhalb einer Stunde so zugetropft, dass die Temperatur der Reaktionsmischung -7°C nicht überstieg. Es wurde 90 Minuten bei ca. -8°C nachgerührt und anschließend 700 mL Wasser innerhalb von 30 Minuten zugetropft. Die Mischung wurde 30 Minuten bei ca. 10°C nachgerührt und anschließend mit 8.1 L Natronlauge (2 mol/L) langsam versetzt. Das Reaktionsgemisch wurde anschließend auf 50°C erwärmt und die Phasen getrennt. Die organische Phase wurde mit 2 L Wasser gewaschen. Danach wurden aus der organischen Phase 10 L Lösemittel abdestilliert, 15 L Butylacetat zum Rückstand zugegeben und hiervon nochmals 8 L abdestilliert. Durch langsames Abkühlen auf 0°C wurde das Produkt kristallisiert. Der Niederschlag wurde abgesaugt, mit 2L Butylacetat gewaschen und bei 40°C getrocknet.
Ausbeute: 1108 g (78.8% d. Th.)
ESI-MS: m/z = 389/391 (M+H)⁺

### Stufe 3: 4-(2-Oxo-2,3-dihydro-imidazo[4,5-b]pyridin-1-yl)-piperidin-1-carbonsäure-Benzylester

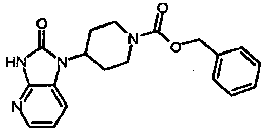

1108 g (2.85 mol) 4-[1-(2-Chlor-pyridin-3-yl)-ureido]-piperidin-1-carbonsäurebenzylester wurden mit 720 g (8.57 mol) Natriumhydrogencarbonat in 14.5 L tert-Amylalkohol unter Rückfluss erhitzt. Hierbei wurden 3 L Lösemittel abdestilliert. Das Reaktionsgemisch wurde auf 35°C abgekühlt und mit 11 mL Wasser versetzt. Anschließend wurden 13 g (0.058 mol) Palladiumacetat und 49 g (0.115 mol) 1,4-Bis-(diphenylphosphino)-butan (DPPB) zugegeben und auf Rückflusstemperatur erhitzt. Es wurde bei 100°C bis zur Vollständigkeit der Reaktion nachgerührt, auf RT abgekühlt und 7.5 L Wasser zugegeben. Die organische Phase wurde abgetrennt, mit 5 L Wasser gewaschen und anschließend eingeengt. Der ölige Rückstand wurde zweimal mit je 3 L Isopropylacetat versetzt und abdestilliert. Anschließend wurde der Rückstand in 7 L Isopropylacetat heiß gelöst und langsam auf Raumtemperatur abgekühlt. Der auskristallisierte Feststoff wurde abgesaugt, mit je 2 L Isopropylacetat und tert.-Butyl-methylether gewaschen und bei 50°C getrocknet.
Ausbeute: 690 g (69% d. Th.)
ESI-MS: m/z = 353 (M+H)⁺

### Stufe 4: 1-Piperidin-4-yl-1,3-dihydro-imidazo[4,5-b]pyridin-2-on

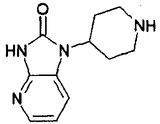

690 g (1.96 mol) 4-(2-Oxo-2,3-dihydro-imidazo[4,5-b]pyridin-1-yl)-piperidin-1-carbonsäure-benzylester wurden in 5.4 L Methanol gelöst und unter Zusatz von 46 g Pd/C (10%ig; 6.6 Gew.%) bei 60°C und einem Wasserstoffdruck von 60 psi bis zur vollständigen Wasser-stoffaufnahme hydriert. Der Katalysator wurde abfiltriert. Vom Filtrat wurden 4 L Methanol abdestilliert. Es wurden 2 L Methylcyclohexan zugegeben und weitere 1.5 L Lösemittel abdestilliert. Die so erhaltene Suspension wurde abgesaugt, der Rückstand mit Methylcyclohexan gewaschen und bei 40°C getrocknet.
Ausbeute: 446 g (100% d. Th.)
ESI-MS: m/z = 219 (M+H)⁺

### Intermediat 2

### 3-Piperidin-4-yl-1,3,4,5-tetrahydro-1,3-benzodiazepin-2-on

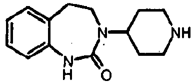

Diese Verbindung und deren Vorstufen wurden wie in der Europäischen Patentanmeldung Nr. 1 619 187 beschrieben synthetisiert.

| | |
|---|---|
| ESI-MS: | m/z = 246.2 (M+H)⁺ |

### Intermediat 3

### 7-Methoxy-3-piperidin-4-yl-1,3,4,5-tetrahydro-1,3-benzodiazepin-2-on

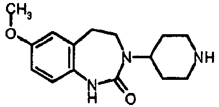

### Stufe 1: (5-Methoxy-2-nitrophenyl)acetonitril

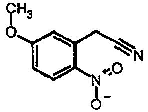

Zu einer Lösung aus 13.17 g (86.0 mmol) 4-Nitroanisol und 18.0 g (107 mmol) 4-Chlorphenoxyacetonitril in 50 m L DMF wurden 24.0 g (214 mmol) Kalium-*tert*-butylat in 100 mL DMF langsam zugetropft. Der Reaktionsansatz wurde 30 min bei -10°C gerührt und anschließend in 300 g einer 1:1 Mischung aus konz. HCl und Eis gegossen. Nach Extraktion mit EtOAc wurde die organische Phase mit Wasser gewaschen, getrocknet und im Vakuum unter gelindem Erwärmen bis zur Trockne einrotiert. Der Rückstand wurde mit einer 1:1 Mischung PE/EtOAc behandelt und das auskristallisierte Produkt abgesaugt. Nach dem Nachwaschen mit einer 1:1 Mischung PE/EtOAc wurden die Kristalle an der Luft getrocknet. Man erhielt 6.5 g des gewünschten Produkts.

| | |
|---|---|
| Ausbeute: | 6.5 g (39% d. Th.) |
| ESI-MS: | m/z = 210 (M+NH₄)⁺ |
| R_{f}: | 0.45 (Kieselgel; PE/EtOAc = 1:1) |

### Stufe 2: 2-(5-Methoxy-2-nitrophenyl)-ethylamin

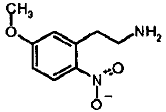

Unter einer Stickstoffatmosphäre wurden bei RT zu 12.6 g (65.7 mmol) (5-Methoxy-2-nitrophenyl)-acetonitril in 380 mL THF langsam 200 mL (200 mmol) einer 1 M Boran in THF Lösung zugetropft. Der Reaktionsansatz wurde 2 h unter Rückfluss gekocht. Nach dem Erkalten wurden innerhalb von 20 min 30 mL Methanol zugetropft. Dabei wurde mit einem Eisbad die Temperatur bei 10°C bis 20°C gehalten. Man ließ den Reaktionsansatz 30 min bei RT nachrühren und tropfte im Anschluss daran innerhalb von 30 min 45 mL einer 2M HCl-Lösung hinzu. Das Reaktionsgemisch wurde unter gelindem Erwärmen i.vac. einrotiert. Der Rückstand wurde mit Wasser auf ca. 200 mL verdünnt und mit 200 mL EtOAc extrahiert. Die wässrige Phase wurde mit einer 15%igen (w/v) wässrigen Kaliumcarbonat-Lösung alkalisch gestellt und über Nacht mit einem Rotations-Perforator nach Ludwig (Fa. Normag) mit Diethylether kontinuierlich extrahiert. Das organische Extrakt wurde bis zur Trockene einrotiert. Man erhielt 9.98 g des gewünschten Produkts.

| | |
|---|---|
| Ausbeute: | 9.98 g (77% d. Th.) |
| ESI-MS: | m/z = 197 (M+H)⁺ |
| Rₜ(HPLC) = | 2.13 min (Methode C) |

### Stufe 3: (1-Benzylpiperidin-4-yl)-[2-(5-methoxy-2-nitrophenyl)-ethyl]-amin

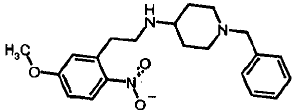

Unter Stickstoffatmosphäre wurde in einem Eisbad ein Gemisch aus 9.98 g (50.9 mmol) 2-(5-Methoxy-2-nitrophenyl)-ethylamin, 9.80 mL (54.9 mmol) *N-*Benzylpiperidon und 6.30 mL (114 mmol) Essigsäure in 270 mL DCM auf 0°C gekühlt. Bei dieser Temperatur wurden 14.2 g (67.0 mmol) Natriumtriacetoxyborhydrid portionsweise innerhalb von 20 min zugegeben. Man ließ den Reaktionsansatz weitere 4 h bei 0°C nachrühren und über Nacht auf RT aufwärmen. Anschließend wurde der Ansatz mit 400 mL einer 15%igen (w/v) wässrigen Kaliumcarbonat-Lösung versetzt und 1 h bei RT nachgerührt. Die organische Phase wurde abgetrennt, getrocknet und einrotiert. Man erhielt 18.8 g des gewünschten Produkts.

| | |
|---|---|
| Ausbeute: | 18.8 g (quantitativ) |
| ESI-MS: | m/z = 370 (M+H)⁺ |
| Rₜ(HPLC) | 1.93 min (Methode C) |

### Stufe 4: [2-(2-Amino-5-methoxyphenyl)-ethyl]-(1-benzylpiperidin-4-yl)-amin

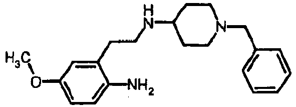

26.0 g (70.3 mmol) (1-Benzylpiperidin-4-yl)-[2-(5-methoxy-2-nitrophenyl)-ethyl]-amin wurden mit 5.00 g (2.45 mmol) Rhodiumkohle (5%, wasserfeucht) in 350 mL Methanol in einer 3 bar Wasserstoffatmosphäre 3 h bei RT hydriert. Der Katalysator wurde abgesaugt und die Lösung einrotiert. Man erhielt 23.9 g Rückstand, der ohne weitere Aufreinigung sofort weiter umgesetzt wurde.

| | |
|---|---|
| Ausbeute: | 23.9 g (quantitativ) |
| Rₜ(HPLC) | Rₜ = 0.99 min (Methode D) |

### Stufe 5: 3-(1-Benzylpiperidin-4-yl)-7-methoxy-1,3,4,5-tetrahydro-1,3-benzodiazepin-2-on

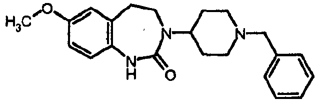

Zu 23.9 g (70.3 mmol) [2-(2-Amino-5-methoxyphenyl)-ethyl]-(1-benzylpiperidin-4-yl)-amin in 175 mL DMF wurden 35.0 g (216 mmol) *N,N*'-Carbonyldiimidazol zugegeben und die Mischung 2 h bei 100°C gerührt. Der Reaktionsansatz wurde auf ca. 1 kg Eiswasser gegossen und über Nacht gerührt. Das ausgefallene Produkt wurde abgesaugt, mit 100 mL Wasser gewaschen und bei 45°C im ULTS getrocknet. Der Rückstand wurde mit 150 mL DIPE verrührt und abgesaugt. Das Festprodukt wurde mit 50 mL DIPE nachgewaschen. Nach Trocknen im ULTS bei 35°C erhielt man 21.6 g des gewünschten Produkts.

| | |
|---|---|
| Ausbeute: | 21.6 g (84% d. Th.) |
| ESI-MS: | m/z = 366 (M+H)⁺ |
| Rₜ(HPLC) = | 2.12 min (Methode C) |

### Stufe 6: 7-Methoxy-3-piperidin-4-yl-1,3,4,5-tetrahydro-1,3-benzodiazepin-2-on

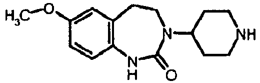

Eine Mischung aus 21.6 g (59.2 mmol) 3-(1-Benzyl-piperidin-4-yl)-7-methoxy-1,3,4,5-tetrahydro-1,3-benzodiazepin-2-on und 2.5 g Palladium auf Kohle (10%) in 300 mL Methanol wurde in einer 3 bar Wasserstoffatmosphäre bei 50°C bis zur vollständigen Umsetzung hydriert. Der Katalysator wurde abgesaugt und die Mutterlauge einrotiert. Der Rückstand wurde mit 150 mL DIPE verrieben, abgesaugt, mit 100 mL DIPE nachgewaschen und im ULTS bei 40°C getrocknet. Man erhielt 13.2 g des gewünschten Produkts.

| | |
|---|---|
| Ausbeute: | 13.2 g (81% d. Th.) |
| ESI-MS: | m/z = 276 (M+H)⁺ |
| Rₜ(HPLC) = | 0.73 min (Methode A) |

### Intermediat 4

### 3-Piperidin-4-yl-1,3-dihydroimidazo[4,5-c]chinolin-2-on

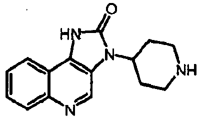

### Stufe1: 3-Bromchinolin-1-oxid

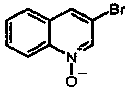

Zu einer auf 5°C gekühlten Lösung von 85.0 g (0.409 mol) 3-Bromchinolin in 100 mL DCM wurde eine Lösung aus 72%iger 3-Chlorperbenzoesäure (97.8 g (0.408 mol) gelöst in 1000 mL DCM, über Natriumsulfat getrocknet und abfiltriert) zugetropft. Dabei wurde darauf geachtet, dass die Temperatur der Reaktionsmischung nicht über 10°C anstieg. Nach Beendigung der Zugabe wurde 5 h gerührt, dann wurde nochmals eine Lösung von 72%iger 3-Chlorperbenzoesäure (25.0 g (0.104 mol) gelöst in 200 mL DCM, über Natriumsulfat getrocknet und abfiltriert) zugetropft und über Nacht bei RT gerührt. Es wurde gesättigte wässrige Natriumcarbonat-Lösung zugegeben, die Phasen getrennt und die organische Phase über Natriumsulfat getrocknet. Die Lösung wurde über Aktivkohle filtriert und anschließend i.vac. eingeengt.

| | |
|---|---|
| Ausbeute: | 224 g (99% d. Th.) |
| ESI-MS: | m/z = 223 / 225 (Br) |
| R_{f} = | 0.15 (Kieselgel, PE / EtOAc = 2:1) |

### Stufe2: 3-Brom-4-nitrochinolin-1-oxid

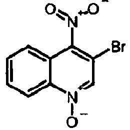

Eine Lösung aus 190 g (0.848 mol) 3-Bromchinolin-1-oxid in 500 mL konzentrierter Schwefelsäure wurde auf 90°C erhitzt. Dann wurden 120 g (1.19 mol) Kaliumnitrat in kleinen Portionen derart zugegeben, dass die Temperatur der Reaktion nicht über 95°C anstieg. Der Ansatz wurde 3 h bei 90°C gerührt; man ließ auf RT abkühlen und goss die Mischung auf Eis. Das ausgefallene Produkt wurde abfiltriert und der Filterkuchen mit Wasser gewaschen. Der Rückstand wurde in DCM gelöst und mit gesättigter, wässriger Natriumhydrogencarbonat-Lösung gewaschen, bis die Lösung alkalisch reagierte. Die Phasen wurden getrennt und die wässrige Phase mit DCM extrahiert. Die vereinigten organischen Phasen wurden über Natriumsulfat getrocknet und i.vac. eingeengt. Nach Zerkleinerung des Rückstandes und ausgiebiger Trocknung i.vac. wurde das Produkt als gelber Feststoff erhalten.

| | |
|---|---|
| Ausbeute: | 104 g (46% d. Th.) |
| ESI-MS: | m/z = 268/270 (M+H)⁺ (Br) |
| R_{f} = | 0.77 (Kieselgel, EtOAc) |

### Stufe 3: (1-Benzylpiperidin-4-yl)-(4-nitro-1-oxychinolin-3-yl)-amin

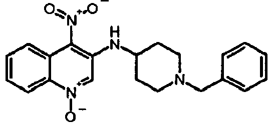

Zu 320 mL (1.54 mol) 4-Amino-1-benzylpiperidin wurden 104 g (0.387 mol) 3-Brom-4-nitrochinolin-1-oxid gegeben. Dann wurden 500 mL THF hinzugefügt und die Mischung bis zur vollständigen Lösung der Substanzen etwas erwärmt. Anschließend wurde 3 h bei 70°C gerührt und die Reaktionsmischung darauf i.vac. eingeengt. Der erhaltene Rückstand wurde in 2.5 L DCM gelöst und mit gesättigter wässriger Natriumhydrogencarbonat-Lösung gewaschen. Die wässrige Phase wurde mit 300 mL DCM extrahiert. Anschließend wurden die organischen Phasen vereinigt, über Natriumsulfat getrocknet und i.vac. eingeengt. Der Rückstand wurde in 250 mL Methanol gelöst. Das als Feststoff ausgefallene Produkt wurde abgesaugt und i.vac. getrocknet.

| | |
|---|---|
| Ausbeute: | 104 g (71% d. Th.) |
| ESI-MS: | m/z = 379 (M+H)⁺ |
| R_{f} = | 0.75 (Kieselgel, EtOAc) |

### Stufe 4: N³⁻(1-Benzylpiperidin-4-yl)chinolin-3,4-diamin

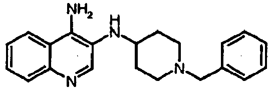

Zu 76.0 g (0.20 mol) (1-Benzylpiperidin-4-yl)-(4-nitro-1-oxychinolin-3-yl)-amin in 1.0 L THF wurden 12.0 g Rhodiumkohle (5%, wasserfeucht) gegeben. Die Reaktion wurde 4.5 h unter Wasserstoffatmosphäre (50 psi) bei RT geschüttelt. Der Katalysator wurde abfiltriert und das Lösungsmittel i.vac. entfernt. Wegen seiner Oxidationsempfindlichkeit wurde das Rohprodukt sogleich für die nächste Stufe eingesetzt.

| | |
|---|---|
| Ausbeute: | 66.0 g |
| R_{f} = | 0.30 (Kieselgel, DCM/MeOH/Cyc/NH₄OH = 70:15:15:2) |

### Stufe 5: 3-(1-Benzylpiperidin-4-yl)-1,3-dihydroimidazo[4,5-c]chinolin-2-on

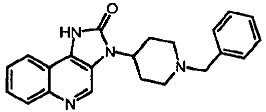

Zu 9.00 g (27.1 mmol) *N*³-(1-Benzylpiperidin-4-yl)-chinolin-3,4-diamin in 100 mL DMF wurden 22.6 g (139 mmol) 1,1'-Carbonyldiimidazol gegeben. Die Mischung wurde auf 100°C erhitzt und 1.5 h gerührt. Nach Abkühlen der Reaktionsmischung wurde diese auf 300 mL Wasser gegossen. Der ausgefallene Feststoff wurde abfiltriert, mit Wasser gewaschen und bei 30°C i.vac. getrocknet. Der Rückstand wurde mit Diethylether verrieben, abgesaugt und das Festprodukt i.vac. getrocknet.

| | |
|---|---|
| Ausbeute: | 7.42 g (77% d. Th.) |
| ESI-MS: | m/z = 359 (M+H)⁺ |
| Rₜ(HPLC) | 1.57 min (Methode C) |

### Stufe 6: 3-Piperidin-4-yl-1,3-dihydroimidazo[4,5-c]chinolin-2-on

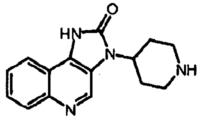

Eine Mischung aus 44.0 g (0.123 mol) 3-(1-Benzylpiperidin-4-yl)-1,3-dihydroimidazo-[4,5-c]chinolin-2-on und 10.0 g Palladium auf Kohle (Pd/C 10%) in 500 mL Methanol wurde 16 h bei 50 C in einer Wasserstoff-Atmosphäre von 50 psi hydriert. Nach Filtration des Reaktionsgemisches wurde das Lösungsmittel im Vakuum entfernt. Durch Zugabe von Isopropanol fiel das Produkt als Niederschlag aus. Dieser wurde abfiltriert und anschließend im Vakuum getrocknet.

| | |
|---|---|
| Ausbeute: | 31.2 g (95% d. Th.) |
| ESI-MS: | m/z = 269 (M+H)⁺ |
| R_{f}: | 0.20 (Kieselgel, DCM/MeOH/Cyc/NH₄OH = 70:15:15:2) |

### Intermediat 5

### 6-Chlorpyrimidin-4-carbonsäurechlorid

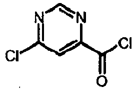

### Stufe 1: 6-Hydroxypyrimidin-4-carbonsäure

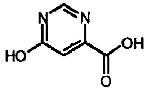

Zu 24.1 g (597 mmol) NaOH in 3.6 L Wasser wurden 63.5 g (287 mmol) Natriumdiethyl-Oxalacetat und 30.2 g (287 mmol) Formamidin-Acetat zugegeben. Der Ansatz wurde über Nacht bei RT gerührt. Anschließend fügte man Aktivkohle hinzu und kochte den Ansatz für 1h unter Rückfluss. Es wurde heiß abfiltriert und nach dem Erkalten mit wässriger HCl angesäuert. Die Lösung wurde bis zur Trockne einrotiert. Der Rückstand enthielt das gewünschte Produkt und wurde ohne weitere Aufreinigung in die nächste Stufe eingesetzt.

| | |
|---|---|
| Ausbeute: | 83.0 g |

### Stufe 2: 6-Chlorpyrimidin-4-carbonsäurechlorid

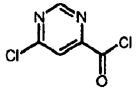

50 g (0.35 mol) 6-Hydroxypyrimidin-4-carbonsäure wurde vorgelegt und 500 mL Phosphoroxychlorid zugegeben. Anschließend wurde unter Rühren 150 g (0.72 mol) Phosphorpentachlorid portionsweise zugegeben. Der Reaktionsansatz wurde 5 h unter Rückfluss gekocht. Das Phosphoroxychlorid wurde abdestilliert und der Rückstand durch Vakuumdestillation über eine Kolonne aufgereinigt.

| | |
|---|---|
| Ausbeute: | 52 (83% d. Th.) |
| ESI-MS: | m/z = 176/178/ 180 (M)⁺(2 Cl) |

### Intermediat 6

### 4-Methyl-3H-benzoxazol-2-on

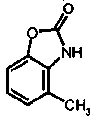

25.0 g (200 mmol) 2-Amino-*m*-cresol und 70.4 mL (400 mmol) DIPEA wurden in 1.0 L DCM vorgelegt und auf 0°C gekühlt. Dazu wurde eine Lösung aus 38.0 g (227 mmol) CDI über 30 min zugetropft. Der Ansatz wurde 30 min bei 0°C, dann über Nacht bei RT gerührt. Nach Einengen des Reaktionsgemisches i.vac. auf die Hälfte des Volumens wurde die wässrige Phase mit Wasser (2 x 250 mL), 1 M wässriger Kaliumhydrogensulfatlösung (1 x 250 mL) und nochmals Wasser (1 x 250 mL) gewaschen. Die organische Phase wurde i.vac. eingeengt. Das als Feststoff zurückgebliebene Rohprodukt wurde mit einem Gemisch aus Diethylether und PE verrieben, der ausgefallene Feststoff abgesaugt, mit, PE nachgewaschen und i.vac. getrocknet.

| | |
|---|---|
| Ausbeute: | 25.0 g (86% d. Th.) |
| ESI-MS: | m/z =150(M+H)⁺ |
| Rₜ (HPLC): | 2.67 min (Methode C) |

### Intermediat 7

### 6-(6-Chlorpyrimidin-4-carbonyl)-4-methyl-3H-benzoxazol-2-on

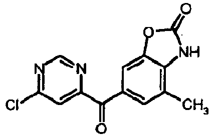

Ein gut gerührtes Gemisch aus 2.34 g (13.2 mmol) 6-Chlorpyrimidin-4-carbonsäurechlorid, 8.00 g (60.0 mmol) Aluminiumtrichlorid und 1.79 g (12.0 mmol) 4-Methyl-3H-benzoxazol-2-on wurde 1.5 h auf 130°C erhitzt. Nach dem Abkühlen auf RT wurde der Ansatz mit Eiswasser zersetzt, dann mit Ethylacetat extrahiert, die organische Phase über Natriumsulfat getrocknet und i.vac. eingeengt. Das als Feststoff zurückgebliebene Rohprodukt wurde mit Diethylether verrieben, abgesaugt und an der Luft getrocknet.

| | |
|---|---|
| Ausbeute: | 2.00 g (52% d. Th.) |
| ESI-MS: | m/z = 290 / 292 (M+H)⁺ (Cl) |
| Rₜ (HPLC): | 3.17 min (Methode C) |

### Intermediat 8

### 6-(6-Chlorpyrimidin-4-carbonyl)-3,4-dimethyl-3H-benzoxazol-2-on

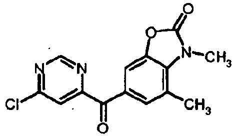

Zu 2.2 g (7.6 mmol) 6-(6-Chlorpyrimidin-4-carbonyl)-4-methyl-3*H*-benzoxazol-2-on in 10 mL *N,N*'-Dimethylformamid wurden 0.35 g (8.0 mmol) Natriumhydrid (55%, Suspension in Mineralöl) zugegeben. Der Reaktionsansatz wurde 30 min bei RT gerührt. Anschließend wurden 0.95 mL (15.0 mmol) Iodmethan zugegeben und 1 h bei RT gerührt. Der Reaktionsansatz wurde mit Eiswasser versetzt, die wässrige Phase mehrmals mit EtOAc extrahiert. Die vereinigten organischen Phasen wurden über Natriumsulfat getrocknet, filtriert und bis zur Trockne einrotiert. Der Rückstand wurde mit Diethylether verrieben, abgesaugt und getrocknet.

| | |
|---|---|
| Ausbeute: | 1.6 g (69% d. Th.) |
| ESI-MS: | m/z = 304 / 306 (Cl) (M+H)⁺ |
| Rₜ (HPLC) | 3.55 min (Methode C) |

### Intermediat 9

### (6-Chlor-pyrimidin-4-yl)-(3,4-dichlor-phenyl)-methanon

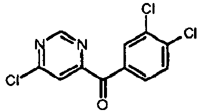

3.00 g (17.0 mmol) 6-Chlor-pyrimidin-4-carbonsäurechlorid, 11.3 g (84.8 mmol) Aluminiumtrichlorid und 1.80 mL (16.0 mmol) o-Dichlorbenzol wurden 1.5 h auf 130°C erhitzt. Nach dem Abkühlen auf RT wurde der Ansatz mit Eiswasser versetzt, dann mit EtOAc extrahiert, die organische Phase über Magnesiumsulfat getrocknet und i.vac. eingeengt. Der Rückstand wurde mit DIPE verrieben, abgesaugt und getrocknet.

| | |
|---|---|
| Ausbeute: | 2.40 g (49% d. Th.) |
| EI-MS: | m/z = 286/288/290/292 (3 x Cl) (M⁺) |
| R_{f}: | 0.76 (Kieselgel, PE / EtOAc = 2/1) |

### Intermediat 10

### (6-Chlor-pyrimidin-4-yl)-(3,4-dimethyl-phenyl)-methanon

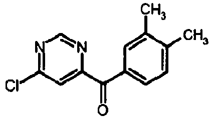

1.0 g (5.7 mmol) 6-Chlor-pyrimidin-4-carbonsäurechlorid wurden in 10 mL DCM gelöst, zu 3.7 g (28 mmol) Aluminiumtrichlorid in 10 mL DCM gegeben und der Ansatz 30 min bei RT gerührt. Dann wurden 0.70 mL (5.8 mmol) Xylol (gelöst in 10 mL DCM) langsam zu dem Reaktionsgemisch getropft und dieses 14 h bei RT gerührt. Es wurde dann mit DCM und 15%iger Kaliumcarbonatlösung versetzt und die wässrige Phase mehrmals mit DCM extrahiert. Die organischen Phasen wurden vereinigt, über Magnesiumsulfat getrocknet und i.vac. eingeengt.

| | |
|---|---|
| Ausbeute: | 0.88 g (63% d. Th.) |
| ESI-MS: | m/z = 247/249 (Cl) (M+H)⁺ |
| R_{f}: | 0.71 (Kieselgel, PE / EtOAc = 2/1) |

### Intermediat 11

### (6-Chlor-pyrimidin-4-yl)-(3,4-diethyl-phenyl)-methanon

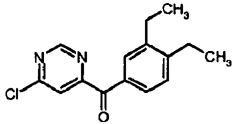

1.0 g (5.7 mmol) 6-Chlor-pyrimidin-4-carbonsäurechlorid wurden in 10 mL DCM gelöst, zu 3.7 g (28 mmol) Aluminiumtrichlorid in 10 mL DCM gegeben und der Ansatz 30 min bei RT gerührt. Dann wurden 0.93 mL (5.5 mmol) 1,2-Diethylbenzol (gelöst in 10 mL DCM) langsam zu dem Reaktionsgemisch getropft und dieses 14 h bei RT gerührt. Es wurde dann mit DCM und 15%iger Kaliumcarbonatlösung versetzt und die wässrige Phase wurde mehrmals mit DCM extrahiert. Die organischen Phasen wurden vereinigt, über Magnesiumsulfat getrocknet und i.vac. eingeengt.

| | |
|---|---|
| Ausbeute: | 1.4 g (90% d. Th.) |
| ESI-MS: | m/z = 275 (M+H)⁺ |
| R_{f}: | 0.77 (Kieselgel, PE / EtOAc = 2/1) |

### Intermediat 12

### (6-Chlor-pyrimidin-4-yl)-(3,4,5-trimethyl-phenyl)-methanon

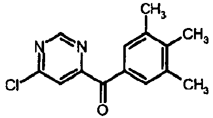

1.0 g (5.7 mmol) 6-Chlor-pyrimidin-4-carbonsäurechlorid wurden in 10 mL DCM gelöst, zu 3.7 g (28 mmol) Aluminiumtrichlorid in 10 mL DCM gegeben und der Ansatz 30 min bei RT gerührt. Dann wurden 0.70 g (5.8 mmol) 1,2,3-Trimethylbenzol (gelöst in 10 mL DCM) langsam zu dem Reaktionsgemisch getropft und dieses 14 h bei RT gerührt. Es wurde dann mit DCM und 15%iger Kaliumcarbonatlösung versetzt und die wässrige Phase mehrmals mit DCM extrahiert. Die organischen Phasen wurden vereinigt, über Magnesiumsulfat getrocknet und i.vac. eingeengt.

| | |
|---|---|
| Ausbeute: | 0.90 g (61 % d. Th.) |
| ESI-MS: | m/z = 261/263 (Cl) (M+H)⁺ |
| R_{f}: | 0.71 (Kieselgel, PE / EtOAc = 2 / 1) |

### Intermediat 13

### 3-{1-(6-(4-Benzyloxy-3,5-dimethyl-benzoyl)-pyrimidin-4-yl]-piperidin-4-y}-7-methoxy-1,3,4,5-tetrahydro-benzo[d][1,3]diazepin-2-on

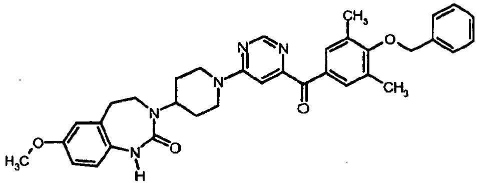

### Stufe 1: (4-Benzyloxy-3,5-dimethyl-phenyl)-(6-Chlor-pyrimidin-4-yl)-methanon

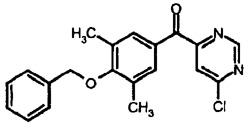

150 mg (1.00 mmol) 4,6-Dichlorpyrimidin, 367 mg (1.50 mmol) 4-Benzyloxy-3,5-Dimethylbenzaldehyd und 166 mg (0.600 mmol) 1,3-Dimethyl-3H-benzimidazol-1-ium-iodid (Chem. Pharm. Bull. 1990, 1147-52) in 10.0 mL THF wurden bei RT verrührt. Dann wurden 73.0 mg (1.51 mmol) Natriumhydrid (50% in Mineralöl) zugefügt und der Reaktionsansatz 2.5 h unter Rückfluss gekocht. Das Reaktionsgemisch wurde auf Eiswasser gegeben und mit DCM extrahiert. Die organische Phase wurde getrocknet und eingeengt.

| | |
|---|---|
| Ausbeute: | 100 mg (28% d. Th.) |
| ESI-MS: | m/z = 353/55 (Cl) (M+H)⁺ |

### Stufe 2: 3-{1-[6-(4-Benzyloxy-3,5-dimethyl-benzoyl)-pyrimidin-4-yl]-piperidin-4-yl}-7-methoxy-1,3,4,5-tetrahydro-benzo[d][1,3]diazepin-2-on

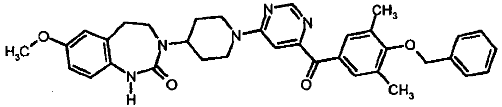

200 mg (0.730 mmol) 7-Methoxy-3-piperidin-4-yl-1,3,4,5-tetrahydro-1,3-benzodiazepin-2-on, 240 mg (0.680 mmol) (4-Benzyloxy-3,5-dimethyl-phenyl)-(6-Chlor-pyrimidin-4-yl)-methanon und 0.400 mL (2.30 mmol) DIPEA wurden in 2.0 mL DMF zusammen gegeben und 3 h bei RT gerührt. Der Ansatz wurde auf Eiswasser gegeben, der Niederschlag wurde abgesaugt und getrocknet.

| | |
|---|---|
| Ausbeute: | 400 mg (89% d. Th.) |
| Reinheit: | 90%ig |
| ESI-MS: | m/z = 592 (M+H)⁺ |
| Rₜ (HPLC): | 5.0 min (Methode C) |

### Intermediat 14

### 6-Chlor-pyrimidin-4-carbonsäuremethoxy-methyl-amid

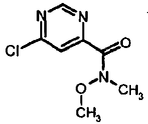

Zu 10.7 g (54.4 mmol) 6-Chlorpyrimidin-4-carbonsäurechlorid und 5.50 g (56.4 mmol) N,O-Dimethylhydroxylamin in 150 mL DCM wurden bei 0°C 20.7 mL (0.120 mol) DIPEA zugegeben und 1 h bei 0°C und 1h bei RT nachgerührt. Der Ansatz wurde mit DCM verdünnt und mit Wasser gewaschen. Die organische Phase wurde mit Natriumsulfat getrocknet, filtriert und eingeengt.

| | |
|---|---|
| Ausbeute: | 11.8 g (100% d. Th.) |
| Reinheit: | 93%ig |

### Intermediat 15

### 6-(4-Chlor-pyridin-2-carbonyl)-4-methyl-3H-benzoxazol-2-on

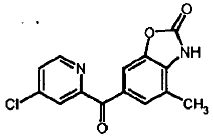

Zu 2.00 g (12.7 mmol) 4-Chlorpicolinsäure in 30 mL DCM wurden 2.80 mL (38.1 mmol) Thionylchlorid und 0.50 mL DMF gegeben und das Gemisch 2 h unter Rückfluss gekocht. Der Reaktionsansatz wurde bis zur Trockne eingeengt und zweimal mit Toluol coevaporiert. Der Rückstand wurde mit 8.00 g (60.0 mmol) Aluminiumtrichlorid und 1.79 g (12.0 mmol) 4-Methyl-3H-benzoxazol-2-on versetzt und bei 130°C gerührt. Der Ansatz wurde mit Eiswasser zersetzt und zweimal mit EtOAc extrahiert. Die organischen Phasen wurden vereinigt, über Magnesiumsulfat getrocknet, filtriert und i.vac. eingeengt. Der Rückstand wurde mit DiPE verrieben und abgesaugt. Zur weiteren Reinigung wurde der Rückstand mittels präparativer HPLC-MS gereinigt. Die Produkt enthaltenden Fraktionen wurden vereinigt und gefriergetrocknet.

| | |
|---|---|
| Ausbeute: | 30 mg (1 % d. Th.) |
| ESI-MS: | m/z = 289/291 (Cl) (M+H)⁺ |
| | m/z = 287/289 (Cl) (M-H)⁻ |
| R_{f}: | 0.17 (Kieselgel, PE / EtOAc = 2 / 1) |

### Intermediat 16

### 5-Phenyl-2-piperidin-4-yl-2,4-dihydro-[1,2,4]triazol-3-on

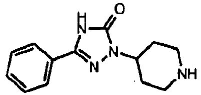

Diese Verbindung und deren Vorstufen wurden analog zu US2001/36946 A1 (US2001-789391) synthetisiert.

| | |
|---|---|
| R_{f}: | 0.28 (Kieselgel, DCM/Cyc/MeOH/ NH₄OH = 70/15/15/2) |

### Intermediat 17

### 1-{1-[6-(4-Benzyloxy-3,5-dimethyl-benzoyl)-pyrimidin-4-yl]-piperidin-4-yl}1,3-dihydroimidazo[4,5-b]pyridin-2-on

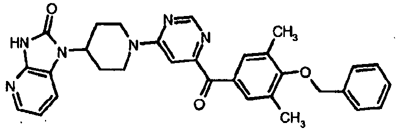

### Stufe 1: 2-Benzyloxy-5-Brom-1,3-dimethyl-benzol

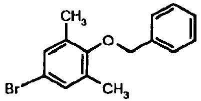

Zu 20 g (96.5 mmol) 2,6-Dimethyl-4-bromphenol und 16.5 g (118 mmol) Kaliumcarbonat in 300 mL Aceton wurden 11.7 mL (96.5 mmol) Benzylbromid getropft und über Nacht bei RT gerührt. Der ausgefallene Niederschlag wurde abgesaugt, mit Aceton nachgewaschen und das Filtrat eingeengt. Der Rückstand wurde in DCM gelöst, über Alox filtriert und mit DCM nachgewaschen. Das Filtrat wurde eingeengt.

| | |
|---|---|
| Ausbeute: | 28.5 g (99 % d. Th.) |
| Reinheit: | 98%ig |
| Rₜ (HPLC): | 1.43 min (Methode E) |

### Stufe 2: (4-Benzyloxy-3,5-dimethyl-phenyl)-(6-Chlor-pyrimidin-4-yl)-methanon

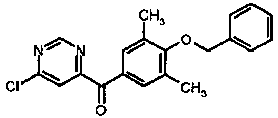

Unter einer Argonatmosphäre wurden 2.8 mL (4.5 mmol) einer 1.6 M n-Butyllithium-Lösung zu einem auf -75°C gekühlten Gemisch aus 1.2 g (4.0 mmol) 2-Benzyloxy-5-Brom-1,3-dimethyl-benzol in 40 mL THF gegeben und 1 h bei -75°C gerührt. Dann wurden 0.96 g (4.3 mmol) 6-Chlor-pyrimidin-4-carbonsäuremethoxy-methyl-amid, gelöst in 10 mL THF, zugetropft. Nach weiteren 30 min Rühren bei -75°C wurde der Reaktionsansatz langsam auf 0°C erwärmt. Der Ansatz wurde mit gesättigter Natriumhydrogencarbonat-Lösung versetzt und mit Diethylether extrahiert. Die organische Phase wurde getrocknet und eingeengt. Der Rückstand wurde mittels Flash-Chromatographie gereinigt.

| | |
|---|---|
| Ausbeute: | 0.43 g (29% d. Th.) |

### Stufe 3: 1-{1-[6-(4-Benzyloxy-3,5-dimethyl-benzoyl)-pyrimidin-4-yl]-piperidin-4-yl}-1,3-dihydro-imidazo[4,5-b]pyridin-2-on

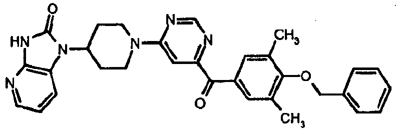

170 mg (0.779 mmol) 1-Piperidin-4-yl-1,3-dihydro-imidazo[4,5-b]pyridin-2-on, 270 mg (0.727 mmol) (4-Benzyloxy-3,5-dimethyl-phenyl)-(6-Chlor-pyrimidin-4-yl)-methanon und 0.200 mL (1.42 mmol) TEA wurden in 2.0 mL DMF zusammen gegeben und über Nacht bei RT gerührt. Das Reaktionsgemisch wurde in Eiswasser eingerührt, der Niederschlag abgesaugt und getrocknet.

| | |
|---|---|
| Ausbeute: | 390 mg (95% d. Th.) |

### Intermediat 18

### 7-Methoxy-3-(1-{6-[7-methyl-1-(2-trimethylsilanyl-ethoxymethyl)-1H-indazol-5-carbonyl]-pyrimidin-4-yl}-piperidin-4-yl)-1,3,4,5-tetrahydro-benzo[d][1,3]diazepin-2-on

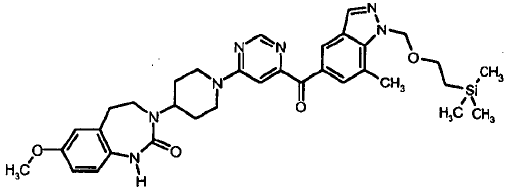

### Stufe1: 4-Brom-2,6-dimethylphenyldiazonium tetrafluoroborat

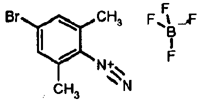

21.2 g (0.104 mol) 4-Brom-2,6-dimethylanilin in 58.0 mL (0.444 mol) Tetrafluorborsäure (48%ig in Wasser) wurden mit Wasser zu einer rührbaren Suspension verdünnt. Zu dem auf 0°C abgekühlten Reaktionsansatz wurden 7.18 g (0.104 mol) Natriumnitrit, gelöst in Wasser, langsam zugetropft. Nach beendeter Zugabe wurde 1 h bei RT nachgerührt. Das als Feststoff ausgefallene Produkt wurde abgesaugt, dreimal mit Diethylether gewaschen und getrocknet.

| | |
|---|---|
| Ausbeute: | 26.5 g (85% d. Th.) |

### Stufe 2: 5-Brom-7-methyl-1H-indazol

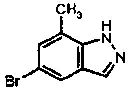

Zu 25.5 g (0.260 mol) Kaliumacetat und 1.72 g (6.50 mmol) 18-Krone-6 in 700 mL Chloroform wurden 39.0 g (0.130 mol) 4-Brom-2,6-dimethylphenyldiazonium-tetrafluoroborat unter mechanischem Rühren portionsweise zugegeben, weitere 3 h bei RT nachgerührt und anschließend über Nacht bei RT stehen gelassen. Das ausgefallene Produkt wurde abgesaugt und mit 100 mL Chloroform gewaschen. Der Niederschlag wurde mit 500 mL Wasser verrührt und mit 800 mL DCM extrahiert. Die organische Phase wurde getrocknet und i.vac eingdampft. Das noch bestehende Filtrat wurde mit 1 L Wasser gewaschen, mit Natriumsulfat getrocknet und eingeengt. Der Rückstand wurde mit DIPE versetzt, die ausgefallene Substanz abgesaugt, nochmals mit DIPE nachgewaschen und getrocknet. Die erhaltenen Feststoffe wurden vereinigt.

| | |
|---|---|
| Ausbeute: | 19.8 g (72% d. Th.) |
| ESI-MS: | m/z = 209/211 (Br) (M-H)⁻ |
| R_{f}: | 0.5 (Kieselgel, DCM/MeOH = 90 / 10) |

### Stufe3: 5-Brom-7-methyl-1-(2-trimethylsilanyl-ethoxymethyl)-1H-indazol

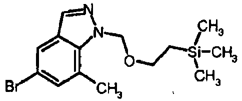

Zu 2.11 g (10.0 mmol) 5-Brom-7-methyl-1H-indazol und 1.80 mL (12.3 mmol) N-Methyldicyclohexylamin in 50 mL THF wurden 1.95 mL (11.0 mmol) (2-Chlormethoxy-ethyl)-trimethyl-silan zugegeben und über Nacht bei RT gerührt. Der entstandene Niederschlag wurde abfiltriert und das Filtrat eingeengt. Der Rückstand wurde mittels Flash-Chromatographie aufgereinigt.

| | |
|---|---|
| Ausbeute: | 0.78 g (23% d. Th.) |
| ESI-MS: | m/z = 341/343 (Br) (M+H)⁺ |
| Rₜ (HPLC): | 1.6 min (Methode E) |

### Stufe 4: (6-Chlor-pyrimidin-4-yl)-[7-methyl-1-(2-trimethylsilanyl-ethoxymethyl)-1H-indazol-5-yl]-methanon

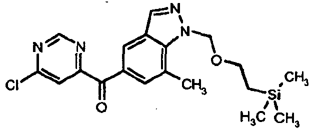

Unter einer Argonatmosphäre wurden 0.34 g (1.00 mmol) 5-Brom-7-methyl-1-(2-trimethylsilanyl-ethoxymethyl)-1H-indazol in 10 mL THF auf -75°C gekühlt, mit 0.70 mL (1.1 mmol) einer 1.6 molare n-Butyllithium-Lösung versetzt und 1 h bei -75°C gerührt. Anschließend wurden 0.25 g (1.1 mmol) 6-Chlor-pyrimidin-4-carbonsäuremethoxy-methyl-amid, in wenig THF gelöst, zugetropft. Das Kältebad wurde entfernt und der Ansatz auf 0°C erwärmt und eine weitere Stunde im Eisbad gerührt. Der Ansatz wurde mit gesättigter Natriumhydrogencarbonat-Lösung verrührt, mit EtOAc extrahiert, die organische Phase getrocknet und eingeengt. Der Rückstand wurde mittels Flash-Chromatogrphie aufgereinigt.

| | |
|---|---|
| Ausbeute: | 58 mg (14% d. Th.) |
| Rₜ (HPLC): | 1.4 min (Methode E) |

### Stufe 5: 7-Methoxy-3-(1-{6-[7-methyl-1-(2-trimethylsilanyl-ethoxymethyl)-1H-indazol-5-carbonyl]-pyrimidin-4-yl}-piperidin-4-vl)-1,3,4,5-tetrahydro-benzo[d][1,3]diazepin-2-on

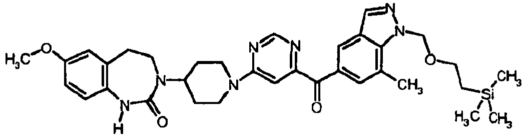

36 mg (0.13 mmol) 7-Methoxy-3-piperidin-4-yl-1,3,4,5-tetrahydro-1,3-benzodiazepin-2-on, 50 mg (0.12 mmol) (6-Chlor-pyrimidin-4-yl)-[7-methyl-1-(2-trimethylsilanyl-ethoxymethyl)-1 H-indazol-5-yl]-methanon und 30 µL (0.21 mmol) TEA wurden in 0.5 mL DMF zusammen gegeben und über Nacht bei RT gerührt. Das Reaktionsgemisch wurde mit Eiswasser versetzt, das als Feststoff ausgefallene Produkt wurde abfiltriert und getrocknet.

| | |
|---|---|
| Ausbeute: | 71 mg (84% d. Th.) |
| ESI-MS: | m/z = 642 (M+H)⁺ |
| Rₜ (HPLC): | 1.78 min (Methode B) |

### Intermediat 19

### 6-(6-Chlor-pyrimidin-4-carbonyl)-3-methyl-3H-benzoxazol-2-on

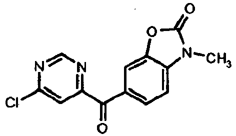

### Stufe 1: 6-(6-Chlor-pyrimidin-4-carbonyl)-3H-benzoxazol-2-on

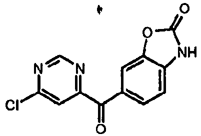

3.93 g (22.2 mmol) 6-Chlorpyrimidin-4-carbonsäurechlorid, 3.00 g (22.2 mmol) 2-Benzoxazolinon und 14.8 g (111 mmol) Aluminiumtrichlorid wurden zusammen gegeben und 3 h bei 130°C erhitzt. Anschließend wurde der Ansatz mit Eiswasser und EtOAc versetzt, der Feststoff abgesaugt und die Phasen getrennt. Die wässrige Phase wurde zweimal mit EtOAc extrahiert, die organischen Phasen wurden vereinigt, getrocknet und eingeengt. Der Rückstand wurde in DIPE verrieben, abgesaugt und getrocknet.

| | |
|---|---|
| Ausbeute: | 1.4 g (23% d. Th.) |
| ESI-MS: | m/z = 274/276 (M-H)⁻ |
| Rₜ (HPLC): | 1.17 min (Methode B) |

### Stufe2: 6-(6-Chlor-pyrimidin-4-carbonyl)-3-methyl-3H-benzoxazol-2-on

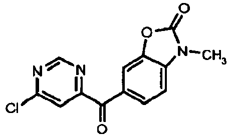

Zu 800 mg (2.90 mmol) 6-(6-Chlor-pyrimidin-4-carbonyl)-3H-benzoxazol-2-on in 10 mL DMF wurden 135 mg (3.10 mmol) Natriumhydrid (55%, Suspension in Mineralöl) gegeben und 30 min bei RT gerührt. Dann wurden 0.368 mL (5.80 mmol) lodmethan zugefügt und eine weitere Stunde bei RT gerührt. Der Ansatz wurde mit Eiswasser versetzt und zweimal mit EtOAc extrahiert. Die organischen Phasen wurden vereinigt, mit Wasser gewaschen, getrocknet und eingeengt. Der Rückstand wurde mit DIPE verrieben, abgesaugt und getrocknet.

| | |
|---|---|
| Ausbeute: | 600 mg (71% d. Th.) |
| ESI-MS: | m/z = 290/292 (M+H)⁺ |
| Rₜ (HPLC): | 1.33 min (Methode B) |

### Intermediat 20:

### 5-(6-Chlor-pyrimidin-4-carbonyl)-1,3-dihydro-indol-2-on

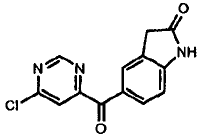

3.93 g (22.2 mmol) 6-Chlorpyrimidin-4-carbonsäurechlorid, 2.96 g (22.2 mmol) 2-Oxindol und 14.8 g (111 mmol) Aluminiumtrichlorid wurden zusammen gegeben und 3 h auf 130°C erhitzt. Anschließend wurde der Ansatz mit Eiswasser versetzt und zweimal mit EtOAc extrahiert. Die organischen Phasen wurden vereinigt, getrocknet und eingeengt. Der Rückstand wurde in DIPE verrieben, abgesaugt, nachgewaschen und getrocknet.

| | |
|---|---|
| Ausbeute: | 1.4 g (23% d. Th.) |
| ESI-MS: | m/z = 272/274 (M-H)⁻ |
| Rₜ (HPLC): | 1.12 min (Methode B) |

### Intermediat 21:

### 6-(2-Brom-pyridin-4-carbonyl)-4-methyl-3H-benzoxazol-2-on

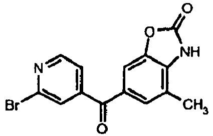

Zu 2.00 g (9.90 mmol) 2-Brompyridin-4-carbonsäure in 30 mL DCM wurden 2.20 mL (30.0 mmol) Thionylchlorid und 0.390 mL (4.80 mmol) DMF gegeben und 2 h unter Rückfluss gekocht. Das Reaktionsgemisch wurde bis zur Trockene eingedampft und zweimal mit Toluol coevaporiert. Der Rückstand wurde mit 6.24 g (46.8 mmol) Aluminiumtrichlorid und 1.50 g (10.1 mmol) 4-Methyl-3H-benzoxazol-2-on versetzt und das erhaltene Gemisch bei 110°C über Nacht gerührt und 5 Stunden bei 130°C gerührt. Der Ansatz wurde mit Eiswasser zersetzt und zweimal mit DCM extrahiert. Die organischen Phasen wurden über Magnesiumsulfat getrocknet, filtriert und i.vac. eingeengt. Der Rückstand wurde mit DIPE / MeOH verrieben und abgesaugt. Das erhaltene Produkt ist ein Gemisch aus der entsprechenden Brom- und Chlorverbindung (40/60).

| | |
|---|---|
| Ausbeute: | 1.0 g (∼30% d. Th.) |

### Intermediat 22

### 7-Chlor-3-piperidin-4-yl-1,3,4,5-tetrahydro-benzo[d][1,3]diazepin-2-on

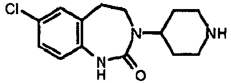

Zu 1.23 g (5.00 mmol) 3-Piperidin-4-yl-1,3,4,5-tetrahydro-benzo[d][1,3]diazepin-2-on in 10.0 mL Tetrachlorkohlenstoff wurden 801 mg (6.00 mmol) N-Chlorsuccinimid gegeben und das Gemisch 72 h unter Rückfluss gekocht. Das Lösungsmittel wurde i.vac. entfernt und das Rohprodukt mittels Flash-Chromatographie gereinigt. Die Produkt enthaltenden Fraktionen wurden einrotiert und mittels präparativer HPLC gereinigt.

| | |
|---|---|
| Ausbeute: | 420 mg (30% d. Th.) |
| ESI-MS: | m/z = 280/282 (Cl) (M+H)⁺ |
| Rₜ (HPLC): | 1.96 min (Methode C) |

### Intermediat 23

### 6-(6-Chlor-pyrimidin-4-carbonyl)-3-ethyl-4-methyl-3H-benzoxazol-2-on

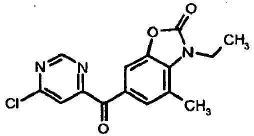

Zu 0.25 g (0.86 mmol) 6-(6-Chlorpyrimidin-4-carbonyl)-4-methyl-3H-benzoxazol-2-on in 2.0 mL DMF wurden 44 mg (1.0 mmol) Natriumhydrid (55%, Suspension in Mineralöl) gegeben und das Gemisch 30 min bei RT gerührt. Anschließend wurden 98 µL (1.2 mmol) Ethyljodid zugegeben und 1 h bei RT gerührt. Der Reaktionsansatz wurde mit Eiswasser verdünnt und mit EtOAc extrahiert. Die organische Phase wurde mit Wasser gewaschen, über Natriumsulfat getrocknet, filtriert und i.vac. eingeengt. Der Rückstand wurde mittels Flashchromatographie gereinigt. Die Produkt enthaltenden Fraktionen wurden vereinigt und eingeengt.

| | |
|---|---|
| Ausbeute: | 110 mg (40 % d. Th.) |
| Rₜ(HPLC): | 3,83 min (Methode C) |

### Intermediat 24

### 6-(6-Chlor-pyrimidin-4-carbonyl)-4-methyl-3H-benzothiazol-2-on

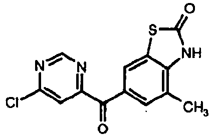

### Stufe 1: 4-Methyl-3H-benzothiazol-2-on

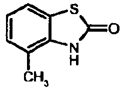

5.00 g (30.5 mmol) 2-Amino-4-Methylbenzothiazol in 15.0 mL Ameisensäure, 6.10 mL Eisessig und 112 mL konz. Salzsäure wurden unter Rühren auf -5°C abgekühlt und langsam mit einer Lösung von 2.10 g (30.5 mmol) Natriumnitrit in 5.0 mL Wasser versetzt. Der Reaktionsansatz wurde 20 min bei dieser Temperatur nachgerührt, dann auf RT erwärmt und anschließend über Nacht unter Rückfluss gekocht. Der abgekühlte Ansatz wurde dann mit Wasser versetzt und mehrmals mit EtOAc extrahiert. Die vereinigten organischen Phasen wurden mit gesättigter Natriumchlorid-Lösung gewaschen, über Natriumsulfat getrocknet, filtriert und das Filtrat eingeengt.

| | |
|---|---|
| Ausbeute: | 3.70 g (74% d. Th.) |
| ESI-MS: | m/z =164 (M-H)⁻ |
| Rₜ (HPLC): | 0.89 min (Methode B) |

### Stufe 2: 6-(6-Chlor-pyrimidin-4-carbonyl)-4-methyl-3H-benzothiazol-2-on

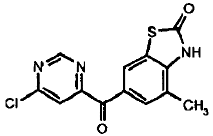

1.93 g (10.0 mmol) 6-Chlorpyrimidin-4-carbonsäurechlorid, 1.80 g (10.9 mmol) 4-Methyl-3H-benzothiazol-2-on und 7.33 g (55.0 mmol) Aluminiumtrichlorid wurden zusammen gegeben und unter Rühren 3 h auf 130°C erhitzt. Der Ansatz wurde mit Eiswasser und EtOAc versetzt, die entstandenen Flocken abgesaugt und die Phasen getrennt. Die wässrige Phase wurde mit EtOAc extrahiert. Die organischen Phasen wurden vereinigt, über Natriumsulfat getrocknet, filtriert und das Filtrat i.vac. eingeengt. Der Rückstand wurde mit DIPE verrieben, der Niederschlag wurde abgesaugt und getrocknet.

| | |
|---|---|
| Ausbeute: | 1.10 g (33% d. Th.) |
| Rₜ(HPLC): | 1.40 min (Methode B) |

### Intermediat 25

### 6-(2-Chlor-pyridin-4-carbonyl)-3,4-dimethyl-3H-benzoxazol-2-on

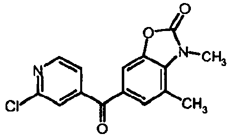

### Stufe 1: 6-(2-Chlor-pyridin-4-carbonyl)-4-methyl-3H-benzoxazol-2-on

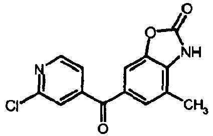

Zu 2.10 g (12.9 mmol) 2-Chlorisonicotinsäure in 30.0 mL DCM wurden 2.80 mL (38.1 mmol) Thionylchlorid und 0.50 mL DMF gegeben und das Gemisch 2 h unter Rückfluss gekocht. Der Reaktionsansatz wurde bis zur Trockene eingeengt und zweimal mit Toluol coevaporiert. Der Rückstand wurde mit 8.00 g (60.0 mmol) Aluminiumtrichlorid und 1.79 g (12.0 mmol) 4-Methyl-3H-benzoxazol-2-on versetzt und bei 130°C über Nacht gerührt. Der Ansatz wurde mit Eiswasser zersetzt und zweimal mit EtOAc extrahiert. Die organischen Phasen wurden vereinigt, über Magnesiumsulfat getrocknet, filtriert und i.vac. eingeengt. Der Rückstand wurde mit DIPE und Isopropanol verrieben, abgesaugt und getrocknet.

| | |
|---|---|
| Ausbeute: | 1.70 g (46% d. Th.) |
| ESI-MS: | m/z = 287/289 (Cl) (M-H)⁻ |
| R_{f}: | 0.13 (Kieselgel, PE / EtOAc = 2 / 1) |

### Stufe 2: 6-(2-Chlor-pyridin-4-carbonyl)-3,4-dimethyl-3H-benzoxazol-2-0n

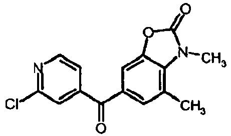

Zu 500 mg (1.73 mmol) 6-(2-Chlor-pyridin-4-carbonyl)-4-methyl-3H-benzoxazol-2-on in 10.0 mL THF wurden 220 mg (1.94 mmol) Kalium-*tert*-butylat gegeben und das Gemisch 30 min bei RT gerührt. Anschließend wurden 0.220 mL (3.46 mmol) Methyljodid zugegeben und über Nacht bei RT gerührt. Der Reaktionsansatz wurde mit EtOAc verdünnt und mehrmals mit gesättigter Natriumchlorid-Lösung gewaschen. Die organische Phase wurde über Magnesiumsulfat getrocknet, filtriert und i.vac. eingeengt. Der Rückstand wurde mit DIPE verrieben und abgesaugt.

| | |
|---|---|
| Ausbeute: | 390 mg (74% d. Th.) |
| ESI-MS: | m/z = 303/5 (Cl) (M+H)⁺ |
| Rₜ(HPLC): | 1.42 min (Methode B) |

### Intermediat 26:

### 3-(3-Fluor-piperidin-4-yl)-7-methoxy-1,3,4,5-tetrahydro-benzo[d][1,3]diazepin-2-on

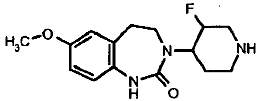

### Stufe 1: (5-Methoxy-2-nitro-phenyl)-acetonitril

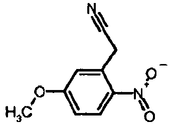

Zu 161 g (1.44 mol) Kalium-*tert-*butylat in 1.50 L DMF wurden innerhalb von 1.5 h bei -30°C eine Lösung aus 110 g (0.653 mol) 4-(Chlor-phenoxy)-acetonitril und 100 g (0.653 mol) 1-Methoxy-4-nitrobenzol in DMF zugetropft. Der Reaktionsansatz wurde 30 min bei -30°C gerührt, anschließend in 2.0 L einer 2N wässrigen Salzsäure-Lösung gegossen und 1 h gerührt. Der entstandene Niederschlag wurde abgesaugt, mit Wasser gewaschen und dann in EtOAc gelöst. Diese Lösung wurde über Natriumsulfat getrocknet, filtriert und das Filtrat eingeengt. Der Rückstand wurde in EtOAc / PE = 1/1 gegeben und kühl gestellt. Das als Feststoff ausgefallene Produkt wurde abgesaugt, mit DIPE gewaschen und getrocknet.

| | |
|---|---|
| Ausbeute: | 55 g (44% d. Th.) |
| ESI-MS: | m/z = 191 (M-H)⁻ |
| R_{f}: | 0.4 (Kieselgel: EtOAc / PE = 1 / 9) |

### Stufe 2: 2-(5-Methoxy-2-nitro-phenyl)-ethylamin

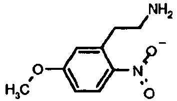

Zu 110 g (0.572 mol) (5-Methoxy-2-nitro-phenyl)-acetonitril in 0.5 L THF wurden langsam bei 10°C 1.5 L einer 1 M Boran-Lösung in THF zugetropft und 16 h bei RT nachgerührt. Der Reaktionsansatz wurde bei 0°C mit 250 ml MeOH versetzt und 1 h bei RT gerührt. Im Anschluss daran wurden 250 mL einer 2N wässrigen Salzsäure-Lösung hinzugefügt und das überschüssige THF eingeengt. Die wässrige Phase wurde mit EtOAc extrahiert, dann mit gesättigter Natriumcarbonat-Lösung basisch gestellt und anschließend mit EtOAc extrahiert. Die organische Phase wurde mit Wasser und gesättigter NaCl-Lösung gewaschen, über Natriumsulfat getrocknet, filtriert und das Filtrat eingeengt.

| | |
|---|---|
| Ausbeute: | 67.0 g (60% d. Th.) |
| ESI-MS: | m/z = 197 (M+H)⁺ |
| R_{f}: | 0.3 (Kieselgel: MeOH / Chloroform = 1 / 9) |

### Stufe 3: 3-Fluor-4-[2-(5-methoxy-2-nitro-phenyl)-ethylamino]-piperidin-1-carbonsäure-tert-butylester

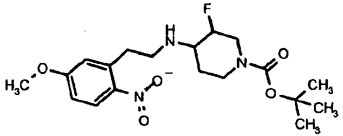

Zu 1.50 g (7.65 mmol) 2-(5-Methoxy-2-nitro-phenyl)-ethylamin und 1.80 g (8.29 mmol) 3-Fluor-4-oxo-piperidin-1-carbonsäure-*tert*-butylester in 25 mL DCM wurden bei 0°C 1.00 mL (17.7 mmol) Essigsäure und 2.09 g (9.86 mmol) Natriumtriacetoxyborhydrid zugefügt und im Anschluss 3 h bei RT gerührt. Der Reaktionsansatz wurde bei 0°C mit einer gesättigten Kaliumcarbonat-Lösung versetzt und die wässrige Phase mehrmals mit DCM extrahiert. Die vereinigten organischen Phasen wurden mit Wasser und gesättigter Natriumchlorid-Lösung gewaschen, über Natriumsulfat getrocknet, filtriert und das Filtrat eingeengt. Der Rückstand wurde mittels Flash-Chromatographie gereinigt.

| | |
|---|---|
| Ausbeute: | 1.75 g (58% d. Th.) |
| R_{f}: | 0.65 (Kieselgel: MeOH / Chloroform = 1 / 9) |

### Stufe 4: 4-[2-(2-Amino-5-methoxy-phenyl)-ethylamino]-3-fluor-piperidin-1-carbon säure-tert-butylester

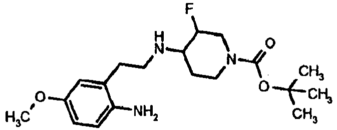

3.50 g (8.81 mmol) 3-Fluor-4-[2-(5-methoxy-2-nitro-phenyl)-ethylamino]-piperidin-1-carbonsäure-*tert*-butylester wurden mit 0.8 g Palladium auf Kohle (Pd/C 10%) und 17.5 mL (359 mmol) Hydrazinhydrat in 50 mL EtOH 16 h bei RT gerührt. Der Katalysator wurde über Kieselgur abgesaugt und die Lösung einrotiert.

| | |
|---|---|
| Ausbeute: | 3 g (93% d. Th.) |
| R_{f}: | 0.5 (Kieselgel: MeOH/Chloroform 1 / 9) |

### Stufe 5: 3-Fluor-4-(7-methoxy-2-oxo-1,2,4,5-tetrahydro-benzo[d][1,3]diazepin-3-yl)-piperidin-1-carbonsäure-tert-butylester

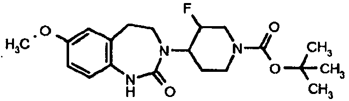

Zu 3.00 g (8.16 mmol) 4-[2-(2-Amino-5-methoxy-phenyl)-ethylamino]-3-fluor-piperidin-1-carbonsäure-*tert*-butylester in 20 mL DMF wurden 3.97 g (24.5 mmol) CDI gegeben und die Mischung 2 h unter Rückfluss gekocht. Der Reaktionsansatz wurde abgekühlt und mit Eiswasser versetzt. Die wäßrige Phase wurde mehrmals mit EtOAc extrahiert, die organischen Phasen vereinigt, über Natriumsulfat getrocknet, filtriert und das Filtrat eingeengt. Der Rückstand wurde mittels Flash-Chromatographie gereinigt.

| | |
|---|---|
| Ausbeute: | 2.10 g (65% d. Th.) |
| ESI-MS: | m/z = 394 (M+H)⁺ |
| R_{f}: | 0.65 (Kieselgel: MeOH / Chloroform = 1 / 9) |

### Stufe 6: 3-(3-Fluor-piperidin-4-yl)-7-methoxy-1,3,4,5-tetrahydro-benzo[d][1,3]diazepin-2-on

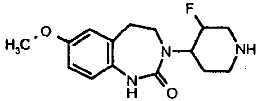

3.20 g (8.13 mmol) 3-Fluor-4-(7-methoxy-2-oxo-1,2,4,5-tetrahydro-benzo[d][1,3]diazepin-3-yl)-piperidin-1-carbonsäure-*tert*-butylester wurden auf 0°C abgekühlt und bei dieser Temperatur langsam mit 50 mL einer Salzsäure-Lösung (4 M in Dioxan) versetzt. Im Anschluß wurde auf RT erwärmt und 16 h bei RT gerührt. Der entstandene Niederschlag wurde abgesaugt, mit Diethylether gewaschen und getrocknet.

| | |
|---|---|
| Ausbeute: | 2.20 g (92% d. Th.) |
| ESI-MS: | m/z = 294 (M+H)⁺ |
| R_{f}: | 0.2 (Kieselgel: MeOH / Chloroform = 1 / 9) |

### Intermediat 27

### 7-Methoxy-3-(3-methyl-piperidin-4-yl)-1,3,4,5-tetrahydro-benzo[d][1,3]diazepin-2-on

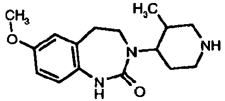

### Stufe 1: 4-[2-(5-Methoxy-2-nitro-phenyl)ethylamino]-3-methyl-piperidin-1-carbonsäure-tert-butylester

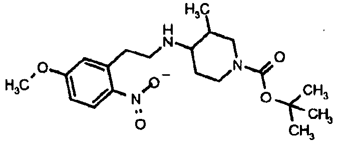

Zu 2.50 g (12.7 mmol) 2-(5-Methoxy-2-nitro-phenyl)-ethylamin und 2.93 g (13.7 mmol) 3-Methyl-4-oxo-piperidin-1-carbonsäure-*tert*-butylester (WO2004/41777) in 75 mL DCM wurden bei 0°C 1.74 mL (29.0 mmol) Essigsäure und 3.48 g (16.4 mmol) Natriumtriacetoxyborhydrid gegeben und das Gemisch 3 h bei RT gerührt. Der Reaktionsansatz wurde bei 0°C mit einer gesättigten Kaliumcarbonat-Lösung versetzt und die wässrige Phase mehrmals mit DCM extrahiert. Die vereinigten organischen Phasen wurden mit Wasser und gesättigter Natriumchlorid-Lösung gewaschen, über Natriumsulfat getrocknet, filtriert und das Filtrat eingeengt. Der Rückstand wurde mittels Flash-Chromatographie gereinigt.

| | |
|---|---|
| Ausbeute: | 4.50 g (90% d. Th.) |
| ESI-MS: | m/z = 394 (M+H)⁺ |
| R_{f}: | 0.65 (Kieselgel: MeOH / Chloroform = 1 / 9) |

### Stufe 2: 4-[2-(2-Amino-5-methoxy-phenyl)-ethylamino]-3-methyl-piperidin-1-carbon-säure-tert-butylester

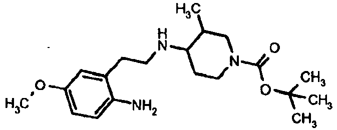

4.50 g (11.4 mmol) 4-[2-(5-Methoxy-2-nitro-phenyl)-ethylamino]-3-methyl-piperidin-1-carbonsäure-*tert*-butylester wurden mit 0.6 g Palladium auf Kohle (Pd/C 10%) und 22.5 mL (460 mmol) Hydrazinhydrat in 100 mL EtOH 16 h bei RT gerührt. Der Katalysator wurde über Kieselgur abgesaugt und das Filtrat i.vac. eingeengt.

| | |
|---|---|
| Ausbeute: | 4 g (96% d. Th.) |
| ESI-MS: | m/z = 364 (M+H)⁺ |
| R_{f}: | 0.5 (Kieselgel: MeOH / Chloroform = 1 / 9) |

### Stufe 3: 4-(7-Methoxy-2-oxo-1,2,4,5-tetrahydro-benzo[d][1,3]diazepin-3-yl)-3-methyl-piperidin-1-carbonsäure-tert-butylester

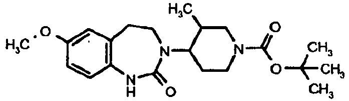

Zu 5.40 g (14.9 mmol) 4-[2-(2-Amino-5-methoxy-phenyl)-ethylamino]-3-methyl-piperidin-1-carbonsäure-*tert*-butylester in 75 mL DMF wurden 7.20 g (44.4 mmol) CDI gegeben und die Mischung 2 h unter Rückfluss gekocht. Der Reaktionsansatz wurde auf RT abgekühlt und 16 h gerührt. Nach Zugabe von Eiswasser wurde die wäßrige Phase mehrmals mit EtOAc extrahiert. Die organische Phase wurde über Natriumsulfat getrocknet, filtriert und das Filtrat i.vac. eingeengt. Der erhaltene Rückstand wurde mittels Flash-Chromatographie gereinigt.

| | |
|---|---|
| Ausbeute: | 4.00 g (69% d. Th.) |
| R_{f}: | 0.65 (Kieselgel: MeOH / Chloroform = 1 / 9) |

### Stufe 4: 7-Methoxy-3-(3-methyl-Piperidin-4-yl)-1,3,4,5-tetrahydro-benzo[d][1,3]diazepin-2-on

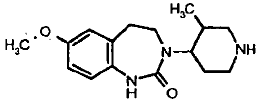

2.50 g (6.42 mmol) 4-(7-Methoxy-2-oxo-1,2,4,5-tetrahydro-benzo[d][1,3]diazepin-3-yl)-3-methyl-piperidin-1-carbonsäure-*tert*-butylester wurden bei 0°C langsam mit 50 mL Salzsäure-Lösung (4 M in Dioxan) versetzt. Dann wurde auf RT erwärmt und 16 h nachgerührt. Der entstandene Niederschlag wurde abgesaugt, mit Diethylether gewaschen und getrocknet.

| | |
|---|---|
| Ausbeute: | 1.50 g (81 % d. Th.) |
| ESI-MS: | m/z = 290 (M+H)⁺ |
| R_{f}: | 0.2 (Kieselgel: MeOH / Chloroform = 1 / 9) |

### Intermediat 28

### 7-Methoxy-3-(2-methyl-piperidin-4-yl)-1,3,4,5-tetrahydro-benzo[d][1,3]diazepin-2-on

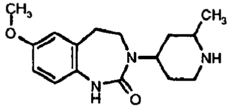

### Stufe 1: 4-[2-(5-Methoxy-2-nitro-phenyl)-ethylamino]-2-methyl-piperidin-1-carbon-säurebenzylester

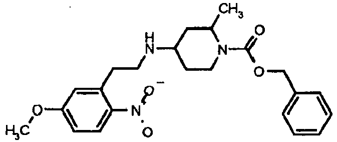

Zu 1.50 g (7.65 mmol) 2-(5-Methoxy-2-nitro-phenyl)-ethylamin und 2.04 g (8.25 mmol) 2-Methyl-4-oxo-piperidin-1-carbonsäurebenzylester (WO2007/11810) in 75 mL DCM wurden bei 0°C 1.04 mL (17.3 mmol) Essigsäure und 2.09 g (9.86 mmol) Natriumtriacetoxyborhydrid zugefügt und 3 h bei RT nachgerührt. Dann wurde der Reaktionsansatz bei 0°C mit einer gesättigten Kaliumcarbonat-Lösung versetzt und die wässrige Phase mehrmals mit DCM extrahiert. Die vereinigten organischen Phasen wurden mit Wasser und gesättigter Natriumchlorid-Lösung gewaschen, über Natriumsulfat getrocknet, filtriert und das Filtrat eingeengt. Der Rückstand wurde mittels Flash-Chromatographie gereinigt.

| | |
|---|---|
| Ausbeute: | 3.00 g (92% d. Th.) |
| ESI-MS: | m/z = 428 (M+H)' |
| R_{f}: | 0.6 (Kieselgel: MeOH / Chloroform = 1 / 9) |

### Stufe 2: 4-[2-(2-Amino-5-methoxy-phenyl)-ethylaminol]-2-methyl-piperidin-1-carbon-säurebenzylester

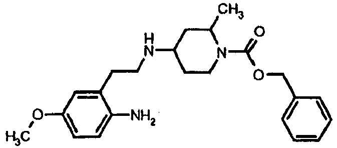

5.30 g (12.4 mmol)_4-[2-(5-Methoxy-2-nitro-phenyl)-ethylamino]-2-methyl-piperidin-1-carbonsäurebenzylester wurden mit 1.5 g Palladium auf Kohle (Pd/C 10%) und 24.3 mL (499 mmol) Hydrazinhydrat in 100 mL EtOH 16 h bei RT gerührt. Der Katalysator wurde über Kieselgur abgesaugt und das Filtrat i.vac. eingeengt.

| | |
|---|---|
| Ausbeute: | 4.60 g (93% d. Th.) |
| ESI-MS: | m/z = 398 (M+H)⁺ |
| R_{f}: | 0.5 (Kieselgel: MeOH / Chloroform = 1 / 9) |

### Stufe 3: 4-(7-Methoxy-2-oxo-1,2,4,5-tetrahydro-benzo[d][1,3]diazepin-3-yl)-2-methyl-piperidin-1-carbonsäurebenzylester

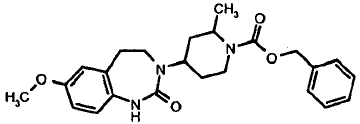

Zu 2.60 g (6.54 mmol) 4-[2-(2-Amino-5-methoxy-phenyl)-ethylamino]-2-methyl-piperidin-1-carbonsäurebenzylester in 20 mL DMF wurden 3.17 g (19.6 mmol) CDI gegeben und die Mischung 2 h unter Rückfluss gekocht. Der Reaktionsansatz wurde auf RT abgekühlt und 16 h gerührt. Anschließend wurde Eiswasser zugesetzt. Die wäßrige Phase wurde mehrmals mit EtOAc extrahiert. Die organischen Phasen wurden vereinigt, über Natriumsulfat getrocknet, filtriert und das Filtrat i.vac. eingeengt. Der Rückstand wurde mittels Flash-Chromatographie gereinigt.

| | |
|---|---|
| Ausbeute: | 2.24 g (81% d. Th.) |
| ESI-MS: | m/z = 424 (M+H)⁺ |
| R_{f}: | 0.65 (Kieselgel: MeOH / Chloroform = 1 / 9) |

### Stufe 4: 7-Methoxy-3-(2-methyl-piperidin-4-yl)-1,3,4,5-tetrahydrobenzo[d][1,3]diazepin-2-on

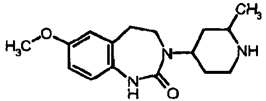

2.00 g (4.88 mmol) 4-(7-Methoxy-2-oxo-1,2,4,5-tetrahydro-benzo[d][1,3]diazepin-3-yl)-2-methyl-piperidin-1-carbonsäurebenzylester wurden auf 0°C abgekühlt und bei dieser Temperatur langsam mit 50 mL Salzsäure-Lösung (4 M in Dioxan) versetzt. Dann wurde auf RT erwärmt und 16 h nachgerührt. Der entstandene Niederschlag wurde abgesaugt, mit Diethylether gewaschen und getrocknet.

| | |
|---|---|
| Ausbeute: | 1.20 g (85% d. Th.) |
| ESI-MS: | m/z = 290 (M+H)⁺ |
| R_{f}: | 0.2 (Kieselgel: MeOH / Chloroform = 1 / 9) |

### Intermediat 29

### 6-(2,6-Dichlor-pyridin-4-carbonyl)-3,4-dimethyl-3H-benzoxazol-2-on

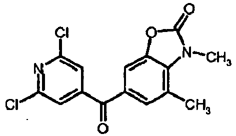

### Stufe 1: 6-(2,6-Dichlor-pyridin-4-carbonyl)-4-methyl-3H-benzoxazol-2-on

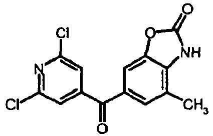

3.00 g (14.3 mmol) 2,6-Dichlorpyridin-4-carbonsäurechlorid, 1.80 g (11.0 mmol) 4-Methyl-3H-benzoxazol-2-on und 9.47 g (71.0 mmol) Aluminiumtrichlorid wurden zusammen gegeben und unter Rühren 2 h auf 125°C erhitzt. Anschließend wurde der Ansatz mit Eiswasser und EtOAc zersetzt und die Phasen getrennt. Die wässrige Phase wurde mehrmals mit EtOAc extrahiert. Die organischen Phasen wurden vereinigt, über Natriumsulfat getrocknet, filtriert und das Filtrat i.vac. eingeengt. Der Rückstand wurde mit Diethylether verrieben, der Niederschlag wurde abgesaugt und i.vac. getrocknet.

| | |
|---|---|
| Ausbeute: | 2.90 g (63 % d. Th.) |
| ESI-MS: | m/z = 321/323/325 (2 x Cl) (M+H)⁺ |
| Rₜ(HPLC): | 1.49 min (Methode B) |

### Stufe 2: 6-(2,6-Dichlor-pyridin-4-carbonyl)-3,4-dimethyl-3H-benzoxazol-2-on

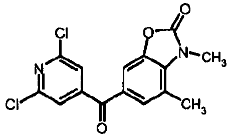

Unter einer Stickstoffatmosphäre wurden 2.30 g (7.12 mmol) 6-(2,6-Dichlor-pyridin-4-carbonyl)-4-methyl-3H-benzoxazol-2-on in 10 mL DMF bei 0°C mit 310 mg (7.10 mmol) Natriumhydrid (55%, Suspension in Mineralöl) versetzt. Der Reaktionsansatz wurde 30 min bei RT gerührt. Anschließend wurden 0.44 mL (7.10 mmol) lodmethan zugegeben und über Nacht bei RT gerührt. Der Reaktionsansatz wurde auf Wasser gegossen und das ausgefallene Produkt abgesaugt, mit Wasser gewaschen und getrocknet.

| | |
|---|---|
| Ausbeute: | 2.40 g (quantitativ) |
| ESI-MS: | m/z = 409/411 (2 x Cl) (M+H)⁺ |
| Rₜ(HPLC): | 1.59 min (Methode B) |

### Intermediat 30

### 6-(2-Benzyloxy-6-chlor-pyridin-4-carbonyl)-3,4-dimethyl-3H-benzoxazol-2-on

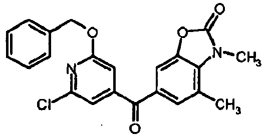

### Stufe 1: 6-(2-Benzyloxy-6-chlor-pyridin-4-carbonyl)-4-methyl-3H-benzoxazol-2-on

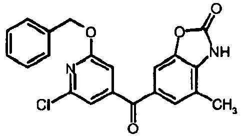

Zu 1.24 g (3.85 mmol) 6-(2,6-Dichlor-pyridin-4-carbonyl)-4-methyl-3H-benzoxazol-2-on in 50 mL THF wurden 3.85 mL (3.85 mmol) Natriumbenzylat-Lösung (1 M in Benzylalkohol) gegeben und über Nacht bei RT gerührt. Der Reaktionsansatz wurde mit gesättigter Natriumhydrogencarbonat-Lösung versetzt und mit EtOAc extrahiert. Die vereinigten organischen Phasen wurden getrocknet und i.vac. eingeengt.

| | |
|---|---|
| Ausbeute: | 1.10 g (72 % d. Th.) |
| Rₜ(HPLC): | 2.60 min (Methode F) |

### Stufe 2: 6-(2-Benzyloxy-6-chlor-pyridin-4-carbonyl)-3,4-dimethyl-3H-benzoxazol-2-on

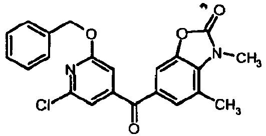

Unter Stickstoffatmosphäre und Eiskühlung wurden zu 2.00 g (5.07 mmol) 6-(2-Benzyl-oxy-6-chlor-pyridin-4-carbonyl)-4-methyl-3H-benzoxazol-2-on in 10 mL DMF 220 mg (5.06 mmol) Natriumhydrid (55%, Suspension in Mineralöl) gegeben. Der Reaktionsansatz wurde 30 min bei RT gerührt. Dann wurden 0.312 mL (5.06 mmol) lodmethan zugegeben und über Nacht bei RT gerührt. Der Reaktionsansatz wurde auf Wasser gegossen und das ausgefallene Produkt abgesaugt, mit Wasser gewaschen und getrocknet. Der Rückstand wurde mit Diethylether ausgerührt, abgesaugt und getrocknet.

| | |
|---|---|
| Ausbeute: | 2.00 g (97 % d. Th.) |
| ESI-MS: | m/z = 409 (Cl) (M+H)⁺ |
| Rₜ(HPLC): | 1.80 min (Methode B) |

### Intermediat 31

### 6-(3-Brom-benzoyl)-3,4-dimethyl-3H-benzoxazol-2-on

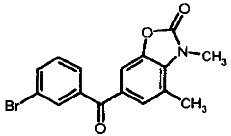

### Stufe 1: 6-(3-Brom-benzoyl)-4-methyl-3H-benzoxazol-2-on

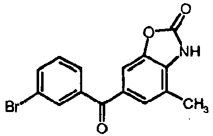

2.08 g (9.48 mmol) 3-Brom-benzoesäurechlorid, 1.41 g (9.48 mmol) 4-Methyl-3H-benzoxazol-2-on und 5.33 g (40.0 mmol) Aluminiumtrichlorid wurden unter Rühren 1.5 h auf 125°C erhitzt. Anschließend wurde der Ansatz mit Eiswasser versetzt und die ausgefallene Schmiere vom Lösungsmittel durch Abdekantieren getrennt. Der Rückstand wurde in EtOAc gelöst, eingeengt und wenig MeOH verrieben. Der ausgefallene Niederschlag wurde abgesaugt, mit Diethylether gewaschen und i.vac. getrocknet.

| | |
|---|---|
| Ausbeute: | 1.00 g (32 % d. Th.) |
| ESI-MS: | m/z = 332 (M+H)⁺ |
| Rₜ(HPLC): | 1.53 min (Methode B) |

### Stufe 2: 6-(3-Brom-benzoyl)-3,4-dimethyl-3H-benzoxazol-2-on

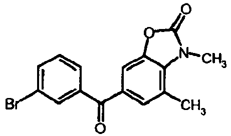

Zu 1.00 g (3.01 mmol) 6-(3-Brom-benzoyl)-4-methyl-3H-benzoxazol-2-on in 4 mL DMF wurden bei RT 144 mg (3.30 mmol) Natriumhydrid (55%, Suspension in Mineralöl) gegeben. Der Reaktionsansatz wurde 30 min bei RT gerührt. Anschließend wurden 0.285 mL (4.50 mmol) lodmethan zugegeben und 1 h bei RT gerührt. Der Reaktionsansatz wurde auf Eiswasser gegossen und der ausgefallene Niederschlag abgesaugt, mit Wasser gewaschen und i.vac. getrocknet.

| | |
|---|---|
| Ausbeute: | 0.98 g (94 % d. Th.) |
| ESI-MS: | m/z = 346 (M+H)⁺ |
| Rₜ(HPLC): | 1.61 min (Methode B) |

### Intermediat 32

### 6-Methoxy-3-piperidin-4-yl-1H-chinolin-2-on

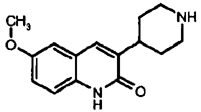

### Stufe 1: 1-Benzyl-4-(2-chlor-6-methoxy-chinolin-3-yl)-piperidin-4-ol

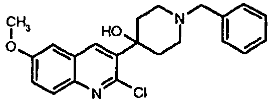

Unter Argonatmosphäre wurden 14.0 mL (28.0 mmol) einer 2 M Lithiumdiisopropylamid-lösung in 50 mL THF auf -78°C gekühlt und unter Rühren mit einer Lösung aus 5.00 g (25.1 mmol) 2-Chlor-6-methoxy-chinolin in THF versetzt. Nach 1 h Rühren bei -78°C wurden 4.5 mL (25.2 mmol) N-Benzylpiperidon zugetropft. Nach 1 h Rühren bei -78°C ließ man den Ansatz auf RT kommen und rührte über Nacht nach. Der Ansatz wurde i.vac. eingeengt und mittels Flash-Chromatographie über Alox gereinigt. Die Produkt enthaltenden Fraktionen wurden vereinigt und eingeengt.

| | |
|---|---|
| Ausbeute: | 2.10 g (13% d. Th.) |
| Reinheit: | 60 % |
| ESI-MS: | m/z = 383 (M+H)⁺ |
| Rₜ(HPLC): | 1.14 min (Methode B) |

### Stufe 2: 3-(1-Benzyl-1,2,3,6-tetrahydro-pyridin-4-yl)-6-methoxy-chinolin-2-ol

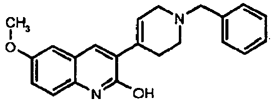

1.90 g (4.96 mmol) 1-Benzyl-4-(2-chlor-6-methoxy-chinolin-3-yl)-piperidin-4-ol wurden in 25 mL einer 4N wässrigen Salzsäure-Lösung gegeben und über Nacht bei 100°C gerührt. Anschließend wurden 15 ml einer konzentrierten wässrigen Salzsäure-Lösung zugetropft und nochmals über Nacht gerührt. Der Ansatz wurde i.vac. auf die Hälfte eingeengt, mit Wasser verdünnt und mit EtOAc extrahiert. Die organische Phase wurde über Natriumsulfat getrocknet, filtriert und eingeengt. Der Rückstand wurde mit PE/EtOAc verrieben und das als Feststoff zurückgebliebene Produkt abgesaugt und getrocknet.

| | |
|---|---|
| Ausbeute: | 165 mg (8 % d. Th.) |
| Reinheit: | 80 % |
| ESI-MS: | m/z = 347 (M+H)⁺ |

### Stufe 3: 6-Methoxy-3-piperidin-4-yl-1H-chinolin-2-on

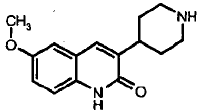

Ein Gemisch aus 160 mg (0.462 mmol) 3-(1-Benzyl-1,2,3,6-tetrahydro-pyridin-4-yl)-6-methoxy-chinolin-2-ol und 20 mg Palladium auf Kohle (Pd/C 10 %) in 30 mL MeOH wurde zunächst 17.5 h bei 50°C in einer Wasserstoffatmosphäre von 50 psi hydriert. Anschließend wurden 10 mL THF und 20 mg Palladium auf Kohle (Pd/C 10 %) hinzugefügt und weitere 2 h unter den gleichen Bedingungen hydriert. Es wurden nochmals 20 mg Palladium auf Kohle (Pd/C 10 %) zugegeben und über Nacht bei 50°C in einer Wasserstoffatmosphäre von 60 psi hydriert. Dann wurde das Reaktionsgemisch filtriert, mit DMF nachgewaschen und das Filtrat eingeengt. Der Rückstand wurde in EtOAc gegeben, mit PE verrieben und filtriert. Der Niederschlag wurde mit DIPE nachgewaschen und getrocknet.

| | |
|---|---|
| Ausbeute: | 56 mg (35 % d. Th.) |
| Reinheit: | 75 % |
| ESI-MS: | m/z = 259 (M+H)⁺ |
| Rₜ(HPLC): | 0.90 min (Methode B) |

### Intermediat 33

### 6-(3,6-Dichlor-pyridazin-4-carbonyl)-4-methyl-3H-benzoxazol-2-on

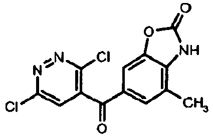

Zu 1.92 g (9.95 mmol) 3,6-Dichlor-pyridazin-4-carbonsäure in 10 mL 1,2-Dichlorethan wurden 2.00 mL (27.4 mmol) Thionylchlorid zugegeben und 2 h unter Rückfluss gekocht. Der Reaktionsansatz wurde bis zur Trockene eingeengt und mit 1,2-Dichlorethan coevaporiert. Dann wurden 5.30 g (39.8 mmol) Aluminiumtrichlorid und 1.56 g (10.5 mmol) 4-Methyl-3H-benzoxazol-2-on zugegeben und das Gemisch unter einer Stickstoffatmosphäre 1 h bei 100°C gerührt. Anschließend wurden weitere 2 h bei 120°C, 3 h bei 130°C gerührt und dann 48 h bei RT abgekühlt. Der Ansatz wurde mit Eiswasser zersetzt und mit DCM extrahiert. Die organischen Phasen wurden vereinigt, mit Wasser gewaschen, über Natriumsulfat getrocknet, filtriert und i.vac. eingeengt. Der Rückstand wurde mit DIPE verrieben, abgesaugt und getrocknet.

| | |
|---|---|
| Ausbeute: | 0.660 g (18% d. Th.) |
| ESI-MS: | m/z = 324 (M+H)⁺ |
| Rₜ(HPLC): | 1.43 min (Methode B) |

### Intermediat 34

### 6-(5-Brom-1-oxy-pyridin-3-carbonyl)-3,4-dimethyl-3H-benzoxazol-2-on

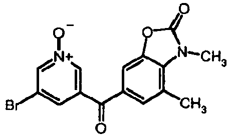

### Stufe 1: 5-Brom-nicotinsäurechlorid Hydrochlorid

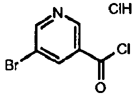

2.00 g (9.90 mmol) 5-Bromnicotinsäure wurden mit 20 mL Thionylchlorid vermischt und 4 h gekocht. Der Ansatz wurde i.vac. bis zur Trockne eingeengt und zweimal mit Toluol coevaporiert. Der Rückstand wurde als Rohprodukt weiter umgesetzt.

| | |
|---|---|
| Ausbeute: | 2.35 g (92% d. Th.) |

### Stufe 2: 6-(5-Brom-pyridin-3-carbonyl)-4-methyl-3H-benzoxazol-2-on

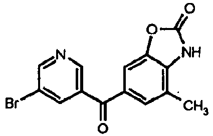

2.57 g (10.0 mmol) 5-Brom-nicotinsäurechlorid Hydrochlorid, 1.49 g (10.0 mmol) 4-Methyl-3H-benzoxazol-2-on und 5.33 g (40.0 mmol) Aluminiumtrichlorid wurden unter Rühren 1.5 h auf 125°C erhitzt. Nach dem Abkühlen auf RT wurde der Ansatz mit Eiswasser versetzt. Der ausgefallene Niederschlag wurde abgesaugt, mit Wasser gewaschen und i.vac. getrocknet.

| | |
|---|---|
| Ausbeute: | 2.30 g (69 % d. Th.) |
| ESI-MS: | m/z = 333 (M+H)⁺ |
| Rₜ(HPLC): | 1.41 min (Methode B) |

### Stufe 3: 6-(5-Brom-pyridin-3-carbonyl)-3,4-dimethyl-3H-benzoxazol-2-on

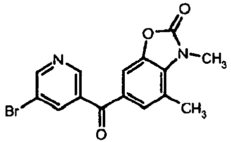

Zu 1.27 g (3.80 mmol) 6-(5-Brom-pyridin-3-carbonyl)-4-methyl-3H-benzoxazol-2-on in 5 mL DMF wurden bei RT 0.18 g (4.00 mmol) Natriumhydrid (55%, Suspension in Mineralöl) gegeben. Der Reaktionsansatz wurde 30 min bei RT gerührt. Anschließend wurden 0.32 mL (5.00 mmol) lodmethan zugegeben und über Nacht bei RT gerührt. Der Reaktionsansatz wurde auf Eiswasser gegossen und der ausgefallene Niederschlag wurde abgesaugt, mit Wasser nachgewaschen und i.vac. getrocknet.

| | |
|---|---|
| Ausbeute: | 1.15 g (87% d. Th.) |
| ESI-MS: | m/z = 347 (M+H)⁺ |
| Rₜ(HPLC): | 1.54 min (Methode B) |

### Stufe 4: 6-(5-Brom-1-oxy-pyridin-3-carbonyl)-3,4-dimethyl-3H-benzoxazol-2-on

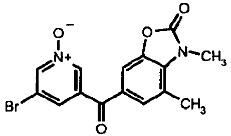

Zu 1.05 g (3.03 mmol) 6-(5-Brom-pyridin-3-carbonyl)-3,4-dimethyl-3H-benzoxazol-2-on in 15 mL DCM wurden 0.690 g (4.00 mmol) m-Chlor-perbenzoesäure zugegeben und 4 h bei RT gerührt. Nach Zugabe von weiteren 100 mg m-Chlor-perbenzoesäure wurde der Reaktionsansatz über Nacht bei RT gerührt. Dann wurde mit DCM verdünnt und zweimal mit 1 N wässriger Natronlauge ausgeschüttelt. Die organsiche Phase wurde über Natriumsulfat getrocknet, filtriert und i.vac. eingeengt und der erhaltene Rückstand getrocknet.

| | |
|---|---|
| Ausbeute: | 1.05 g (96 % d. Th.) |
| ESI-MS: | m/z = 363 (M+H)⁺ |
| Rₜ(HPLC): | 1.27 min (Methode B) |

### Intermediat 35

### 6-(2-Chlor-6-methoxy-pyridin-4-carbonyl)-3,4-dimethyl-3H-benzoxazol-2-on

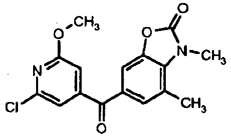

### Stufe 1: 6-(2,6-Dichlor-pyridin-4-carbonyl)-4-methyl-3H-benzoxazol-2-on

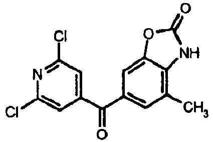

10.0 g (47.5 mmol) 2,6-Dichlorpyridin-4-carbonsäurechlorid, 7.08 g (47.5 mmol) 4-Methyl-3H-benzoxazol-2-on und 32.0 g (240 mmol) Aluminiumtrichlorid wurden unter Rühren 1 h auf 120°C erhitzt. Der Ansatz wurde mit Eiswasser versetzt und mit EtOAc mehrmals extrahiert. Der aus EtOAc ausgefallene Feststoff wurde abgesaugt und mit EtOAc nachgewaschen. Die zurückgebliebene organische Phase wurde abgetrennt, über Natriumsulfat getrocknet, filtriert und eingeengt. Der Rückstand wurde mit wenig EtOAc versetzt, der ausgefallene Feststoff wurde abgesaugt und mit wenig EtOAc gewaschen. Zur weiteren Reinigung wurde aus EtOAc umkristallisiert.

| | |
|---|---|
| Ausbeute: | 7.00 g (46 % d. Th.) |
| ESI-MS: | m/z = 323 (M+H)⁺ |
| Rₜ(HPLC): | 1.6 min (Methode B) |

### Stufe 2: 6-(2-Chlor-6-methoxy-pyridin-4-carbonyl)-4-methyl-3H-benzoxazol-2-on

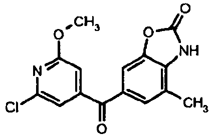

Unter Stickstoffatmosphäre wurden zu 50 mL MeOH 0.21 g (9.3 mmol) Natrium portionsweise gegeben. Nachdem sich das Natrium vollständig gelöst hatte, wurden 1.0 g (3.1 mmol) 6-(2,6-Dichlor-pyridin-4-carbonyl)-4-methyl-3H-benzoxazol-2-on zugegeben und das Gemisch 5 h unter Rückfluss gekocht. Anschließend wurde der Reaktionsansatz i.vac. eingeengt, der Rückstand mit 50 mL Wasser versetzt und der ausgefallene Feststoff abgesaugt. Dieser wurde mit wenig Wasser nachgewaschen und getrocknet.

| | |
|---|---|
| Ausbeute: | 0.9 g (91 % d. Th.) |
| ESI-MS: | m/z = 319 (M+H)⁺ |
| Rₜ(HPLC): | 1.45 min (Methode B) |

### Stufe 3: 6-(2-Chlor-6-methoxy-pyridin-4-carbonyl)-3,4-dimethyl-3H-benzoxazol-2-on

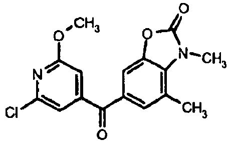

Unter Stickstoffatmosphäre wurden zu 0.90 g (2.8 mmol) 6-(2-Chlor-6-methoxy-pyridin-4-carbonyl)-4-methyl-3H-benzoxazol-2-on in 10 mL DMF bei RT 0.13 g (2.9 mmol) Natriumhydrid (55%, Suspension in Mineralöl) gegeben. Der Reaktionsansatz wurde 30 min bei RT gerührt. Anschließend wurden 0.18 mL (5.0 mmol) lodmethan in 1 mL DMF zugetropft und über Nacht bei RT gerührt. Der Reaktionsansatz wurde auf Wasser gegossen und der ausgefallene Niederschlag abgesaugt und mit Wasser nachgewaschen. Der Rückstand wurde mit Diethylether ausgerührt, abgesaugt und getrocknet.

| | |
|---|---|
| Ausbeute: | 0.84 g (89 % d. Th.) |
| ESI-MS: | m/z = 333 (M+H)⁺ |
| Rₜ(HPLC): | 1.70 min (Methode B) |

### Intermediat 36

### 3-(1-{5-[Hydroxy-(4-methyl-2-oxo-2,3-dihydro-benzoxazol-6-yl)-methyl]-pyridazin-3-yl}-piperidin-4-yl)-7-methoxy-1,3,4,5-tetrahydro-benzo[d][1,3]diazepin-2-on

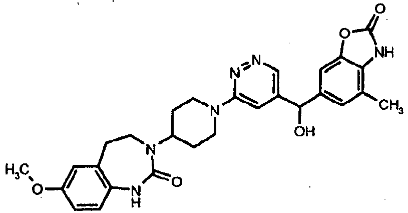

Eine Mischung aus 0.25 g (0.40 mmol) 3-{1-[6-Chloro-5-(4-methyl-2-oxo-2,3-dihydro-benzoxazol-6-carbonyl)-pyridazin-3-yl]-piperidin-4-yl}-7-methoxy-1,3,4,5-tetrahydro-benzo[d][1,3]diazepin-2-on, 0.10 mL (0.71 mmol)TEA und 50 mg Palladium auf Kohle (Pd/C 10%) in MeOH wurde 3 h bei 50°C in einer Wasserstoff-Atmosphäre von 50 psi hydriert. Nach Filtration des Reaktionsgemisches wurde das Filtrat auf ca. 3 mL eingeengt und mit wenig Eiswasser versetzt. Der entstandene Niederschlag wurde abgesaugt und getrocknet. Der Rückstand enthielt das gewünschte Produkt im Gemisch und wurde ohne weitere Aufreinigung in die nächste Stufe eingesetzt.

| | |
|---|---|
| Ausbeute: | 130 mg (12% d. Th. ) |
| Reinheit: | 20 % |

### Intermediat 37

### 6-(3-Brom-4-fluor-benzoyl)-3,4-dimethyl-3H-benzoxazol-2-on

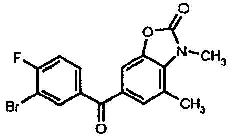

### Stufe 1: 3-Brom-4-fluor-benzoesäurechlorid

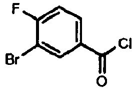

2.17 g (9.41 mmol) 3-Brom-4-Fluorbenzoesäure wurden mit 20 mL Thionylchlorid vermischt und anschließend 2 h gekocht. Der Reaktionsansatz wurde bis zur Trockene eingeengt und zweimal mit Toluol coevaporiert. Der Rückstand wurde als Rohprodukt weiter umgesetzt.

| | |
|---|---|
| Ausbeute: | 2.23 g |

### Stufe 2: 6-(3-Brom-4-fluor-benzoyl)-4-methyl-3H-benzoxazol-2-on

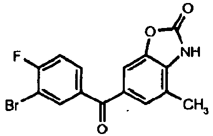

2.23 g (9.40 mmol) 3-Brom-4-fluor-benzoesäurechlorid, 1.40 g (9.40 mmol) 4-Methyl-3H-benzoxazol-2-on und 5.01 g (37.60 mmol) Aluminiumtrichlorid wurden unter Rühren 1.5 h auf 125°C erhitzt. Nach dem Abkühlen auf RT wurde der Ansatz mit Eiswasser versetzt. Der ausgefallene Niederschlag wurde abgesaugt und mit Wasser gewaschen. Dann wurde der Niederschlag mit MeOH verrieben, abgesaugt, mit MeOH nachgewaschen und i.vac. getrocknet.

| | |
|---|---|
| Ausbeute: | 1.90 g (58% d. Th.) |
| ESI-MS: | m/z = 350 (M+H)⁺ |
| Rₜ(HPLC): | 3.89 min (Methode C) |

### Stufe 3: 6-(3-Brom-4-fluor-benzoyl)-3,4-dimethyl-3H-benzoxazol-2-on

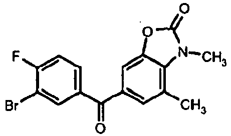

Zu 1.33 g (3.80 mmol) 6-(3-Brom-4-fluor-benzoyl)-4-methyl-3H-benzoxazol-2-on in 5 mL DMF wurden bei RT 0.175 g (4.00 mmol) Natriumhydrid (55%, Suspension in Mineralöl) gegeben. Der Reaktionsansatz wurde 30 min bei RT gerührt. Anschließend wurden 0.317 mL (5.00 mmol) lodmethan zugegeben und 1 h bei RT gerührt. Dann wurden 0.100 mL (15.8 mmol) lodmethan zugegeben und über Nacht bei RT gerührt. Nach Zugabe von Eiswasser wurde der Reaktionsansatz mit EtOAc extrahiert. Die organische Phase wurde mit Wasser gewaschen, über Natriumsulfat getrocknet und i.vac. eingeengt. Der Rückstand wurde mittels Flash-Chromatographie gereinigt. Die Produkt enthaltenden Fraktionen wurden vereinigt, eingeengt und i.vac. getrocknet.

| | |
|---|---|
| Ausbeute: | 1.38 g (quantitativ) |
| ESI-MS: | m/z = 364 (M+H)⁺ |
| Rₜ(HPLC): | 1.32 min (Methode E) |

### Intermediat 38

### 3-{1-[6-Chlor-5-(3,4-dimethyl-2-oxo-2,3-dihydro-benzoxazol-6-carbonyl)-pyridazin-3-yl]-piperidin-4-yl}-7-methoxy-1,3,4,5-tetrahydro-benzo[d][1,3]diazepin-2-on

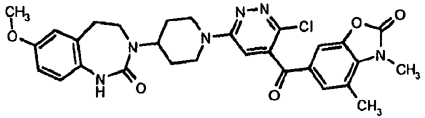

### Stufe 1: 6-(3,6-Dichlor-pyridazin-4-carbonyl)-3,4-dimethyl-3H-benzoxazol-2-on

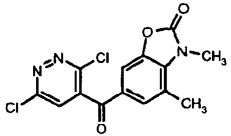

Zu 0.65 g (2.0 mmol) 6-(3,6-Dichlor-pyridazin-4-carbonyl)-4-methyl-3H-benzoxazol-2-on in 10 mL DMF wurden unter Eiskühlung 85 mg (2.1 mmol) Natriumhydrid (55%, Suspension in Mineralöl) gegeben. Der Reaktionsansatz wurde 20 min gerührt. Dann wurden 0.19 mL (3.1 mmol) lodmethan unter Eiskühlung zugegeben und der Ansatz 2 h bei RT gerührt. Der Reaktionsansatz wurde mit Eiswasser versetzt, der ausgefallene Niederschlag wurde abgesaugt und getrocknet.

| | |
|---|---|
| Ausbeute: | 0.62 g (82% d. Th.) |

### Stufe 2: 3-{1-[6-Chlor-5-(3,4-dimethyl-2-oxo-2,3-dihydro-benzoxazol-6-carbonyl)-pyridazin-3-yl]-piperidin-4-yl}-7-methoxy-1,3,4,5-tetrahydro-benzo[d][1,3]diazepin-2-on

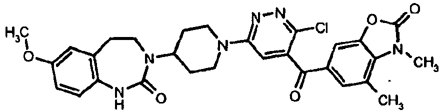

550 mg (2.00 mmol) 7-Methoxy-3-piperidin-4-yl-1,3,4,5-tetrahydro-benzo[d][1,3]diazepin-2-on, 610 mg (1.80 mmol) 6-(3,6-Dichlor-pyridazin-4-carbonyl)-3,4-dimethyl-3H-benz-oxazol-2-on, 630 mg (4.39 mmol) Kupfer(I)bromid und 430 mL (2.50 mmol) DIPEA in 5 mL DMF wurden 2 h bei 110°C gerührt, anschließend abgekühlt und filtriert. Das Filtrat wurde mit 1 mL einer 1N wässrigen Salzsäure-Lösung versetzt und mit 40 mL Eiswasser gefällt. Der Niederschlag wurde abgesaugt, mit Wasser nachgewaschen und getrocknet. Der Feststoff wurde mit 120 mL DCM/MeOH (11:1) verrührt, über Kieselgel filtriert und mit DCM/MeOH nachgewaschen. Das Filtrat wurde eingeengt. Der Rückstand wurde mit Diethylether verrieben, abgesaugt und getrocknet. Es wurde ein Isomerengemisch erhalten, das weiter umgesetzt wurde.

| | |
|---|---|
| Ausbeute: | 870 mg (50% d. Th.) |
| Reinheit: | 60 % |
| ESI-MS: | m/z = 575(M-H)⁻ |
| Rₜ(HPLC): | 3.70 min (Methode C) |

### Intermediat 39

### 6-(3,5-Difluor-benzoyl)-3,4-dimethyl-3H-benzoxazol-2-on

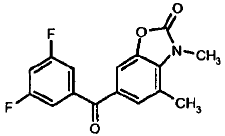

### Stufe 1: 3,5-Difluor-benzoesäurechlorid

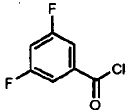

1.63 g (10.00 mmol) 3,5-Difluor-benzoesäure wurden mit 20 mL Thionylchlorid vermischt und 2 h gekocht. Der Reaktionsansatz wurde bis zur Trockene eingeengt und zweimal mit Toluol coevaporiert. Der Rückstand wurde als Rohprodukt weiter umgesetzt.

| | |
|---|---|
| Ausbeute: | 1.45 g (82% d. Th.) |

### Stufe 2: 6-(3,5-Difluor-benzoyl)-4-methyl-3H-benzoxazol-2-on

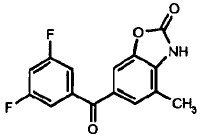

1.45 g (8.21 mmol) 3,5-Difluorbenzoesäurechlorid, 1.22 g (8.21 mmol) 4-Methyl-3H-benzoxazol-2-on und 4.40 g (33.0 mmol) Aluminiumtrichlorid wurden unter Rühren 1.5 h auf 125°C erhitzt. Der Ansatz wurde mit Eiswasser versetzt und das als Feststoff ausgefallene Produkt abgesaugt und mit Wasser gewaschen. Nach dem Verreiben des Niederschlages mit MeOH wurde wurde dieser abgesaugt, mit MeOH nachgewaschen und i.vac. getrocknet.

| | |
|---|---|
| Ausbeute: | 2.20 g (93% d. Th.) |
| ESI-MS: | m/z = 290 (M+H)⁺ |
| Rₜ(HPLC): | 1.61 min (Methode B) |

### Stufe 3: 6-(3,5-Difluor-benzoyl)-3,4-dimethyl-3H-benzoxazol-2-on

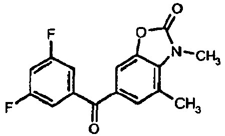

Zu 2.20 g (7.61 mmol) 6-(3,5-Difluor-benzoyl)-4-methyl-3H-benzoxazol-2-on in 10 mL DMF wurden bei RT 0.349 g (8.00 mmol) Natriumhydrid (55%, Suspension in Mineralöl) gegeben. Der Reaktionsansatz wurde 30 min bei RT gerührt. Anschließend wurden 0.634 mL (10.0 mmol) lodmethan zugegeben und 1 h bei RT gerührt. Dann wurden nochmals 0.1 mL lodmethan zugegeben und weiter bei RT gerührt. Nach Zugabe von Eiswasser wurde der Ansatz mit EtOAc extrahiert. Die organische Phase wurde mit Wasser gewaschen, über Natriumsulfat getrocknet und i.vac. eingeengt. Der Rückstand wurde mittels Flash-Chromatographie gereinigt. Die Produkt enthaltenden Fraktionen wurden vereinigt, eingeengt und i.vac. getrocknet.

| | |
|---|---|
| Ausbeute: | 1.00 g (43% d. Th.) |
| ESI-MS: | m/z = 304 (M+H)⁺ |
| Rₜ(HPLC): | 1.68 min (Methode B) |

### Intermediat 40

### 6-(6-Chlor-pyrimidin-4-carbonyl)-3-methyl-4-pyrazol-1-ylmethyl-3H-benzoxazol-2-on

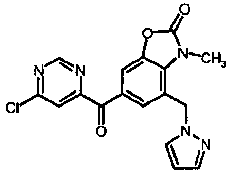

Unter Stickstoffatmosphäre wurden 0.15 g (0.49 mmol) 6-(6-Chlorpyrimidin-4-carbonyl)-3,4-dimethyl-3*H*-benzoxazol-2-on, 95 mg (0.53 mmol) N-Bromsuccinimid und 5.0 mg (30 mmol) 2,2'-Azobis-isobutyronitril (AIBN) in 15 mL Tetrachlorkohlenstoff zusammen gegeben und 4 h unter Rückfluss gekocht. Der ausgefallene Niederschlag wurde abfiltriert und das Filtrat mit 20 µL Isopropanol versetzt. Nach kurzem Rühren wurden 36 mg (0.53 mmol) Pyrazol und 0.17 mL (0.99 mmol) DIPEA zugegeben und das Gemisch 6 h unter Rückfluss erhitzt. Anschließend wurde der Reaktionsansatz über Kieselgel filtriert und mit DCM EtOAc (1/1) nachgewaschen. Das Filtrat wurde i.vac. eingeengt und der Rückstand als Rohprodukt weiter umgesetzt.

| | |
|---|---|
| Ausbeute: | 210 mg (58% d. Th.) |
| Reinheit: | 50% |
| Rₜ(HPLC): | 3.35 min (Methode C) |

### Intermediat 41

### 4-(3,4-Dimethyl-2-oxo-2,3-dihydro-benzoxazol-6-carbonyl)-2-fluor-benzonitril

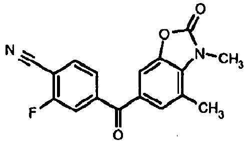

### Stufe 1: 4-Cyan-3-fluor-benzoesäurechlorid

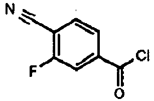

0.66 g (4.0 mmol) 4-Cyan-3-fluor-benzoesäure wurden mit 10 mL Thionylchlorid vermischt und 2 h gekocht. Der Ansatz wurde bis zur Trockene eingeengt und zweimal mit Toluol coevaporiert. Der Rückstand wurde als Rohprodukt weiter umgesetzt.

| | |
|---|---|
| Ausbeute: | 0.73 g (quantitativ) |

### Stufe 2: 2-Fluor-4-(4-methyl-2-oxo-2,3-dihydro-benzoxazol-6-carbonyl)-benzonitril

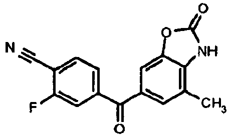

0.73 g (4.0 mmol) 4-Cyan-3-fluor-benzoesäurechlorid, 0.60 g (4.0 mmol) 4-Methyl-3H-benzoxazol-2-on und 2.1 g (16 mmol) Aluminiumtrichlorid wurden unter Rühren 1.5 h auf 125°C erhitzt. Nach dem Abkühlen auf RT wurde der Ansatz mit Eiswasser versetzt. Der ausgefallene Niederschlag wurde abgesaugt, mit Wasser gewaschen und i.vac. getrocknet.

| | |
|---|---|
| Ausbeute: | 1.2 g (quantitativ) |
| ESI-MS: | m/z = 295 (M-H)⁻ |
| Rₜ(HPLC): | 1.45 min (Methode B) |

### Stufe 3: 4-(3,4-Dimethyl-2-oxo-2,3-dihydro-benzoxazol-6-carbonyl)-2-fluor-benzonitril

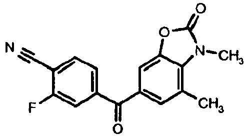

Zu 1.15 g (3.88 mmol) 2-Fluor-4-(4-methyl-2-oxo-2,3-dihydro-benzoxazol-6-carbonyl)-benzonitril in 5.0 mL DMF wurden bei RT 0.190 g (4.30 mmol) Natriumhydrid (55%, Suspension in Mineralöl) gegeben. Der Reaktionsansatz wurde 30 min bei RT gerührt. Anschließend wurden 0.320 mL (5.00 mmol) Iodmethan zugegeben und 1 h bei RT gerührt. Dann wurden nochmals 0.1 mL lodmethan zugegeben und weiter bei RT gerührt. Zum Reaktionsansatz wurde Eiswasser zugefügt und der ausgefallene Niederschlag abgesaugt. Der Rückstand wurde mittels Flash-Chromatographie gereinigt. Die Produkt enthaltenden Fraktionen wurden vereinigt, eingeengt, mit Diethylether verrieben, abgesaugt und nochmals mit Diethylether nachgewaschen. Der Rückstand wurde i.vac. getrocknet.

| | |
|---|---|
| Ausbeute: | 0.60 g (50% d. Th.) |
| ESI-MS: | m/z = 311 (M+H)⁺ |
| Rₜ(HPLC): | 1.54 min (Methode B) |

### Intermediat 42

### 6-(6-Chlor-2-methyl-pyrimidin-4-carbonyl)-4-methyl-3H-benzoxazol-2-on

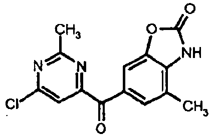

### Stufe 1: 6-Chlor-2-methyl-pyrimidin-4-carbonsäurechlorid

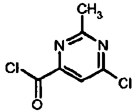

2.00 g (13.0 mmol) 6-Hydroxy-2-methylpyrimidin-4-carbonsäure wurden mit 11.9 mL (130 mmol) Phosphoroxychlorid 2 h unter Rückfluss gekocht. Nach dem Abkühlen auf RT wurden 2.70 g (13.0 mmol) Phosphor-(V)-chlorid zugefügt und 2 h gekocht. Der Reaktionsansatz wurde auf RT abgekühlt, i.vac. bis zur Trockene eingeengt und zweimal mit Toluol coevaporiert. Der Rückstand wurde mehrmals mit DCM verrieben und das überschüssige DCM abdekantiert. Die vereinigten DCM-Phasen wurden eingeengt und der Rückstand als Rohprodukt weiter umgesetzt.

| | |
|---|---|
| Ausbeute: | 2.48 g (quantitativ) |

### Stufe 2: 6-(6-Chlor-2-methyl-pyrimidin-4-carbonyl)-4-methyl-3H-benzoxazol-2-on

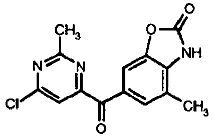

2.48 g (13.0 mmol) 6-Chlor-2-methyl-pyrimidin-4-carbonsäurechlorid, 1.94 g (13.0 mmol) 4-Methyl-3H-benzoxazol-2-on und 6.93 g (52.0 mmol) Aluminiumtrichlorid wurden unter Rühren 1.5 h auf 125°C erhitzt. Der Ansatz wurde mit Eiswasser versetzt und der ausgefallene Niederschlag abgesaugt und mit Wasser gewaschen. Dann wurde der Niederschlag in MeOH/DCM gelöst und über Kieselgel abgesaugt. Das Filtrat wurde eingeengt und der Rückstand mittels Flash-Chromatographie gereinigt. Die Produkt enthaltenden Fraktionen wurden vereinigt, eingeengt und mit Diethylether verrieben. Der Niederschlag wurde abgesaugt, mit Diethylether gewaschen und i.vac. getrocknet.

| | |
|---|---|
| Ausbeute: | 0.600 g (15% d. Th.) |
| ESI-MS: | m/z = 304 (M+H)⁺ |
| Rₜ(HPLC): | 1.42 min (Methode B) |

### Intermediat 43

### 6-(6-Chlor-2-methyl-pyrimidin-4-carbonyl)-3,4-dimethyl-3H-benzoxazol-2-on

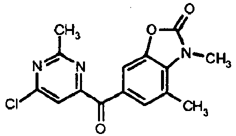

Zu 0.37 g (1.2 mmol) 6-(6-Chlor-2-methyl-pyrimidin-4-carbonyl)-4-methyl-3H-benzoxazol-2-on in 5.0 mL DMF wurden bei RT 59 mg (1.4 mmol) Natriumhydrid (55%, Suspension in Mineralöl) gegeben. Der Reaktionsansatz wurde 30 min bei RT gerührt. Anschließend wurden 0.10 mL (1.60 mmol) lodmethan zugegeben und 1 h bei RT gerührt. Dann wurden nochmals 0.10 mL (1.60 mmol) lodmethan zugegeben und über Nacht bei RT gerührt. Der Reaktionsansatz wurde mit Eiswasser verdünnt und der ausgefallene Niederschlag abgesaugt. Der Rückstand wurde mit Wasser nachgewaschen und i.vac. getrocknet.

| | |
|---|---|
| Ausbeute: | 0.37 g (96% d. Th.) |
| ESI-MS: | m/z = 318 (M+H)⁺ |
| Rₜ(HPLC): | 1.53 min (Methode B) |

### Intermediat 44

### 6-(2-Chlor-5-methyl-pyridin-4-carbonyl)-3,4-dimethyl-3H-benzoxazol-2-on

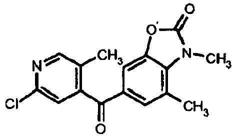

### Stufe 1: 6-(2-Chlor-5-methyl-pyridin-4-carbonyl)-4-methyl-3H-benzoxazol-2-on

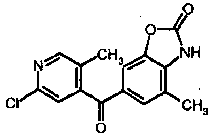

1.53 g (10.0 mmol) 3-Methyl-1-oxy-isonicotinsäure wurden mit 9.32 mL (100 mmol) Phosphoroxychlorid verrührt und 4 h gekocht. Der Reaktionsansatz wurde i.vac. bis zur Trockene eingeengt und zweimal mit Toluol coevaporiert. Das so erhaltene Rohprodukt (welches im Gemisch mit 2-Chlor-3-methyl-isonicotinsäurechlorid vorlag) wurde mit 1.49 g (10.0 mmol) 4-Methyl-3H-benzoxazol-2-on und 5.33 g (40.0 mmol) Aluminiumtrichlorid wurden zusammen gegeben und unter Rühren 1.5 h auf 125°C erhitzt. Anschließend wurde der Ansatz mit Eiswasser versetzt und die ausgefallene Schmiere vom Lösungsmittel durch Abdekantieren getrennt. Der Rückstand wurde mit MeOH verrieben. Der ausgefallene Niederschlag wurde abgesaugt, mit MeOH und Diethylether gewaschen i.vac. getrocknet. Das so erhaltene Produkt, welches im Gemisch mit 6-(2-Chlor-3-methyl-pyridin-4-carbonyl)-4-methyl-3H-benzoxazol-2-on vorlag, wurde ohne weitere Reinigung weiter umgesetzt.

| | |
|---|---|
| Ausbeute: | 400 mg (~13%) |
| ESI-MS: | m/z = 303 (M+H)⁺ |
| Rₜ(HPLC): | 1.40 min (Methode B) |

### Stufe 2: 6-(2-Chlor-5-methyl-pyridin-4-carbonyl)-3,4-dimethyl-3H-benzoxazol-2-on

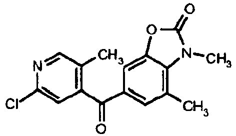

Zu 0.61 g (2.0 mmol) 6-(2-Chlor-5-methyl-pyridin-4-carbonyl)-4-methyl-3H-benzoxazol-2-on im Gemisch mit 6-(2-Chlor-3-methyl-pyridin-4-carbonyl)-4-methyl-3H-benzoxazol-2-on in 5 mL DMF wurden bei RT 0.10 g (2.2 mmol) Natriumhydrid (55%, Suspension in Mineralöl) gegeben. Der Reaktionsansatz wurde 30 min bei RT gerührt. Anschließend wurden 0.17 mL (2.6 mmol) lodmethan zugegeben und 1 h bei RT gerührt. Dann wurden nochmals 0.1 mL lodmethan zugegeben und weiter bei RT gerührt. Zum Reaktionsansatz wurde Eiswasser gegeben und der ausgefallene Niederschlag wurde abgesaugt. Der Rückstand wurde mittels Flash-Chromatographie gereinigt. Die Produkt enthaltenden Fraktionen wurden vereinigt, eingeengt, mit Diethylether verrieben, abgesaugt und getrocknet. Das Produkt wurde im Gemisch mit 6-(2-Chlor-3-methyl-pyridin-4-carbonyl)-3,4-dimethyl-3H-benzoxazol-2-on erhalten und als solches weiter umgesetzt.

| | |
|---|---|
| Ausbeute: | 0.60 g (94% d. Th.) als Gemisch |
| ESI-MS: | m/z = 311 (M+H)⁺ |
| Rₜ(HPLC): | 1.46 min (Methode B) |

### Intermediat 45

### 3-Fluor-5-(4-methyl-2-oxo-2,3-dihydro-benzoxazol-6-carbonyl)-benzonitril

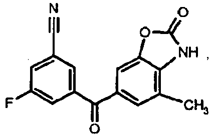

### Stufe 1: 3-Cyan-5-fluor-benzoesäurechlorid

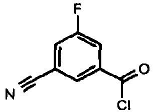

1.65 g (10.0 mmol) 3-Cyan-5-fluorbenzoesäure wurden mit 7.27 mL (100 mmol) Thionylchlorid unter Rühren 2 h gekocht. Der Reaktionsansatz wurde i.vac. bis zur Trockene eingeengt und zweimal mit Toluol coevaporiert. Der Rückstand wurde als Rohprodukt weiter umgesetzt.

| | |
|---|---|
| Ausbeute: | 1.84 g (quantitativ) |

### Stufe 2: 3-Fluor-5-(4-methyl-2-oxo-2,3-dihydro-benzoxazol-6-carbonyl)-benzonitril

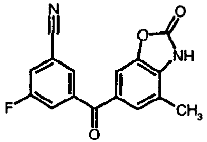

1.84 g (10.0 mmol) 3-Cyan-5-fluor-benzoesäurechlorid, 1.49 g (10.0 mmol) 4-Methyl-3H-benzoxazol-2-on und 5.33 g (40.0 mmol) Aluminiumtrichlorid wurden unter Rühren 1.5 h auf 125°C erhitzt. Anschließend wurde der Ansatz mit Eiswasser versetzt. Der ausgefallene Niederschlag wurde abgesaugt, mit Wasser gewaschen und i.vac. getrocknet.

| | |
|---|---|
| Ausbeute: | 1.75 g (59% d. Th.) |
| ESI-MS: | m/z = 295 (M-H)⁻ |
| Rₜ(HPLC): | 1.33 min (Methode B) |

### Intermediat 46

### 9-Fluor-3-piperidin-4-yl-1,3,4,5-tetrahydro-benzo[d][1,3]diazepin-2-on

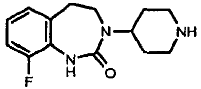

### Stufe 1: 2-(3-Fluor-2-nitro-phenyl)-malonsäuredimethylester

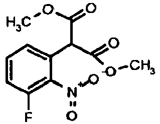

Zu 6.00 g (37.7 mmol) 2,6-Difluornitrobenzol in 60 mL DMF wurden nacheinander 5.40 g (38.3 mmol) Kaliumcarbonat und 4.50 mL (38.0 mmol) Dimethylmalonat gegeben und das Gemisch über Nacht bei 65°C gerührt. Dann wurde der Reaktionsansatz auf RT abgekühlt und langsam auf 75 mL 1 N wässrige Salzsäure-Lösung gegossen. Die wässrige Phase wurde mehrmals mit EtOAc extrahiert. Die vereinigten organischen Phasen wurden mit Wasser und gesättigter Natriumchloridlösung gewaschen, über Magnesiumsulfat getrocknet, filtriert und i.vac. eingeengt. Der Rückstand wurde mit n-Hexan verrührt, abgesaugt und getrocknet.

| | |
|---|---|
| Ausbeute: | 2.18 g (21% d. Th.) |
| ESI-MS: | m/z = 272 (M+H)⁺ |
| R_{f}: | 0.32 (Kieselgel; PE / EtOAc = 2 / 1) |

### Stufe 2: (3-Fluor-2-nitro-phenyl)-essigsäure

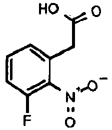

28.0 g (103 mmol) 2-(3-Fluor-2-nitro-phenyl)-malonsäuredimethylester wurden in 120 mL Wasser und 120 mL konz. Salzsäure zusammen gegeben und 5 h unter Rückfluss gekocht. Der Ansatz wurde auf RT abgekühlt und mehrmals mit EtOAc extrahiert. Die vereinigten organischen Phasen wurden über Magnesiumsulfat getrocknet, filtriert und i.vac. eingeengt. Der Rückstand wurde mit PE / EtOAc = 3/1 verrieben und abgesaugt.

| | |
|---|---|
| Ausbeute: | 17.3 g (84% d. Th.) |
| ESI-MS: | m/z = 217 (M+NH₄)⁺ |
| Rₜ(HPLC): | 1.13 min (Methode B) |

### Stufe 3: N-(1-Benzyl-piperidin-4-yl)-2-(3-fluor-2-nitro-phenyl)-acetamid

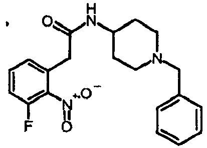

Zu 18.3 g (91.9 mmol) (3-Fluor-2-nitro-phenyl)-essigsäure in 500 mL THF wurden bei RT 17.0 g (102 mmol) CDI gegeben und 30 min nachgerührt. Dann wurden 20.0 mL (95.8 mmol) 4-Amino-1-benzylpiperidin zugegeben und weitere 2 h gerührt. Der Reaktionsansatz wurde mit EtOAc verdünnt und die organische Phase mit Wasser und mit gesättigter Natriumchloridlösung gewaschen. Die organische Phase wurde über Magnesiumsulfat getrocknet, filtriert und i.vac. eingeengt. Der Rückstand wurde mit DIPE verrieben und abgesaugt.

| | |
|---|---|
| Ausbeute: | 33.0 g (97% d. Th.) |
| ESI-MS: | m/z = 372 (M+H)⁺ |
| Rₜ(HPLC): | 1.29 min (Methode B) |

### Stufe 4: (1-Benzyl-piperidin-4-yl)-[2-(3-fluor-2-nitro-phenyl)-ethyl]-amin

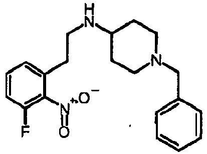

Zu 18.9 g (50.9 mmol) N-(1-Benzyl-piperidin-4-yl)-2-(3-fluor-2-nitro-phenyl)-acetamid in 500 mL THF wurden unter Rühren langsam 23.4 mL (183 mmol) Chlortrimethylsilan getropft. Es wurde 1 h bei RT nachgerührt. Dann wurden 2.70 g (124 mmol) Lithiumborhydrid portionsweise hinzugefügt und 1 h bei RT nachgerührt. Anschließend wurde der Ansatz 5 h unter Rückfluss gekocht und über Nacht auf RT abgekühlt. Unter Rühren wurden 16 mL MeOH, 40 mL Wasser und 35 mL konz. Salzsäure zugetropft und nach beendeter Zugabe wurde der Reaktionsansatz 3 h unter Rückfluss gekocht. Nach dem Abkühlen auf RT wurde die organische Phase abgetrennt und die wässrige Phase mehrmals mit EtOAc extrahiert. Die vereinigten organischen Phasen wurden mit gesättigter Natriumchloridlösung gewaschen, über Magnesiumsulfat getrocknet, filtriert und i.vac. eingeengt. Der Rückstand wurde mittels Flash-Chromatographie gereinigt. Die Produkt enthaltenden Fraktionen wurden vereinigt und eingeengt. (Produkt als Gemisch!)

| | |
|---|---|
| Ausbeute: | 14.4 g (79% d. Th.) |
| ESI-MS: | m/z = 358 (M+H)⁺ |
| R_{f}: | 0.39 (Kieselgel; DCM/Cyc/MeOH/ NH₄OH = 70/15/15/2) |

### Stufe 5: [2-(2-Amino-3-fluor-phenyl)-ethyl]-(1-benzyl-piperidin-4-yl)-amin

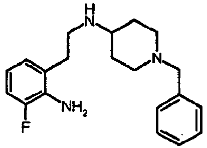

14.4 g (40.3 mmol) (1-Benzyl-piperidin-4-yl)-[2-(3-fluor-2-nitro-phenyl)-ethyl]-amin in 100 mL MeOH wurden mit 2.00 g Rhodiumkohle (10%) verrührt und unter Wasserstoffatmosphäre (3 bar) bei RT geschüttelt. Der Katalysator wurde abfiltriert und das Lösungsmittel i.vac. entfernt. Der Rückstand wurde als Rohprodukt sofort weiter umgesetzt.

| | |
|---|---|
| Ausbeute: | 13.1 g (99% d. Th.) |
| Rₜ(HPLC): | 0.64 min (Methode B) |

### Stufe 6: 3-(1-Benzyl-piperidin-4-yl)-9-fluor-1,3,4,5-tetrahydro-benzo[d][1,3]diazepin-2-on

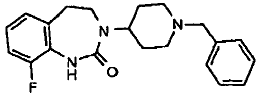

Zu 13.1 g (40.0 mmol) [2-(2-Amino-3-fluor-phenyl)-ethyl]-(1-benzyl-piperidin-4-yl)-amin in 120 mL DMF wurden 11.0 g (65.8 mmol) CDI gegeben. Die Mischung wurde auf 100°C erhitzt und 1 h gerührt. Nach Abkühlen des Reaktionsansatzes auf RT wurde dieser auf 300 mL Eiswasser gegossen. Die wässrige Phase wurde mehrmals mit DCM extrahiert, die organischen Phasen wurden vereinigt, über Magnesiumsulfat getrocknet, filtriert und i.vac. eingeengt. Der Rückstand wurde mittels Flash-Chromatographie gereinigt. Die Produkt enthaltenden Fraktionen wurden vereinigt und eingeengt.

| | |
|---|---|
| Ausbeute: | 13.7 g (97% d. Th.) |
| ESI-MS: | m/z = 354 (M+H)⁺ |
| Rₜ(HPLC): | 1.00 min (Methode B) |

### Stufe 7: 9-Fluor-3-piperidin-4-yl-1,3,4,5-tetrahydro-benzo[d][1,3]diazepin-2-on

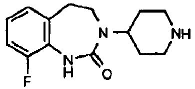

13.7 g (38.8 mmol) 3-(1-Benzyl-piperidin-4-yl)-9-fluor-1,3,4,5-tetrahydro-benzo[d][1,3]diazepin-2-on in 100 mL MeOH wurden mit 2.00 g Palladium auf Kohle (Pd/C 10%) unter Wasserstoffatmosphäre (3 bar) bei RT geschüttelt. Der Katalysator wurde abfiltriert und das Lösungsmittel eingeengt. Der Rückstand wurde mittels Flash-Chromatographie gereinigt. Die Produkt enthaltenden Fraktionen wurden vereinigt und eingeengt. Der Rückstand wurde mit DIPE / EtOAc verührt, abgesaugt und getrocknet.

| | |
|---|---|
| Ausbeute: | 3.10 g (30% d. Th.) |
| ESI-MS: | m/z = 264 (M+H)⁺ |
| Rₜ(HPLC): | 2.43 min (Methode B) |

### Intermediat 47

### 6-(2,6-Dichlor-pyrimidin-4-carbonyl)-4-methyl-3H-benzoxazol-2-on

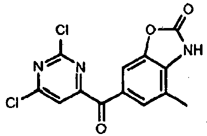

### Stufe 1: 2,6-Dihydroxy-pyrimidin-4-carbonsäure

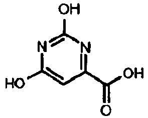

23.00 g (127.74 mmol) Orotsäure Lithiumsalz Monohydrat in 400 mL DMF wurden auf 70°C erhitzt und bei dieser Temperatur portionsweise innerhalb von 2 h mit Salzsäure versetzt. Der Reaktionsansatz wurde eine weitere Stunde bei 70°C gerührt und anschließend im Vakuum eingeengt. Der Rückstand wurde mit Wasser verrührt, abgsaugt und im ULTS bei 50°C getrocknet.

| | |
|---|---|
| Ausbeute: | 21.40 g (quantitativ) |
| ESI-MS: | m/z = 155 (M-H)⁻ |

### Stufe 2: 2,6-Dichlor-pyrimidin-4-carbonsäurechlorid

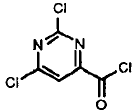

20 g (0.13 mol) 2,6-Dihydroxy-pyrimidin-4-carbonsäure wurden zusammen mit 40 mL Phosphoroxychlorid unter Rückfluss gekocht. Nach dem Abkühlen auf RT wurden 60 g (0.29 mol) Phosphor-(V)-chlorid zugefügt und weitere 3 h gekocht. Durch fraktionierte Destillation wurde das Produkt erhalten.

| | |
|---|---|
| Ausbeute: | 6.00 g (22% d. Th.) |

### Stufe 3: 6-(2,6-Dichlor-pyrimidin-4-carbonyl)-4-methyl-3H-benzoxazol-2-on

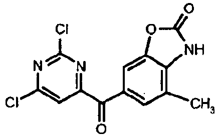

2.75 g (13.0 mmol) 2,6-Dichlor-pyrimidin-4-carbonsäurechlorid, 1.94 g (13.0 mmol) 4-Methyl-3H-benzoxazol-2-on und 6.93 g (52.0 mmol) Aluminiumtrichlorid wurden unter Rühren 1.5 h auf 125°C erhitzt. Der Ansatz mit Eiswasser versetzt und der das als Feststoff ausgefallene Produkt wurde abgesaugt, mit Wasser gewaschen und i.vac. getrocknet.

| | |
|---|---|
| Ausbeute: | 3.70 g (88% d. Th.) |
| ESI-MS: | m/z = 322 (M-H)⁻ |
| Rₜ(HPLC): | 1.41 min (Methode B) |

### Intermediat 48

### 3-(3,4-Dimethyl-2-oxo-2,3-dihydro-benzoxazol-6-carbonyl)-5-fluor-benzonitril

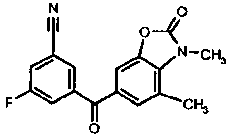

Zu 1.00 g (3.38 mmol) 3-Fluor-5-(4-methyl-2-oxo-2,3-dihydro-benzoxazol-6-carbonyl)-benzonitril in 5 mL DMF wurden bei RT 0.161 g (3.70 mmol) Natriumhydrid (55%, Suspension in Mineralöl) gegeben und 30 min bei RT gerührt. Anschließend wurden 0.317 mL (5.00 mmol) lodmethan zugegeben und 1 h bei RT gerührt. Dann wurden nochmals 0.1 mL lodmethan gegeben und weiter bei RT gerührt. Nach dem Verdünnen mit Eiswasser wurde die wässrige Phase mit EtOAc extrahiert. Die organische Phase wurde mit Wasser gewaschen, über Natriumsulfat getrocknet, filtriert und i.vac. eingeengt. Der Rückstand wurde mittels Flash-Chromatographie gereinigt. Die Produkt enthaltenden Fraktionen wurden vereinigt, eingeengt und i.vac. getrocknet.

| | |
|---|---|
| Ausbeute: | 0.960 g (92% d. Th.) |
| ESI-MS: | m/z = 311 (M+H)⁺ |
| Rₜ(HPLC): | 1.47 min (Methode B) |

### Intermediat 49

### 5-(6-Chlor-pyrimidin-4-carbonyl)-7-methyl-1,3-dihydro-indol-2-on

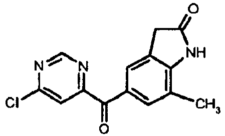

### Stufe 1: 7-Methyl-1,3-dihydro-indol-2-on

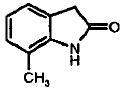

5.00 g (31.0 mmol) 7-Methyl-1H-indol-2,3-dion in 18.1 mL (372 mmol) Hydrazinhydrat wurden 3 h bei 110°C erhitzt. Dann wurde der Reaktionsansatz abgekühlt, der ausgefallene Niederschlag abgesaugt und mit etwas Wasser nachgewaschen. Der Niederschlag wurde in etwas Wasser suspendiert, mit konz. Salzsäure sauer gestellt und 10 min nachgerührt. Dann wurde der Reaktionsansatz im Eisbad noch 30 min gerührt, der Niederschlag abgesaugt, mit etwas Wasser nachgewaschen und getrocknet.

| | |
|---|---|
| Ausbeute: | 1.60 g (35% d. Th.) |
| ESI-MS: | m/z = 148 (M+H)⁺ |
| Rₜ(HPLC): | 2.72 min (Methode C) |

### Stufe 2: 5-(6-Chlor-pyrimidin-4-carbonyl)-7-methyl-1,3-dihydro-indol-2-on

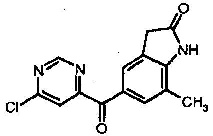

1.92 g (10.9 mmol) 6-Chlorpyrimidin-4-carbonsäurechlorid, 1.60 g (10.9 mmol) 7-Methyl-1,3-dihydro-indol-2-on und 7.33 g (55.0 mmol) Aluminiumtrichlorid wurden unter Rühren 3 h auf 130°C erhitzt. Der Ansatz wurde zuerst mit Eiswasser und dann mit EtOAc versetzt. Der als schwarze Flocken ausgefallene Niederschlag wurde abgesaugt und die Phasen getrennt. Die wässrige Phase wurde mehrmals mit EtOAc extrahiert. Die vereinigten organischen Phasen wurden über Natriumsulfat getrocknet, filtriert und i.vac. eingeengt.

| | |
|---|---|
| Ausbeute: | 250 mg (8% d. Th.) |
| ESI-MS: | m/z = 288/90 (Cl) (M+H)⁺ |
| Rₜ(HPLC): | 1.25 min (Methode B) |

### Intermediat 50

### 3-[6'-Benzyloxy-4'-(3,4-dimethyl-2-oxo-2,3-dihydro-benzoxazol-6-carbonyl)-3,4,5,6-tetrahydro-2H-[1,2']bipyridinyl-4-yl]-7-methoxy-1,3,4,5-tetrahydro-benzo[d][1,3]diazepin-2-on

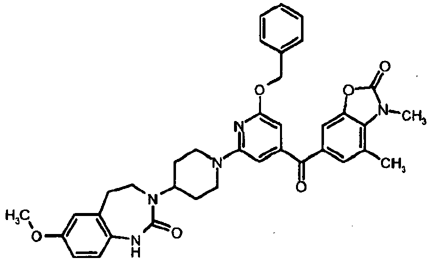

991 mg (3.60 mmol) 7-Methoxy-3-piperidin-4-yl-1,3,4,5-tetrahydro-1,3-benzodiazepin-2-on und 500 mg (1.22 mmol) 6-(2-Benzyloxy-6-chlor-pyridin-4-carbonyl)-3,4-dimethyl-3H-benzoxazol-2-on wurden in 10 mL NMP zusammen gegeben und über Nacht bei 120°C gerührt. Der Reaktionsansatz wurde mittels präparativer HPLC-MS gereinigt. Die Produkt enthaltenden Fraktionen wurden vereinigt und eingeengt.

| | |
|---|---|
| Ausbeute: | 130 mg (6% d. Th.) |
| ESI-MS: | m/z = 648 (M+H)⁺ |
| Rₜ(HPLC): | 1.80 min (Methode B) |

### Intermediat 51

### (6-Chlor-pyrimidin-4-yl)-(1,7-dimethyl-1H-indazol-5-yl)-methanon

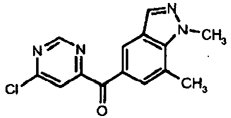

### Stufe 1: 5-Brom-1,7-dimethyl-1H-indazol

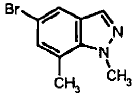

2.11 g (10.0 mmol) 5-Brom-7-methyl-1H-indazol und 1.20 g (10.7 mmol) Kalium-*tert-*butylat in 50 mL THF wurden mit 0.700 mL (11.2 mmol) lodmethan über Nacht bei RT gerührt. Anschließend wurde der Niederschlag abfiltriert und das Filtrat i.vac. eingeengt. Der Rückstand wurde mittels Flash-Chromatographie gereinigt.

| | |
|---|---|
| Ausbeute: | 1.92 g (43% d. Th.) |
| ESI-MS: | m/z = 225/27 (M+H)⁺ |
| Rₜ(HPLC): | 1.13 min (Methode E) |

### Stufe 2: (6-Chlor-pyrimidin-4-yl)-(1,7-dimethyl-1H-indazol-5-yl)methanon

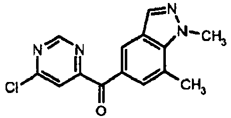

Unter einer Argonatmosphäre wurden 0.450 g (2.00 mmol) 5-Brom-1,7-dimethyl-1H-indazol in 25 mL THF auf -75°C gekühlt, mit 1.40 mL (2.24 mmol) einer 1.6 molaren n-Butyllithium-Lösung versetzt und 1 h bei -75°C gerührt. Anschließend wurden 0.480 g (2.14 mmol) 6-Chlor-pyrimidin-4-carbonsäuremethoxy-methyl-amid zugetropft. Der Ansatz wurde auf 0°C gebracht und eine weitere Stunde gerührt. Dann wurde gesättigte Natriumhydrogencarbonat-Lösung eingerührt, mit EtOAc extrahiert, die organische Phase getrocknet und i.vac. eingeengt. Der Rückstand wurde mit DIPE verrieben, abgesaugt und getrocknet. Der Niederschlag wurde mittels Flash-Chromatogrphie (Alox) aufgereinigt.

| | |
|---|---|
| Ausbeute: | 100 mg (14% d. Th.) |
| Rₜ (HPLC): | 1.46 min (Methode B) |

### Intermediat 52

### (6-Iod-pyrimidin-4-yl)-(8-methyl-2,3-dihydro-benzo[1,4]dioxin-6-yl)-methanon

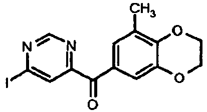

Zu 0.677 g (2.00 mmol) 4,6-Diiodpyrimidin, 0.535 g (3.00 mmol) 8-Methyl-2,3-dihydrobenzo[1,4]dioxin-6-carbaldehyd (US2005/256099) und 0.133 g (1.00 mmol) 1,3-Dimethylimidazoliumchlorid in 10 mL THF wurden 0.131 g (3.00 mmol) Natriumhydrid (55%, Suspension in Mineralöl) gegeben und 4 h unter Rückfluss gekocht. Dann wurde der Reaktionsansatz mit Eiswasser versetzt und mit EtOAc extrahiert. Die organische Phase wurde über Natriumsulfat getrocknet, i.vac. eingeengt und der Rückstand mittels präparativer HPLC-MS gereinigt. Die Produkt enthaltenden Fraktionen wurden vereinigt und das organische Lösungsmittel i.vac. eingeengt. Der Rückstand wurde mit 1 N wässriger Natronlauge alkalisch gestellt und die ausgefallene Schmiere mit EtOAc extrahiert. Die organische Phase wurde über Natriumsulfat getrocknet, filtriert, eingeengt und i.vac. getrocknet.

| | |
|---|---|
| Ausbeute: | 200 mg (26% d. Th.) |
| ESI-MS: | m/z = 383 (M+H)⁺ |
| Rₜ (HPLC): | 4.19 min (Methode B) |

### Intermediat 53

### (6-Chlor-pyrimidin-4-yl)-(7-methyl-2,3-dihydro-benzofuran-5-yl)-methanon

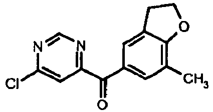

### Stufe 1: 7-Methyl-2,3-dihydro-benzofuran-3-ol

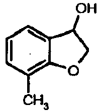

Unter einer Stickstoffatmosphäre wurden 0.945 g (7.35 mmol) Trimethylsulfoxoniumchlorid in 20 mL THF vorgelegt und mit 0.300 g (7.50 mmol) Natriumhydrid (55%, Suspension in Mineralöl) portionsweise versetzt. Der Reaktionsanstz wurde 2 h unter Rückfluss gekocht. Dann wurden 1.00 g (7.35 mmol) 2-Hydroxy-3-methylbenzaldehyd in 20 mL THF zum Reaktionsansatz getropft und über Nacht unter Rückfluss gekocht. Anschließend wurde PE hinzugefügt und die erhaltene Suspension filtriert. Das Filtrat wurde i.vac. eingeengt und mittels Flash-Chromatographie gereinigt. Die Produkt enthaltenden Fraktionen wurden vereinigt und eingeengt.

| | |
|---|---|
| Ausbeute: | 0.615 g (56% d. Th.) |
| ESI-MS: | m/z = 133 (M-H₂O+H)⁺ |
| Rₜ (HPLC): | 1.09 min (Methode B) |

### Stufe 2: 7-Methyl-2,3-dihydro-benzofuran

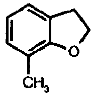

Unter einer Stickstoffatmosphäre wurden 0.610 g (4.06 mmol) 7-Methyl-2,3-dihydrobenzofuran-3-ol in 5 mL Essigsäure mit 770 µL (8.16 mmol) Essigsäureanhydrid 2 h unter Rückfluss gekocht. Nach Abkühlen auf RT wurden 60 mg Palladium auf Kohle (Pd/C 10%) hinzugefügt und der Ansatz 3.5 h unter einer Wasserstoffatmosphäre (3 bar) hydriert. Der Katalysator wurde abfiltriert und das Lösungsmittel eingeengt.

| | |
|---|---|
| Ausbeute: | 0.350 g (64% d. Th.) |
| ESI-MS: | m/z = 134 (M+) |

### Stufe 3: (6-Chlor-pyrimidin-4-yl)-(7-methyl-2,3-dihydro-benzofuran-5-yl)-methanon

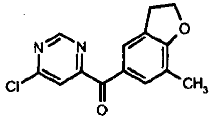

0.396 g (2.24 mmol) 6-Chlorpyrimidin-4-carbonsäurechlorid und 0.328 g (2.46 mmol) Aluminiumtrichlorid in 10 mL DCM wurden 20 min bei RT gerührt. Dann wurden 0.300 g (2.24 mmol) 7-Methyl-2,3-dihydro-benzofuran in DCM zu dem Reaktionsansatz getropft und dieser 1.5 h bei RT gerührt. Nach Zugabe von Wasser und DCM zum Reaktionsgemisch wurden die Phasen getrennt und die wässrige Phase mit DCM extrahiert. Die vereinigten organischen Phasen wurden mit gesättigter wässriger Natriumhydrogencarbonat-Lösung gewaschen, über Natriumsulfat getrocknet, filtriert und i.vac. eingeengt.

| | |
|---|---|
| Ausbeute: | 0.550 g (62% d. Th.) |
| Reinheit: | 70% |
| ESI-MS: | m/z = 275/277 (Cl) (M+H)⁺ |
| Rₜ (HPLC): | 1.54 min (Methode B) |

### Intermediat 54

### (6-Chloro-pyrimidin-4-yl)-(1,3,3,7-tetramethy-2,3-dihydro-1H-indol-5-yl)-methanon

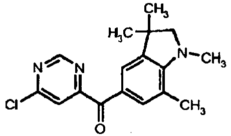

### Stufe 1: 1,3,3,7-Tetramethyl-1,3-dihydro-indol-2-on

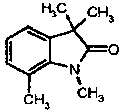

Zu 2.34 g (3.58 mmol) 7-Methyl-1,3-dihydro-indol-2-on in 20 mL DMF wurden bei 0°C 2.00 g (50.0 mmol) Natriumhydrid (55%, Suspension in Mineralöl) gegeben und 30 min gerührt. Anschließend wurden 3.00 mL (48.2 mmol) lodmethan zugegeben und 2 h bei RT gerührt. Zum Reaktionsansatz wurde Eiswasser gegeben, anschließend wurde mit DCM und wässriger Natriumhydrogencarbonat-Lösung extrahiert. Die organische Phase wurde über Natriumsulfat getrocknet, filtriert und i.vac. eingeengt. Der Rückstand wurde mittels präparativer HPLC-MS gereinigt. Die Produkt enthaltenden Fraktionen wurden vereinigt und gefriergetrocknet.

| | |
|---|---|
| Ausbeute: | 1.300 g (43% d. Th.) |
| Reinheit: | 90% |
| ESI-MS: | m/z = 190 (M+H)⁺ |
| Rₜ(HPLC): | 1.41 min (Methode B) |

### Stufe 2: 1,3,3,7-Tetramethyl-2,3-dihydro-1H-indol

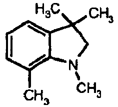

Unter einer Argonatmosphäre wurden zu 600 mg (2.85 mmol) 1,3,3,7-Tetramethyl-1,3-dihydro-indol-2-on in 20 mL THF 5.71 mL einer 1 molaren Lithiumaluminiumhydrid-Lösung in THF zugetropft. Der Reaktionsansatz wurde 2.5 h bei 60°C gerührt und anschließend auf RT abgekühlt. Unter Eisbadkühlung wurde gesättigte, wässrige Natriumsulfatlösung langsam zugetropft und die entstandene Suspension über Celite filtriert. Das Filtrat wurde mit EtOAc und gesättigter Natriumchloridlösung versetzt. Die organsiche Phase wurde über Natriumsulfat getrocknet, filtriert und das Filtrat eingeengt.

| | |
|---|---|
| Ausbeute: | 520 mg (99% d. Th.) |
| Reinheit: | 95% |
| ESI-MS: | m/z = 176 (M+H)⁺ |

### Stufe 3: (6-Chlor-pyrimidin-4-yl)-(1,3,3,7-tetramethyl-2,3-dihydro-1H-indol-5-yl)-methanon

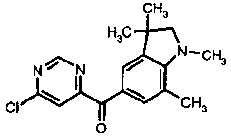

Unter Argonatmosphäre wurden 0.473 g (2.67 mmol) 6-Chlorpyrimidin-4-carbonsäure-chlorid und 0.427 g (3.20 mmol) Aluminiumtrichlorid in 20 mL DCM für 30 min gerührt. Dann wurden 0.520 g (2.67 mmol) 1,3,3,7-Tetramethyl-2,3-dihydro-1H-indol in DCM zu dem Reaktionsansatz getropft und es wurde 45 min bei RT, dann 45 min bei 40°C gerührt. Im Anschluss wurde der Reaktionsansatz mit Eiswasser / Natronlauge zersetzt, mit DCM extrahiert und die organische Phase i.vac. eingeengt. Der Rückstand wurde in MeOH aufgenommen und mittels präparativer HPLC-MS gereinigt. Die Produkt enthaltenden Fraktionen wurden vereinigt und gefriergetrocknet.

| | |
|---|---|
| Ausbeute: | 170 mg (20% d. Th.) |
| ESI-MS: | m/z = 316 (M+H)⁺ |
| Rₜ(HPLC): | 1.70 min (Methode B) |

### Intermediat 55

### [4'-(3,4-Dimethyl-2-oxo-2,3-dihydro-benzoxazol-6-carbonyl)-4-(2-oxo-2,3-dihydro-imidazo-[4,5-b]pyridin-1-yl)-3,4,5,6-tetrahydro-2H-[1,2']bipyridinyl-6'-yl]-methyl-carbaminsäure-tert-butylester

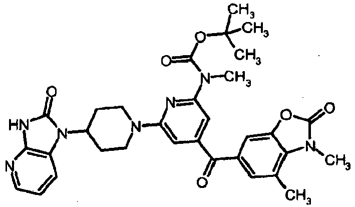

Unter Argonatmosphäre wurden zu 50 mg (0.10 mmol) 1-[6'-Chlor-4'-(3,4-dimethyl-2-oxo-2,3-dihydro-benzoxazol-6-carbonyl)-3,4,5,6-tetrahydro-2H-[1,2']bipyridinyl-4-yl]-1,3-dihydro-imidazo[4,5-b]pyridin-2-on in 1.00 mL Dioxan 13 mg (0.96 mmol) Methyl-carbaminsäure-*tert*-butylester, 11 mg (0.019 mmol) Xantphos, 8.8 mg (0.010 mmol) Pd₂dba₃ und 47 mg (0.15 mmol) Cäsiumcarbonat gegeben und der Ansatz 15 h am Rückfluss gerührt. Der Reaktionsansatz wurde i.vac. eingeengt und der erhaltene Rückstand ohne weitere Aufreinigung in der nächsten Stufe eingesetzt.

| | |
|---|---|
| Ausbeute: | 59 mg (quantitativ) |
| Rₜ (HPLC): | 1.70 min (Methode B) |

### Intermediat 56

### [4'-(3,4-Dimethyl-2-oxo-2,3-dihydro-benzoxazol-6-carbonyl)-4-(7-methoxy-2-oxo-1,2,4,5-tetrahydro-benzo[d][1,3]diazepin-3-yl)-3,4,5,6-tetrahydro-2H-[1,2']bipyridinyl-6'-yl]-carbaminsäure-tert-butylester

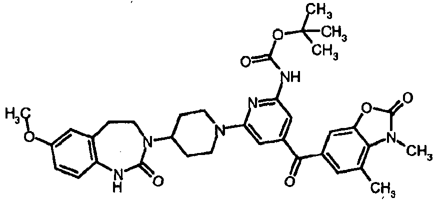

Unter Argonatmosphäre wurden zu 50 mg (0.10 mmol) 3-[6'-Chlor-4'-(3,4-dimethyl-2-oxo-2,3-dihydro-benzoxazol-6-carbonyl)-3,4,5,6-tetrahydro-2H-[1,2']bipyridinyl-4-yl]-7-methoxy-1,3,4,5-tetrahydro-benzo[d][1,3]diazepin-2-on in 1.0 mL Dioxan 31 mg (0.26 mmol) Carbaminsäure-*tert*-butylester, 10 mg (0.017 mmol) Xantphos, 8.0 mg (0.009 mmol) Pd₂dba₃ und 42 mg (0.13 mmol) Cäsiumcarbonat zugefügt und 15 h am Rückfluss gerührt. Der Reaktionsansatz wurde i.vac. eingeengt und der erhaltene Rückstand ohne weitere Aufreinigung in der nächsten Stufe eingesetzt.

| | |
|---|---|
| Ausbeute: | 57 mg (quantitativ) |
| Rₜ (HPLC): | 1.72 min (Methode B) |

### Intermediat 57

### (6-Chlor-pyrimidin-4-yl)-phenyl-methanon

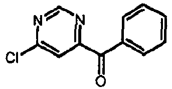

250 mg (1.68 mmol) 4,6-Dichlorpyrimidin, 258 mg (2.52 mmol) Benzaldehyd und 152 mg (0.56 mmol) 1,3-Dimethyl-3H-benzimidazol-1-ium-iodid (Chem. Pharm. Bull. 1990, 1147-52) in 3.0 mL THF wurden bei RT verrührt. Dann wurde 121 mg (2.52 mmol) 50%iges Natriumhydrid (Suspension in Mineralöl) hinzugefügt und der Reaktionsansatz 30 min bei RT gerührt und dann unter Rückfluss gekocht. Das Reaktionsgemisch wurde i.vac. eingeengt, der Rückstand mit Eiswasser versetzt und das Produkt mit DCM extrahiert. Die organischen Phasen wurden vereinigt, getrocknet und über Kieselgel filtriert. Das Filtrat wurde i.vac. eingeengt und der Rückstand als Rohprodukt weiter umgesetzt.

| | |
|---|---|
| Ausbeute: | 300 mg (39% d. Th.) |
| Reinheit: | 48% |
| ESI-MS: | m/z = 219/221 (Cl) (M+H)⁺ |
| R_{f}: | 0.5 (Kieselgel; Cyclohexan/EtOAc 5/1) |

### Intermediat 58

### 6-[4-(7-Methoxy-2-oxo-1,2,4,5-tetrahydro-benzo[d][1,3]diazepin-3-yl)-piperidin-1-yl]-pyrimidin-4-carbonsäuremethoxy-methyl-amid

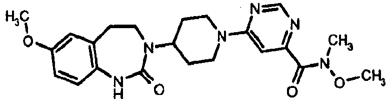

0.20 g (0.99 mmol) 6-Chlor-pyrimidin-4-carbonsäuremethoxy-methyl-amid, 0.28 g 7-Methoxy-3-piperidin-4-yl-1,3,4,5-tetrahydro-1,3-benzodiazepin-2-on und 0.20 mL (1.16 mmol) DIPEA in 1.5 mL THF wurden 30 min in der Mikrowelle auf 120°C erhitzt. Der Ansatz mit Wasser verdünnt und mit DCM extrahiert. Die vereinigten organischen Phasen wurden mit Natriumsulfat getrocknet, filtriert und i.vac. eingeengt. Der Rückstand wurde mit Diethylether verrieben, abgesaugt und getrocknet.

| | |
|---|---|
| Ausbeute: | 290 mg (66% d. Th.) |
| ESI-MS: | m/z = 441 (M+H)⁺ |
| Rₜ (HPLC): | 1.06 min (Methode B) |

### Intermediat 59

### 5-(6-Chlor-pyrimidin-4-carbonyl)-1,3-dimethyl-1,3-dihydro-benzimidazol-2-on

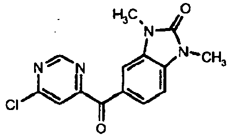

### Stufe 1: 5-(6-Chlor-pyrimidin-4-carbonyl)-1,3-dihydro-benzimidazol-2-on

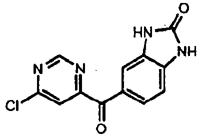

3.00 g (17.0 mmol) 6-Chor-pyrimidin-4-carbonsäurechlorid, 11.1 g (83.2 mmol) Aluminiumtrichlorid und 2.40 g (17.4 mmol) Benzimidazol wurden 3 h bei 130°C gerührt. Anschließend wurde das Reaktionsgemisch mit DCM, Wasser und 15%iger (w/v) wässriger Kaliumcarbonatlösung versetzt und die Phasen getrennt. Die wässrige Phase wurde mehrfach mit DCM extrahiert. Die vereinigten organischen Phasen wurden vereinigt, über Magnesiumsulfat getrocknet, filtriert und i.vac. eingeengt. Der Rückstand wurde mit DIPE und Isopropanol verrieben, abgesaugt und i.vac. getrocknet.

| | |
|---|---|
| Ausbeute: | 570 mg (12% d. Th.) |
| ESI-MS: | m/z = 273 (M-H)⁻ |
| R_{f}: | 0.61 (Kieselgel; DCM/Cyc/MeOH/ NH₄OH = 70/15/15/2) |

### Stufe 2: 5-(6-Chlor-pyrimidin-4-carbonyl)-1,3-dimethyl-1,3-dihydro-benzimidazol-2-on

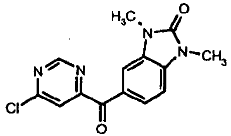

570 mg (2.08 mmol) 5-(6-Chlor-pyrimidin-4-carbonyl)-1,3-dihydro-benzoimidazol-2-on in 10.0 mL DMF wurden bei 0°C mit 190 mg (4.35 mmol) Natriumhydrid (55%, Suspension in Mineralöl) versetzt. Nach 1 h Rühren bei 0°C wurden 0.300 mL (4.73 mmol) lodmethan zugegeben. Man ließ die Reaktion auf RT kommen und rührte über Nacht. Dann wurde nochmals lodmethan zugefügt und weitere 2 h bei RT gerührt. Anschließend wurde der Reaktionsansatz auf Wasser gegeben und 30 min gerührt. Der ausgefallene Niederschlag wurde abgesaugt und i.vac. getrocknet.

| | |
|---|---|
| Ausbeute: | 400 mg (64% d. Th.) |
| ESI-MS: | m/z = 303/305 (Cl) (M+H)⁺ |
| R_{f}: | 0.84 (Kieselgel; DCM/Cyc/MeOH/ NH₄OH = 70/15/15/2) |

### Herstellung der Endverbindungen:

### Beispiel 1

### 1-{1-[6-(4-Methyl-2-oxo-2,3-dihydrobenzoxazol-6-carbonyl)-pyrimidin-4-yl]-piperidin-4-yl}-1,3-dihydro-imidazo[4,5-b]pyridin-2-on

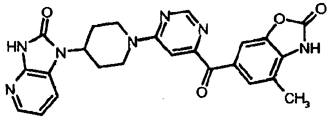

64 mg (0.22 mmol) 1-Piperidin-4-yl-1,3-dihydroimidazo[4,5-b]pyridin-2-on-dihydrochlorid, 64 mg (0.221 mmol) 6-(6-Chlorpyrimidin-4-carbonyl)-4-methyl-3*H*-benzoxazol-2-on und 0.174 mL (1.00 mmol) DIPEA wurden in 2.0 mL DMF bei RT über Nacht gerührt. Der Ansatz wurde mittels präparativer HPLC-MS gereinigt. Die Produkt enthaltenden Fraktionen wurden vereinigt, das organische Lösungsmittel *i.vac*. entfernt und die zurückgebliebene wässrige Phase mit 4N wässrigen NaOH-Lösung neutralisiert. Der Niederschlag wurde abgesaugt, mit Wasser nachgewaschen und im ULTS getrocknet.

| | |
|---|---|
| Ausbeute: | 56 mg (54% d. Th.) |
| ESI-MS: | m/z = 472 (M+H)⁺ |
| Rₜ (HPLC): | 2.37 min (Methode C) |

### Beispiel 2

### 7-Methoxy-3-{1-[6-(4-methyl-2-oxo-2,3-dihydrobenzoxazol-6-carbonyl)-pyrimidin-4-yl]-piperidin-4-yl}-1,3,4,5-tetrahydro-1,3-benzodiazepin-2-on

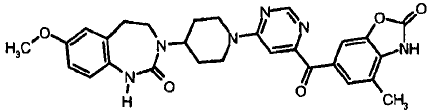

275 mg (1.00 mmol) 7-Methoxy-3-piperidin-4-yl-1,3,4,5-tetrahydro-1,3-benzodiazepin-2-on, 289 mg (1.00 mmol) 6-(6-Chlorpyrimidin-4-carbonyl)-4-methyl-3*H*-benzoxazol-2-on und 0.348 mL (2.00 mmol) DIPEA wurden in 10 mL DMF bei RT über Nacht gerührt. Der Ansatz wurde mittels präparativer HPLC-MS gereinigt. Die Produkt enthaltenden Fraktionen wurden vereinigt, das organische Lösungsmittel *i.vac.* entfernt und die zurückgebliebene wässrige Phase mit 4N wässrigen NaOH-Lösung neutralisiert. Der Niederschlag wurde abgesaugt, mit Wasser nachgewaschen und im ULTS getrocknet.

| | |
|---|---|
| Ausbeute: | 270 mg (51% d. Th.) |
| ESI-MS: | m/z = 529 (M+H)⁺ |
| Rₜ (HPLC): | 2.86 min (Methode C) |

### Beispiel 3

### 4-Methyl-6-{6-[4-(5-oxo-3-phenyl-4,5-dihydro-1,2,4-triazol-1-yl)-piperidin-1-yl]-pyrimidin-4-carbonyl}-3H-benzoxazol-2-on

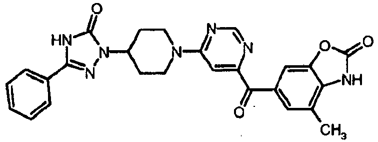

378 mg (1.00 mmol) 5-Phenyl-2-piperidin-4-yl-2,4-dihydro-1,2,4-triazol-3-on, 289 mg (1.00 mmol) 6-(6-Chlorpyrimidin-4-carbonyl)-4-methyl-3*H*-benzoxazol-2-on und 0.348 mL (2.0 mmol) DIPEA wurden in 10 mL DMF bei RT über Nacht gerührt. Der Ansatz wurde mittels präparativer HPLC-MS gereinigt. Die Produkt enthaltenden Fraktionen wurden vereinigt, das organische Lösungsmittel *i.vac*. entfernt und die zurückgebliebene wässrige Phase mit einer 4N wässrigen NaOH-Lösung neutralisiert. Der Niederschlag wurde abgesaugt, mit Wasser nachgewaschen und im ULTS getrocknet.

| | |
|---|---|
| Ausbeute: | 280 mg (56% d. Th.) |
| ESI-MS: | m/z = 498 (M+H)⁺ |
| Rₜ (HPLC) = | 2.72 min (Methode C) |

### Beispiel 4

### 3-{1-[6-(4-Methyl-2-oxo-2,3-dihydro-benzoxazol-6-carbonyl)-pyrimidin-4-yl]-piperidin-4-yl}-1,3-dihydroimidazo[4,5-c]chinolin-2-on

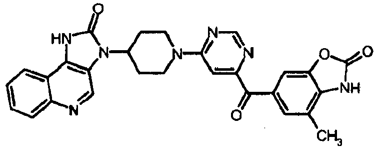

134 mg (0.500 mmol) 3-Piperidin-4-yl-1,3-dihydroimidazo[4,5-*c*]chinolin-2-on, 145 mg (0.500 mmol) 6-(6-Chlorpyrimidin-4-carbonyl)-4-methyl-3*H*-benzoxazol-2-on und 0.174 mL (1.00 mmol) DIPEA wurden in 5 mL DMF bei RT über Nacht gerührt. Der Ansatz wurde mittels präparativer HPLC-MS gereinigt. Die Produkt enthaltenden Fraktionen wurden vereinigt, das organische Lösungsmittel *i.vac*. entfernt und die zurückgebliebene wässrige Phase mit einer 4N wässrigen NaOH-Lösung neutralisiert. Der Niederschlag wurde abgesaugt, mit Wasser nachgewaschen und im ULTS getrocknet.

| | |
|---|---|
| Ausbeute: | 100 mg (38% d. Th.) |
| ESI-MS: | m/z = 522 (M+H)⁺ |
| Rₜ (HPLC) = | 1.99 min (Methode C) |

### Beispiel 5

### 1-{1-[6-(3,4-Dimethyl-2-oxo-2,3-dihydro-benzoxazol-6-carbonyl)-pyrimidin-4-yl]-piperidin-4-yl}-1,3-dihydro-imidazo[4,5-b]pyridin-2-on

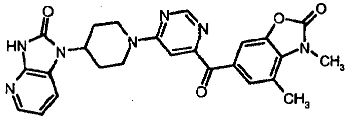

72.8 mg (0.25 mmol) 1-Piperidin-4-yl-1,3-dihydroimidazo[4,5-b]pyridin-2-on-dihydrochlorid, 75 mg (0.25 mmol) 6-(6-Chlorpyrimidin-4-carbonyl)-3,4-dimethyl-3*H*-benzoxazol-2-on und 0.22 mL (1.25 mmol) DIPEA wurden in 2 mL DMF 2h bei RT gerührt. Der Ansatz wurde mittels präparativer HPLC-MS gereinigt. Die Produkt enthaltenden Fraktionen wurden vereinigt, das organische Lösungsmittel *i.vac.* entfernt und die zurückgebliebene wässrige Phase mit einer 4N wässrigen NaOH-Lösung neutralisiert. Der Niederschlag wurde abgesaugt, mit Wasser nachgewaschen und im ULTS getrocknet.

| | |
|---|---|
| Ausbeute: | 71 mg (59% d. Th.) |
| ESI-MS: | m/z = 486 (M+H)⁺ |
| Rₜ (HPLC) | 2.67 min (Methode C) |

### Beispiel 6

### 3-{1-[6-(3,4-Dimethyl-2-oxo-2,3-dihydro-benzoxazol-6-carbonyl)-pyrimidin-4-yl]-piperidin-4-yl}-7-methoxy-1,3,4,5-tetrahydro-1,3-benzodiazepin-2-on

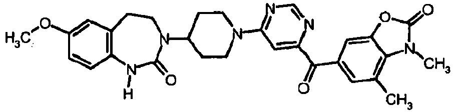

63.3 mg (0.23 mmol) 7-Methoxy-3-piperidin-4-yl-1,3,4,5-tetrahydro-1,3-benzodiazepin-2-on, 70 mg (0.23 mmol) 6-(6-Chlorpyrimidin-4-carbonyl)-3,4-dimethyl-3*H*-benzoxazol-2-on und 0.08 mL (0.46 mmol) DIPEA wurden in 2 mL DMF 2 h bei RT gerührt. Der Ansatz wurde mit Methanol verdünnt. Der Niederschlag wurde abgesaugt, mit Methanol und Diethylether nachgewaschen und im ULTS getrocknet.

| | |
|---|---|
| Ausbeute: | 85 mg (68% d. Th.) |
| ESI-MS: | m/z = 543 (M+H)⁺ |
| Rₜ (HPLC) | 3.2 min (Methode C) |

### Beispiel 7

### 1-{1-[6-(3,4-Dimethyl-benzoyl)-pyrimidin-4-yl]-piperidin-4-yl}-1,3-dihydro-imidazo[4,5-b]-pyridin-2-on

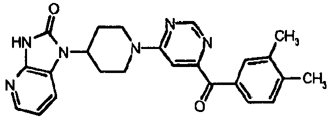

150 mg (0.515 mmol) 1-Piperidin-4-yl-1,3-dihydroimidazo[4,5-b]pyridin-2-on-dihydro-chlorid, 150 mg (0.527 mmol) (6-Chlor-pyrimidin-4-yl)-(3,4-dimethyl-phenyl)-methanon und 0.300 mL (1.74 mmol) DIPEA wurden in 5.0 mL DMF über Nacht bel RT gerührt. Das Reaktionsgemisch wurde i.vac. eingeengt, der Rückstand mit Wasser versetzt und 10 min nachgerührt. Der Niederschlag wurde abgesaugt und mittels präparativer HPLC-MS gereinigt. Die Produkt enthaltenden Fraktionen wurden vereinigt und gefriergetrocknet.

| | |
|---|---|
| Ausbeute: | 125 mg (57% d. Th.) |
| ESI-MS: | m/z = 429 (M+H)⁺ |
| R_{f}: | 0.52 (Kieselgel, DCM/Cyc/MeOH/ NH₄OH = 70/15/15/2) |

### Beispiel 8

### 1-{1-[6-(3,4-Diethyl-benzoyl)-pyrimidin-4-yl]-piperidin-4-yl}-1,3-dihydro-imidazo[4,5-b]-pyridin-2-on

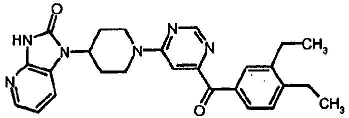

150 mg (0.515 mmol) 1-Piperidin-4-yl-1,3-dihydroimidazo[4,5-b]pyridin-2-on-dihydro-chlorid, 150 mg (0.546 mmol) (6-Chlor-pyrimidin-4-yl)-(3,4-diethyl-phenyl)-methanon und 0.300 mL (1.74 mmol) DIPEA wurden in 5.0 mL DMF über Nacht bei RT gerührt. Das Reaktionsgemisch wurde i.vac. eingeengt, der Rückstand mit Wasser versetzt und 10 min nachgerührt. Der Niederschlag wurde abgesaugt und mittels präparativer HPLC-MS gereinigt. Die Produkt enthaltenden Fraktionen wurden vereinigt und gefriergetrocknet.

| | |
|---|---|
| Ausbeute: | 125 mg (53% d. Th.) |
| ESI-MS: | m/z = 457 (M+H)⁺ |
| R_{f}: | 0.53 (Kieselgel, DCM/Cyc/MeOH/ NH₄OH = 70/15/15/2) |

### Beispiel 9:

### 3-[1-(6-Benzoyl-pyrimidin-4-yl)-piperidin-4-yl]-7-methoxy-1,3,4,5-tetrahydro-benzo[d]-[1,3]diazepin-2-on

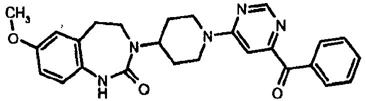

Unter einer Stickstoffatmosphäre wurden 100 mg (0.227 mmol) 6-[4-(7-Methoxy-2-oxo-1,2,4,5-tetrahydro-benzo[d][1,3]diazepin-3-yl)-piperidin-1-yl]-pyrimidin-4-carbonsäure-methoxy-methyl-amid in 5.00 mL THF auf-10°C abgekühlt und mit 0.400 mL (0.400 mmol) Phenylmagnesiumbromidlösung (1 M in THF) versetzt. Der Ansatz wurde 1 h bei -10°C gerührt und dann auf 0°C erwärmt. Dann wurden 0.200 mL (0.200 mmol) Phenyl-magnesiumbromidlösung (1 M in THF) zugesetzt, 1 h nachgerührt und dann auf RT erwärmt. Der Reaktionsansatz wurde mit einer gesättigten Ammoniumchlorid-Lösung versetzt und mit EtOAc extrahiert. Die vereinigten organischen Phasen wurden getrocknet und i.vac. eingeengt. Der Rückstand wurde mittels präparativer HPLC-MS gereinigt. Die Produkt enthaltenden Fraktionen wurden vereinigt und bis zur Hälfte eingeengt. Der ausgefallene Niederschlag wurde abgesaugt und getrocknet.

| | |
|---|---|
| Ausbeute: | 19 mg (18% d. Th.) |
| ESI-MS: | m/z = 548 (M+H)⁺ |
| Rₜ (HPLC): | 3.24 min (Methode C) |

### Beispiel 10

### 1-{1-[6-(3,4-Dichlor-benzoyl)-pyrimidin-4-yl]-piperidin-4-yl)-1,3-dihydro-imidazo[4,5-b]-pyridin-2-on

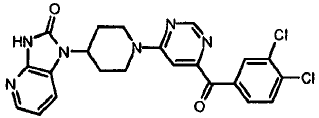

150 mg (0.515 mmol) 1-Piperidin-4-yl-1,3-dihydroimidazo[4,5-b]pyridin-2-on-dihydro-chlorid, 150 mg (0.522 mmol) (6-Chlor-pyrimidin-4-yl)-(3,4-dichlor-phenyl)-methanon und 0.300 mL (1.74 mmol) DIPEA wurden in 5.0 mL DMF über Nacht bei RT gerührt. Das Reaktionsgemisch wurde i.vac. eingeengt, der Rückstand mit Wasser versetzt und 10 min nachgerührt. Danach wurde der Niederschlag abgesaugt und mittels präparativer HPLC-MS gereinigt. Die Produkt enthaltenden Fraktionen wurden vereinigt und gefriergetrocknet.

| | |
|---|---|
| Ausbeute: | 125 mg (52% d. Th.) |
| ESI-MS: | m/z = 469/471/473 (2 CI) (M+H)⁺ |
| R_{f}: | 0.67 (Kieselgel, DCM/Cyc/MeOH/ NH₄OH = 70/15/15/2) |

### Beispiel 11:

### 3-[4'-(3,4-Dimethyl-2-oxo-2,3-dihydro-benzooxazole-6-carbonyl)-6'-oxo-3,4,5,6,1',6'-hexa-hydro-2H-[1,2']bipyridinyl-4-yl]-7-methoxy-1,3,4,5-tetrahydro-benzo[d][1,3]diazepin-2-on

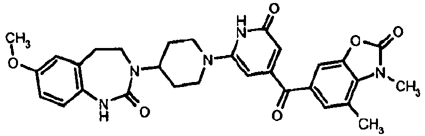

Zu 0.13 g (0.20 mmol) 3-[6'-Benzyloxy-4'-(3,4-dimethyl-2-oxo-2,3-dihydro-benzoxazol-6-carbonyl)-3,4,5,6-tetrahydro-2H-[1,2']bipyridinyl-4-yl]-7-methoxy-1,3,4,5-tetrahydro-benzo[d][1,3]diazepin-2-on in 20 mL MeOH wurden 30 mg Palladium auf Kohle (Pd/C 10%), 30 mL THF und 30 mL DCM gegeben und 1.5 h unter einer Wasserstoffatmosphäre hydriert. Nach Filtration des Reaktionsansatzes wurde das Lösungsmittel i.vac. eingeengt. Der Rückstand wurde mittels präparativer HPLC-MS gereinigt. Die Produkt enthaltenden Fraktionen wurden vereinigt und gefriergetrocknet.

| | |
|---|---|
| Ausbeute: | 3 mg (3% d. Th.) |
| ESI-MS: | m/z =558(M+H)⁺ |
| Rₜ (HPLC): | 1.35 min (Methode B) |

### Beispiel 12:

### 7-Methoxy-3-{1-[5-(4-methyl-2-oxo-2,3-dihydro-benzoxazol-6-carbonyl)-pyridazin-3-yl]-piperidin-4-yl}-1,3,4,5-tetrahydro-benzo[d][1,3]diazepin-2-on

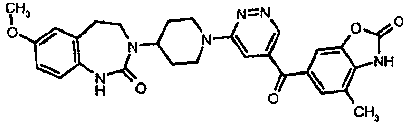

0.13 g (0.049 mmol) 3-(1-{5-[Hydroxy-(4-methyl-2-oxo-2,3-dihydro-benzoxazol-6-yl)-methyl]-pyridazin-3-yl}-piperidin-4-yl)-7-methoxy-1,3,4,5-tetrahydro-benzo[d][1,3]diazepin-2-on und 1.0 g (12 mmol) Mangan(IV)-oxid in 100 mL DCM wurden über Nacht bei RT gerührt. Anschließend wurden 500 mg Mangan(IV)-oxid zugegeben und weitere 20 h gerührt. Dann wurde der Reaktionsansatz mit 10 mL MeOH versetzt, abfiltriert und eingeengt. Der Rückstand wurde mittels präparativer HPLC-MS gereinigt. Die Produkt enthaltenden Fraktionen wurden vereinigt und gefriergetrocknet.

| | |
|---|---|
| Ausbeute: | 5 mg (17% d. Th.) |
| ESI-MS: | m/z = 529 (M+H)⁺ |
| Rₜ (HPLC): | 1.23 min (Methode G) |

### Beispiel 13:

### 7-Methoxy-3-{1-[2-methyl-6-(4-methyl-2-oxo-2,3-dihydro-benzoxazol-6-carbonyl)-pyrimidin-4-yl]-piperidin-4-yl}-1,3,4,5-tetrahydro-benzo[d][1,3]diazepin-2-on

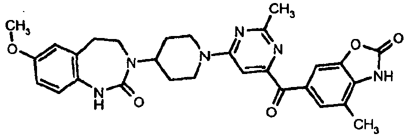

83 mg (0.30 mmol) 7-Methoxy-3-piperidin-4-yl-1,3,4,5-tetrahydro-1,3-benzodiazepin-2-on, 91 mg (0.30 mmol) 6-(6-Chlor-2-methyl-pyrimidin-4-carbonyl)-4-methyl-3H-benzoxazol-2-on und 0.11 mL (0.60 mmol) DIPEA wurden in 3 mL DMF zusammen über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel eingeengt. Der Rückstand wurde mit 4N wässriger Natronlauge neutralisiert, der ausgefallene Niederschlag abgesaugt, mit Wasser nachgewaschen und i.vac. getrocknet.

| | |
|---|---|
| Ausbeute: | 115 mg (71% d. Th.) |
| ESI-MS: | m/z = 543 (M+H)⁺ |
| Rₜ (HPLC): | 1.33 min (Methode B) |

### Beispiel 14:

### 3-[5'-(3,4-Dimethyl-2-oxo-2,3-dihydro-benzoxazol-6-carbonyl)-1'-oxy-3,4,5,6-tetrahydro-2H-[1,3']bipyridinyl-4-yl]-7-methoxy-1,3,4,5-tetrahydro-benzo[d][1,3]diazepin-2-on

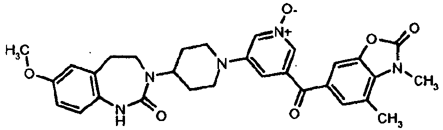

Unter einer Argonatmosphäre wurden 275 mg (1.00 mmol) 7-Methoxy-3-piperidin-4-yl-1,3,4,5-tetrahydro-benzo[d][1,3]diazepin-2-on, 363 mg (1.00 mmol) 6-(5-Brom-1-oxy-pyri-din-3-carbonyl)-3,4-dimethyl-3H-benzoxazol-2-on und 489 mg (1.50 mmol) Cäsiumcarbonat in 16 mL Dioxän zusammen gegeben, anschließend mit 63 mg (0.10 mmol) BINAP und 23 mg (0.10 mmol) Palladium(II)acetat versetzt und 6 h bei 120°C gerührt. Der Ansatz wurde i.vac. eingeengt und der Rückstand mittels präparativer HPLC-MS gereinigt. Die Produkt enthaltenden Fraktionen wurden vereinigt und das Acetonitril i.vac. entfernt. Der wässrige Rückstand wurde mit 1 N wässriger Natronlauge basisch gestellt, der ausgefallene Niederschlag abgesaugt und mit Wasser gewaschen und i.vac. getrocknet.

| | |
|---|---|
| Ausbeute: | 35 mg (6% d. Th.) |
| ESI-MS: | m/z = 558 (M+H)⁺ |
| Rₜ (HPLC): | 1.38 min (Methode B) |

### Beispiel 15:

### 4'-(3,4-Dimethyl-2-oxo-2,3-dihydro-benzoxazol-6-carbonyl)-4-(2-oxo-2,3-dihydro-imidazo[4,5-b]pyridin-1-yl)-3,4,5,6-tetrahydro-2H,1'H-[1,2']bipyridinyl-6'-on

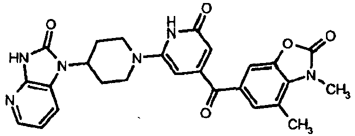

20 mg (0.034 mmol) 1-[6'-Benzyloxy-4'-(3,4-dimethyl-2-oxo-2,3-dihydro-benzoxazol-6-carbonyl)-3,4,5,6-tetrahydro-2H-[1,2']bipyridinyl-4-yl]-1,3-dihydro-imidazo[4,5-b]pyridin-2-on in 0.10 g (0.87 mmol) Pyridinhydrochlorid wurden 4 min in der Schmelze gehalten. Nach dem Abkühlen wurde der Ansatz mittels präparativer HPLC-MS gereinigt. Die Produkt enthaltenden Fraktionen wurden vereinigt und gefriergetrocknet.

| | |
|---|---|
| Ausbeute: | 11 mg (65% d. Th.) |
| ESI-MS: | m/z =501(M+H)⁺ |
| Rₜ (HPLC): | 1.17 min (Methode B) |

### Beispiel 16:

### 3-{1-[2-Chlor-6-(4-methyl-2-oxo-2,3-dihydro-benzoxazol-6-carbonyl)-pyrimidin-4-yl]-piperidin-4-yl}-7-methoxy-1,3,4,5-tetrahydro-benzo[d][1,3]diazepin-2-on

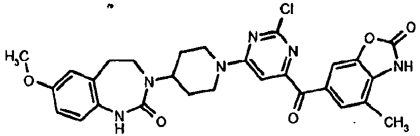

275 mg (1.00 mmol) 7-Methoxy-3-piperidin-4-yl-1,3,4,5-tetrahydro-1,3-benzodiazepin-2-on, 324 mg (1.00 mmol) 6-(2,6-Dichlor-pyrimidin-4-carbonyl)-4-methyl-3H-benzoxazol-2-on und 0.348 mL (2.00 mmol) DIPEA in 5.00 mL DMF wurden über Nacht bei RT gerührt. Anschließend wurde der Ansatz mittels präparativer HPLC-MS gereinigt. Die Produkt enthaltenden Fraktionen wurden vereinigt und das organische Lösungsmittel i.vac. eingeengt. Der Rückstand wurde mit 4N wässriger Natronlauge neutralisiert und der ausgefallene Niederschlag abgesaugt, mit Wasser nachgewaschen und getrocknet.

| | |
|---|---|
| Ausbeute: | 180 mg (32% d. Th.) |
| ESI-MS: | m/z =563 (M+H)⁺ |
| Rₜ (HPLC): | 1.15 min (Methode B) |

### Beispiel 17:

### 7-Methoxy-3-{1-[6-methoxy-5-(4-methyl-2-oxo-2,3-dihydro-benzoxazol-6-carbonyl)-pyridazin-3-yl]-piperidin-4-yl}-1,3,4,5-tetrahydro-benzo[d][1,3]diazepin-2-on

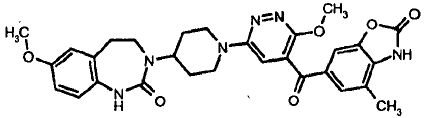

0.13 g (0.11 mmol) (Reinheit 50%) 3-{1-[6-Chloro-5-(4-methyl-2-oxo-2,3-dihydro-benzoxa-zol-6-carbonyl)-pyridazin-3-yl]-piperidin-4-yl}-7-methoxy-1,3,4,5-tetrahydro-benzo[d][1,3]-diazepin-2-on und 0.050 mL (0.29 mmol) DIPEA wurden mit 1 mL MeOH in einem Mikrowellengefäß 2 h bei 125°C erhitzt. Dann wurde Kalium-*tert*-butylat zugegeben und 48 h bei RT gerührt. Im Anschluss wurde weitere 6 h erhitzt und dann wurde der Reaktionsansatz mit Essigsäure neutralisiert und mittels präparativer HPLC-MS gereinigt.

| | |
|---|---|
| Ausbeute: | 13 mg (20% d. Th.) |
| Rₜ (HPLC): | 2.14 min (Methode H) |

### Beispiel 18:

### 3-{1-[6-(3,4-Dimethyl-2-oxo-2,3-dihydro-benzoxazol-6-carbonyl)-2-methyl-pyrimidin-4-yl]-piperidin-4-yl}-7-methoxy-1,3,4,5-tetrahydro-benzo[d][1,3]diazepin-2-on

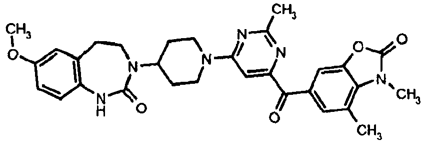

83 mg (0.30 mmol) 7-Methoxy-3-piperidin-4-yl-1,3,4,5-tetrahydro-1,3-benzodiazepin-2-on, 95 mg (0.30 mmol) 6-(6-Chlor-2-methyl-pyrimidin-4-carbonyl)-3,4-dimethyl-3H-benzoxazol-2-on und 0.11 mL (0.60 mmol) DIPEA wurden in 3 mL DMF über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel i.vac. eingeengt. Der Rückstand wurde mit 4N wässriger Natronlauge neutralisiert, der ausgefallene Niederschlag wurde abgesaugt, mit Wasser gewaschen und i.vac. getrocknet.

| | |
|---|---|
| Ausbeute: | 115 mg (71% d. Th.) |
| ESI-MS: | m/z = 543 (M+H)⁺ |
| Rₜ (HPLC): | 1.33 min (Methode B) |

### Beispiel 19:

### 3-[4'-(3,4-Dimethyl-2-oxo-2,3-dihydro-benzoxazol-6-carbonyl)-3,4,5,6-tetrahydro-2H-[1,2']bipyridinyl-4-yl]-7-methoxy-1,3,4,5-tetrahydro-benzo[d][1,3]diazepin-2-on

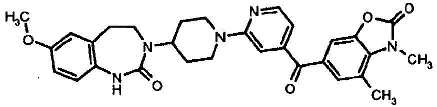

350 mg (1.27 mmol) 7-Methoxy-3-piperidin-4-yl-1,3,4,5-tetrahydro-1,3-benzodiazepin-2-on und 180 mg (0.600 mmol) 6-(2-Chlor-pyridin-4-carbonyl)-3,4-dimethyl-3H-benzoxazol-2-on wurden in 3 mL NMP über Nacht bei 120°C gerührt. Der Reaktionsansatz wurde mit MeOH verdünnt und mittels präparativer HPLC-MS gereinigt. Die Produkt enthaltenden Fraktionen wurden vereinigt und gefriergetrocknet.

| | |
|---|---|
| Ausbeute: | 60 mg (19% d. Th.) |
| ESI-MS: | m/z = 542 (M+H)⁺ |
| Rₜ (HPLC): | 4.24 min (Methode L) |

### Beispiel 20:

### 3-{1-[6-(3,4-Dimethyl-2-oxo-2,3-dihydro-benzoxazol-6-carbonyl)-pyrimidin-4-yl]-3-fluor-piperidin-4-yl}-7-methoxy-1,3,4,5-tetrahydro-benzo[d][1,3]diazepin-2-on

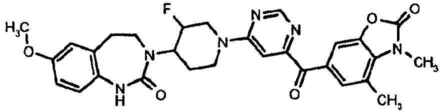

60 mg (0.21 mmol) 3-(3-Fluor-piperidin-4-yl)-7-methoxy-1,3,4,5-tetrahydro-benzo[d]-[1,3]diazepin-2-on, 60 mg (0.20 mmol) 6-(6-Chlor-pyrimidin-4-carbonyl)-3,4-dimethyl-3H-benzoxazol-2-on und 0.040 mL (0.30 mmol) TEA wurden in 1 mL DMF über Nacht bei RT gerührt. Der Reaktionsansatz wurde mit 2 mL MeOH versetzt und der entstandene Niederschlag abgesaugt, mit MeOH und Diethylether gewaschen und getrocknet.

| | |
|---|---|
| Ausbeute: | 77 mg (70% d. Th.) |
| ESI-MS: | m/z = 561 (M+H)⁺ |
| Rₜ (HPLC): | 3.40 min (Methode C) |

### Beispiel 21:

### 1-{1-[2-Methyl-6-(4-methyl-2-oxo-2,3-dihydro-benzoxazol-6-carbonyl)-pyrimidin-4-yl]-piperidin-4-yl}-1,3-dihydro-imidazo[4,5-b]pyridin-2-on

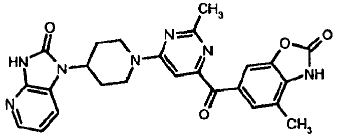

65 mg (0.30 mmol) 1-Piperidin-4-yl-1,3-dihydroimidazo[4,5-b]pyridin-2-on, 91 mg (0.30 mmol) 6-(6-Chlor-2-methyl-pyrimidin-4-carbonyl)-4-methyl-3H-benzoxazol-2-on und 0.11 mL (0.60 mmol) DIPEA wurden in 3 mL DMF über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel eingeengt. Der Rückstand wurde mit 4N wässriger Natronlauge neutralisiert, der ausgefallene Niederschlag wurde abgesaugt, mit Wasser nachgewaschen und getrocknet.

| | |
|---|---|
| Ausbeute: | 90 mg (62% d. Th.) |
| ESI-MS: | m/z = 486 (M+H)⁺ |
| Rₜ (HPLC): | 1.17 min (Methode B) |

### Beispiel 22:

### 7-Methoxy-3-[4'-(4-methyl-2-oxo-2,3-dihydro-benzoxazol-6-carbonyl)-3,4,5,6-tetrahydro-2H-[1,2']bipyridinyl-4-yl]-1,3,4,5-tetrahydro-benzo[d][1,3]diazepin-2-on

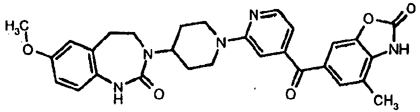

Unter einer Stickstoffatmosphäre wurden 0.21 g (0.80 mmol) 7-Methoxy-3-piperidin-4-yl-1,3,4,5-tetrahydro-benzo[d][1,3]diazepin-2-on, 0.38 g (1.2 mmol) Cäsiumcarbonat, 50 mg (0.10 mmol) BINAP und 25 mg (0.10 mmol) Palladium(II)acetat in 20 mL Xylol 10 min bei RT gerührt. 0.25 g (0.80 mmol) 6-(2-Brom-pyridin-4-carbonyl)-4-methyl-3H-benzoxazoi-2-on wurden zugefügt und über Nacht bei 120°C gerührt. Der Ansatz wurde eingeengt; der Rückstand mit 15 mL DMF versetzt und 25 mg (0.11 mmol) Palladium(II)acetat und 50 mg (0.10 mmol) BINAP zugegeben. Das Reaktionsgemisch wurde 48 h bei 120°C gerührt. Nach dem Erkalten wurde der Feststoff abfiltriert und das Filtrat i.vac. eingeengt. Der Rückstand wurde mit EtOAc verrieben, abgesaugt und in DMF gelöst. Das Produkt wurde mittels präparativer HPLC-MS gereinigt. Die Produkt enthaltenden Fraktionen wurden vereinigt und gefriergetrocknet.

| | |
|---|---|
| Ausbeute: | 10 mg (3% d. Th.) |
| ESI-MS: | m/z = 528 (M+H)⁺ |
| R_{f}: | 0.57 (Kieselgel, DCM/Cyc/MeOH/ NH₄OH = 70/15/15/2) |

### Beispiel 23:

### 1-[1-[6-(3,4-Dimethyl-2-oxo-2,3-dihydro-benzoxazol-6-carbonyl)-2-methyl-pyrimidin-4-yl]-piperidin-4-yl)-1,3-dihydro-imidazo[4,5-b]pyridin-2-on

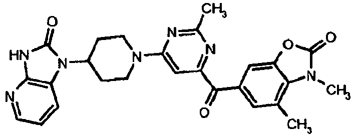

65 mg (0.30 mmol) 1-Piperidin-4-yl-1,3-dihydroimidazo[4,5-b]pyridin-2-on, 95 mg (0.30 mmol) 6-(6-Chlor-2-methyl-pyrimidin-4-carbonyl)-3,4-dimethyl-3H-benzoxazol-2-on und 0.11 mL (0.60 mmol) DIPEA wurden in 3 mL DMF über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel i.vac. eingeengt. Der Rückstand wurde mit 4N wässriger Natronlauge neutralisiert, der ausgefallene Niederschlag wurde abgesaugt, mit Wasser nachgewaschen und getrocknet.

| | |
|---|---|
| Ausbeute: | 80 mg (53% d. Th.) |
| ESI-MS: | m/z = 500 (M+H)⁺ |
| Rₜ (HPLC): | 1.24 min (Methode B) |

### Beispiel 24:

### 1-[4'-(3,4-Dimethyl-2-oxo-2,3-dihydro-benzoxazol-6-carbonyl)-3,4,5,6-tetrahydro-2H-[1,2']bipyridinyl-4-yl]-1,3-dihydro-imidazo[4,5-b]pyridin-2-on

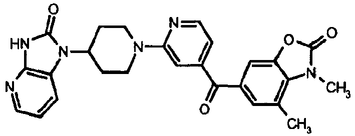

0.40 g (1.8 mmol) 1-Piperidin-4-yl-1,3-dihydroimidazo[4,5-b]pyridin-2-on und 0.18 g (0.60 mmol) 6-(2-Chlor-pyridin-4-carbonyl)-3,4-dimethyl-3H-benzoxazol-2-on wurden in 3 mL NMP über Nacht bei 120°C gerührt. Der Reaktionsansatz wurde mit MeOH verdünnt und mittels präparativer HPLC-MS gereinigt. Die Produkt enthaltenden Fraktionen wurden vereinigt und gefriergetrocknet.

| | |
|---|---|
| Ausbeute: | 65 mg (23% d. Th.) |
| ESI-MS: | m/z = 485 (M+H)⁺ |
| Rₜ (HPLC): | 1.23 min (Methode B) |

### Beispiel 25:

### 3-{1-[6-Chloro-5-(4-methyl-2-oxo-2,3-dihydro-benzoxazol-6-carbonyl)-pyridazin-3-yl]-piperidin-4-yl}-7-methoxy-1,3,4,5-tetrahydro-benzo[d][1,3]diazepin-2-on

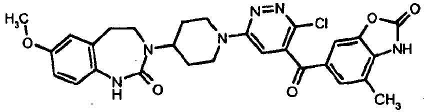

360 mg (1.31 mmol) 7-Methoxy-3-piperidin-4-yl-1,3,4,5-tetrahydro-1,3-benzodiazepin-2-on, 480 mg (1.19 mmol) 6-(3,6-Dichlor-pyridazin-4-carbonyl)-4-methyl-3H-benzoxazol-2-on und 0.300 mL (1.74 mmol) DIPEA wurden in 3 mL DMF 4 h bei 100°C gerührt. Anschließend wurde der Reaktionsansatz abgekühlt, mit 0.5 mLI Ameisensäure und 15 mL Wasser versetzt, der ausgefallene Niederschlag abgesaugt und getrocknet. Dieser wurde mittels präparativer HPLC-MS gereinigt. Die Produkt enthaltenden Fraktionen wurden vereinigt und gefriergetrocknet.

| | |
|---|---|
| Ausbeute: | 10 mg (2% d. Th.) |
| ESI-MS: | m/z = 563 (M+H)⁺ |
| Rₜ (HPLC): | 1.31 min (Methode G) |

### Beispiel 26:

### 3-[4-(7-Methoxy-2-oxo-1,2,4,5-tetrahydro-benzo[d][1,3]diazepin-3-yl)-piperidin-1-yl]-5-(4-methyl-2-oxo-2,3-dihydro-benzoxazol-6-carbonyl)-benzonitril

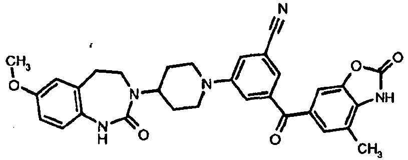

0.22 g (0.80 mmol) 7-Methoxy-3-piperidin-4-yl-1,3,4,5-tetrahydro-1,3-benzodiazepin-2-on und 0.12 g (0.40 mmol) 3-Fluor-5-(4-methyl-2-oxo-2,3-dihydro-benzoxazol-6-carbonyl)-benzonitril wurden ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag abgesaugt, mit Wasser nachgewaschen und getrocknet.

| | |
|---|---|
| Ausbeute: | 30 mg (14% d. Th.) |
| ESI-MS: | m/z =552 (M+H)⁺ |
| Rₜ (HPLC): | 1.48 min (Methode B) |

### Beispiel 27:

### 1-[1-(6-Benzoyl-pyrimidin-4-yl)-piperidin-4-yl]-1,3-dihydro-imidazo[4,5-b]pyridin-2-on

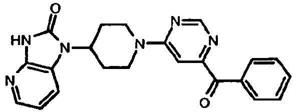

0.20 g (0.67 mmol) 1-Piperidin-4-yl-1,3-dihydroimidazo[4,5-b]pyridin-2-on, 0.15 g (0.33 mmol) (6-Chlor-pyrimidin-4-yl)-phenyl-methanon und 0.20 mL (1.1 mmol) DIPEA in 3 mL DMF wurden bei 80°C 1 h erhitzt. Anschließend wurde der Reaktionsansatz mittels präparativer HPLC-MS gereinigt.Die Produkt enthaltenden Fraktionen wurden vereinigt und gefriergetrocknet.

| | |
|---|---|
| Ausbeute: | 87 mg (66% d. Th.) |
| ESI-MS: | m/z = 401 (M+H)⁺ |
| Rₜ (HPLC): | 1.59 min (Methode M) |

### Beispiel 28:

### 3-{1-[6-(3,4-Dimethyl-2-oxo-2,3-dihydro-benzoxazol-6-carbonyl)-pyrimidin-4-yl]-piperidin-4-yl}-1,3,4,5-tetrahydro-benzo[d][1,3]diazepin-2-on

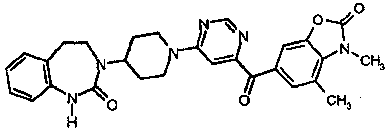

98 mg (0.40 mmol) 3-Piperidin-4-yl-1,3,4,5-tetrahydro-1,3-benzodiazepin-2-on, 0.12 g (0.40 mmol) 6-(6-Chlorpyrimidin-4-carbonyl)-3,4-dimethyl-3H-benzoxazol-2-on und 0.14 mL (0.80 mmol) DIPEA wurden in 3 mL DMF 2 h bei RT gerührt. Der Ansatz wurde mit Methanol verdünnt, und der ausgefallene Niederschlag wurde abgesaugt, mit MeOH und Diethylether gewaschen und getrocknet.

| | |
|---|---|
| Ausbeute: | 185 mg (90% d. Th.) |
| ESI-MS: | m/z = 513 (M+H)⁺ |
| Rₜ (HPLC): | 1.36 min (Methode B) |

### Beispiel 29:

### 7-Methoxy-3-{1-[5-(4-methyl-2-oxo-2,3-dihydro-benzoxazol-6-carbonyl)-6-oxo-5,6-dihydro-pyridazin-3-yl]-piperidin-4-yl}-1,3,4,5-tetrahydro-benzo[d][1,3]diazepin-2-on

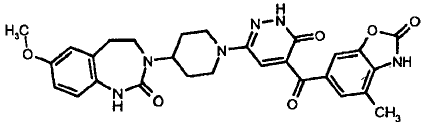

Zu 0.13 g (0.20 mmol) 3-{1-[6-Chloro-5-(4-methyl-2-oxo-2,3-dihydro-benzoxazol-6-carbo-nyl)-pyridazin-3-yl]-piperidin-4-yl}-7-methoxy-1,3,4,5-tetrahydro-benzo[d][1,3]diazepin-2-on (Gemisch) in 3 mL Essigsäure wurden 0.10 g (1.0 mmol) Kaliumacetat gegeben und unter Stickstoffatmosphäre 7 h gekocht. Dann wurde der Reaktionsansatz eingeengt und der Rückstand wurde mit Wasser verrieben, abgesaugt und getrocknet. Der Rückstand wurde mittels präparativer HPLC-MS gereinigt. Die Produkt enthaltenden Fraktionen wurden vereinigt und gefriergetrocknet.

| | |
|---|---|
| Ausbeute: | 1.5 mg (1% d. Th.) |
| ESI-MS: | m/z = 545 (M+H)⁺ |
| Rₜ (HPLC): | 4.22 min (Methode I) |

### Beispiel 30:

### 3-{1-[5-(3,4-Dimethyl-2-oxo-2,3-dihydro-benzoxazol-6-carbonyl)-6-oxo-1,6-dihydro-pyridazin-3-yl]-piperidin-4-yl}-7-methoxy-1,3,4,5-tetrahydro-benzo[d][1,3]diazepin-2-on

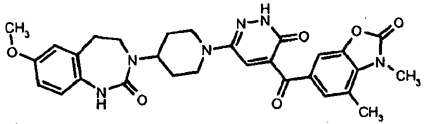

Zu 0.13 g (0.20 mmol) 3-{1-[6-Chlor-5-(3,4-dimethyl-2-oxo-2,3-dihydro-benzoxazol-6-carbonyl)-pyridazin-3-yl]-piperidin-4-y}-7-methoxy-1,3,4,5-tetrahydro-benzo[d]-[1,3]diazepin-2-on (Gemisch) in 2 mL Essigsäure wurden 0.10 g (1.02 mmol) Kaliumacetat gegeben und unter Stickstoffatmosphäre 16 h gekocht. Dann wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 1.6 mg (13% d. Th.) |
| Reinheit: | 90 % |
| ESI-MS: | m/z = 559 (M+H)⁺ |
| Rₜ (HPLC): | 1.52 min (Methode K) |

### Beispiel 31

### 7-Chlor-3-{1-[6-(3,4-dimethyl-2-oxo-2,3-dihydro-benzoxazol-6-carbonyl)-pyrimidin-4-yl]-piperidin-4-yl}-1,3,4,5-tetrahydro-benzo[d][1,3]diazepin-2-on

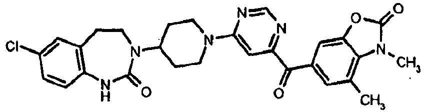

56 mg (0.20 mmol) 7-Chlor-3-piperidin-4-yl-1,3,4,5-tetrahydro-benzo[d][1,3]diazepin-2-on, 61 mg (0.20 mmol) 6-(6-Chlorpyrimidin-4-carbonyl)-3,4-dimethyl-3H-benzoxazol-2-on und 0.070 mL (0.40 mmol) DIPEA wurden in 1.5 mL DMF über Nacht bei RT gerührt. Der Ansatz wurde mit 1.5 mL MeOH verdünnt, abgesaugt, mit DIPE nachgewaschen und getrocknet.

| | |
|---|---|
| Ausbeute: | 87 mg (80 % d. Th.) |
| ESI-MS: | m/z = 547/549 (M+H)⁺ |
| Rₜ (HPLC): | 3.6 min (Methode C) |

### Beispiel 32:

### 3-{1-[6-(3,4-Dimethyl-2-oxo-2,3-dihydro-benzoxazol-6-carbonyl)-pyrimidin-4-yl]-piperidin-4-yl}-1,3-dihydro-imidazo[4,5-c]chinolin-2-on

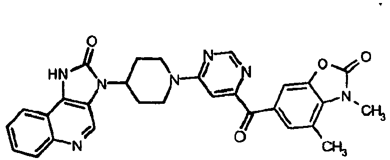

0.11 g (0.40 mmol) 3-Piperidin-4-yl-1,3-dihydroimidazo[4,5-c]chinolin-2-on, 0.12 g (0.40 mmol) 6-(6-Chlorpyrimidin-4-carbonyl)-3,4-dimethyl-3H-benzoxazol-2-on und 0.14 mL (0.80 mmol) DIPEA wurden in 3 mL DMF über Nacht bei RT gerührt. Der Ansatz wurde mittels präparativer HPLC-MS gereinigt. Die Produkt enthaltenden Fraktionen wurden vereinigt, das organische Lösungsmittel i.vac. entfernt und die zurückgebliebene wässrige Phase mit 4M wässriger NaOH-Lösung neutralisiert. Der Niederschlag wurde abgesaugt, mit Wasser nachgewaschen und i.vac. getrocknet.

| | |
|---|---|
| Ausbeute: | 150 mg (70% d. Th.) |
| ESI-MS: | m/z = 536 (M+H)⁺ |
| Rₜ (HPLC): | 1.0 min (Methode B) |

### Beispiel 33

### 3-[6'-Chlor-4'-(3,4-dimethyl-2-oxo-2,3-dihydro-benzoxazol-6-carbonyl)-3,4,5,6-tetrahydro-2H-[1,2']bipyridinyl-4-yl]-7-methoxy-1,3,4,5-tetrahydro-benzo[d][1,3]diazepin-2-on

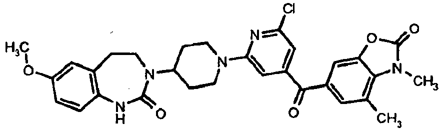

0.41 g (1.5 mmol) 7-Methoxy-3-piperidin-4-yl-1,3,4,5-tetrahydro-benzo[d][1,3]diazepin-2-on und 0.20 g (0.49 mmol) 6-(2,6-Dichlor-pyridin-4-carbonyl)3,4-dimethyl-3H-benzoxazol-2-on wurden in 3 mL NMP 3 h bei 120°C gerührt. Der Reaktionsansatz wurde mittels präparativer HPLC gereinigt. Die Produkt enthaltenden Fraktionen wurden vereinigt und gefriergetrocknet.

| | |
|---|---|
| Ausbeute: | 150 mg (53% d. Th.) |
| ESI-MS: | m/z = 576 / 78 (Cl) (M+H)⁺ |
| Rₜ (HPLC): | 1.67 min (Methode B) |

### Beispiel 34

### 3-{1-[6-Chlor-5-(3,4-dimethyl-2-oxo-2,3-dihydro-benzoxazol-6-carbonyl)-pyridazin-3-yl]-piperidin-4-yl}-7-methoxy-1,3,4,5-tetrahydro-benzo[d][1,3]diazepin-2-on

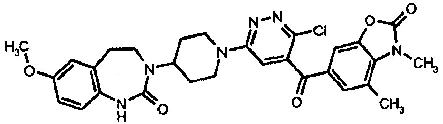

Zu 0.13 g (0.20 mmol) 3-{1-[6-Chlor-5-(3,4-dimethyl-2-oxo-2,3-dihydro-benzoxazol-6-carbonyl)-pyridazin-3-yl]-piperidin-4-yl}-7-methoxy-1,3,4,5-tetrahydro-benzo[d][1,3]di-azepin-2-on (Gemisch) in 2 mL Essigsäure wurden 0.10 g (1.0 mmol) Kaliumacetat gegeben und unter Stickstoffatmosphäre 16 h gekocht. Dann wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 5 mg (4% d. Th.) |
| Reinheit: | 90 % |
| ESI-MS: | m/z = 577 (M+H)⁺ |
| Rₜ (HPLC): | 3.67 min (Methode C) |

### Beispiel 35:

### 1-[4'-(4-Methyl-2-oxo-2,3-dihydro-benzoxazol-6-carbonyl)-3,4,5,6-tetrahydro-2H-[1,2']bipyridinyl-4-yl]-1,3-dihydro-imidazo[4,5-b]pyridin-2-on

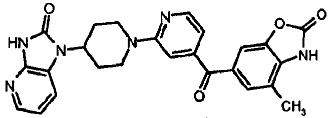

0.20 g (0.70 mmol) 1-Piperidin-4-yl-1,3-dihydroimidazo[4,5-b]pyridin-2-on-dihydrochlorid, 0.20 g (0.70 mmol) 6-(6-Chlorpyrimidin-4-carbonyl)-4-methyl-3H-benzoxazol-2-on und 0.30 g (2.2 mmol) Kaliumcarbonat wurden in 3.0 mL NMP über Nacht bei 130°C gerührt. Das Reaktionsgemisch wurde filtriert, das Filtrat mit wenig Wasser verdünnt und anschließend mittels präparativer HPLC-MS gereinigt. Die Produkt enthaltenden Fraktionen wurden vereinigt und gefriergetrocknet.

| | |
|---|---|
| Ausbeute: | 40 mg (12% d. Th.) |
| ESI-MS: | m/z = 471 (M+H)⁺ |
| R_{f}: | 0.62 (Kieselgel, DCM/Cyc/MeOH/ NH₄OH = 70/15/15/2) |

### Beispiel 36:

### 3-(3,4-Dimethyl-2-oxo-2,3-dihydro-benzoxazol-6-carbonyl)-5-[4-(7-methoxy-2-oxo-1,2,4,5-tetrahydro-benzo[d][1,3]diazepin-3-yl)piperidin-1-yl]-benzonitril

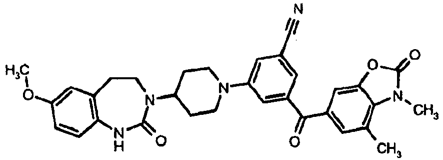

0.22 g (0.80 mmol) 7-Methoxy-3-piperidin-4-yl-1,3,4,5-tetrahydro-1,3-benzodiazepin-2-on und 0.12 g (0.40 mmol) 3-(3,4-Dimethyl-2-oxo-2,3-dihydro-benzoxazol-6-carbonyl)-5-fluor-benzonitril wurden vereinigt und ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und i.vac. getrocknet.

| | |
|---|---|
| Ausbeute: | 25 mg (11% d. Th.) |
| ESI-MS: | m/z =566 (M+H)⁺ |
| Rₜ (HPLC): | 1.56 min (Methode B) |

### Beispiel 37:

### 4-Methyl-6-[4-(2-oxo-2,3-dihydro-benzimidazol-1-yl)-3,4,5,6-tetrahydro-2H-[1,4']bipyridinyl-2'-carbonyl]-3H-benzoxazol-2-on

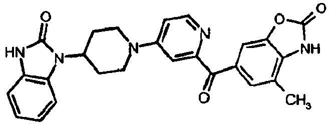

0.20 g (0.90 mmol) 1-Piperidin-4-yl-1,3-dihydro-benzimidazol-2-on, 0.25 g (0.90 mmol) 6-(4-Chlor-pyridin-2-carbonyl)-4-methyl-3H-benzoxazol-2-on und 0.13 g (0.90 mmol) Kaliumcarbonat wurden in 3 mL NMP über Nacht bei 130°C gerührt. Der Feststoff wurde abfiltriert, das Filtrat mit DMF verdünnt und mittels präparativer HPLC-MS gereinigt. Die Produkt enthaltenden Fraktionen wurden vereinigt und gefriergetrocknet.

| | |
|---|---|
| Ausbeute: | 20 mg (5% d. Th.) |
| ESI-MS: | m/z = 471 (M+H)⁺ |
| R_{f}: | 0.51 (Kieselgel, DCM/Cyc/MeOH/ NH₄OH = 70/15/15/2) |

### Beispiel 38

### 3-[4'-(3,4-Dimethyl-2-oxo-2,3-dihydro-benzoxazol-6-carbonyl)-5'-methyl-3,4,5,6-tetra-hydro-2H-[1,2']bipyridinyl-4-yl]-7-methoxy-1,3,4,5-tetrahydro-benzo[d][1,3]diazepin-2-on

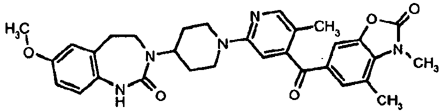

0.33 g (1.2 mmol) 7-Methoxy-3-piperidin-4-yl-1,3,4,5-tetrahydro-1,3-benzodiazepin-2-on und 0.13 g (0.40 mmol) 6-(2-Chlor-5-methyl-pyridin-4-carbonyl)-3,4-dimethyl-3H-benz-oxazol-2-on (Gemisch) wurden vereinigt und ca. 10 min auf 300°C erhitzt. Anschließend wurde der Ansatz in DMF gelöst und mittels präparatlver HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser gewaschen und i.vac. getrocknet.

| | |
|---|---|
| Ausbeute: | 40 mg (18% d. Th.) |
| ESI-MS: | m/z = 556 (M+H)⁺ |
| Rₜ (HPLC): | 1.51 min (Methode B) |

### Beispiel 39

### 3-{1-[3-(3,4-Dimethyl-2-oxo-2,3-dihydro-benzoxazol-6-carbonyl-5-fluor-phenyl]-piperidin-4-yl}-7-methoxy-1,3,4,5-tetrahydro-benzo[d][1,3]diazepin-2-on

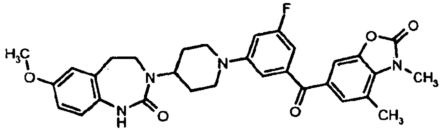

440 mg (1.60 mmol) 7-Methoxy-3-piperidin-4-yl-1,3,4,5-tetrahydro-1,3-benzodiazepin-2-on und 121 mg (0.40 mmol) 6-(3,5-Difluor-benzoyl)-3,4-dimethyl-3H-benzoxazol-2-on wurden vereinigt und mit der Hotgun ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und i.vac. getrocknet.

| | |
|---|---|
| Ausbeute: | 50 mg (22% d. Th.) |
| ESI-MS: | m/z =559 (M+H)⁺ |
| Rₜ (HPLC): | 1.77 min (Methode B) |

### Beispiel 40

### 1-[6'-Chlor-4'-(3,4-dimethyl-2-oxo-2,3-dihydro-benzoxazol-6-carbonyl)-3,4.5,6-tetrahydro-2H-[1,2']bipyridinyl-4-yl]-1,3-dihydro-imidazo[4,5-b]pyridin-2-on

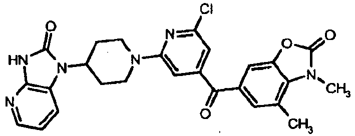

0.33 g (1.5 mmol) 1-Piperidin-4-yl-1,3-dihydroimidazo[4,5-b]pyridin-2-on und 0.20 g (0.49 mmol) 6-(2,6-Dichlor-pyridin-4-carbonyl)-3,4-dimethyl-3H-benzoxazol-2-on wurden in 3 mL NMP 3 h bei 120°C gerührt. Der Reaktionsansatz wurde mittels präparativer HPLC gereinigt. Die Produkt enthaltenden Fraktionen wurden vereinigt und gefriergetrocknet.

| | |
|---|---|
| Ausbeute: | 160 mg (63% d. Th.) |
| ESI-MS: | m/z = 519/21 (Cl) (M+H)⁺ |
| Rₜ (HPLC): | 1.54 min (Methode B) |

### Beispiel 41

### 7-Methoxy-3-{1-[6-(7-methyl-1H-indazol-5-carbonyl)-pyrimidin-4-yl]-piperidin-4-yl}-1,3,4,5-tetrahydro-benzo[d][1,3]diazepin-2-on

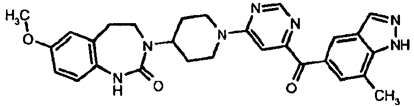

65 mg (0.10 mmol) 7-Methoxy-3-(1-{6-[7-methyl-1-(2-trimethylsilanyl-ethoxymethyl)-1H-indazol-5-carbonyl]-pyrimidin-4-yl}-piperidin-4-yl)-1,3,4,5-tetrahydro-benzo[d][1,3]diazepin-2-on in 0.4 mL MeOH und 0.6 mL (2.4 mmol) einer 4 molaren HCl-Lösung in Dioxan wurde über Nacht bei RT gerührt. Der Ansatz wurde mit 0.4 mL einer 6 molaren methanolischen Ammoniaklösung neutralisiert, mit etwas Wasser versetzt und mittels präparativer HPLC-MS gereinigt. Die Produkt enthaltenden Fraktionen wurden vereinigt und gefriergetrocknet.

| | |
|---|---|
| Ausbeute: | 16 mg (28% d. Th.) |
| Reinheit: | 90%ig |
| ESI-MS: | m/z = 512 (M+H)⁺ |
| Rₜ (HPLC): | 2.76 min (Methode C) |

### Beispiel 42

### 3-{1-[6-(3,4-Dimethyl-2-oxo-2,3-dihydro-benzoxazol-6-carbonyl)-pyrimidin-4-yl]-3-methyl-piperidin-4-yl}-7-methoxy-1,3,4,5-tetrahydro-benzo[d][1,3]diazepin-2-on

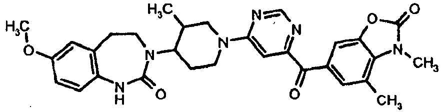

60 mg (0.21 mmol) 7-Methoxy-3-(3-methyl-piperidin-4-yl)-1,3,4,5-tetrahydro-benzo[d]-[1,3]diazepin-2-on, 60 mg (0.20 mmol) 6-(6-Chlor-pyrimidin-4-carbonyl)-3,4-dimethyl-3H-benzoxazol-2-on und 0.040 mL (0.29 mmol) TEA wurden in 1 mL DMF über Nacht bei RT gerührt. Anschließend wurde der Reaktionsansatz mit 2 mL MeOH versetzt, der entstandene Niederschlag abgesaugt, mit MeOH und Diethylether gewaschen und getrocknet.

| | |
|---|---|
| Ausbeute: | 80 mg (66% d. Th.) |
| ESI-MS: | m/z = 557 (M+H)⁺ |
| Rₜ (HPLC): | 1.41 min (Methode C) |

### Beispiel 43

### 4-(3,4-Dimethyl-2-oxo-2,3-dihydro-benzoxazol-6-carbonyl)-2-[4-(7-methoxy-2-oxo-1,2,4,5-tetrahydro-benzo[d][1,3]diazepin-3-yl)-piperidin-1-yl]-benzonitril

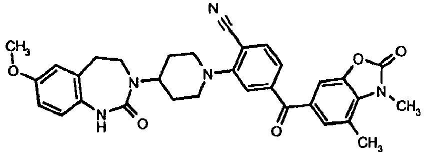

440 mg (1.60 mmol) 7-Methoxy-3-piperidin-4-yl-1,3,4,5-tetrahydro-1,3-benzodiazepin-2-on und 124 mg (0.400 mmol) 4-(3,4-Dimethyl-2-oxo-2,3-dihydro-benzoxazol-6-carbonyl)-2-fluor-benzonitril wurden vereinigt und ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und getrocknet.

| | |
|---|---|
| Ausbeute: | 125 mg (55% d. Th.) |
| ESI-MS: | m/z =566 (M+H)⁺ |
| Rₜ (HPLC): | 1.65 min (Methode B) |

### Beispiel 44

### 7-Methoxy-3-{1-[6-(4-methyl-2-oxo-2,3-dihydro-benzothiazol-6-carbonyl)-pyrimidin-4-yl]-piperidin-4-yl}-1,3,4,5-tetrahydro-benzo[d][1,3]diazepin-2-on

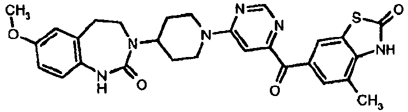

63 mg (0.23 mmol) 7-Methoxy-3-piperidin-4-yl-1,3,4,5-tetrahydro-benzo[d][1,3]diazepin-2-on, 71 mg (0.23 mmol) 6-(6-Chlor-pyrimidin-4-carbonyl)-4-methyl-3H-benzothiazol-2-on und 0.047 mL (0.27 mmol) DIPEA wurden in 2 mL DMF zusammen gegeben und über Nacht bei RT geschüttelt. Der Reaktionsansatz wurde in Acetonitril/Wasser aufgenommen und mittels präparativer HPLC gereinigt. Die Produkt enthaltenden Fraktionen wurden vereinigt und gefriergetrocknet.

| | |
|---|---|
| Ausbeute: | 25 mg (20% d. Th.) |
| ESI-MS: | m/z = 545 (M+H)⁺ |
| Rₜ (HPLC): | 3.48 min (Methode C) |

### Beispiel 45

### 3-(1-[3-(3,4-Dimethyl-2-oxo-2,3-dihydro-benzoxazol-6-carbonyl)-phenyl]-piperidin-4-yl}-7-methoxy-1,3,4,5-tetrahydro-benzo[d][1,3]diazepin-2-on

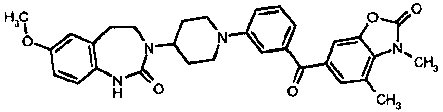

Unter einer Argonatmosphäre wurden 330 mg (1.20 mmol) 7-Methoxy-3-piperidin-4-yl-1,3,4,5-tetrahydro-benzo[d][1,3]diazepin-2-on, 350 mg (1.01 mmol) 6-(3-Brom-benzoyl)-3,4-dimethyl-3H-benzoxazol-2-on und 586 mg (1.80 mmol) Cäsiumcarbonat in 10 mL Xylol verrührt, anschließend mit 56 mg (0.090 mmol) BINAP und 20 mg (0.089 mmol) Palladium(I)acetat versetzt und 48 h bei 100°C gerührt. Der Ansatz wurde i.vac. eingeengt, der Rückstand in DMF/MeOH gelöst und mittels präparativer HPLC-MS gereinigt. Die Produkt enthaltenden Fraktionen wurden vereinigt und das Acetonitril eingeengt. Der wässrige Rückstand wurde mit 1 N wässriger Natronlauge basisch gestellt, der ausgefallene Niederschlag wurde abgesaugt und mit Wasser gewaschen und getrocknet. Das Produkt wurde nochmals mittels präparativer HPLC-MS gereinigt. Die Produkt enthaltenden Fraktionen wurden vereinigt und gefriergetrocknet.

| | |
|---|---|
| Ausbeute: | 7 mg (1 % d. Th.) |
| ESI-MS: | m/z = 541 (M+H)⁺ |
| Rₜ (HPLC): | 1.58 min (Methode B) |

### Beispiel 46

### 4-(3,4-Dimethyl-2-oxo-2,3-dihydro-benzoxazol-6-carbonyl)-2-[4-(2-oxo-2,3-dihydro-imidazo[4,5-b]pyridin-1-yl)-piperidin-1-yl]-benzonitril

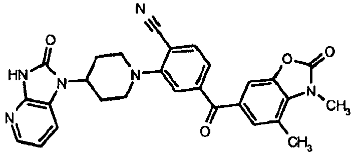

0.26 g (1.2 mmol) 1-Piperidin-4-yl-1,3-dihydroimidazo[4,5-b]pyridin-2-on und 93 mg (0.30 mmol) 4-(3,4-Dimethyl-2-oxo-2,3-dihydro-benzoxazol-6-carbonyl)-2-fluor-benzonitril wurden vereinigt und ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und getrocknet.

| | |
|---|---|
| Ausbeute: | 7 mg (5% d. Th.) |
| ESI-MS: | m/z = 509 (M+H)⁺ |
| Rₜ (HPLC): | 1.50 min (Methode B) |

### Beispiel 47

### 1-[4'-(3,4-Dimethyl-2-oxo-2,3-dihydro-benzoxazol-6-carbonyl)-6'-methoxy-3,4,5,6-tetra-hydro-2H-[1,2']bipyridinyl-4-yl]-1,3-dihydro-imidazo[4,5-b]pyridin-2-on

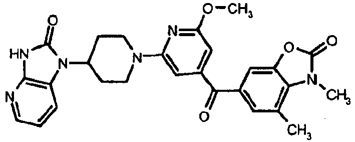

785 mg (3.60 mmol) 1-Piperidin-4-yl-1,3-dihydroimidazo[4,5-b]pyridin-2-on und 420 mg (1.26 mmol) 6-(2-Chlor-6-methoxy-pyridin-4-carbonyl)-3,4-dimethyl-3H-benzoxazol-2-on wurden in 3 mL NMP zusammen gegeben und 12 h bei 120°C gerührt. Der Ansatz wurde mittels präparativer HPLC-MS gereinigt. Die Produkt enthaltenden Fraktionen wurden vereinigt und das Acetonitril i.vac. entfernt. Der Rückstand wurde mit Wasser verdünnt, der ausgefallene Feststoff abgesaugt, mit Wasser gewaschen und getrocknet.

| | |
|---|---|
| Ausbeute: | 180 mg (10% d. Th.) |
| ESI-MS: | m/z = 515 (M+H)⁺ |
| Rₜ (HPLC): | 1.60 min (Methode B) |

### Beispiel 48

### 3-[4'-(3,4-Dimethyl-2-oxo-2,3-dihydro-benzoxazol-6-carbonyl)-6'-methoxy-3,4,5,6-tetra-hydro-2H-[1,2']bipyridinyl-4-yl]-7-methoxy-1,3,4,5-tetrahydro-benzo[d][1,3]diazepin-2-on

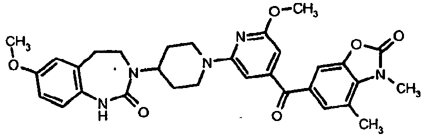

991 mg (3.60 mmol) 7-Methoxy-3-piperidin-4-yl-1,3,4,5-tetrahydro-benzo[d][1,3]diazepin-2-on und 420 mg (1.26 mmol) 6-(2-Chlor-6-methoxy-pyridin-4-carbonyl)-3,4-dimethyl-3H-benzoxazol-2-on wurden in 3 mL NMP zusammen gegeben und 12 h bei 120°C gerührt. Der Ansatz wurde mittels präparativer HPLC-MS gereinigt. Die Produkt enthaltenden Fraktionen wurden vereinigt und das Acetonitril i.vac. entfernt. Der Rückstand wurde mit Wasser verdünnt, der ausgefallene Feststoff abgesaugt, mit Wasser gewaschen und getrocknet.

| | |
|---|---|
| Ausbeute: | 290 mg (14% d. Th.) |
| ESI-MS: | m/z = 572 (M+H)⁺ |
| Rₜ (HPLC): | 1.70 min (Methode B) |

### Beispiel 49

### 3-{1-[6-(3,4-Dimethyl-2-oxo-2,3-dihydro-benzoxazol-6-carbonyl)-pyrimidin-4-yl]-piperidin-4-yl}-1H-chinolin-2-on

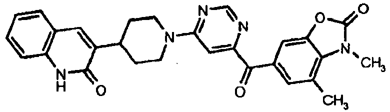

60 mg (0.26 mmol) 3-Piperidin-4-yl-1 H-chinolin-2-on, 80 mg (0.26 mmol) 6-(6-Chlorpyrimi-din-4-carbonyl)-3,4-dimethyl-3*H*-benzoxazol-2-on und 0.15 mL (0.86 mmol) DIPEA wurden in 3 mL DMF zusammen gegeben und bei RT über Nacht gerührt. Der Reaktionsansatz wurde auf Wasser gegeben und der entstandene Niederschlag wurde abgesaugt, mit MeOH nachgewaschen und getrocknet.

| | |
|---|---|
| Ausbeute: | 110 mg (80% d. Th.) |
| ESI-MS: | m/z = 496 (M+H)⁺ |
| Rₜ (HPLC): | 1.34 min (Methode B) |

### Beispiel 50:

### 3,4-Dimethyl-6-{6-[4-(5-oxo-3-phenyl-4,5-dihydro-[1,2,4]triazol-1-yl)piperidin-1-yl]-pyrimidin-4-carbonyl}-3H-benzoxazol-2-on

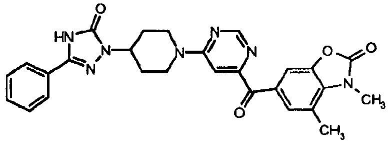

98 mg (0.40 mmol) 5-Phenyl-2-piperidin-4-yl-2,4-dihydro-[1,2,4]triazol-3-on, 0.12 g (0.40 mmol) 6-(6-Chlor-pyrimidin-4-carbonyl)-3,4-dimethyl-3H-benzoxazol-2-on und 0.14 mL (0.80 mmol) DIPEA wurden in 3 mL DMF zusammen gegeben und 48 h bei RT gerührt. Der Ansatz wurde mit MeOH verdünnt, abgesaugt, mit MeOH und Diethylether nachgewaschen und getrocknet.

| | |
|---|---|
| Ausbeute: | 158 mg (77% d. Th.) |
| ESI-MS: | m/z = 512 (M+H)⁺ |
| Rₜ (HPLC): | 1.27 min (Methode A) |

### Beispiel 51

### 7-Methoxy-3-{1-[6-(7-methyl-2-oxo-2,3-dihydro-1H-indol-5-carbonyl)-pyrimidin-4-yl]-piperidin-4-yl}-1,3,4,5-tetrahydro-benzo[d][1,3]diazepin-2-on

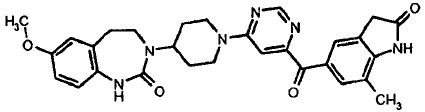

0.12 g (0.42 mmol) 7-Methoxy-3-piperidin-4-yl-1,3,4,5-tetrahydro-1,3-benzodiazepin-2-on, 0.12 g (0.42 mmol) 5-(6-Chlor-pyrimidin-4-carbonyl)-7-methyl-1,3-dihydro-indol-2-on und 0.08 mL (0.46 mmol) DIPEA wurden in 2 mL DMF zusammen gegeben und über Nacht bei RT geschüttelt. Anschließend wurde der Reaktionsansatz in Acetonitril/Wasser aufgenommen und mittels präparativer HPLC-MS gereinigt. Die Produkt enthaltenden Fraktionen wurden vereinigt und gefriergetrocknet.

| | |
|---|---|
| Ausbeute: | 30 mg (14% d. Th.) |
| ESI-MS: | m/z = 527 (M+H)⁺ |
| Rₜ (HPLC): | 1.18 min (Methode B) |

### Beispiel 52

### 1-{4'-(3,4-Dimethyl-2-oxo-2,3-dihydro-benzoxazol-6-carbonyl)-5'-methyl-3,4,5,6-tetra-hydro-2H-[1,2']bipyridin-4-yl]-1,3-dihydro-imidazo[4,5-b]pyridin-2-on

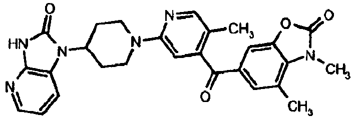

0.26 g (1.2 mmol) 1-Piperidin-4-yl-1,3-dihydroimidazo[4,5-b]pyridin-2-on und 0.13 g (0.40 mmol) 6-(2-Chlor-5-methyl-pyridin-4-carbonyl)-3,4-dimethyl-3H-benzoxazol-2-on (Gemisch) wurden vereinigt und ca. 10 min auf 250°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und getrocknet.

| | |
|---|---|
| Ausbeute: | 52 mg (26% d. Th.) |
| ESI-MS: | m/z =499 (M+H)⁺ |
| Rₜ (HPLC): | 1.34 min (Methode B) |

### Beispiel 53

### 3-{1-[6-(3,4-Dimethyl-2-oxo-2,3-dihydro-benzoxazol-6-carbonyl)-pyrimidin-4-yl]-piperidin-4-yl}-6-methoxy-1H-chinolin-2-on

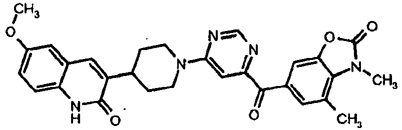

30 mg (0.087 mmol) 6-Methoxy-3-piperidin-4-yl-1 H-quinolin-2-on, 30 mg (0.10 mmol) 6-(6-Chlor-pyrimidin-4-carbonyl)-3,4-dimethyl-3H-benzoxazol-2-on und 50 µl (0.36 mmol) TEA wurden in 3 mL DMF zusammen gegeben und über Nacht bei RT gerührt. Anschließend wurde der Reaktionsansatz auf Wasser gegeben, der ausgefallene Niederschlag abgesaugt und mit MeOH nachgewaschen. Im Anschluss wurde der Niederschlag in DMF/MeOH verkocht und der Niederschlag abgesaugt.

| | |
|---|---|
| Ausbeute: | 12 mg (24% d. Th.) |
| ESI-MS: | m/z = 526 (M+H)⁺ |
| Rₜ (HPLC): | 1.33 min (Methode B) |

### Beispiel 54

### 3-{1-[6-(4-Hydroxy-3,5-dimethyl-benzoyl)-pyrimidin-4-yl]-piperidin-4-yl}-7-methoxy-1,3,4,5-tetrahydro-benzo[d][1,3]diazepin-2-on

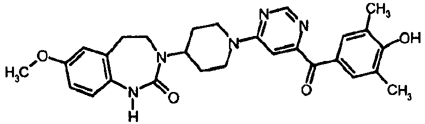

70 mg (0.10 mmol) 3-{1-[6-(4-Benzyloxy-3,5-dimethyl-benzoyl)-pyrimidin-4-yl]-piperidin-4-yl}-7-methoxy-1,3,4,5-tetrahydro-benzo[d][1,3]diazepin-2-on und 20 mg Palladium auf Kohle (Pd/C 10%) in 10 mL MeOH wurden 30 min bei RT in einer Wasserstoffatmosphäre von 10 psi hydriert. Nach Filtration des Reaktionsgemisches wurde das Lösungsmittel i.vac. entfernt und der Rückstand mittels präparativer HPLC-MS gereinigt. Die Produkt enthaltenden Fraktionen wurden vereinigt und gefriergetrocknet.

| | |
|---|---|
| Ausbeute: | 11 mg (21 % d. Th.) |
| ESI-MS: | m/z = 502 (M+H)⁺ |
| Rₜ (HPLC): | 3.0 min (Methode C) |

### Beispiel 55

### 1-{1-[6-(4-Methyl-2-oxo-2,3-dihydro-benzothiazol-6-carbonyl)-pyrimidin-4-yl]-piperidin-4-yl}-1,3-dihydro-imidazo[4,5-]pyridin-2-on

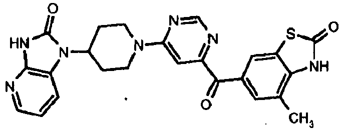

50 mg (0.23 mmol) 1-Piperidin-4-yl-1,3-dihydroimidazo[4,5-b]pyridin-2-on, 70.6 mg (0.23 mmol) 6-(6-Chlor-pyrimidin-4-carbonyl)-4-methyl-3H-benzothiazol-2-on und 0.05 mL (0.27 mmol) DIPEA wurden in 2 mL DMF zusammen gegeben und über Nacht bei RT geschüttelt. Der Reaktionsansatz wurde in Acetonitril/Wasser aufgenommen und mittels präparativer HPLC gereinigt. Die Produkt enthaltenden Fraktionen wurden vereinigt und gefriergetrocknet.

| | |
|---|---|
| Ausbeute: | 12 mg (11% d. Th.) |
| ESI-MS: | m/z = 488 (M+H)⁺ |
| Rₜ (HPLC): | 2.80 min (Methode C) |

### Beispiel 56

### 1-{1-[6-(4-Hydroxy-3,5-dimethyl-benzoyl)-pyrimidin-4-yl]-piperidin-4-yl}-1,3-dihydro-imidazo[4,5-b]pyridin-2-on

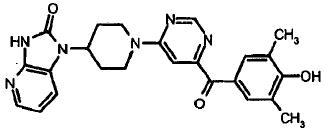

Zu 390 mg (0.7 mmol) 1-{1-[6-(4-Benzyloxy-3,5-dimethyl-benzoyl)-pyrimidin-4-yll-piperidin-4-yl}-1,3-dihydro-imidazo[4,5-b]pyridin-2-on in 50 mL MeOH wurden 50 mg Palladium/Kohle (10 %) gegeben und 40 min in einer 5 psi Wasserstoffatmosphäre bei RT hydriert. Der Katalysator wurde abgesaugt und das Filtrat eingeengt. Der Rückstand wurde mit etwas MeOH verrieben, abgesaugt und mit Diethylether nachgewaschen. Der abgesaugte Katalysator wurde mit 120 mL MeOH 1 h ausgekocht, heiß filtriert und das Filtrat eingeengt. Der Rückstand wurde mit wenig MeOH verrieben, abgesaugt und getrocknet. Die beiden Feststoffchargen wurden vereinigt und mittels präparativer HPLC-MS gereinigt. Die Produkt enthaltenden Fraktionen wurden vereinigt und gefriergetrocknet.

| | |
|---|---|
| Ausbeute: | 30 mg (10% d. Th.) |
| ESI-MS: | m/z = 445 (M+H)⁺ |
| Rₜ (HPLC): | 2.48 min (Methode C) |

### Beispiel 57

### 3-{1-[6-(1,7-Dimethyl-1H-indazol-5-carbonyl)-pyrimidin-4-yl]-piperidin-4-yl}-7-methoxy-1,3,4,5-tetrahydro-benzo[d][1,3]diazepin-2-on

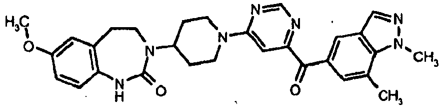

33 mg (0.12 mmol) 7-Methoxy-3-piperidin-4-yl-1,3,4,5-tetrahydro-1,3-benzodiazepin-2-on, 45 mg (0.13 mmol) (6-Chlor-pyrimidin-4-yl)-(1,7-dimethyl-1H-indazol-5-yl)-methanon und 25 µL (0.18 mmol) TEA wurden in 1 mL DMF zusammen gegeben und über Nacht bei RT gerührt. Der Reaktionsansatz wurde mittels präparativer HPLC gereinigt. Die Produkt enthaltenden Fraktionen wurden vereinigt und auf ca. 10 mL eingeengt. Dieser Rückstand wurde mit gesättigter Natriumhydrogencarbonat-Lösung neutralisiert, der entstandene Niederschlag abgesaugt und getrocknet.

| | |
|---|---|
| Ausbeute: | 30 mg (48% d. Th.) |
| ESI-MS: | m/z = 526 (M+H)⁺ |
| Rₜ (HPLC): | 3.11 min (Methode C) |

### Beispiel 58

### 3-{1-[6-(3-Ethyl-4-methyl-2-oxo-2,3-dihydro-benzoxazol-6-carbonyl)-pyrimidin-4-yl]-piperidin-4-yl}-7-methoxy-1.3.4.5-tetrahydro-benzo[d][1,3]diazepin-2-on

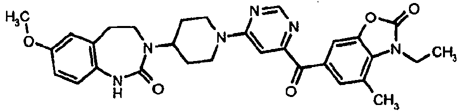

44 mg (0.20 mmol) 7-Methoxy-3-piperidin-4-yl-1,3,4,5-tetrahydro-benzo[d][1,3]diazepin-2-on, 50 mg (0.20 mmol) 6-(6-Chlor-pyrimidin-4-carbonyl)-3-ethyl-4-methyl-3H-benzoxazol-2-on und 56 µL (0.30 mmol) DIPEA wurden in 2 mL DMF zusammen gegeben und über Nacht bei RT gerührt. Der Reaktionsansatz wurde mittels präparativer HPLC gereinigt. Die Produkt enthaltenden Fraktionen wurden vereinigt, das organische Lösungsmittel wurde eingeengt und der Rückstand mit 1 N wäßriger Natronlauge neutralisiert. Der ausgefallene Niederschlag wurde abgesaugt, mit Wasser gewaschen und getrocknet.

| | |
|---|---|
| Ausbeute: | 43 mg (48% d. Th.) |
| ESI-MS: | m/z = 557 (M+H)⁺ |
| Rₜ (HPLC): | 3.41 min (Methode C) |

### Beispiel 59

### 7-Methoxy-3-{1-[6-(3-methyl-2-oxo-2,3-dihydro-benzoxazol-6-carbonyl)-pyrimidin-4-yl]-piperidin-4-yl}-1,3,4,5-tetrahydro-benzo[d][1,3]diazepin-2-on

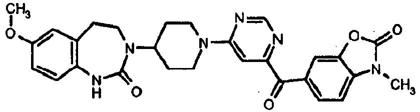

99 mg (0.40 mmol) 7-Methoxy-3-piperidin-4-yl-1,3,4,5-tetrahydro-1,3-benzodiazepin-2-on, 0.10 g (0.40 mmol) 6-(6-Chlor-pyrimidin-4-carbonyl)-3-methyl-3H-benzoxazol-2-on und 0.070 mL (0.40 mmol) DIPEA wurden in 2 mL DMF zusammen gegeben und über Nacht bei RT geschüttelt. Der Ansatz wurde mit Acetonitril/Wasser verdünnt und mittels präparativer HPLC-MS gereinigt. Die Produkt enthaltenden Fraktionen wurden vereinigt und gefriergetrocknet.

| | |
|---|---|
| Ausbeute: | 140 mg (74% d. Th.) |
| ESI-MS: | m/z = 529 (M+H)⁺ |
| Rₜ (HPLC): | 1.27 min (Methode B) |

### Beispiel 60

### 7-Methoxy-3-{1-{6-(2-oxo-2,3-dihydro-benzoxazol-6-carbonyl)-pyrimidin-4-yl]-piperidin-4-yl}-1,3,4,5-tetrahydro-benzo[d][1,3]diazepin-2-on

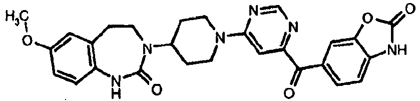

99 mg (0.40 mmol) 7-Methoxy-3-piperidin-4-yl-1,3,4,5-tetrahydro-1,3-benzodiazepin-2-on, 0.10 g (0.40 mmol) 6-(6-Chlor-pyrimidin-4-carbonyl)-3H-benzoxazol-2-on und 0.07 mL (0.40 mmol) DIPEA wurden in 2 mL DMF zusammen gegeben und über Nacht bei RT geschüttelt. Der Ansatz wurde mit Acetonitril/Wasser verdünnt und mittels präparativer HPLC-MS gereinigt. Die Produkt enthaltenden Fraktionen wurden vereinigt und gefriergetrocknet.

| | |
|---|---|
| Ausbeute: | 105 mg (57% d. Th.) |
| ESI-MS: | m/z = 513 (M-H)⁻ |
| Rₜ (HPLC): | 1.18 min (Methode B) |

### Beispiel 61

### 1-{1-[6-(7-Methyl-2-oxo-2,3-dihydro-1H-indol-5-carbonyl)-pyrimidin-4-yl]-piperidin-4-yl}-1,3-dihydro-imidazo[4,5-b]pyridin-2-on

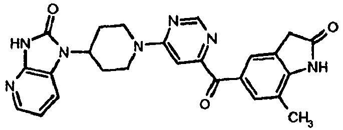

91 mg (0.42 mmol) 1-Piperidin-4-yl-1,3-dihydroimidazo[4,5-b]pyridin-2-on, 120 mg (0.42 mmol) 5-(6-Chlor-pyrimidin-4-carbonyl)-7-methyl-1,3-dihydro-indol-2-on und 0.080 mL (0.46 mmol) DIPEA wurden in 2 mL DMF zusammen gegeben und über Nacht bei RT geschüttelt. Anschließend wurde der Reaktionsansatz in Acetonitril/Wasser aufgenommen und mittels präparativer HPLC-MS gereinigt. Die Produkt enthaltenden Fraktionen wurden vereinigt und gefriergetrocknet.

| | |
|---|---|
| Ausbeute: | 33 mg (17% d. Th.) |
| ESI-MS: | m/z = 468 (M-H)⁻ |
| Rₜ (HPLC): | 1.07 min (Methode B) |

### Beispiel 62

### 1-{1-[6-(3,4,5-Trimethyl-benzoyl)-pyrimidin-4-yl]-piperidin-4-yl}-1,3-dihydro-imidazo[4,5-b]-pyridin-2-on

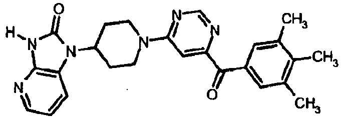

0.20 g (0.70 mmol) 1-Piperidin-4-yl-1,3-dihydroimidazo[4,5-b]pyridin-2-on-dihydrochlorid, 0.18 g (0.70 mmol) (6-Chlor-pyrimidin-4-yl)-(3,4,5-trimethyl-phenyl)-methanon und 0.40 mL (2.3 mmol) DIPEA wurden in 10 mL DMF zusammen gegeben und über Nacht bei RT gerührt. Das Reaktionsgemisch wurde i.vac. eingeengt, der Rückstand mit Wasser versetzt und 10 min nachgerührt. Der Niederschlag wurde abgesaugt und mittels präparativer HPLC-MS gereinigt. Die Produkt enthaltenden Fraktionen wurden vereinigt und gefriergetrocknet.

| | |
|---|---|
| Ausbeute: | 30 mg (10% d. Th.) |
| ESI-MS: | m/z = 443 (M+H)⁺ |
| R_{f}: | 0.66 (Kieselgel, DCM/Cyc/MeOH/ NH₄OH = 70/15/15/2) |

### Beispiel 63

### 1-[6'-Benzyloxy-4'-(3,4-dimethyl-2-oxo-2,3-dihydro-benzoxazol-6-carbonyl)-3,4,5,6-tetra-hydro-2H-[1,2']bipiridinyl-4-yl]-1,3-dihydro-imidazo[4,5-b]pyridin-2-on

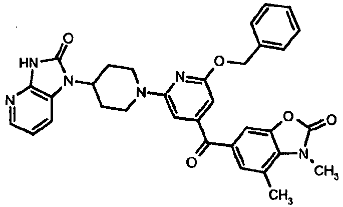

785 mg (3.60 mmol) 1-Piperidin-4-yl-1,3-dihydroimidazo[4,5-b]pyridin-2-on und 500 mg (1.22 mmol) 6-(2-Benzyloxy-6-chlor-pyridin-4-carbonyl)-3,4-dimethyl-3H-benzoxazol-2-on (Gemisch) wurden in 5 mL NMP zusammen gegeben und über Nacht bei 120°C gerührt. Der Reaktionsansatz wurde mittels präparativer HPLC gereinigt. Die Produkt enthaltenden Fraktionen wurden vereinigt und eingeengt. Der Rückstand wurde mit DIPE verrieben, abgesaugt, mit DIPE nachgewaschen und getrocknet.

| | |
|---|---|
| Ausbeute: | 300 mg (14% d. Th.) |
| ESI-MS: | m/z = 591 (M+H)⁺ |
| Rₜ (HPLC): | 1.70 min (Methode B) |

### Beispiel 64

### 7-Methoxy-3-{1-[6-(3-methyl-2-oxo-4-pyrazol-1-ylmethyl-2,3-dihydro-benzoxazol-6-carbonyl)-pyrimidin-4-yl]-piperidin-4-yl}-1,3,4,5-tetrahydro-benzo[d][1,3]diazepin-2-on

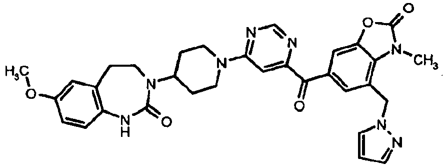

140 mg (0.508 mmol) 7-Methoxy-3-piperidin-4-yl-1,3,4,5-tetrahydro-1,3-benzodiazepin-2-on, 210 mg (0.284 mmol) 6-(6-Chlor-pyrimidin-4-carbonyl)-3-methyl-4-pyrazol-1-yl-methyl-3H-benzoxazol-2-on und 0.100 mL (0.581 mmol) DIPEA wurden in 1.0 mL DMF zusammen gegeben und 48 h bei RT gerührt. Anschließend wurde der Reaktionsansatz mehrmals mittels präparativer HPLC-MS gereinigt. Die Produkt enthaltenden Fraktionen wurden vereinigt und gefriergetrocknet.

| | |
|---|---|
| Ausbeute: | 12 mg (6% d. Th.) |
| ESI-MS: | m/z = 609 (M+H)⁺ |
| Rₜ (HPLC): | 4.33 min (Methode D) |

### Beispiel 65

### 1-{1-[6-(1,7-Dimethyl-1H-imidazol-5-carbonyl)-pirimidin-4-yl]-piperidin-4-yl}-1,3-dihydro-imidazo[4,5-b]pyridin-2-on

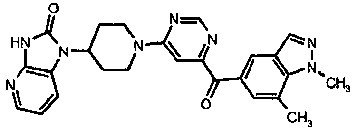

25 mg (0.12 mmol) 1-Piperidin-4-yl-1,3-dihydroimidazo[4,5-b]pyridin-2-on, 35 mg (0.10 mmol) (6-Chlor-pyrimidin-4-yl)-(1,7-dimethyl-1H-indazol-5-yl)-methanon und 25 µL (0.18 mmol) TEA wurden in 1 mL DMF zusammen gegeben und über Nacht bei RT gerührt. Der Reaktionsansatz wurde mittels präparativer HPLC gereinigt. Die Produkt enthaltenden Fraktionen wurden vereinigt und auf ca. 5 mL eingeengt. Dieser Rückstand wurde mit gesättigter Natriumhydrogencarbonat-Lösung neutralisiert, der entstandene Niederschlag abgesaugt und getrocknet.

| | |
|---|---|
| Ausbeute: | 20 mg (44% d. Th.) |
| ESI-MS: | m/z = 469 (M+H)⁺ |
| Rₜ (HPLC): | 2.63 min (Methode C) |

### Beispiel 66

### 7-Methoxy-3-{1-[6-(7-methyl-2,3-dihydro-benzofuran-5-carbonyl)-pyrimidin-4-yl-piperidin-4-yl}-1,3,4,5-tetrahydro-benzo[d][1,3]diazepin-2-on

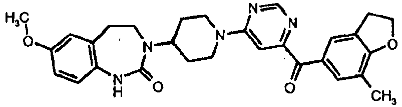

100 mg (0.363 mmol) 7-Methoxy-3-piperidin-4-yl-1,3,4,5-tetrahydro-1,3-benzodiazepin-2-on, 90.0 mg (0.229 mmol) (6-Chlor-pyrimidin-4-yl)-(7-methyl-2,3-dihydro-benzofuran 5-yl)-methanon und 0.200 mL (1.16 mmol) DIPEA wurden in 1.5 mL DMF zusammen gegeben und 48 h bei RT gerührt. Anschließend wurde der Reaktionsansatz mit wenigenTropfen Salzsäure versetzt und mittels präparativer HPLC-MS gereinigt. Die Produkt enthaltenden Fraktionen wurden vereinigt und gefriergetrocknet.

| | |
|---|---|
| Ausbeute: | 75 mg (64% d. Th.) |
| ESI-MS: | m/z = 514 (M+H)⁺ |
| Rₜ (HPLC): | 1.3 min (Methode B) |

### Beispiel 67

### 1-{1-[6-(3-Ethyl-4-methyl-2-oxo-2,3-dihydro-benzoxazol-6-carbonyl)-pyrimidin-4-yl]-piperidin-4-yl}-1,3-dihydro-imidazo[4,5-b]pyridin-2-on

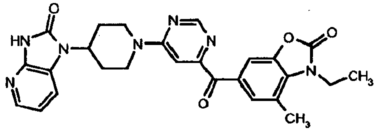

47 mg (0.20 mmol) 1-Piperidin-4-yl-1,3-dihydroimidazo[4,5-b]pyridin-2-on-dihydrochlorid, 50 mg (0.20 mmol) 6-(6-Chlor-pyrimidin-4-carbonyl)-3-ethyl-4-methyl-3H-benzoxazol-2-on und 0.11 mL (0.64 mmol) DIPEA wurden in 2.0 mL DMF zusammen gegeben und über Nacht bei RT gerührt. Der Reaktionsansatz wurde mittels präparativer HPLC gereinigt. Die Produkt enthaltenden Fraktionen wurden vereinigt, das organische Lösungsmittel wurde eingeengt und der Rückstand mit 1 N wäßriger Natronlauge neutralisiert. Der ausgefallene Niederschlag wurde abgesaugt, mit Wasser gewaschen und getrocknet.

| | |
|---|---|
| Ausbeute: | 47 mg (59% d. Th.) |
| ESI-MS: | m/z = 500 (M+H)⁺ |
| Rₜ (HPLC): | 2.89 min (Methode C) |

### Beispiel 68

### 7-Methoxy-3-{1-[6-(1,3,3,7-tetramethyl-2,3-dihydro-1H-indol-5-carbonyl)-pyrimidin-4-yl]-piperidin-4-yl)-1,3,4,5-tetrahydro-benzo[d][1,3]diazepin-2-on

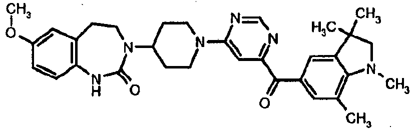

48 mg (0.17 mmol) 7-Methoxy-3-piperidin-4-yl-1,3,4,5-tetrahydro-1,3-benzodiazepin-2-on, 55 mg (0.17 mmol) (6-Chloro-pyrimidin-4-yl)-(1,3,3,7-tetramethyl-2,3-dihydro-1H-indol-5-yl)-methanon und 0.050 mL (0.29 mmol) DIPEA wurden in 2 mL DMF zusammen gegeben und über Nacht bei 40°C gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 50 mg (52% d. Th.) |
| ESI-MS: | m/z = 555 (M+H)⁺ |
| Rₜ (HPLC): | 1.39 min (Methode B) |

### Beispiel 69

### 3-{1-[6-(3,4-Dimethyl-2-oxo-2,3-dihydro-benzoxazol-6-carbonyl)-pyrimidin-4-yl]-piperidin-4-yl}-9-fluor-1,3,4,5-tetrahydro-benzo[d][1,3]diazepin-2-on

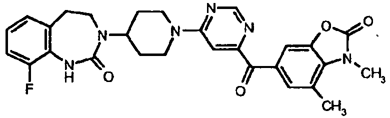

61 mg (0.23 mmol) 9-Fluor-3-piperidin-4-yl-1,3,4,5-tetrahydro-benzo[d][1,3]diazepin-2-on, 70 mg (0.23 mmol) 6-(6-Chlorpyrimidin-4-carbonyl)-3,4-dimethyl-3H-benzoxazol-2-on und 0.080 mL (0.46 mmol) DIPEA wurden in 2 mL DMF zusammen gegeben und 2 h bei RT gerührt. Anschließend wurde der Reaktionsansatz mit MeOH verdünnt, der ausgefallene Niederschlag abgesaugt, mit MeOH und Diethylether nachgewaschen und getrocknet.

| | |
|---|---|
| Ausbeute: | 78 mg (64% d. Th.) |
| ESI-MS: | m/z = 531 (M+H)⁺ |
| Rₜ (HPLC): | 1.38 min (Methode B) |

### Beispiel 70

### 3-{1-[5-(3,4-Dimethyl-2-oxo-2,3-dihydro-benzoxazol-6-carbonyl)-2-fluor-phenyl]-piperidin-4-yl}-7-methoxy-1,3,4,5-tetrahydro-benzo[d][1,3]diazepin-2-on

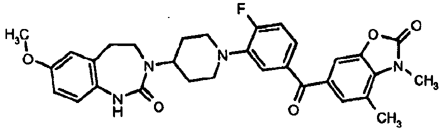

Unter einer Argonatmosphäre wurden 330 mg (1.20 mmol) 7-Methoxy-3-piperidin-4-yl-1,3,4,5-tetrahydro-benzo[d][1,3]diazepin-2-on, 437 mg (1.20 mmol) 6-(3-Brom-4-fluor-benzoyl)-3,4-dimethyl-3H-benzoxazol-2-on und 586 mg (1.80 mmol) Cäsiumcarbonat in 10 mL Dioxan zusammengegeben, anschließend mit 75 mg (0.12 mmol) BINAP und 27 mg (0.12 mmol) Palladium(II)acetat versetzt und 48 h bei 100°C gerührt. Der Ansatz wurde eingeengt, der Rückstand in DMF/MeOH gelöst und mittels präparativer HPLC-MS gereinigt. Die Produkt enthaltenden Fraktionen wurden vereinigt und das Acetonitril eingeengt. Der wässrige Rückstand wurde mit 1 N wässriger Natronlauge basisch gestellt, der ausgefallene Niederschlag wurde abgesaugt und mit Wasser gewaschen und getrocknet. Da das Produkt noch verunreinigt war, wurde anschließend nochmals mittels präparativer HPLC-MS gereinigt.

| | |
|---|---|
| Ausbeute: | 40 mg (6% d. Th.) |
| ESI-MS: | m/z = 559 (M+H)⁺ |
| Rₜ (HPLC): | 4.21 min (Methode C) |

### Beispiel 71

### 1-{1-[6-(7-Methyl-2,3-dihydro-benzofuran-5-carbonyl)-pyrimidin-4-yl]-piperidin-4-yl}-1,3-di-hydro-imidazo[4,5-b]pyridin-2-on-formiat

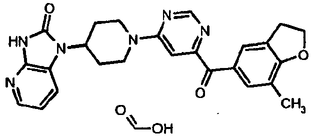

0.11 g (0.36 mmol) 1-Piperidin-4-yl-1,3-dihydroimidazo[4,5-b]pyridin-2-on-dihydrochlorid, 90 mg (0.23 mmol) (6-Chlor-pyrimidin-4-yl)-(7-methyl-2,3-dihydro-benzofuran-5-yl)-methanon und 0.20 mL (1.16 mmol) DIPEA wurden in 1.5 mL DMF zusammen gegeben und 48 h bei RT gerührt. Anschließend wurde der Reaktionsansatz mit wenigenTropfen Salzsäure versetzt und mittels präparativer HPLC-MS gereinigt. Die Produkt enthaltenden Fraktionen wurden vereinigt und gefriergetrocknet.

| | |
|---|---|
| Ausbeute: | 60 mg (52% d. Th.) |
| ESI-MS: | m/z = 457 (M+H)⁺ |
| Rₜ (HPLC): | 1.18 min (Methode B) |

### Beispiel 72

### 1-{1-[6-(2-Oxo-2,3-dihydro-benzoxazol-6-carbonyl)-pyrimidin-4-yl]-piperidin-4-yl}-3-di-hydro-imidazo[4,5-b]pyridin-2-on

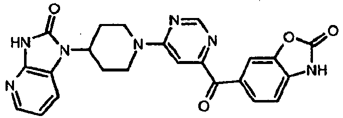

78 mg (0.40 mmol) 1-Piperidin-4-yl-1,3-dihydro-imidazo[4,5-b]pyridin-2-on, 0.10 g (0.40 mmol) 6-(6-Chlor-pyrimidin-4-carbonyl)-3H-benzoxazol-2-on und 0.070 mL (0.40 mmol) DIPEA wurden in 2 mL DMF zusammen gegeben und über Nacht bei RT geschüttelt. Der Ansatz wurde mit Acetonitril/Wasser verdünnt und mittels präparativer HPLC-MS gereinigt. Die Produkt enthaltenden Fraktionen wurden vereinigt und gefriergetrocknet.

| | |
|---|---|
| Ausbeute: | 98 mg (60% d. Th.) |
| ESI-MS: | m/z = 456 (M-H)⁻ |
| Rₜ (HPLC): | 1.02 min (Methode B) |

### Beispiel 73

### 7-Methoxy-3-{1-[6-(8-methyl-2,3-dihydro-benzo[1,4]dioxin-6-carbonyl)-pyrimidin-4-yl]-piperidin-4-yl}-1,3,4,5-tetrahydro-benzo[d][1,3]diazepin-2-on

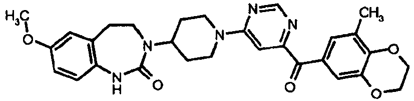

61 mg (0.22 mmol) 7-Methoxy-3-piperidin-4-yl-1,3,4,5-tetrahydro-1,3-benzodiazepin-2-on, 84 mg ,(0.22 mmol) (6-Iod-pyrimidin-4-yl)-(8-methyl-2,3-dihydro-benzo[1,4]dioxin-6-yl)-methanon und 0.08 mL (0.44 mmol) DIPEA wurden in 2.5 mL DMF zusammen gegeben und über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge versetzt, der ausgefallene Niederschlag wurde abgesaugt, mit Wasser nachgewaschen und getrocknet.

| | |
|---|---|
| Ausbeute: | 65 mg (56% d. Th.) |
| ESI-MS: | m/z = 530 (M+H)⁺ |
| Rₜ (HPLC): | 3.38 min (Methode B) |

### Beispiel 74

### 1-{1-[6-(8-Methyl-2,3-dihydro-benzo[1,4]dioxin-6-carbonyl)pyrimidin-4-yl]-piperidin-4-yl}-1,3-dihydro-imidazo[4,5-b]pyridin-2-on

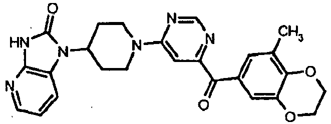

64 mg (0.22 mmol) 1-Piperidin-4-yl-1,3-dihydroimidazo[4,5-b]pyridin-2-on-dihydrochlorid, 84 mg (0.22 mmol) (6-Iod-pyrimidin-4-yl)-(8-methyl-2,3-dihydro-benzo[1,4]dioxin-6-yl)-methanon und 0.15 mL (0.88 mmol) DIPEA wurden in 2.5 mL DMF zusammen gegeben und über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge versetzt, der ausgefallene Niederschlag wurde abgesaugt, mit Wasser nachgewaschen und getrocknet.

| | |
|---|---|
| Ausbeute: | 70 mg (67% d. Th.) |
| ESI-MS: | m/z = 473 (M+H)⁺ |
| Rₜ (HPLC): | 2.85 min (Methode B) |

### Beispiel 75

### 1-{1-[6-(3-Methyl-2-oxo-2,3-dihydro-benzoxazol-6-carbonyl)-pyrimidin-4-yl]-piperidin-4-yl}-1,3-dihydro-Imidazo[4,5-b]pyridin-2-on

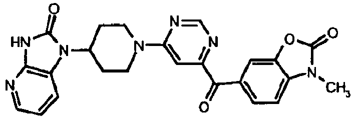

78 mg (0.40 mmol) 1-Piperidin-4-yl-1,3-dihydro-imidazo[4,5-b]pyridin-2-on, 0.10 g (0.40 mmol) 6-(6-Chlor-pyrimidin-4-carbonyl)-3-methyl-3H-benzoxazol-2-on und 0.070 mL (0.40 mmol) DIPEA wurden in 2 mL DMF zusammen gegeben und über Nacht bei RT geschüttelt. Der Ansatz wurde mit Acetonitril/Wasser verdünnt und mittels präparativer HPLC-MS gereinigt. Die Produkt enthaltenden Fraktionen wurden vereinigt und gefriergetrocknet.

| | |
|---|---|
| Ausbeute: | 126 mg (74% d. Th.) |
| ESI-MS: | m/z = 470 (M-H)⁻ |
| Rₜ (HPLC): | 1.1 min (Methode B) |

### Beispiel 76

### 7-Methoxy-3-{1-[6-(2-oxo-2,3-dihydro-1H-indol-5-carbonyl)-pyrimidin-4-yl]-piperidin-4-yl}-1,3,4,5-tetrahydro-benzo[d][1,3]diazepin-2-on

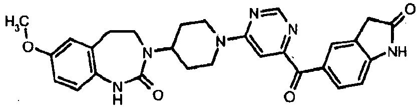

99 mg (0.40 mmol) 7-Methoxy-3-piperidin-4-yl-1,3,4,5-tetrahydro-benzo[d][1,3]diazepin-2-on, 98 mg (0.40 mmol) 5-(6-Chlor-pyrimidin-4-carbonyl)-1,3-dihydro-indol-2-on und 0.070 mL (0.40 mmol) DIPEA wurden in 2 mL DMF zusammen gegeben und über Nacht bei RT geschüttelt. Der Ansatz wurde mit Acetonitril/Wasser verdünnt und mittels präparativer HPLC-MS gereinigt. Die Produkt enthaltenden Fraktionen wurden vereinigt und gefriergetrocknet.

| | |
|---|---|
| Ausbeute: | 91 mg (49% d. Th.) |
| ESI-MS: | m/z = 511 (M-H)⁻ |
| Rₜ (HPLC): | 1.1 min (Methode B) |

### Beispiel 77

### 1-{1-[6-(1,3,3,7-Tetramethyl-2,3-dihydro-1H-indol-5-carbonyl)-pyrimidin-4-yl]-piperidin-4-yl}-1,3-dihydro-imidazo[4,5-b]pyridin-2-on

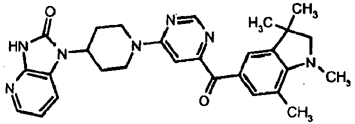

38 mg (0.17 mmol) 1-Piperidin-4-yl-1,3-dihydroimidazo[4,5-b]pyridin-2-on, 55 mg (0.17 mmol) (6-Chloro-pyrimidin-4-yl)-(1,3,3,7-tetramethyl-2,3-dihydro-1H-Indol-5-yl)-methanon und 0.050 mL (0.29 mmol) DIPEA wurden in 2 mL DMF zusammen gegeben und über Nacht bei 40°C gerührt. Anschließend wurde der Reaktionsansatz mittels präparativer HPLC-MS gereinigt. Die Produkt enthaltenden Fraktionen wurden vereinigt und gefriergetrocknet.

| | |
|---|---|
| Ausbeute: | 60 mg (69% d. Th.) |
| ESI-MS: | m/z = 498 (M+H)⁺ |
| Rₜ (HPLC): | 1.24 min (Methode B) |

### Beispiel 78

### 3-{1-[6-(3,4-Dimethyl-2-oxo-2,3-dihydro-benzoxazol-6-carbonyl)-pyrimidin-4-yl]-2-methyl-piperidin-4-yl}-7-methoxy-1,3,4,5-tetrahydro-benzo[d][1,3]diazepin-2-on

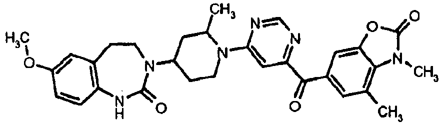

60 mg (0.21 mmol) 7-Methoxy-3-(2-methyl-piperidin-4-yl)-1,3,4,5-tetrahydro-benzo[d]-[1,3]diazepin-2-on, 60 mg (0.20 mmol) 6-(6-Chlorpyrimidin-4-carbonyl)-3,4-dimethyl-3H-benzoxazol-2-on und 0.040 mL (0.29 mmol) TEA wurden in 1 mL DMF zusammen gegeben und drei Tage bei RT gerührt. Anschließend wurde nochmals 7-Methoxy-3-(2-methyl-piperidin-4-yl)-1,3,4,5-tetrahydrobenzo[d][1,3]diazepin-2-on zugegeben und 3 h bei 80°C gerührt. Der Reaktionsansatz wurde mittels präparativer HPLC gereinigt. Die Produkt enthaltenden Fraktionen wurden vereinigt und das Lösungsmittel auf die Hälfte eingeengt. Der wässrige Rückstand wurde mit DCM extrahiert, die vereinten organischen Phasen wurden mit Natriumsulfat getrocknet, filtriert und das Filtrat eingeengt.

| | |
|---|---|
| Ausbeute: | 9 mg (8% d. Th.) |
| ESI-MS: | m/z = 557 (M+H)⁺ |
| Rₜ (HPLC): | 3.54 min (Methode C) |

### Beispiel 79

### 1-{1-[6-(1,3-Dimethyl-2-oxo-2,3-dihydro-1H-benzimidazol-5-carbonyl)-pyrimidin-4-yl]-piperidin-4-yl}1,3-dihydro-imidazo[4,5-b]pyridin-2-on

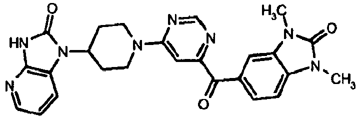

75 mg (0.34 mmol) 1-Piperidin-4-yl-1,3-dihydroimidazo[4,5-b]pyridin-2-on, 0.10 g (0.33 mmol) 5-(6-Chlor-pyrimidin-4-carbonyl)-1,3-dimethyl-1,3-dihydro-benzimidazol-2-on und 0.10 mL (0.58 mmol) DIPEA in 5 mL DMF wurden zusammen gegeben und über Nacht bei RT gerührt. Anschließend wurde der Reaktionsansatz eingeengt, der Rückstand mit Wasser versetzt und 10 min nachgerührt. Der entstandene Niederschlag wurde abgesaugt, in DMF gelöst und mittels präparativer HPLC-MS gereinigt. Die Produkt enthaltenden Fraktionen wurden vereinigt und gefriergetrocknet.

| | |
|---|---|
| Ausbeute: | 75 mg (45% d. Th.) |
| ESI-MS: | m/z = 485 (M+H)⁺ |
| R_{f}: | 0.59 (Kieselgel; DCM/Cyc/MeOH/ NH₄OH = 70/15/15/2) |

### Beispiel 80

### 3-{1-[6-(1,3-Dimethyl-2-oxo-2,3-dihydro-1H-benzoimidazol-5-carbonyl)-pyrimidin-4-yl]-piperidin-4-yl}-7-methoxy-3,4,5-tetrahydro-benzo[d][1,3]diazepin-2-on

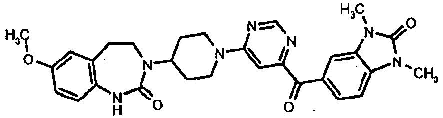

90 mg (0.34 mmol) 7-Methoxy-3-piperidin-4-yl-1,3,4,5-tetrahydro-1,3-benzodiazepin-2-on, 0.10 g (0.33 mmol) 5-(6-Chlor-pyrimidin-4-carbonyl)-1,3-dimethyl-1,3-dihydro-benzimidazol-2-on und 0.10 mL (0.58 mmol) DIPEA in 5 mL DMF wurden zusammen gegeben und über Nacht bei RT gerührt. Anschließend wurde der Reaktionsansatz eingeengt, der Rückstand mit Wasser versetzt und 10 min nachgerührt. Der entstandene Niederschlag wurde abgesaugt, in DMF gelöst und mittels präparativer HPLC-MS gereinigt. Die Produkt enthaltenden Fraktionen wurden vereinigt und gefriergetrocknet.

| | |
|---|---|
| Ausbeute: | 55 mg (31% d. Th.) |
| ESI-MS: | m/z = 542 (M+H)⁺ |
| R_{f}: | 0.64 (Kieselgel; DCM/Cyc/MeOH/ NH₄OH = 70/15/15/2) |

### Beispiel 81

### 3-[6'-Amino-4'-(3,4-dimethyl-2-oxo-2,3-dihydro-benzoxazol-6-carbonyl)-3,4,5,6-tetrahydro-2H-[1,2]bipyridinyl-4-yl]-7-methoxy-1,3,4,5-tetrahydro-benzo[d][1,3]diazepin-2-on

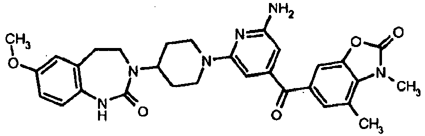

Zu 57 mg (0.10 mmol) [4'-(3,4-Dimethyl-2-oxo-2,3-dihydro-benzoxazol-6-carbonyl)-4-(7-methoxy-2-oxo-1,2,4,5-tetrahydro-benzo[d][1,3]diazepin-3-yl)-3,4,5,6-tetrahydro-2H-[1,2']bipyridinyl-6'-yl]-carbaminsäure-tert-butylester in 5.00 mL DCM wurden 0.50 mL TFA gegeben und 3 h bei RT gerührt. Dann wurden nochmals 0.50 mL TFA hinzugefügt und über Nacht bei RT gerührt. Dann wurde der Reaktionsansatz eingeengt, der Rückstand in DMF gelöst und mittels präparativer HPLC-MS gereinigt. Die Produkt enthaltenden Fraktionen wurden vereinigt und gefriergetrocknet.

| | |
|---|---|
| Ausbeute: | 4 mg (8% d. Th.) |
| ESI-MS: | m/z = 557 (M+H)⁺ |
| Rₜ (HPLC): | 1.30 min (Methode B) |

### Beispiel 82

### 3-{1-[6-(3,4-Dimethyl-2-oxo-2,3-dihydro-benzoxazol-6-carbonyl)-2-methyl-pyrimidin-4-yl]-3-fluor-piperidin-4-yl}-7-methoxy-1,3,4,5-tetrahydro-benzo[d][1,3]diazepin-2-on

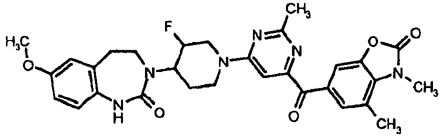

82 mg (0.28 mmol) 3-(3-Fluor-piperidin-4-yl)-7-methoxy-1,3,4,5-tetrahydro-benzo[d]-[1,3]diazepin-2-on, 85 mg (0.27 mmol) 6-(6-Chlor-2-methyl-pyrimidin-4-carbonyl)-3,4-di-methyl-3H-benzoxazol-2-on und 0.10 mL (0.56 mmol) DIPEA wurden in 2 mL DMF zusammen gegeben und über Nacht bei RT gerührt. Anschließend wurde der Reaktionsansatz mittels präparativer HPLC-MS gereinigt. Die Produkt enthaltenden Fraktionen wurden vereinigt und das organische Lösungsmittel eingeengt. Der Rückstand wurde mit 4N wässriger Natronlauge neutralisiert, der ausgefallene Niederschlag wurde abgesaugt, mit Wasser nachgewaschen und getrocknet.

| | |
|---|---|
| Ausbeute: | 64 mg (40% d. Th.) |
| ESI-MS: | m/z = 575 (M+H)⁺ |
| Rₜ (HPLC): | 1.32 min (Methode B) |

### Beispiel 83

### 7-Methoxy-3-[5'-methyl-4-(4-methyl-2-oxo-2,3-dihydro-benzoxazol-6-carbonyl)-3,4,5,6-tetrahydro-2H-[1,2']bipyridinyl-4-yl]-1,3,4,5-tetrahydro-benzo[d][1,3]diazepin-2-on

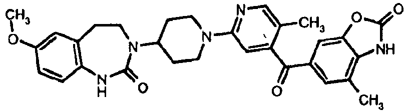

688 mg (2.50 mmol) 7-Methoxy-3-piperidin-4-yl-1,3,4,5-tetrahydro-1,3-benzodiazepin-2-on und 378 mg (1.25 mmol) 6-(2-Chlor-5-methyl-pyridin-4-carbonyl)-4-methyl-3H-benzoxa-zol-2-on (Gemisch) wurden vereinigt und ca. 10 min auf 300°C erhitzt. Anschließend wurde der Ansatz in DMF gelöst und mittels präparativer HPLC-MS gereinigt. Die Produkt enthaltenden Fraktionen wurden vereinigt und das organische Lösungsmittel eingeengt. Der Rückstand wurde mit 1 N wässriger Natronlauge neutralisiert, der ausgefallene Niederschlag wurde abgesaugt, mit Wasser nachgewaschen und getrocknet.

| | |
|---|---|
| Ausbeute: | 115 mg (17% d. Th.) |
| ESI-MS: | m/z = 542 (M+H)⁺ |
| Rₜ (HPLC): | 1.15 min (Methode B) |

### Beispiel 84

### 1-{4'-(3,4-Dimethyl-2-oxo-2,3-dihydro-benzoxazol-6-carbonyl)-6'-methylamino-3,4,5,6-tetrahydro-2H-[1,2']bipyridinyl-4-yl]-1,3-dihydro-imidazo[4,5-b]pyridin-2-on

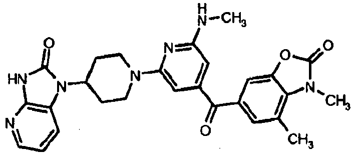

Zu 59 mg (0.096 mmol) [4'-(3,4-Dimethyl-2-oxo-2,3-dihydro-benzoxazol-6-carbonyl)-4-(2-oxo-2,3-dihydro-imidazo[4,5-b]pyridin-1-yl)-3,4,5,6-tetrahydro-2H-[1,2']bipyridinyl-6'-yl]-methyl-carbaminsäure-*tert*-butylester in 5.00 mL DCM wurden 1.00 mL TFA gegeben und 3 h bei RT gerührt. Dann wurde der Reaktionsansatz eingeengt, der Rückstand in DMF gelöst und mittels präparativer HPLC-MS gereinigt. Die Produkt enthaltenden Fraktionen wurden vereinigt und gefriergetrocknet.

| | |
|---|---|
| Ausbeute: | 3 mg (6% d. Th.) |
| ESI-MS: | m/z = 514 (M+H)⁺ |
| Rₜ (HPLC): | 1.28 min (Methode B) |

### Beispiel 85

### 1-[2'-(4-Methyl-2-oxo-2,3-dihydro-benzoxazol-6-carbonyl)-3,4,5,6-tetrahydro-2H-[1.4']bi pyridinyl-4-yl]-1,3-dihydro-imidazo[4,5-b]pyridin-2-on

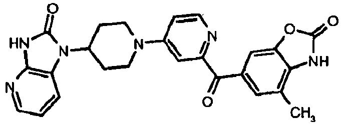

32 mg (0.11 mmol) 1-Piperidin-4-yl-1,3-dihydroimidazo[4,5-b]pyridin-2-on, 30 mg (0.10 mmol) 6-(4-Chlor-pyridin-2-carbonyl)-4-methyl-3H-benzoxazol-2-on und 45 mg (0.33 mmol) Kaliumcarbonat wurden in 1 mL NMP zusammen gegeben und über Nacht bei 130°C gerührt. Der Reaktionsansatz wurde filtriert, das Filtrat mit 1 mL DMF verdünnt und mittels präparativer HPLC-MS gereinigt. Die Produkt enthaltenden Fraktionen wurden vereinigt und gefriergetrocknet.

| | |
|---|---|
| Ausbeute: | 10 mg (21% d. Th.) |
| ESI-MS: | m/z = 471 (M+H)⁺ |
| R_{f}: | 0.49 (Kieselgel, DCM/Cyc/MeOH/NH₄OH = 70/15/15/2) |

Die nachfolgenden Beispiele beschreiben die Herstellung pharmazeutischer Anwendungsformen, die als Wirkstoff eine beliebige Verbindung der allgemeinen Formel **I** enthalten:

### Beispiele I

Kapseln zur Pulverinhalation mit 1 mg Wirkstoff

**Zusammensetzung:**

| 1 Kapsel zur Pulverinhalation enthält: | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

**Zusammensetzung:**

| | |
|---|---|
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

**Zusammensetzung:**

| 1 Fläschchen enthält: | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

**Zusammensetzung:**

| 1 Hub enthält: | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

**Zusammensetzung:**

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

**Zusammensetzung:**

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

**Zusammensetzung:**

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (WfI) auflösen; Human-Serum-Albumin zugeben; Wirkstoff unter Erwärmen auflösen; mit WfI auf Ansatzvolumen auffüllen; in Ampullen abfüllen.

### Beispiel VIII

Lyophilisat mit 10 mg Wirksubstanz

**Zusammensetzung:**

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (WfI) auflösen; Human-Serum-Albumin zugeben; Wirkstoff unter Erwärmen auflösen; mit Wfl auf Ansatzvolumen auffüllen; in Vials abfüllen; gefriertrocknen.

**Lösungsmittel für Lyophilisat:**

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (WfI) auflösen; in Ampullen abfüllen.

### Beispiel IX

Tabletten mit 20 mg Wirksubstanz

**Zusammensetzung:**

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

Kapseln mit 20 mg Wirksubstanz

**Zusammensetzung:**

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

**Zusammensetzung:**

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

**Zusammensetzung:**

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (WfI); Human-Serum-Albumin zugeben; Wirkstoff unter Erwärmen auflösen; mit WfI auf Ansatzvolumen auffüllen; unter Stickstoff-Begasung in Ampullen abfüllen.

## Patentansprüche

1. Verbindungen der allgemeinen Formel **I** 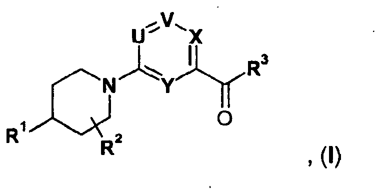 in der
**R¹** eine Gruppe der allgemeinen Formel 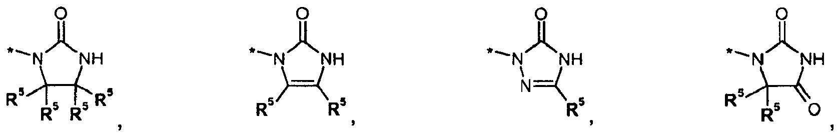 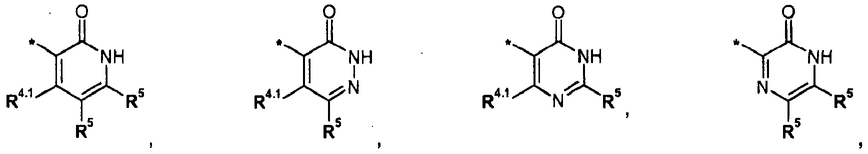 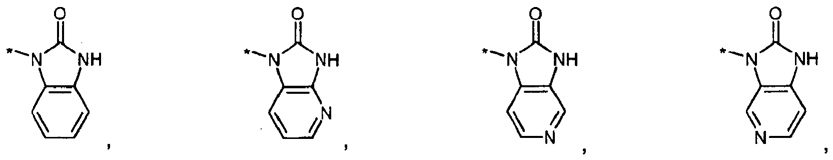 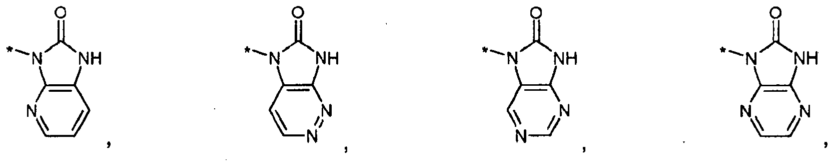 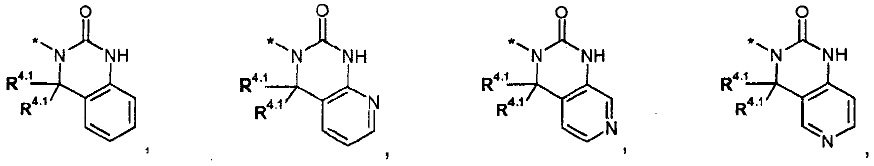 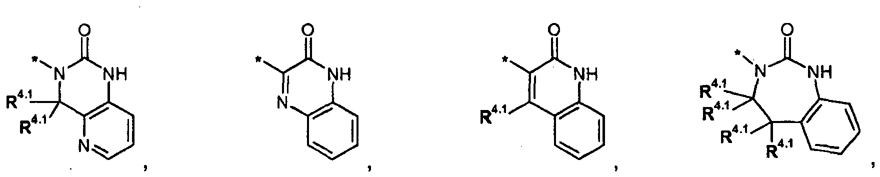 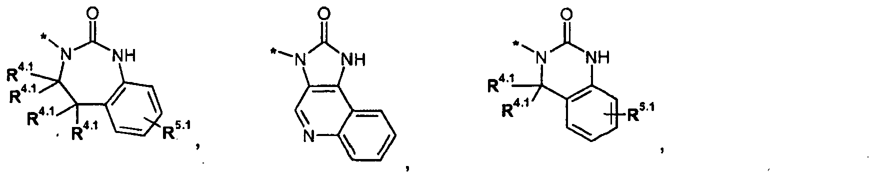
**R²**
(a) H,
(b) F, -CN, C₁₋₃-Alkyl, -CO₂-**R**^{2.1} oder
(c) eine C₁₋₃-Alkylgruppe, in der jede Methylengruppe mit bis zu zwei Fluoratomen und jede Methylgruppe mit bis zu drei Fluoratomen substituiert ist,
**R**^{**2**.1} H oder C₁₋₆-Alkyl,
**R³** eine mit den Resten **R**^{3.1}**,** R^{3.2} und **R^{3.3}** substituierte 6- oder 10-gliedrige Arylgruppe oder,
eine mit den Resten **R^{3.1}, R^{3.2}** und **R^{3.3}** substituierte 6-gliedrige Heteroarylgruppe, die über ein Kohlenstoffatom angebunden ist,
**R**^{3.1}
(a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, **R**^{3.1.1}-C₁₋₃-Alkylen, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R**^{3.1.2},
(f) -S(O)₂-**R**^{3.1.3},
**R**^{3.1.1}
(a) H,
(b) C₃₋₆-Cycloalkyl, C₅₋₆-Cycloalkenyl,
(c) (**R**^{3.1.1.1})₂N-,
(d) einen gesättigten, einfach oder zweifach ungesättigten 5- oder 6-gliedrigen Heterocyclus, der an einem Stickstoffatom mit einem Rest **R^{3.1.1.1}** und an einem Kohlenstoffatom mit einem oder zwei Resten **R^{3.1.1.2}** substituiert ist, oder
(e) eine Heteroarylgruppe, die an einem Kohlenstoffatom mit einem Rest **R**^{**3.1.1.**2} substituiert ist,
**R**^{3.1.1.1} unabhängig voneinander
(a) H, C₁₋₄-Alkyl, C₃₋₆-Cyloalkyl,
(b) Heterocyclyl,
(c) Aryl-C₀₋₃-alkylen oder Heteroaryl-C₀₋₃-alkylen,
**R^{3.1.1.2}** unabhängig voneinander
(a) H, F, C₁₋₃-Alkyl, -CN, -OH, -O-C₁₋₃-Alkyl, -CO(O)**R^{3.1.1.2.1}**, H₂N-, (C₁₋₄-Alkyl)-NH-, (C₁₋₄-Alkyl)₂N-,
(b) Phenyl oder Phenyl-CH₂-,
(c) eine C₁₋₃-Alkyl- oder -O-C₁₋₃-Alkylgruppe, in der jede Methylengruppe mit bis zu zwei Fluoratomen und jede Methylgruppe mit bis zu drei Fluoratomen substituiert ist, oder
**R^{3.1.1.2.1}** H, C₁₋₆-Alkyl, Benzyl,
**R^{3.1.2}** -O-C₁₋₃-Alkyl, -OH, -N**R**^{3.1.2.1}**R^{3.1.2.2},**
**R^{3.1.2.1}** H, C₁₋₃-Alkyl,
**R^{3.1.2.2}** H, C₁₋₃-Alkyl,
**R^{3.1.2.1}** und **R^{3.1.2.1}** zusammen auch einen Ring ausbilden können, der ausgewählt ist aus der Gruppe bestehend aus Azetidinyl, Pyrrolidinyl, Piperidinyl, Piperazinyl und Morpholinyl,
**R^{3.1.3}** -O-C₁₋₃-Alkyl, -N**R^{3.1.3.1}R^{3.1.3.2}**,
**R^{3.1.3.1}** H, C₁₋₃-Alkyl,
**R^{3.1.3.2}** H, C₁₋₃**-**Alkyl,
**R^{3.1.3.1}** und **R^{3.1.3.1}** zusammen auch einen Ring ausbilden können, der ausgewählt ist aus der Gruppe bestehend aus Azetidinyl, Pyrrolidinyl, Piperidinyl, Piperazinyl und Morpholinyl,
**R^{3.2}**
(a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.2.1}**,
(f) -S(O)₂-**R^{3.2.2}**,
**R^{3.2.1}** -O-C₁₋₃-Alkyl, -OH, **-**N**R^{3.2.1.1}R^{3.2.1.2},**
**R^{3.2.1.1}** H, C₁₋₃-Alkyl,
**R^{3.2.1.2}** H**,** C₁₋₃-Alkyl,
**R^{3.2.1.1}** und **R^{3.2.1.2}** zusammen auch einen Ring ausbilden können, der ausgewählt ist aus der Gruppe bestehend aus Azetidinyl, Pyrrolidinyl, Piperidinyl, Piperazinyl und Morpholinyl,
**R^{3.2.2}** -N**R^{3.2.2.1}R^{3.2.2.2}**,
**R^{3.2.2.1}** H, C₁₋₃-Alkyl,
**R^{3.2.2.2}** H, C₁₋₃-Alkyl,
**R**^{**3.2.2.**1} und **R^{3.2.2.2}** zusammen auch einen Ring ausbilden können, der ausgewählt ist aus der Gruppe bestehend aus Azetidinyl, Pyrrolidinyl, Piperidinyl, Piperazinyl und Morpholinyl,
**R^{3.3}**
(a) H,
(b) Halogen, -NH₂, C₁₋₄-Alkyl-NH-, (C₁₋₄-Alkyl)₂N-, C₁₋₃-Alkyl-C(O)-NH-, C₁₋₃-Alkyl-S(O₂)-NH-, -CN, -OH, -O-C(O)-NH-C₁₋₃-Alkyl,
(c) C₁₋₄-Alkyl, C₂₋₄-Alkenyl, C₂₋₄-Alkinyl, C₁₋₃-Alkyl-O-, C₁₋₃-Alkyl-S(O)ₘ-, Cyclopropyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
(e) -C(O)-**R^{3.3.1}**,
(f) -S(O)₂-**R^{3.3.2}**,
**R^{3.3.1}** -O-C₁₋₃-Alkyl, -OH, -N**R**^{3.3.1.1}**R^{3.3.1.2}**,
**R^{3.3.1.1}** H, C₁₋₃-Alkyl,
**R^{3.3.1.2}** H, C₁₋₃-Alky),
**R^{3.3.1.1}** und **R^{3.3.1.2}** zusammen auch einen Ring ausbilden können, der ausgewählt ist aus der Gruppe bestehend aus Azetidinyl, Pyrrolidinyl, Piperidinyl, Piperazinyl und Morpholinyl,
**R^{3.3.2}** -O-C₁₋₃-Alkyl, **-NR^{3.3.2.1}R^{3.3.2.2},**
**R^{3.3.2.1}** H, C₁₋₃-Alkyl,
**R^{3.3.2.2}** H, C₁₋₃-Alkyl,
**R^{3.3.2.1}** und **R^{3.3.2.2}** zusammen auch einen Ring ausbilden können, der ausgewählt ist aus der Gruppe bestehend aus Azetidinyl, Pyrrolidinyl, Piperidinyl, Piperazinyl und Morpholinyl, oder
**R^{3.2}** und **R³.³** zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
**R^{3.3.3}** unabhängig voneinander
(a) C₁₋₄-Alkyl oder
(b) C₃₋₆-Cycloalkyl,
**R^{3.3.4}** unabhängig voneinander
(a) C₁₋₄-Alkyl oder
(b) C₃₋₆-Cycloalkyl,
(c) Halogen, CN, -O-C₁₋₃-Alkyl, -NH₂,
(d) eine C₁₋₃-Alkyl- oder C₁₋₃-Alkyl-O-gruppe, in der jede Methylengruppe mit bis zu zwei und jede Methylgruppe mit bis zu drei Fluoratomen substituiert ist,
**R^{4.1}** unabhängig voneinander
(a) H,
(b) C₁₋₃-Alkyl,-OH, -O-C₁₋₃-Alkyl,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R⁵** unabhängig voneinander
(a) H, C₁₋₆-Alkyl, C₃₋₆-Cycloalkyl,
(b) eine gegebenenfalls mit 1, 2 oder 3 Substituenten **R^{5.2}** substituierte Phenylgruppe, wobei die Substituenten **R^{5.2}** gleich oder verschieden sein können,
(c) eine gegebenenfalls mit 1, 2 oder 3 Substituenten **R^{5.2}** substituierte Heteroarylgruppe, die ausgewählt ist aus der Gruppe bestehend aus Benzimidazol, Benzothiophen, Furan, Imidazol, Indol, Isoxazol, Oxazol, Pyrazin, Pyrazol, Pyridazin, Pyridin, Pyrimidin, Pyrrol, Thiazol, Thiophen und Triazol, wobei die Substituenten **R^{5.2}** gleich oder verschieden sein können,
(d) einen gegebenenfalls mit 1, 2 oder 3 Substituenten **R^{5.2}** substituierten Heterocyclus, wobei die Substituenten **R^{5.2}** gleich oder verschieden sein können,
**R^{5.1}**
(a) H, Halogen, C₁₋₆-Alkyl, C₃₋₆-Cycloalkyl,
(b) -O-C₁₋₆-Alkylen-N**R⁷R⁸**, -O-**R⁶**, -CO₂-**R⁶**, -C(O)-N**R⁷R⁸**, -SO₂-N**R⁷R⁸**, -N**R⁷**-SO₂-**R⁸**, -S(O)ₘ-**R⁷,** -CN, -N**R⁷R⁸**, -O-C(O)-**R⁶**,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{5.2}**
(a) Halogen, C₁₋₆-Alkyl, C₃₋₆-Cycloalkyl,
(b) -O-**R⁶**, -O-(CH₂)ₛ-O-**R⁶**, -CO₂**R⁶**, -S(O)ₘ-**R⁷,** -CN, -O-C(O)-**R⁶** oder
(c) eine C₁₋₃-Alkylgruppe, in der jede Methylengruppe mit bis zu zwei Fluoratomen und jede Methylgruppe mit bis zu drei Fluoratomen substituiert ist,
**R⁶**
(a) H,
(b) C₁₋₆-Alkyl, C₃₋₆-Cycloalkyl, Aryl, Heteroaryl, Benzyl, die mit einem Rest **R^{6.1}** substituiert sein können, oder
(c) eine C₁₋₃-Alkylgruppe, in der jede Methylengruppe mit bis zu zwei Fluoratomen und jede Methylgruppe mit bis zu drei Fluoratomen substituiert ist,
**R^{6.1}** HO- oder C₁₋₆-Alkyl-O-,
**R⁷**
(a) H,
(b) C₁₋₃-Alkyl, Phenyl oder Benzyl, wobei die Reste unsubstituiert oder substituiert sein können mit Halogen, HO- oder H₃C-O-,
**R⁸**
(a) H,
(b) C₁₋₃-Alkyl, Phenyl oder Benzyl, wobei die Reste unsubstituiert oder substituiert sein können mit Halogen, HO- oder H₃C-O-, oder
**R⁷** und **R⁸** zusammen auch einen Ring ausbilden können, der ausgewählt ist aus der Gruppe bestehend aus Azetidinyl, Pyrrolidinyl, Piperidinyl, Piperazinyl und Morpholinyl, wobei der Ring unsubstituiert oder mit einem Substituenten **R⁶** substituiert sein kann,
**m** eine der Ziffern 0, 1 oder 2,
**s** eine der Ziffern 1, 2 oder 3,
**U** N, N-Oxid oder C-**R⁹**,
**V** N, N-Oxid oder C-**R¹⁰**,
**X** N, N-Oxid oder C-**R¹¹**,
**Y** N oder C-**R¹²**,
wobei höchstens drei der voranstehend genannten Reste **U, V, X** oder **Y** gleichzeitig ein Stickstoffatom darstellen,
**R⁹**
(a) H,
(b) eine C₁₋₆-Alkyl- oder C₁₋₃-Alkyl-O-gruppe, die jeweils mit einem Rest **R^{9.1}** substituiert ist,
(c) **R^{9.2}R^{9.3}**N-, **R^{9.2}R^{9.3}**N-C₁₋₃-alkylen-,
(d) Halogen, -CN, -OH, -COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{9.1}** H, OH oder -O-CH₃,
**R^{9.2}** H oder C₁₋₃-Alkyl,
**R^{9.3}** H oder C₁₋₃-Alkyl, oder **R^{9.2}** und **R^{9.3}** zusammen mit dem Stickstoffatom, an das sie gebunden sind, einen 3- bis 6-gliedrigen Heterocyclus,
**R¹⁰**
(a) H,
(b) eine C₁₋₆-Alkyl- oder C₁₋₃-Alkyl-O-gruppe, die jeweils mit einem Rest **R^{10.1}** substituiert ist,
(c) -N**R^{10.2}R^{10.3}**, N**R^{10.2}R^{10.3}**-C₁₋₃-Alkylen-,
(d) Halogen, -CN, -OH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine Aryl-C₀₋₃-alkylen-O-gruppe,
(f) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{10.1}** H, OH oder -O-CH₃,
**R^{10.2}** H oder C₁₋₆-Alkyl,
**R^{10.3}** H, C₁₋₆-Alkyl oder -SO₂-C₁₋₃-Alkyl, oder
**R^{10.2}** und **R^{10.3}** zusammen mit dem Stickstoffatom, an das sie gebunden sind, einen 3- bis 6-gliedrigen Heterocyclus,
**R¹¹**
(a) H,
(b) eine C₁₋₆-Alkyl- oder C₁₋₃-Alkyl-O-gruppe, die jeweils mit einem Rest **R^{11.1}** substituiert ist,
(c) **R^{11.2}R^{11.3}**N-, **R^{11.2}R^{11.3}**N-C₁₋₃-alkylen-,
(d) Halogen, -CN, -OH, -COOH, C₁₋₃-Alkyl-O-C₁₋₃-alkylen-, C₃₋₆-Cycloalkyl-, C₃₋₆-Cycloalkyl-C₁₋₄-alkylen-, C₁₋₃-Alkyl-C(O)-O-C₁₋₃-alkylen,
(e) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{11.1}** H, OH oder -O-CH₃,
**R^{11.2}** H oder C₁₋₃-Alkyl,
**R^{11.3}** H oder C₁₋₃-Alkyl, oder
**R^{11.2}** und **R^{11.3}** zusammen mit dem Stickstoffatom, an das sie gebunden sind, einen 3- bis 6-gliedrigen Heterocyclus, und
**R¹²** H, Halogen oder C₁₋₃-Alkyl, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Verbindungen der allgemeinen Formel **I** gemäß Anspruch 1, in denen **U, V, X, Y, R²** und **R³** wie in Anspruch 1 definiert sind und
**R¹** eine Gruppe ausgewählt aus 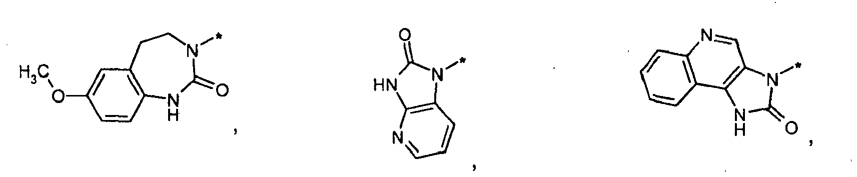 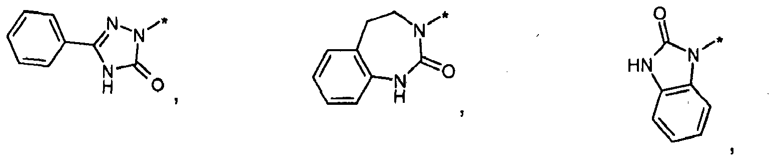 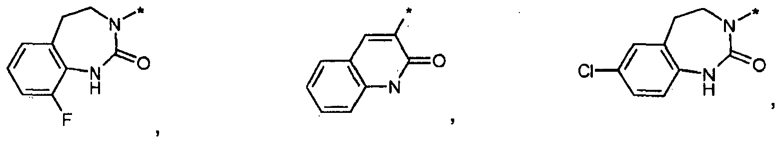
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel I gemäß Anspruch 1, in denen **U, V, X, Y, R¹** und **R³** wie in Anspruch 1 oder 2 definiert sind und **R²** ein Wasserstoffatom bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Verbindungen der allgemeinen Formel I gemäß Anspruch 1, in denen **U, V, X, Y, R**¹ und **R²** wie in Anspruch 1, 2 oder 3 definiert sind und
**R³** eine Gruppe der allgemeinen Formeln **III** 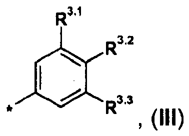
**R^{3.1}**
(a) H,
(b) F, Cl, Br, -NH₂, C₁₋₃-Alkyl-N,H-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}**
(a) H,
(b) F, Cl, Br, H₂N-, (C₁₋₄-Alkyl)-NH-, (C₁₋₄-Alkyl)₂N-, (C₁₋₃-Alkyl)-C(O)-NH-, -OH,
(c) C₁₋₄-Alkyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.3}**
(a) H,
(b) F, Cl, Br, H₂N-, (C₁₋₄-Alkyl)-NH-, (C₁₋₄-Alkyl)₂N-, (C₁₋₃-Alkyl)-C(O)-NH-, -OH,
(c) C₁₋₄-Alkyl,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, oder
**R^{3.2}** und **R^{3.3}** zusammen mit den Kohlenstoffatomen, an denen sie gebunden sind, einen einfach ungesättigten 5-gliedrigen oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
**R^{3.3.3}** unabhängig voneinander
(a) C₁₋₄-Alkyl oder
(b) C₃₋₆-Cycloalkyl, und
**R^{3.3.4}** unabhängig voneinander
(a) C₁₋₄-Alkyl, C₃₋₆-Cycloalkyl,
(b) Halogen, CN, C₁₋₃-Alkyl-O-, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. Verbindungen der allgemeinen Formel **I** gemäß Anspruch 1, in denen **U, V, X, Y, R¹** und **R²** wie in Anspruch 1, 2 oder 3 definiert sind und
**R³** eine Gruppe ausgewählt aus 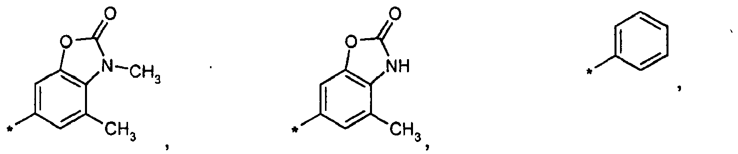 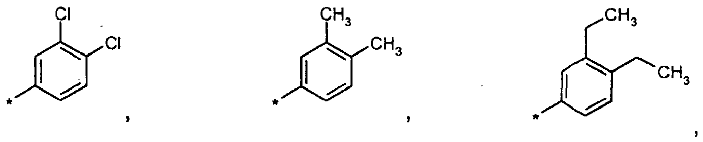 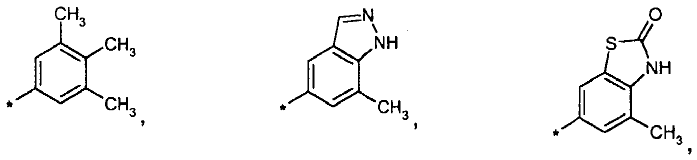 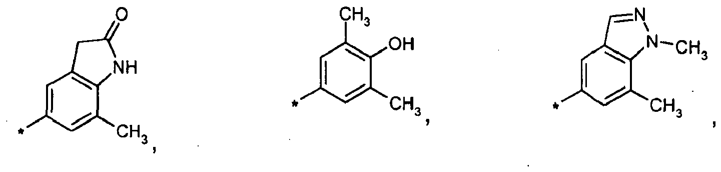 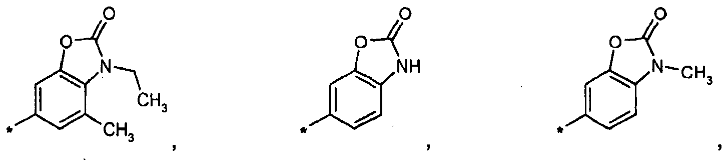 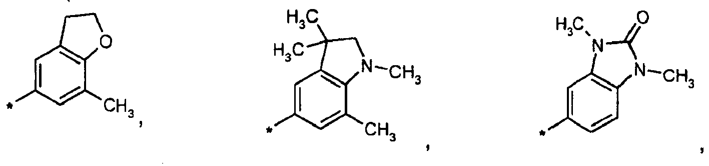 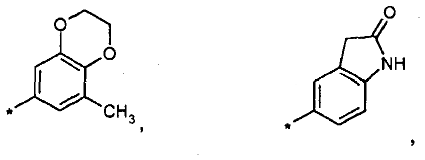 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. Verbindungen der allgemeinen Formel **I** gemäß Anspruch 1, in denen **U, V, X, Y, R**¹ und **R²** wie in Anspruch 1, 2 oder 3 definiert sind und
**R³** eine Gruppe der allgemeinen Formeln **III** 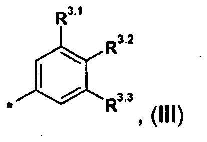
**R^{3.1}**
(a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}** und **R³.³** zusammen mit den Kohlenstoffatomen, an denen sie gebunden sind, einen einfach ungesättigten 5-gliedrigen Heterocyclus oder einen einfach oder zweifach ungesättigten 6-gliedrigen Heterocyclus oder eine 5- bis 6-gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
**R^{3.3.3}** unabhängig voneinander
(a) C₁₋₄-Alkyl- oder
(b) C₃₋₆-Cycloalkyl, und
**R^{3.3.4}** unabhängig voneinander
(a) C₁₋₄-Alkyl-, C₃₋₆-Cycloalkyl-,
(b) Halogen, -CN, -O-C₁₋₃-Alkyl, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

7. Verbindungen der allgemeinen Formel **I** gemäß Anspruch 1, in denen **U, V, X, Y, R**¹ und **R²** wie in Anspruch 1, 2 oder 3 definiert sind und
**R³** eine Gruppe der allgemeinen Formeln **III** 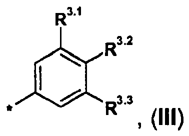
**R^{3.1}**
(a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist,
**R^{3.2}** und **R^{3.3}** zusammen mit den Kohlenstoffatomen, an denen sie gebunden sind, einen einfach ungesättigten 5-gliedrigen Heterocyclus oder eine 5- gliedrige Heteroarylgruppe bilden, wobei
die voranstehend erwähnten Heterocyclen eine Carbonyl-, Thiocarbonyl oder Cyaniminogruppe benachbart zu einem Stickstoffatom enthalten können, und
gegebenenfalls zusätzlich an einem oder zwei Stickstoffatomen durch jeweils einen Rest **R^{3.3.3}** substituiert sein können und
gegebenenfalls zusätzlich an einem oder zwei Kohlenstoffatomen durch jeweils einen oder zwei Reste **R^{3.3.4}** substituiert sein können,
**R^{3.3.3}** unabhängig voneinander
(a) C₁₋₄-Alkyl- oder
(b) C₃₋₆-Cycloalkyl, und
**R^{3.3.4}** unabhängig voneinander
(a) C₁₋₄-Alkyl-, C₃₋₆-Cycloalkyl-,
(b) Halogen, -CN, -O-C₁₋₃-Alkyl, -NH₂,
(c) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

8. Verbindungen der allgemeinen Formel I gemäß Anspruch 1, in denen **U, V, X, Y, R¹** und **R²** wie in Anspruch 1, 2 oder 3 definiert sind und
**R³** eine Gruppe der allgemeinen Formel **IIIa** 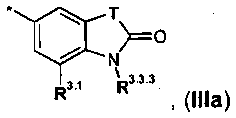
**T** O, S, CH₂, NH oder N-**R^{3.3.3}**,
**R**^{3.1}
(a) H,
(b) F, Cl, Br, -NH₂, C₁₋₃-Alkyl-NH-, (C₁₋₃-Alkyl)₂N-, C₁₋₃-Alkyl-C(O)-NH-, -CN, -OH,
(c) C₁₋₄-Alkyl, C₂₋₄-Alkenyl, C₂₋₄-Alkinyl, C₁₋₃-Alkyl-O-, C₁₋₃-Alkyl-S-,
(d) eine C₁₋₃-Alkyl- oder C₁₋₃-Alkyl-O-gruppe, in der jede Methylengruppe mit bis zu zwei Fluoratomen und jede Methylgruppe mit bis zu drei Fluoratomen substituiert ist, und
**R^{3.3.3}** unabhängig voneinander
(a) C₁₋₄-Alkyl- oder
(b) C₃₋₆-Cycloalkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

9. Verbindungen der allgemeinen Formel **I** gemäß Anspruch 1, in denen **U, V, X, Y, R¹** und **R²** wie in Anspruch 1, 2 oder 3 definiert sind und
**R³** eine Gruppe ausgewählt aus 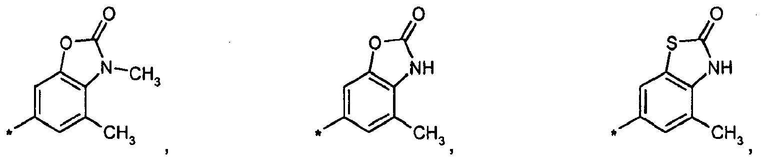 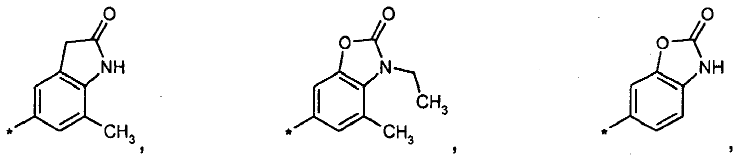 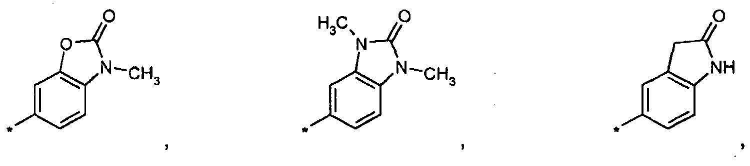
bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

10. Verbindungen der allgemeinen Formel I gemäß Anspruch 1, in denen Y, **R¹, R²** und **R³** wie in Anspruch 1, 2, 3, 4, 5, 6, 7, 8 oder 9 definiert sind und der Ring 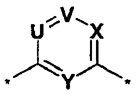 eine Gruppe ausgewählt aus 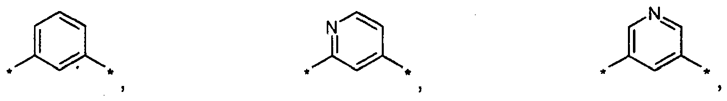 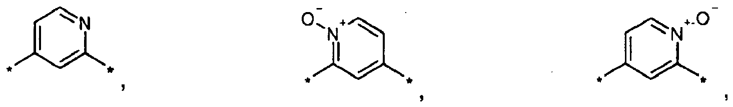 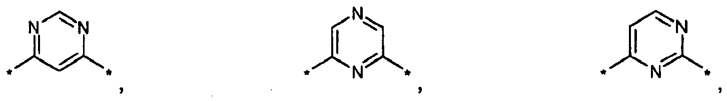 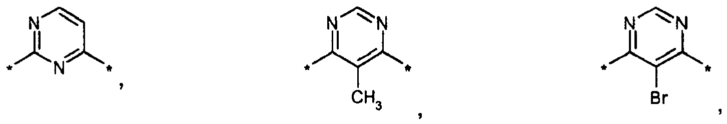 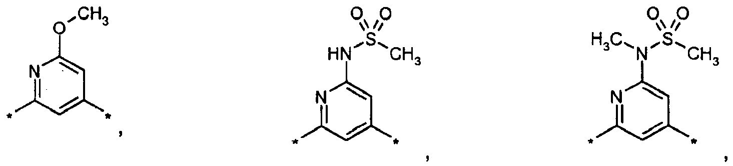 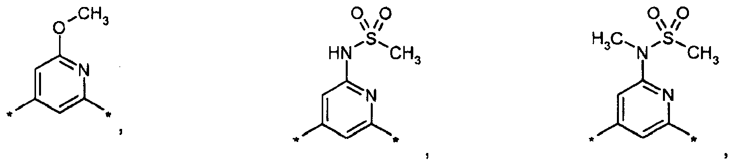 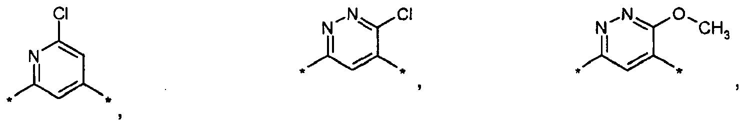 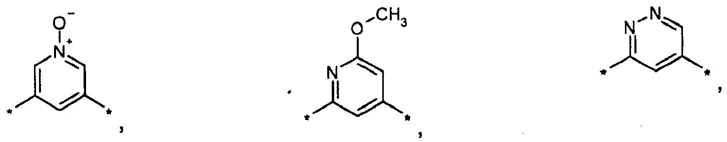 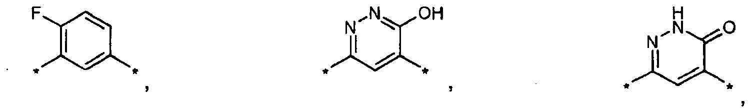 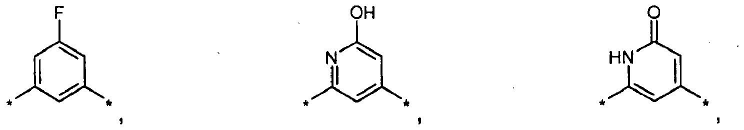 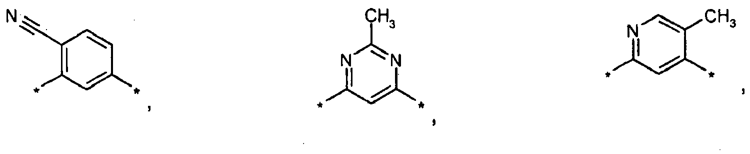 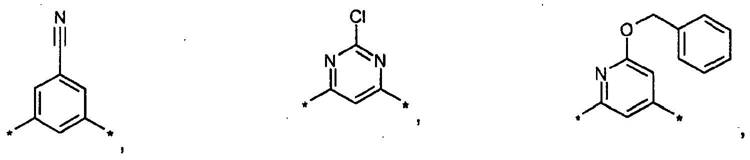 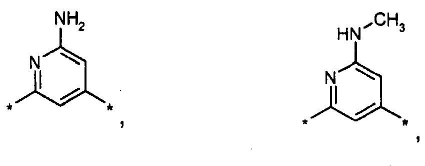 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

11. Verbindungen der allgemeinen Formel I gemäß Anspruch 1, in der
**R¹** eine Gruppe ausgewählt aus 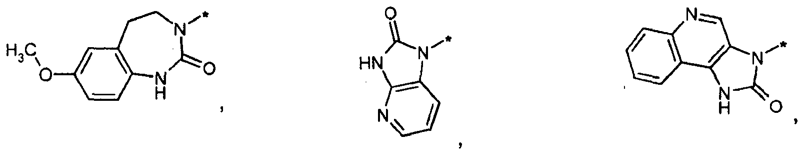 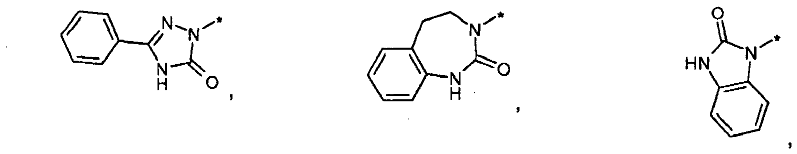 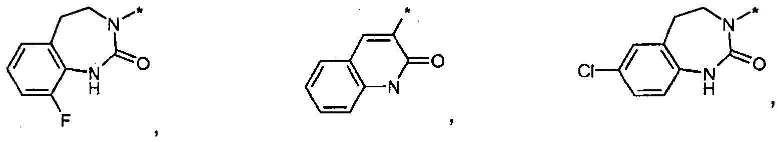
**R²** H und
**R³** eine Gruppe ausgewählt aus 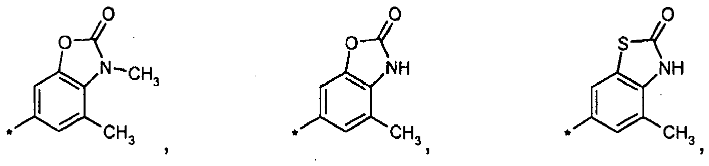 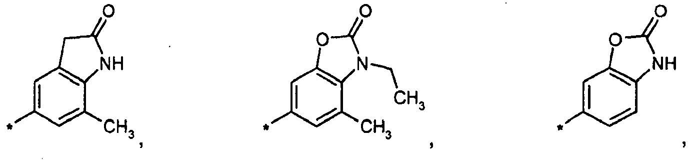 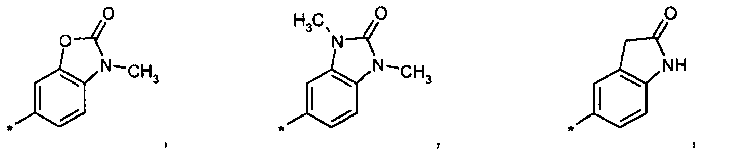 und der Ring 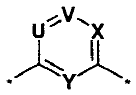 eine Gruppe ausgewählt aus 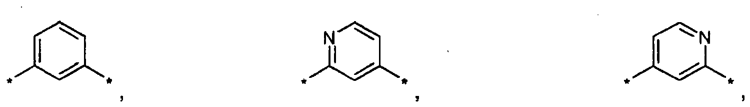 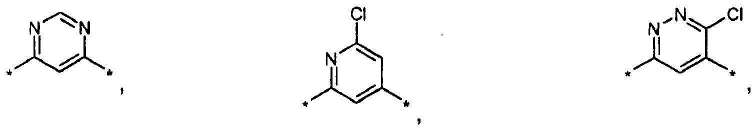 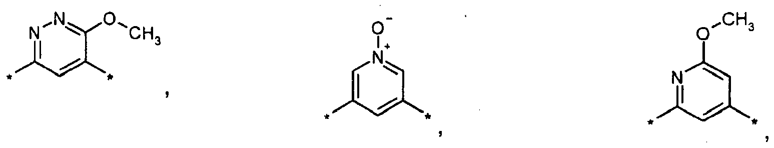 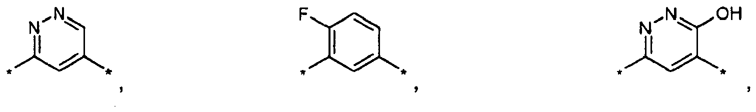 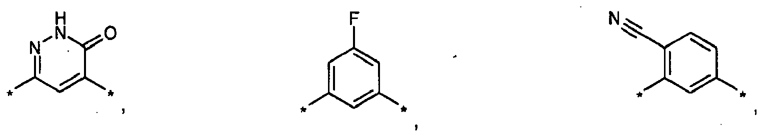 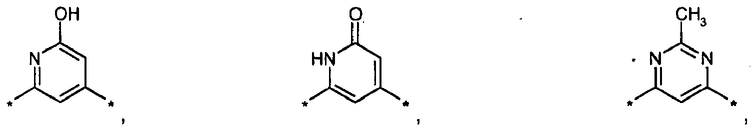 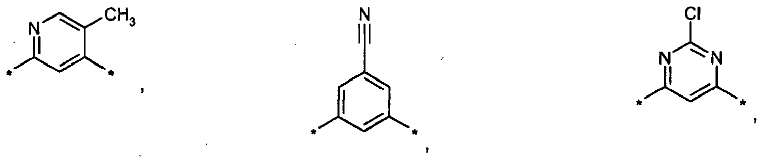 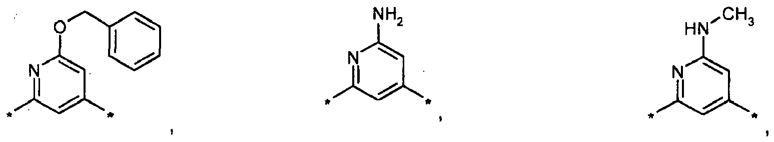 bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

12. Verbindungen der allgemeinen Formel I gemäß Anspruch 1: 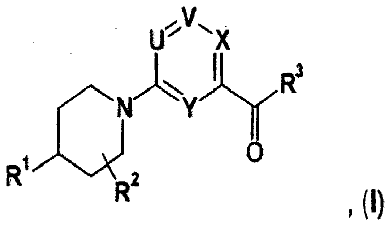
| **Nr.** | **Struktur** |
|---|---|
| (1) | 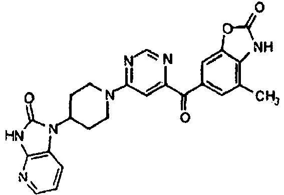 |
| (2) | 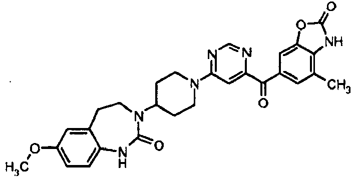 |
| (3) | 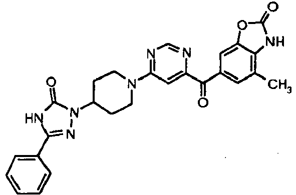 |
| (4) | 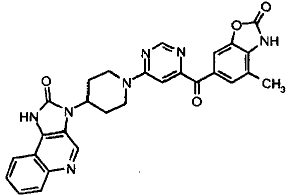 |
| (5) | 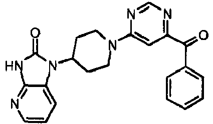 |
| (6) | 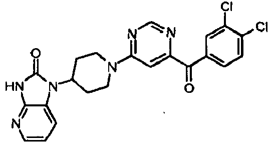 |
| (7) | 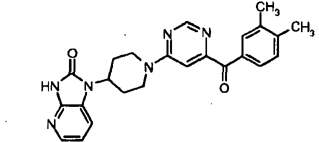 |
| (8) | 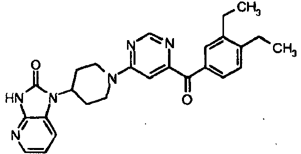 |
| (9) | 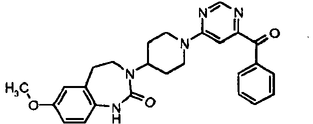 |
| (10) | 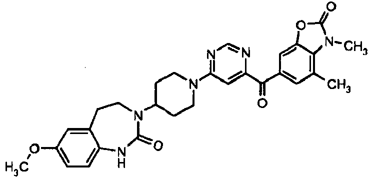 |
| (11) | 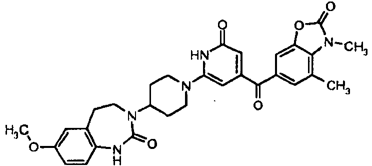 |
| (12) | 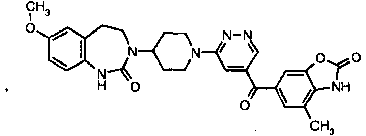 |
| (13) | 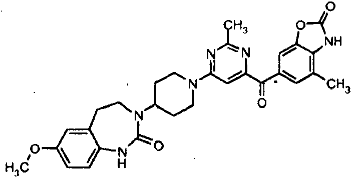 |
| (14) | 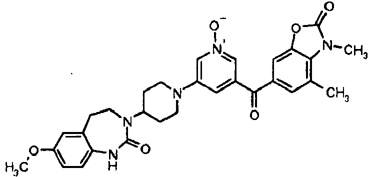 |
| (15) | 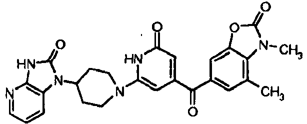 |
| (16) | 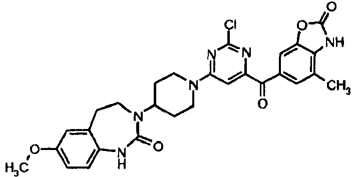 |
| (17) | 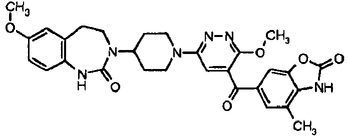 |
| (18) | 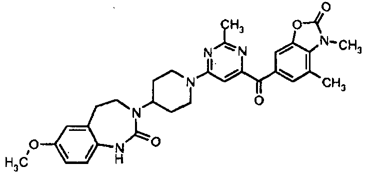 |
| (19) | 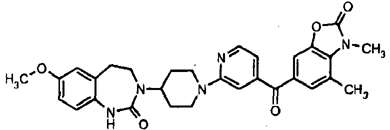 |
| (20) | 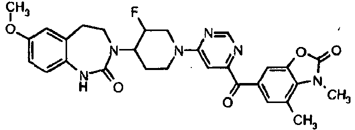 |
| (21) | 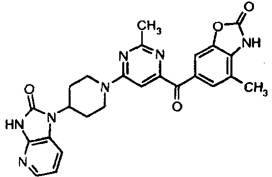 |
| (22) | 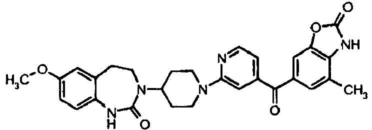 |
| (23) | 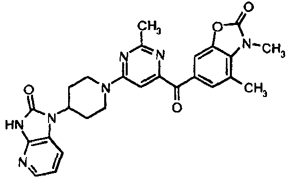 |
| (24) | 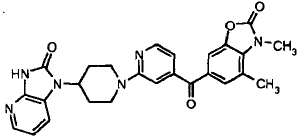 |
| (25) | 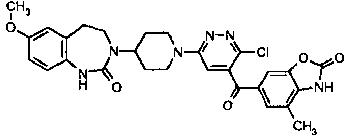 |
| (26) | 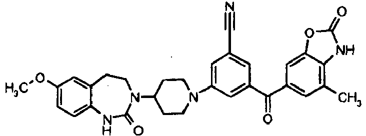 |
| (27) | 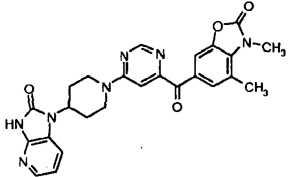 |
| (28) | 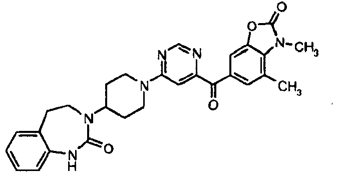 |
| (29) | 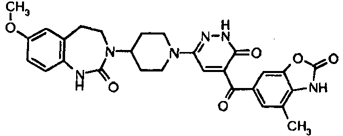 |
| (30) | 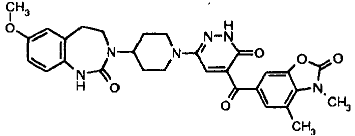 |
| (31) | 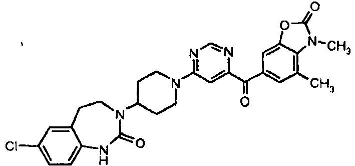 |
| (32) | 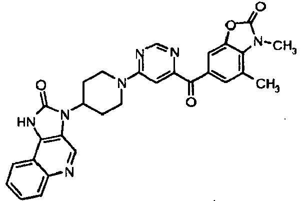 |
| (33) | 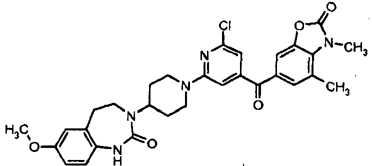 |
| (34) | 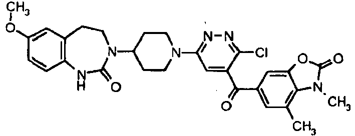 |
| (35) | 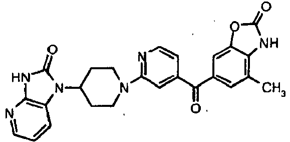 |
| (36) | 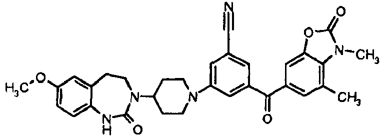 |
| (37) | 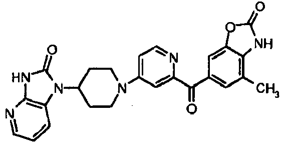 |
| (38) | 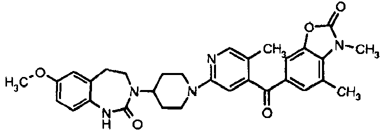 |
| (39) | 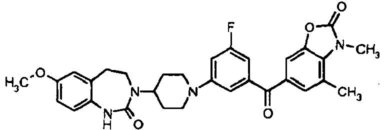 |
| (40) | 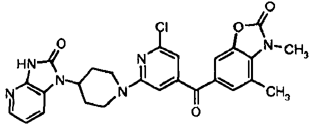 |
| (41) | 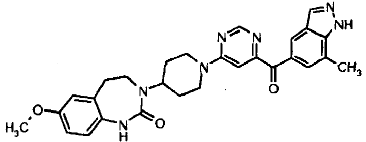 |
| (42) | 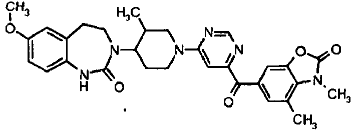 |
| (43) | 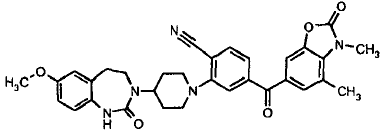 |
| (44) | 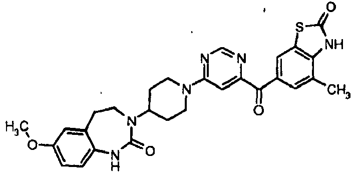 |
| (45) | 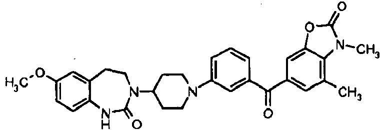 |
| (46) | 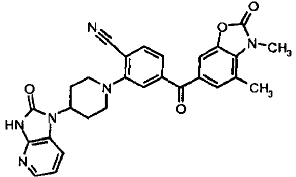 |
| (47) | 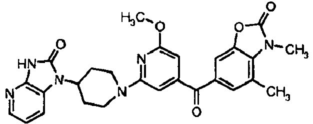 |
| (48) | 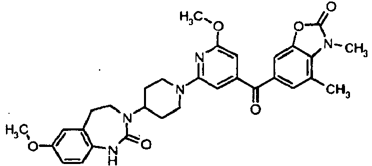 |
| (49) | 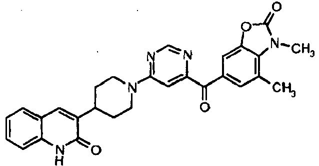 |
| (50) | 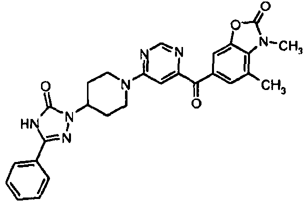 |
| (51) | 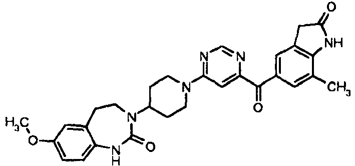 |
| (52) | 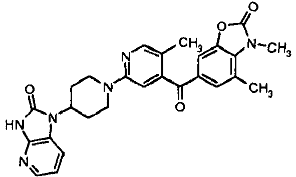 |
| (53) | 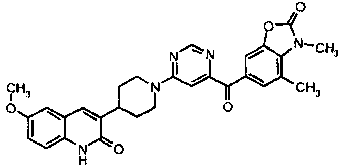 |
| (54) | 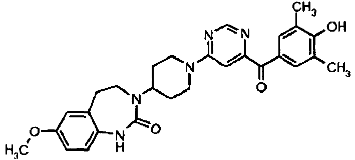 |
| (55) | 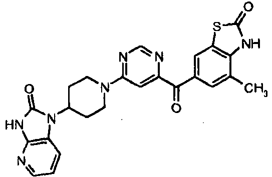 |
| (56) | 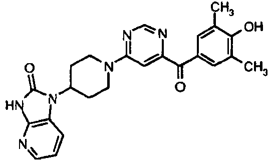 |
| (57) | 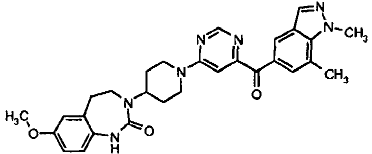 |
| (58) | 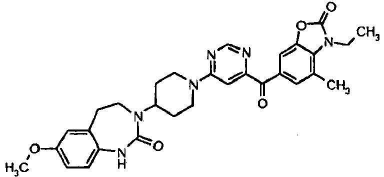 |
| (59) | 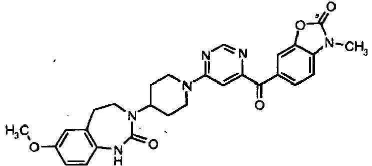 |
| (60) | 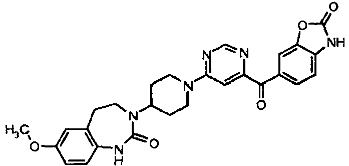 |
| (61) | 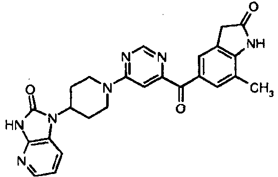 |
| (62) | 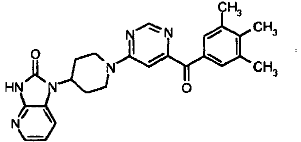 |
| (63) | 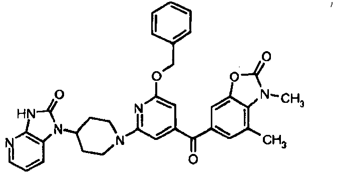 |
| (64) | 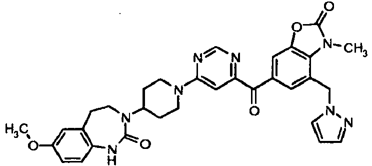 |
| (65) | 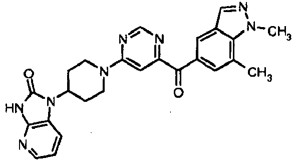 |
| (66) | 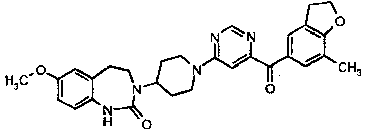 |
| (67) | 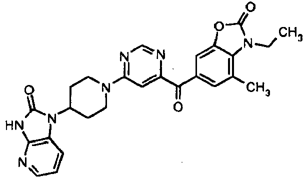 |
| (68) | 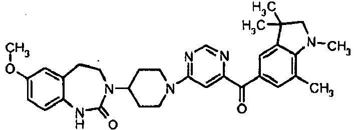 |
| (69) | 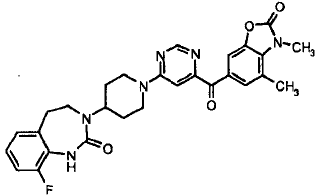 |
| (70) | 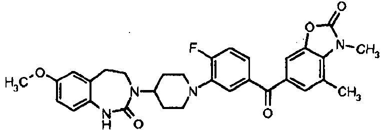 |
| (71) | 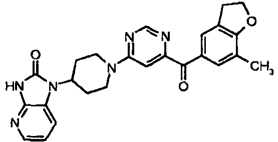 |
| (72) | 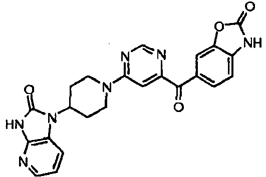 |
| (73) | 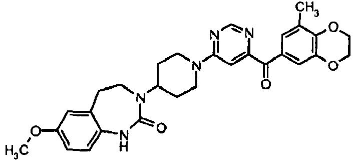 |
| (74) | 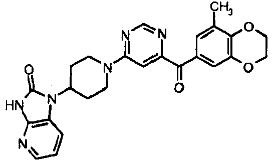 |
| (75) | 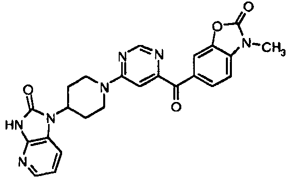 |
| (76) | 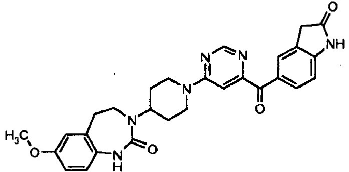 |
| (77) | 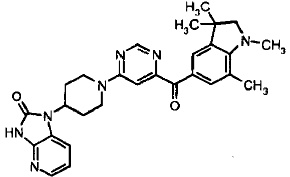 |
| (78) | 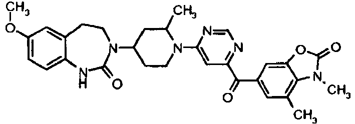 |
| (79) | 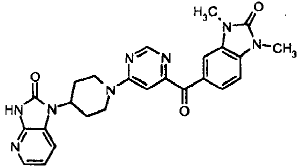 |
| (80) | 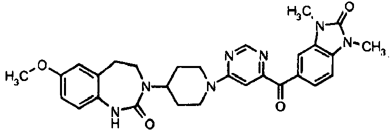 |
| (81) | 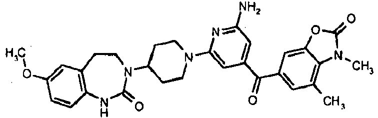 |
| (82) | 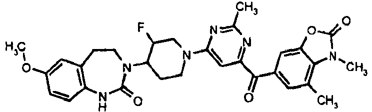 |
| (83) | 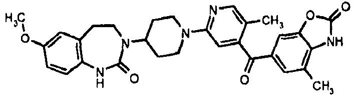 |
| (84) | 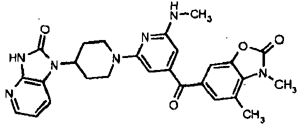 |
| (85) | 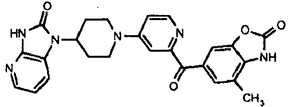 |
deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

13. Physiologisch verträgliche Salze der Verbindungen nach einem der Ansprüche 1 bis 12 mit anorganischen oder organischen Säuren oder Basen.

14. Arzneimittel, enthaltend eine Verbindung nach einem der Ansprüche 1 bis 12 oder ein physiologisch verträgliches Salz gemäß Anspruch 13 neben gegebenenfalls einem oder mehreren inerten Trägerstoffen und/oder Verdünnungsmitteln.

15. Verwendung einer Verbindung nach einem der Ansprüche 1 bis 13 zur Herstellung eines Arzneimittels zur akuten und prophylaktischen Behandlung von Kopfschmerzen, insbesondere Migräne- bzw. Cluster-Kopfschmerz.

16. Verwendung einer Verbindung nach einem der Ansprüche 1 bis 13 zur Herstellung eines Arzneimittels zur Behandlung von nicht insulinabhängigem Diabetes mellitus ("NIDDM"), des complex regional pain syndrome (CRPS1), cardiovaskulärer Erkrankungen, Morphintoleranz, Clostritiumtoxin-bedingten Durchfallerkrankungen, Erkrankungen der Haut, insbesondere thermische und strahlenbedingte Hautschäden inklusive Sonnenbrand, entzündlicher Erkrankungen, z.B. entzündlicher Gelenkerkrankungen (Arthritis), neurogenen Entzündungen der oralen Mucosa, entzündlichen Lungenerkrankungen, allergischer Rhinitis, Asthma, Erkrankungen, die mit einer überschießenden Gefäßerweiterung und dadurch bedingter verringerter Gewebedurchblutung einhergehen, z.B. Schock und Sepsis, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten.

17. Verfahren zur Herstellung eines Arzneimittels gemäß Anspruch 14, **dadurch gekennzeichnet, dass** auf nichtchemischem Weg eine Verbindung nach einem der Ansprüche 1 bis 13 in einen oder mehrere inerte Trägerstoffe und/oder Verdünnungsmittel eingearbeitet wird.

## Claims

1. Compounds of general formula I 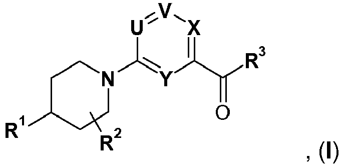 wherein
**R**¹ a group of general formula 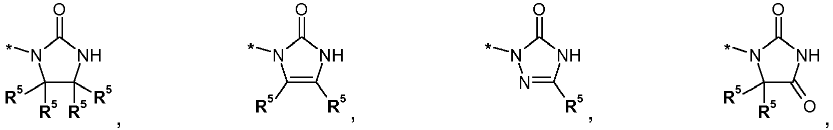 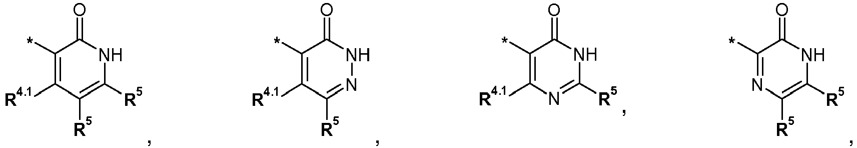 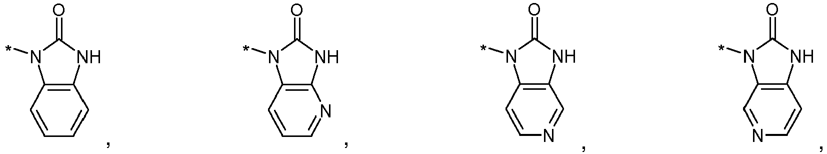 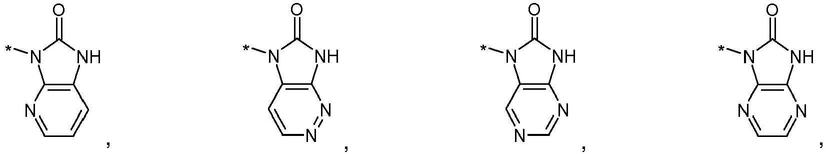 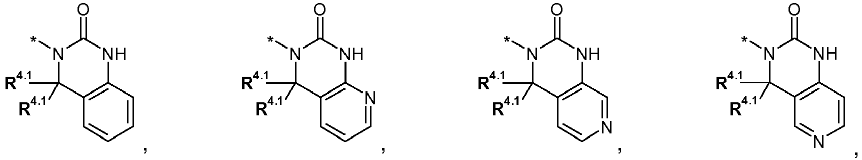 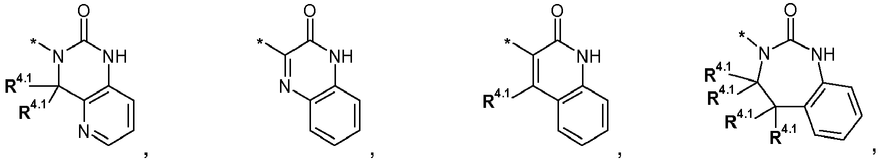 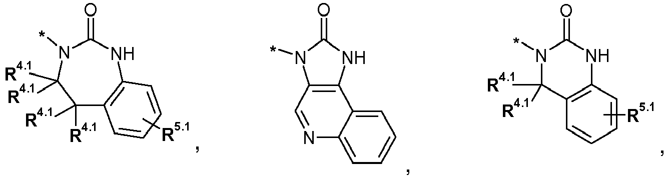
**R²**
(a) H,
(b) F, -CN, C₁₋₃-alkyl, -CO₂-**R^{2.1}** or
(c) a C₁₋₃-alkyl group wherein each methylene group is substituted by up to two fluorine atoms and each methyl group is substituted by up to three fluorine atoms,
**R^{2.1}** H or C₁₋₆-alkyl,
**R³** a 6- or 10-membered aryl group substituted by the groups **R^{3.1}, R^{3.2}** and **R^{3.3}** or a 6-membered heteroaryl group substituted by the groups **R^{3.1}, R^{3.2}** and **R^{3.3}** which is attached via a carbon atom,
**R^{3.1}**
(a) H,
(b) halogen, -NH₂, C₁₋₄-alkyl-NH-, (C₁₋₄-alkyl)₂N-, C₁₋₃-alkyl-C(O)-NH-, C₁₋₃-alkyl-S(O₂)-NH-, -CN, -OH, -O-C(O)-NH-C₁₋₃-alkyl,
(c) C₁₋₄-alkyl, **R^{3.1,1-}**C**₁₋₃-**alkylene**,** C₂₋₄-alkenyl, C₂₋₄-alkynyl, C₁₋₃-alkyl-O-, C₁₋₃-alkyl-S(O)**ₘ**-, cyclopropyl,
(d) a C₁₋₃-alkyl or C₁₋₃-alkyl-O- group wherein each methylene group is substituted by up to two fluorine atoms and each methyl group is substituted by up to three fluorine atoms,
(e) -C(O)-**R^{3.1.2}**,
(f) -S(O)₂-**R^{3.1.3},**
**R^{3.1.1}**
(a) H,
(b) C₃₋₆-cycloalkyl, C₅₋₆-cycloalkenyl,
(c) (**R**^{3.1.1.1})₂N,
(d) a saturated, mono- or diunsaturated 5- or 6-membered heterocyclic group which is substituted at a nitrogen atom by a group **R^{3.1.1.1}** and is substituted at a carbon atom by one or two groups R^{3.1.1.2}, or
(e) a heteroaryl group which is substituted at a carbon atom by a group **R^{3.1.1.2},**
**R^{3.1.1.1}** independently of one another
(a) H, C₁₋₄-alkyl, C₃₋₆-cycloalkyl,
(b) heterocyclyl,
(c) aryl-C₀₋₃-alkylene or heteroaryl-C₀₋₃-alkylene,
**R^{3.1.1.2}** independently of one another
(a) H, F, C₁₋₃-alkyl, -CN, -OH, -O-C₁₋₃-alkyl, -CO(O)**R^{3.1.1.2.1}**, H₂N-, (C₁₋₄-alkyl)-NH-, (C₁₋₄-alkyl)₂N-,
(b) phenyl or phenyl-CH₂-,
(c) a C₁₋₃-alkyl or -O-C₁₋₃-alkyl group wherein each methylene group is substituted by up to two fluorine atoms and each methyl group is substituted by up to three fluorine atoms, or
**R^{3.1.1.2.1}** H, C₁₋₆-alkyl, benzyl,
**R^{3.1.2}** -O-C₁₋₃-alkyl, -OH, -N**R^{3.1.2.1}R^{3.1.2.2}**,
**R^{3.1.2.1}** H, C₁₋₃-alkyl,
**R^{3.1.2.2}** H, C₁₋₃-alkyl,
**R**^{3.1.2.1} and **R**^{3.1.2.1} together may also form a ring which is selected from among azetidinyl, pyrrolidinyl, piperidinyl, piperazinyl and morpholinyl,
**R^{3.1.3}** -O-C₁₋₃-alkyl, **-NR^{3.1.3.1}R^{3.1.3.2},**
**R^{3.1.3.1}** H, C₁₋₃-alkyl,
**R^{3.1.3.2}** H, C₁₋₃-alkyl,
**R^{3.1.3.1}** and **R^{3.1.3.1}** together may also form a ring which is selected from among azetidinyl, pyrrolidinyl, piperidinyl, piperazinyl and morpholinyl,
**R^{3.2}**
(a) H,
(b) halogen, -NH₂, C₁₋₄-alkyl-NH-, (C₁₋₄-alkyl)₂N-, C₁₋₃-alkyl-C(O)-NH-, C₁₋₃-alkyl-S(O₂)-NH-, -CN, -OH, -O-C(O)-NH-C₁₋₃-alkyl,
(c) C₁₋₄-alkyl, C₂₋₄-alkenyl, C₂₋₄-alkynyl, C₁₋₃-alkyl-O, C₁₋₃-alkyl-S(O)ₘ-, cyclopropyl,
(d) a C₁₋₃-alkyl or C₁₋₃-alkyl-O- group wherein each methylene group is substituted by up to two fluorine atoms and each methyl group is substituted by up to three fluorine atoms,
(e) -C(O)-**R^{3.2.1}**,
(f) -S(O)₂-**R^{3.2.2}**,
**R^{3.2.1}** -O-C₁₋₃-alkyl, -OH, -N**R^{3.2.1.1}R^{3.2.1.2}**,
**R^{3.2.1.1}** H, C₁₋₃-alkyl,
**R^{3.2.1.2}** H, C₁₋₃-alkyl,
**R^{3.2.1.1}** and **R^{3.2.1.2}** together may also form a ring which is selected from among azetidinyl, pyrrolidinyl, piperidinyl, piperazinyl and morpholinyl,
**R^{3.2.2}** -N**R**^{3.2,2,1}**R**^{3.2,2,2},
**R^{3.2,2,1}** H, C₁₋₃-alkyl,
**R**^{**3.2,2,**2} H C₁₋₃-alkyl,
**R^{3.2,2,1}** and **R^{3.2,2,2}** together may also form a ring which is selected from among azetidinyl, pyrrolidinyl, piperidinyl, piperazinyl and morpholinyl,
**R^{3.3}**
(a) H,
(b) halogen, -NH₂, C₁₋₄-alkyl-NH-, (C₁₋₄-alkyl)₂N-, C₁₋₃-alkyl-C(O)-NH-, C₁₋₃-alkyl-S(O₂)-NH-, -CN, -OH, -O-C(O)-NH-C₁₋₃-alkyl,
(c) C₁₋₄-alkyl, C₂₋₄-alkenyl, C₂₋₄-alkynyl, C₁₋₃-alkyl-O, C₁₋₃-alkyl-S(O)ₘ-, cyclopropyl,
(d) a C₁₋₃-alkyl- or C₁₋₃-alkyl-O- group wherein each methylene group is substituted by up to two fluorine atoms and each methyl group is substituted by up to three fluorine atoms,
(e) -C(O)-**R**^{3.3.1},
(f) -S(O)₂-**R^{3.3.2}**,
**R^{3.3.1}** -O-C₁₋₃-alkyl, -OH, -N**R^{3.3.1.1}R^{3.3.1.2}**,
**R^{3.3.1.1}** H, C₁₋₃-alkyl,
**R^{3.3.1.2}** H, C₁₋₃-alkyl,
**R^{3.3.1.1}** and **R^{3.3.1.2}** together may also form a ring which is selected from among azetidinyl, pyrrolidinyl, piperidinyl, piperazinyl and morpholinyl,
**R^{3.3.2}** -O-C₁₋₃-alkyl, -N**R^{3.3.2.1}R^{3.3.2.2}**,
**R^{3.3.2.1}** H, C₁₋₃-alkyl,
**R^{3.3.2.2}** H, C₁₋₃-alkyl,
**R^{3.3.2.1}** and **R^{3.3.2.2}** together may also form a ring which is selected from among azetidinyl, pyrrolidinyl, piperidinyl, piperazinyl and morpholinyl, or
**R^{3.2}** and **R^{3.3}** together with the carbon atoms to which they are attached form a monounsaturated 5-membered or a mono- or diunsaturated 6-membered heterocyclic group or a 5- to 6-membered heteroaryl group, while
the heterocycles mentioned previously may contain a carbonyl, thiocarbonyl or cyanimino group adjacent to a nitrogen atom, and
may optionally each additionally be substituted at one or two nitrogen atoms by a group **R^{3.3.3}** and
may optionally each additionally be substituted at one or two carbon atoms by one or two groups **R^{3.3.4},**
**R^{3.3.3}** independently of one another
(a) C₁₋₄-alkyl or
(b) C₃₋₆-cycloalkyl,
**R^{3.3.4}** independently of one another
(a) C₁₋₄-alkyl or
(b) C₃₋₆-cycloalkyl,
(c) halogen, CN, -O-C₁₋₃-alkyl, -NH₂,
(d) a C₁₋₃-alkyl- or C₁₋₃-alkyl-O- group wherein each methylene group is substituted by up to two and each methyl group is substituted by up to three fluorine atoms,
**R^{4.1}** independently of one another
(a) H,
(b) C₁₋₃-alkyl, -OH, -O-C₁₋₃-alkyl,
(c) a C₁₋₃-alkyl- or C₁₋₃-alkyl-O- group wherein each methylene group is substituted by up to two fluorine atoms and each methyl group is substituted by up to three fluorine atoms,
**R⁵** independently of one another
(a) H, C₁₋₆-alkyl, C₃₋₆-cycloalkyl,
(b) a phenyl group optionally substituted by 1, 2, or 3 substituents **R^{5.2}**, wherein the substituents **R^{5.2}** may be identical or different,
(c) a heteroaryl group optionally substituted by 1, 2 or 3 substituents **R^{5.2}** which is selected from among benzimidazole, benzothiophene, furan, imidazole, indole, isoxazole, oxazole, pyrazine, pyrazole, pyridazine, pyridine, pyrimidine, pyrrole, thiazole, thiophene and triazole, wherein the substituents **R^{5.2}** may be identical or different,
(d) a heterocyclic group optionally substituted by 1, 2 or 3 substituents **R^{5.2},** wherein the substituents **R^{5.2}** may be identical or different,
**R^{5.1}**
(a) H, halogen, C₁₋₆-alkyl, C₃₋₆-cycloalkyl,
(b) -O-C₁₋₆-alkylene-N**R**⁷**R**⁸, -O-**R**⁶, -CO₂**R**⁶, -C(O)-N**R**⁷**R**⁸, -SO₂-N**R⁷R⁸,** -N**R**⁷-SO₂-**R**⁸, -S(O)ₘ-**R**⁷, -CN, -N**R**⁷**R**⁸, -O-C(O)-**R**⁶ or
(c) a C₁₋₃-alkyl or C₁₋₃-alkyl-O- group wherein each methylene group is substituted by up to two fluorine atoms and each methyl group is substituted by up to three fluorine atoms,
**R^{5.2}**
(a) halogen, C₁₋₆-alkyl, C₃₋₆-cycloalkyl,
(b) -O-**R**⁶, -O-(CH₂)ₛ-O-**R**⁶, -CO₂**R**⁶, -S(O)ₘ-**R**⁶, -CN, -O-C(O)-**R**⁶ or
(c) a C₁₋₃-alkyl group wherein each methylene group is substituted by up to two fluorine atoms and each methyl group is substituted by up to three fluorine atoms,
**R⁶**
(a) H,
(b) C₁₋₆-alkyl, C₃₋₆-cycloalkyl, aryl, heteroaryl, benzyl, which may be substituted by a group **R**^{6.1}, or
(c) a C₁₋₃-alkyl group wherein each methylene group is substituted by up to two fluorine atoms and each methyl group is substituted by up to three fluorine atoms,
**R^{6.1}** HO- or C₁₋₆-alkyl-O-,
**R⁷**
(a) H,
(b) C₁₋₃-alkyl, phenyl or benzyl, while the groups are unsubstituted or may be substituted by halogen, HO- or H₃C-O-,
**R⁸**
(a) H,
(b) C₁₋₃-alkyl, phenyl or benzyl, while the groups are unsubstituted or may be substituted by halogen, HO- or H₃C-O-, or
**R⁷** and **R⁸** together may also form a ring which is selected from among azetidinyl, pyrrolidinyl, piperidinyl, piperazinyl and morpholinyl, while the ring may be unsubstituted or substituted by a substituent **R⁶,**
**m** one of the numbers 0, 1 or 2, and
**s** one of the numbers 1, 2 or 3,
**U** N, N-oxide or C-**R⁹**,
**V** N, N-oxide or C-**R¹⁰**,
**X** N, N-oxide or C**R¹¹**,
**Y** N or C-**R¹²**,
while at most three of the previously mentioned groups **U, V, X** or **Y** simultaneously denote a nitrogen atom,
**R⁹**
(a) H,
(b) a C₁₋₆-alkyl- or C₁₋₃-alkyl-O- group which may each be substituted by a group **R^{9.1}**,
(c) **R**^{9.2}**R**^{9.3}N, **R^{9.2}R**^{9.3}N-C₁₋₃-alkylene-,
(d) halogen, -CN, -OH, -COOH, C₁₋₃-alkyl-O-C₁₋₃-alkylene, C₃₋₆-cycloalkyl, C₃₋₆-cycloalkyl-C₁₋₄-alkylene-, C₁₋₃-alkyl-C(O)-O-C₁₋₃-alkylene,
(e) a C₁₋₃-alkyl or C₁₋₃-alkyl-O- group wherein each methylene group is substituted by up to two fluorine atoms and each methyl group is substituted by up to three fluorine atoms,
**R^{9.1}** H, OH or -O-CH₃,
**R^{9.2}** H or C₁₋₃-alkyl,
**R^{9.3}** H or C₁₋₃-alkyl, or
**R^{9.2}** and **R^{9.3}** together with the nitrogen atom to which they are attached denote a 3- to 6-membered heterocyclic group,
**R¹⁰**
(a) H,
(b) a C₁₋₆-alkyl- or C₁₋₃-alkyl-O- group which may each be substituted by a group **R^{10.1}**,
(c) -N**R^{10.2}R^{10.3}**, N**R^{10.2}R^{10.3}**-C₁₋₃-alkylene-,
(d) halogen, -CN, -OH, C₁₋₃-alkyl-O-C₁₋₃-alkylene, C₃₋₆-cycloalkyl, C₃₋₆-cycloalkyl-C₁₋₄-alkylene, C₁₋₃-alkyl-C(O)-O-C₁₋₃-alkylene,
(e) an aryl-C₀₋₃-alkylene-O- group,
(f) a C₁₋₃-alkyl or C₁₋₃-alkyl-O- group wherein each methylene group is substituted by up to two fluorine atoms and each methyl group is substituted by up to three fluorine atoms,
**R^{10.1}** H, OH or-O-CH₃,
**R^{10.2}** H or C₁₋₆-alkyl,
**R^{10.3}** H, C₁₋₆-alkyl or -SO₂-C₁₋₃-alkyl, or
**R^{10.2}** and **R^{10.3}** together with the nitrogen atom to which they are attached denote a 3- to 6-membered heterocyclic group,
**R¹¹**
(a) H,
(b) a C₁₋₆-alkyl- or C₁₋₃-alkyl-O- group which may each be substituted by a group **R^{11.1},**
(c) **R^{11.2}R^{11.3}**N, **R^{11.2}R^{11.3}**N-C₁₋₃-alkylene-,
(d) halogen, -CN, -OH, -COOH, C₁₋₃-alkyl-O-C₁₋₃-alkylene-, C₃₋₆-cycloalkyl, C₃₋₆-cycloalkyl-C₁₋₄-alkylene-, C₁₋₃-alkyl-C(O)-O-C₁₋₃-alkylene,
(e) a C₁₋₃-alkyl- or C₁₋₃-alkyl-O- group wherein each methylene group is substituted by up to two fluorine atoms and each methyl group is substituted by up to three fluorine atoms,
**R^{11.1}** H, OH or -O-CH₃,
**R^{11.2}** H or C₁₋₃-alkyl,
**R^{11.3}** H or C₁₋₃-alkyl, or
**R^{11.2}** and **R^{11.3}** together with the nitrogen atom to which they are attached denote a 3- to 6-membered heterocyclic group, and
**R¹²** H, halogen or C₁₋₃-alkyl,
the tautomers, the diastereomers, the enantiomers, the hydrates, the mixtures thereof and the salts thereof as well as the hydrates of the salts, particularly the physiologically acceptable salts thereof with inorganic or organic acids or bases.

2. Compounds of general formula **I** according to claim 1, wherein **U, V, X, Y, R²** and **R³** are defined as in claim 1 and
**R¹** a group selected from 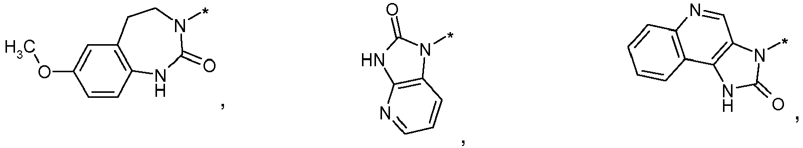 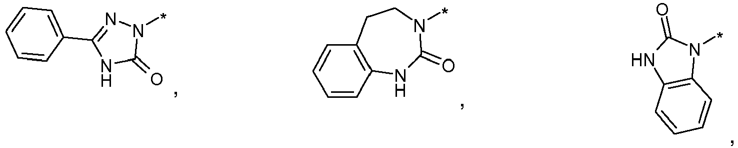 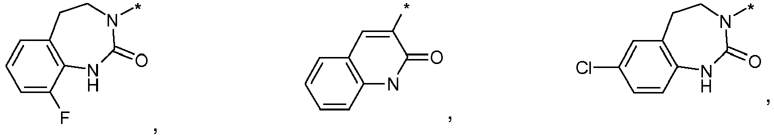
meaning,
the tautomers, the diastereomers, the enantiomers, the hydrates, the mixtures thereof and the salts thereof as well as the hydrates of the salts, particularly the physiologically acceptable salts thereof with inorganic or organic acids or bases.

3. Compounds of general formula **I** according to claim 1, wherein **U, V, X,** Y, **R**¹ and **R³** are defined as in claim 1 or 2 and **R²** denotes a hydrogen atom,
the tautomers, the diastereomers, the enantiomers, the hydrates, the mixtures thereof and the salts thereof as well as the hydrates of the salts, particularly the physiologically acceptable salts thereof with inorganic or organic acids or bases.

4. Compounds of general formula **I** according to claim 1, wherein **U, V, X, Y, R**¹ and **R²** are defined as in claim 1, 2 or 3, and
**R³** a group of general formulae **III** 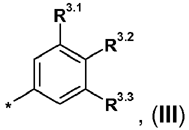
**R^{3.1}**
(a) H,
(b) F, Cl, Br, -NH₂, C₁₋₃-alkyl-NH, (C₁₋₃-alkyl)₂N, C₁₋₃-alkyl-C(O)-NH, -CN, -OH,
(c) C₁₋₄-alkyl, C₂₋₄-alkenyl, C₂₋₄-alkynyl, C₁₋₃-alkyl-O-, C₁₋₃-alkyl-S-,
(d) a C₁₋₃-alkyl or C₁₋₃-alkyl-O- group wherein each methylene group is substituted by up to two fluorine atoms and each methyl group is substituted by up to three fluorine atoms,
**R^{3.2}** (
a) H,
(b) F, Cl, Br, H₂N-, (C₁₋₄-alkyl)-NH-, (C₁₋₄-alkyl)₂N-, (C₁₋₃-alkyl)-C(O)-NH-, -OH,
(c) C₁₋₄-alkyl,
(d) a C₁₋₃-alkyl- or C₁₋₃-alkyl-O- group wherein each methylene group is substituted by up to two fluorine atoms and each methyl group is substituted by up to three fluorine atoms,
**R^{3.3}**
(a) H,
(b) F, Cl, Br, H₂N-, (C₁₋₄-alkyl)-NH-, (C₁₋₄-alkyl)₂N-, (C₁₋₃-alkyl)-C(O)-NH-, -OH,
(c) C₁₋₄-alkyl,
(d) a C₁₋₃-alkyl- or C₁₋₃-alkyl-O- group wherein each methylene group is substituted by up to two fluorine atoms and each methyl group is substituted by up to three fluorine atoms,
**R^{3.2}** and **R^{3.3}** together with the carbon atoms to which they are attached form a monounsaturated 5-membered or a mono- or diunsaturated 6-membered heterocyclic group or a 5- to 6-membered heteroaryl group, while
the heterocycles mentioned previously may contain a carbonyl, thiocarbonyl or cyanimino group adjacent to a nitrogen atom, and
may optionally each additionally be substituted at one or two nitrogen atoms by a group **R^{3.3.3}** and
may optionally each additionally be substituted at one or two carbon atoms by one or two groups **R^{3.3.4},**
**R^{3.3.3}** independently of one another
(a) C₁₋₄-alkyl or
(b) C₃₋₆-cycloalkyl, and
**R^{3.3.4}** independently of one another
(a) C₁₋₄-alkyl, C₃₋₆-cycloalkyl,
(b) halogen, CN, C₁₋₃-alkyl-O-, -NH₂,
(c) a C₁₋₃-alkyl- or C₁₋₃-alkyl-O- group wherein each methylene group is substituted by up to two fluorine atoms and each methyl group is substituted by up to three fluorine atoms,
the tautomers, the diastereomers, the enantiomers, the hydrates, the mixtures thereof and the salts thereof as well as the hydrates of the salts, particularly the physiologically acceptable salts thereof with inorganic or organic acids or bases.

5. Compounds of general formula **I** according to claim 1, wherein **U, V, X, Y, R¹** and **R²** are defined as in claim 1, 2 or 3, and
**R³** a group selected from 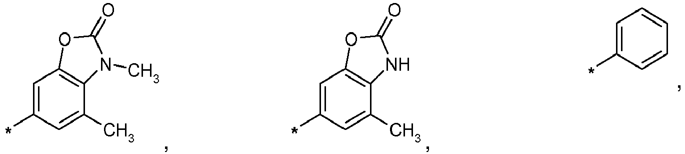 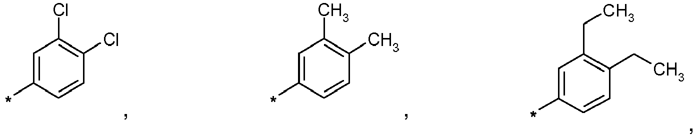 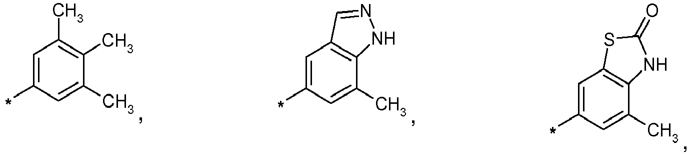 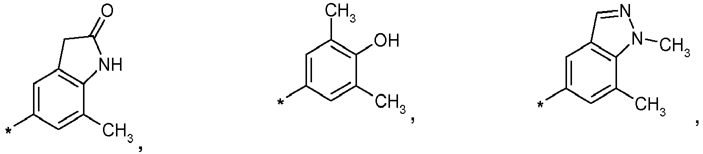 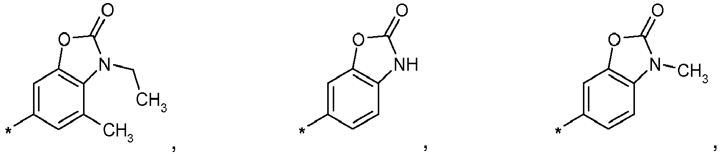 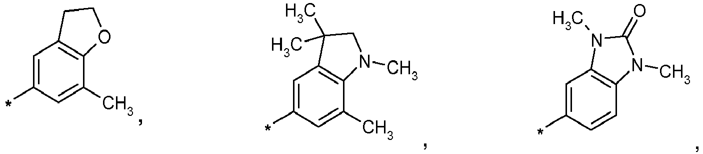 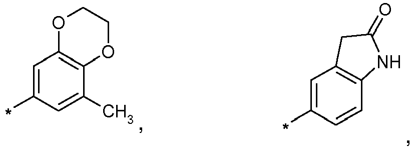
meaning,
the tautomers, the diastereomers, the enantiomers, the hydrates, the mixtures thereof and the salts thereof as well as the hydrates of the salts, particularly the physiologically acceptable salts thereof with inorganic or organic acids or bases.

6. Compounds of general formula **I** according to claim 1, wherein **U, V, X, Y, R¹** and R² are defined as in claim 1, 2 or 3 and
**R³** a group of general formulae **III** 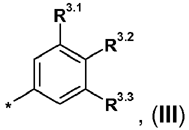
**R^{3.1}**
(a) H,
(b) F, Cl, Br, -NH₂, C₁₋₃-alkyl-NH-, (C₁₋₃-alkyl)₂N, C₁₋₃-alkyl-C(O)-NH-, -CN, -OH,
(c) C₁₋₄-alkyl, C₂₋₄-alkenyl, C₂₋₄-alkynyl, C₁₋₃-alkyl-O-, C₁₋₃-alkyl-S-,
(d) a C₁₋₃-alkyl- or C₁₋₃-alkyl-O- group wherein each methylene group is substituted by up to two fluorine atoms and each methyl group is substituted by up to three fluorine atoms,
**R^{3.2}** and **R^{3.3}** together with the carbon atoms to which they are attached form a monounsaturated 5-membered heterocyclic group or a mono- or diunsaturated 6-membered heterocyclic group or a 5- to 6-membered heteroaryl group, while
the heterocycles mentioned previously may contain a carbonyl, thiocarbonyl or cyanimino group adjacent to a nitrogen atom, and
may optionally each additionally be substituted at one or two nitrogen atoms by a group **R^{3.3.3}** and
may optionally each additionally be substituted at one or two carbon atoms by one or two groups **R^{3.3.4},**
**R^{3.3.3}** independently of one another
(a) C₁₋₄-alkyl or
(b) C₃₋₆-cycloalkyl, and
**R^{3.3.4}** independently of one another
(a) C₁₋₄-alkyl, C₃₋₆-cycloalkyl,
(b) halogen, -CN, -O-C₁₋₃-alkyl, -NH₂,
(c) a C₁₋₃-alkyl- or C₁₋₃-alkyl-O- group wherein each methylene group is substituted by up to two fluorine atoms and each methyl group is substituted by up to three fluorine atoms,
the tautomers, the diastereomers, the enantiomers, the hydrates, the mixtures thereof and the salts thereof as well as the hydrates of the salts, particularly the physiologically acceptable salts thereof with inorganic or organic acids or bases.

7. Compounds of general formula **I** according to claim 1, wherein **U, V, X, Y, R¹** and **R²** are defined as in claim 1, 2 or 3 and
**R³** a group of general formulae **III** 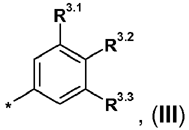
**R^{3.1}**
(a) H,
(b) F, Cl, Br, -NH₂, C₁₋₃-alkyl-NH, (C₁₋₃-alkyl)₂N, C₁₋₃-alkyl-C(O)-NH, -CN, -OH,
(c) C₁₋₄-alkyl, C₂₋₄-alkenyl, C₂₋₄-alkynyl, C₁₋₃-alkyl-O, C₁₋₃-alkyl-S,
(d) a C₁₋₃-alkyl- or C₁₋₃-alkyl-O- group wherein each methylene group is substituted by up to two fluorine atoms and each methyl group is substituted by up to three fluorine atoms,
**R^{3.2}** and **R^{3.3}** together with the carbon atoms to which they are attached form a monounsaturated 5-membered heterocyclic group or a 5- membered heteroaryl group, wherein
the heterocycles mentioned previously may contain a carbonyl, thiocarbonyl or cyanimino group adjacent to a nitrogen atom, and
may optionally each additionally be substituted at one or two nitrogen atoms by a group **R^{3.3.3}** and
may optionally each additionally be substituted at one or two carbon atoms by one or two groups **R^{3.3.4},**
**R^{3.3.3}** independently of one another
(a) C₁₋₄-alkyl- or
(b) C₃₋₆-cycloalkyl, and
**R^{3.3.4}** independently of one another
(a) C₁₋₄-alkyl-, C₃₋₆-cycloalkyl-,
(b) halogen, -CN, -O-C₁₋₃-alkyl, -NH₂,
(c) a C₁₋₃-alkyl or C₁₋₃-alkyl-O- group wherein each methylene group is substituted by up to two fluorine atoms and each methyl group is substituted by up to three fluorine atoms,
the tautomers, the diastereomers, the enantiomers, the hydrates, the mixtures thereof and the salts thereof as well as the hydrates of the salts, particularly the physiologically acceptable salts thereof with inorganic or organic acids or bases.

8. Compounds of general formula **I** according to claim 1, wherein **U, V, X, Y, R¹** and **R²** are defined as in claim 1, 2 or 3 and
**R³** a group of general formula **IIIa** 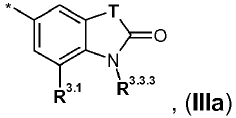
**T** O, S, CH₂, NH or N-**R^{3.3.3},**
**R^{3.1}**
(a) H,
(b) F, Cl, Br, -NH₂, C₁₋₃-alkyl-NH, (C₁₋₃-alkyl)₂N, C₁₋₃-alkyl-C(O)-NH, -CN, -OH,
(c) C₁₋₄-alkyl, C₂₋₄-alkenyl, C₂₋₄-alkynyl, C₁₋₃-alkyl-O, C₁₋₃-alkyl-S-,
(d) a C₁₋₃-alkyl or C₁₋₃-alkyl-O- group wherein each methylene group is substituted by up to two fluorine atoms and each methyl group is substituted by up to three fluorine atoms, and
**R^{3.3.3}** independently of one another
(a) C₁₋₄-alkyl or
(b) C₃₋₆-cycloalkyl,
the tautomers, the diastereomers, the enantiomers, the hydrates, the mixtures thereof and the salts thereof as well as the hydrates of the salts, particularly the physiologically acceptable salts thereof with inorganic or organic acids or bases.

9. Compounds of general formula **I** according to claim 1, wherein **U, V, X, Y, R¹** and **R²** are defined as in claim 1, 2 or 3 and
**R³** a group selected from 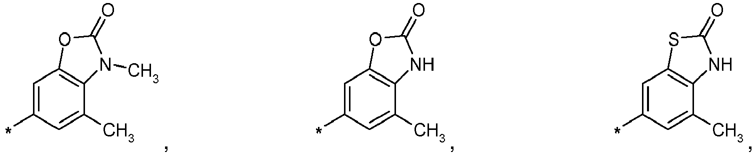 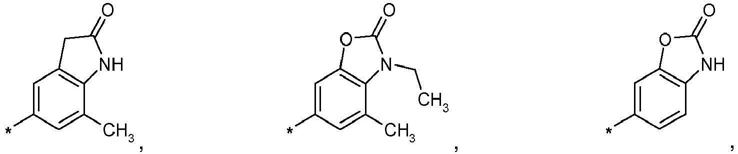 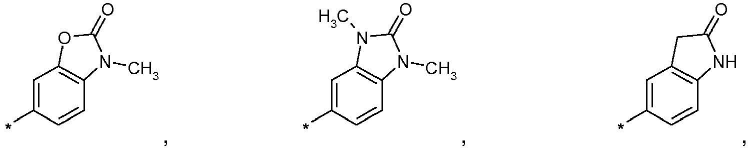
meaning,
the tautomers, the diastereomers, the enantiomers, the hydrates, the mixtures thereof and the salts thereof as well as the hydrates of the salts, particularly the physiologically acceptable salts thereof with inorganic or organic acids or bases.

10. Compounds of general formula **I** according to claim 1, wherein **Y, R¹, R²** and **R³** are defined as in claim 1, 2, 3, 4, 5, 6, 7, 8 or 9 and the ring 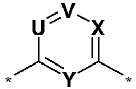 a group selected from 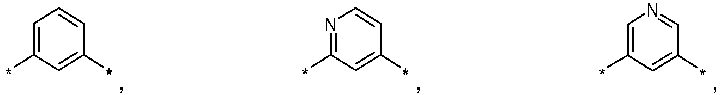 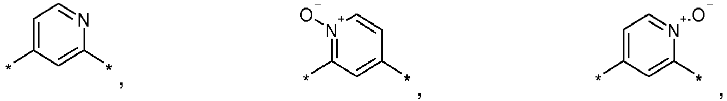 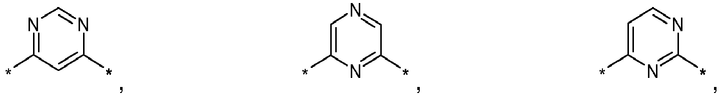 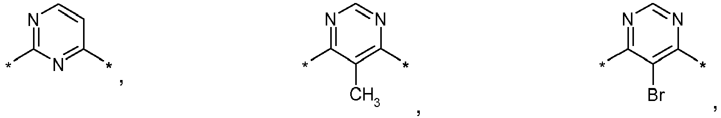 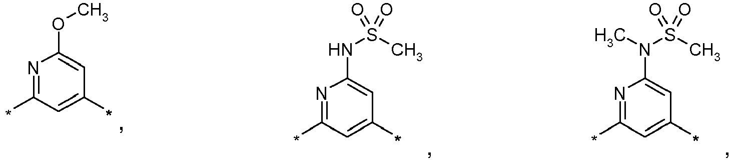 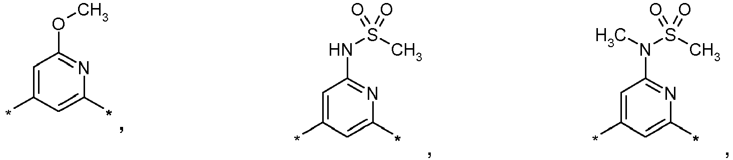 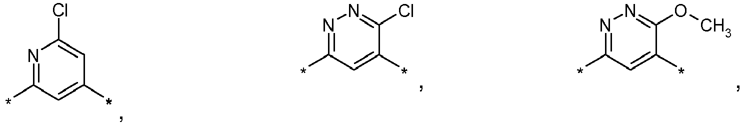 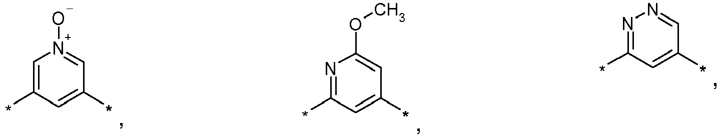 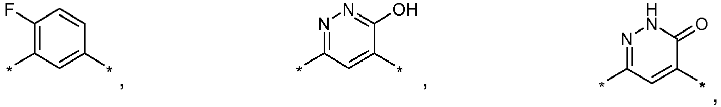 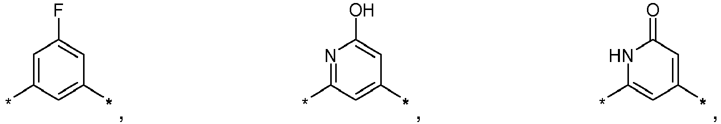 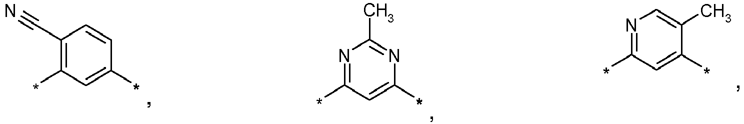 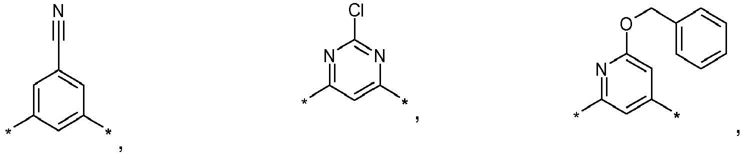 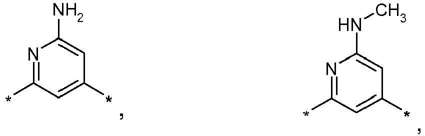 meaning,
the tautomers, the diastereomers, the enantiomers, the hydrates, the mixtures thereof and the salts thereof as well as the hydrates of the salts, particularly the physiologically acceptable salts thereof with inorganic or organic acids or bases.

11. Compounds of general formula **I** according to claim 1, wherein
**R¹** a group selected from 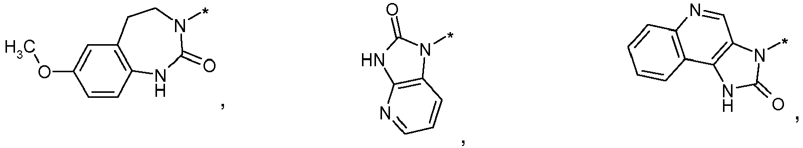 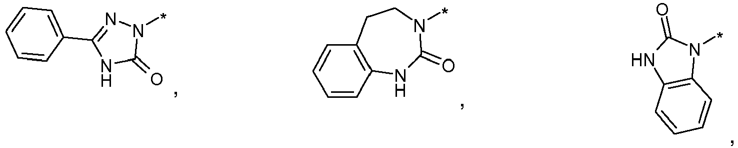 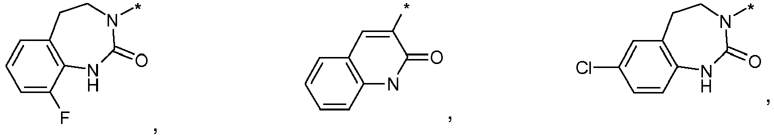
**R²** H and
**R³** a group selected from 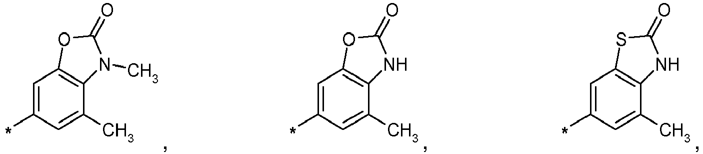 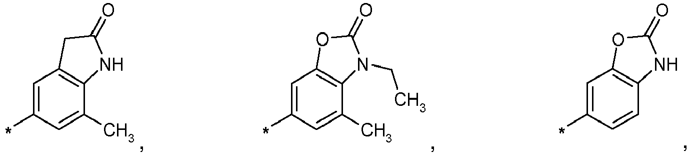 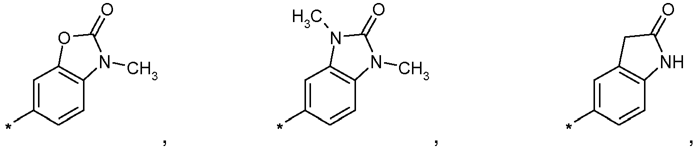 and the ring 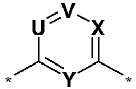 a group selected from 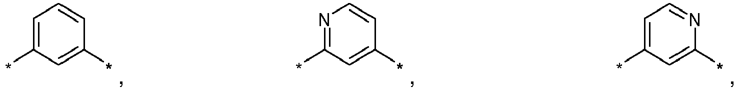 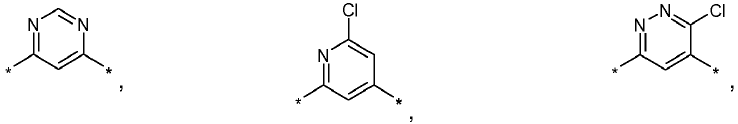 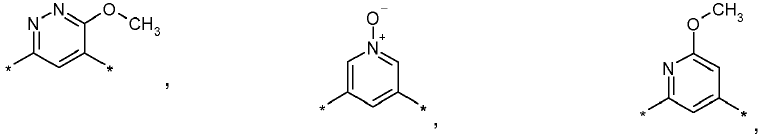 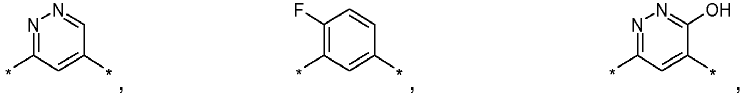 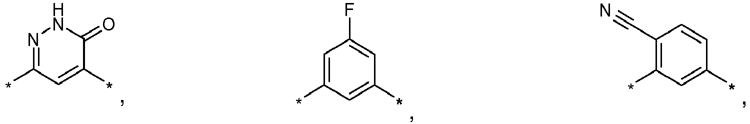 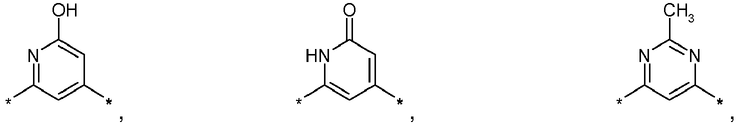 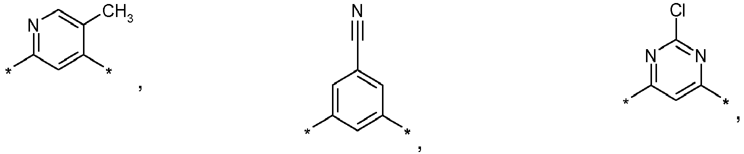 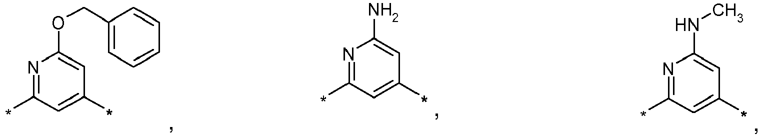 meaning,
the tautomers, the diastereomers, the enantiomers, the hydrates, the mixtures thereof and the salts thereof as well as the hydrates of the salts, particularly the physiologically acceptable salts thereof with inorganic or organic acids or bases.

12. Compounds of general formula **I** according to claim 1: 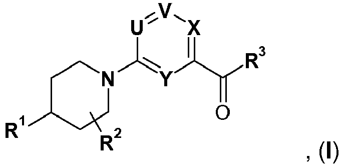
| **No.** | **Structure** |
|---|---|
| (1) | 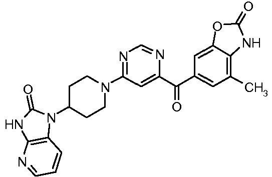 |
| (2) | 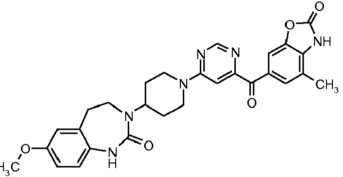 |
| (3) | 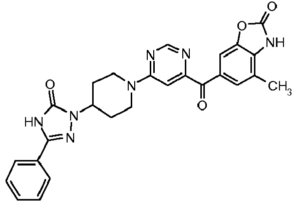 |
| (4) | 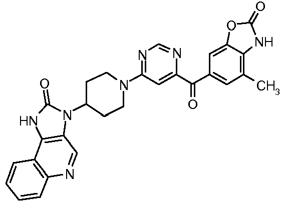 |
| (5) | 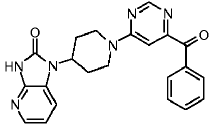 |
| (6) | 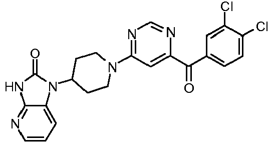 |
| (7) | 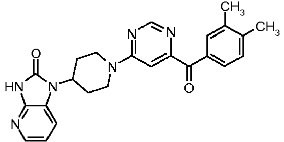 |
| (8) | 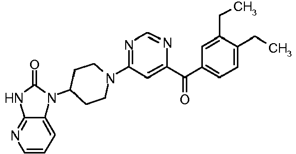 |
| (9) | 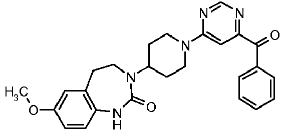 |
| (10) | 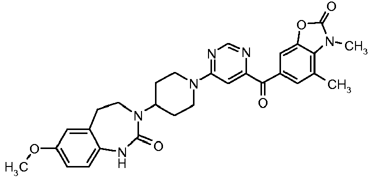 |
| (11) | 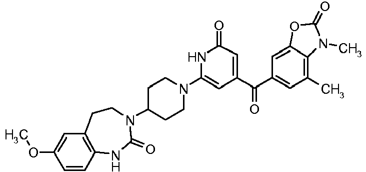 |
| (12) | 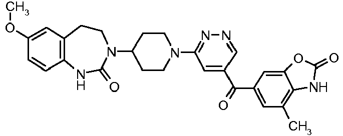 |
| (13) | 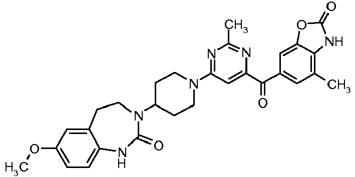 |
| (14) | 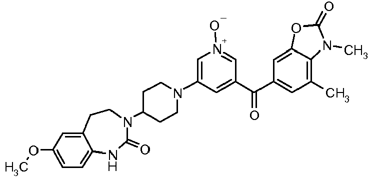 |
| (15) | 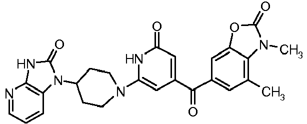 |
| (16) | 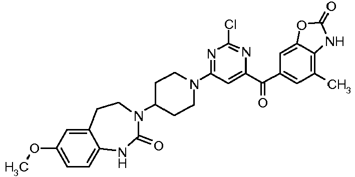 |
| (17) | 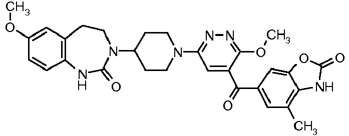 |
| (18) | 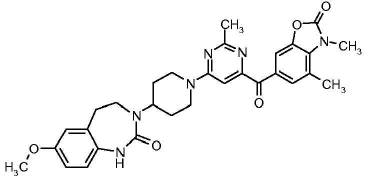 |
| (19) | 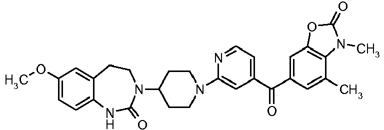 |
| (20) | 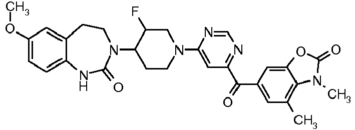 |
| (21) | 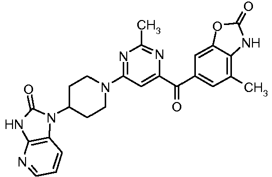 |
| (22) | 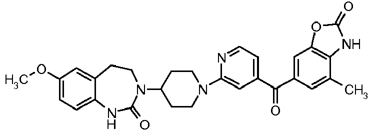 |
| (23) | 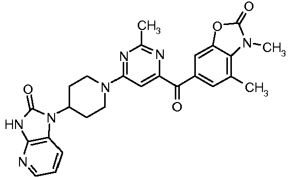 |
| (24) | 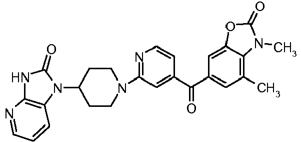 |
| (25) | 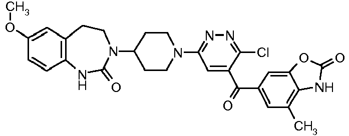 |
| (26) | 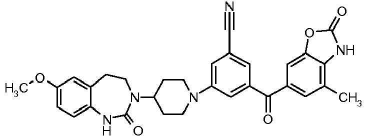 |
| (27) | 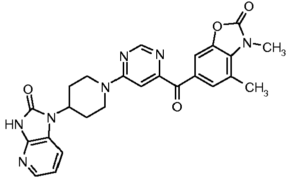 |
| (28) | 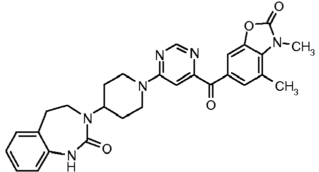 |
| (29) | 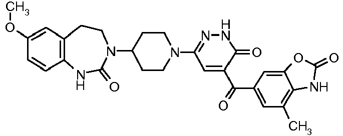 |
| (30) | 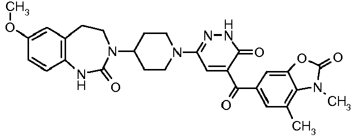 |
| (31) | 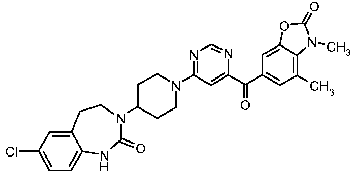 |
| (32) | 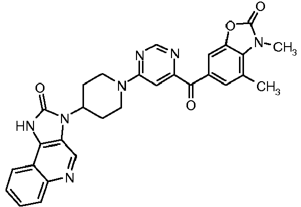 |
| (33) | 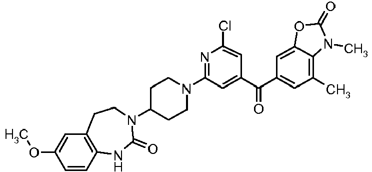 |
| (34) | 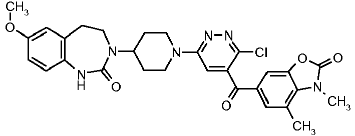 |
| (35) | 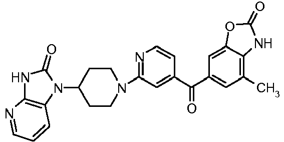 |
| (36) | 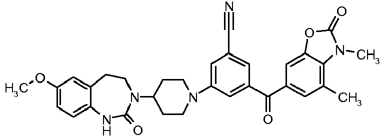 |
| (37) | 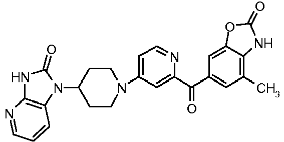 |
| (38) | 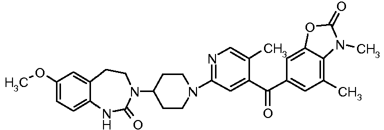 |
| (39) | 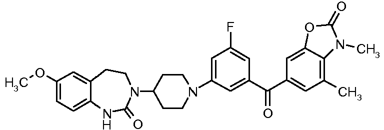 |
| (40) | 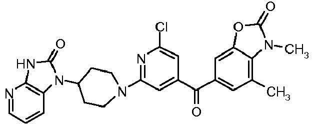 |
| (41) | 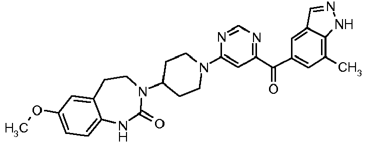 |
| (42) | 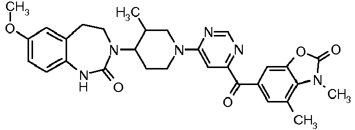 |
| (43) | 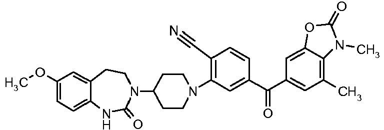 |
| (44) | 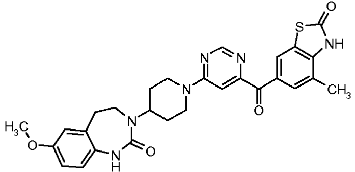 |
| (45) | 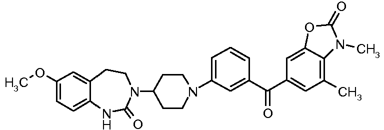 |
| (46) | 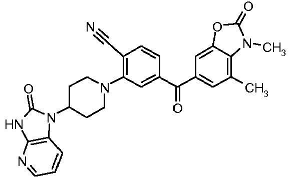 |
| (47) | 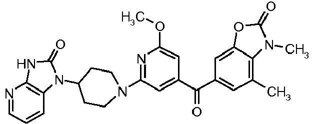 |
| (48) | 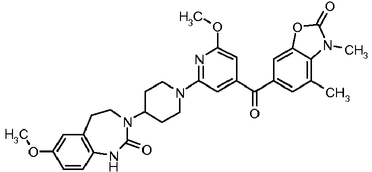 |
| (49) | 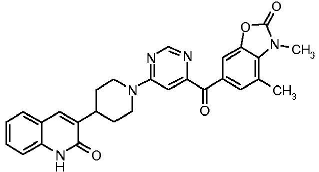 |
| (50) | 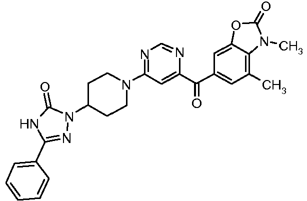 |
| (51) | 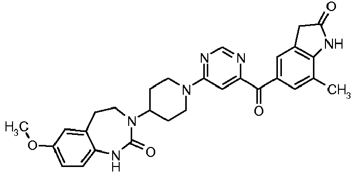 |
| (52) | 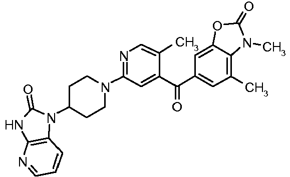 |
| (53) | 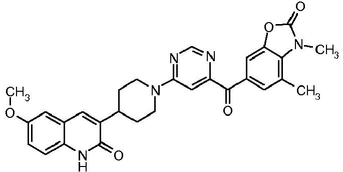 |
| (54) | 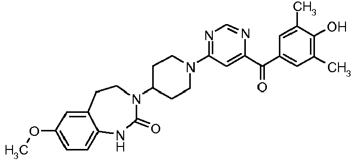 |
| (55) | 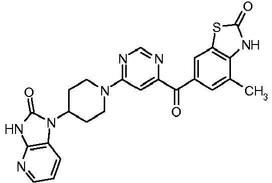 |
| (56) | 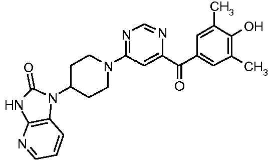 |
| (57) | 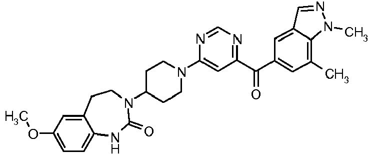 |
| (58) | 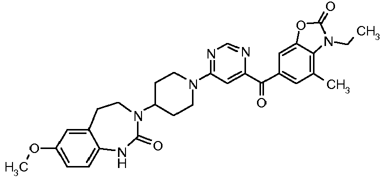 |
| (59) | 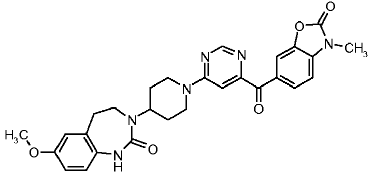 |
| (60) | 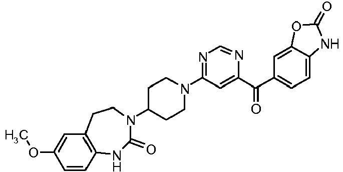 |
| (61) | 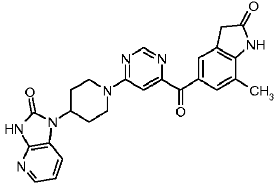 |
| (62) | 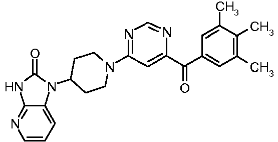 |
| (63) | 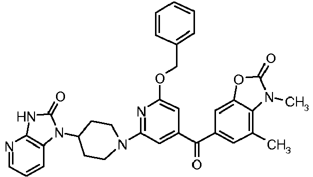 |
| (64) | 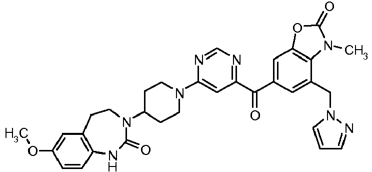 |
| (65) | 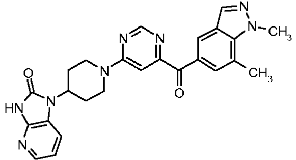 |
| (66) | 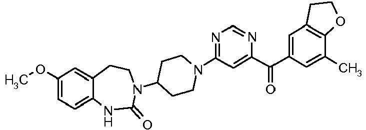 |
| (67) | 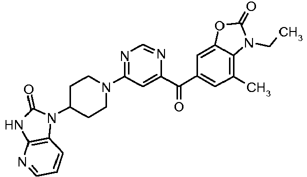 |
| (68) | 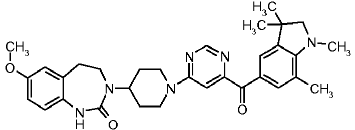 |
| (69) | 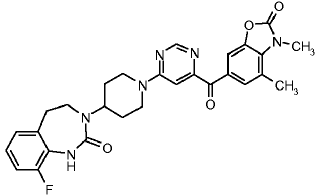 |
| (70) | 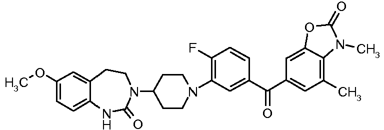 |
| (71) | 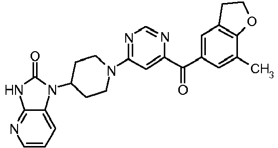 |
| (72) | 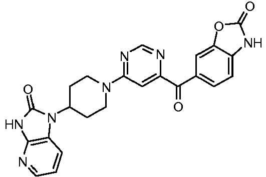 |
| (73) | 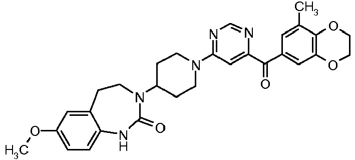 |
| (74) | 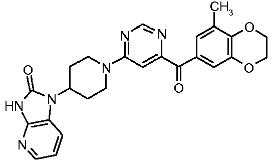 |
| (75) | 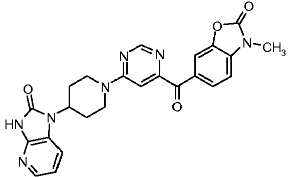 |
| (76) | 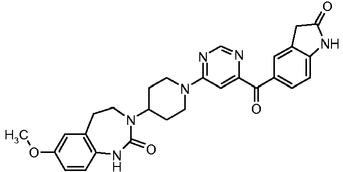 |
| (77) | 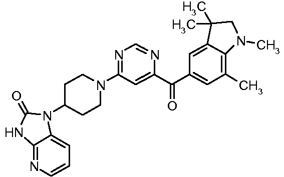 |
| (78) | 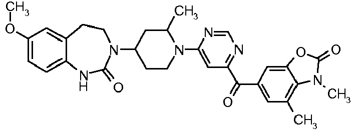 |
| (79) | 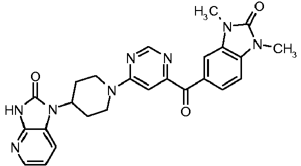 |
| (80) | 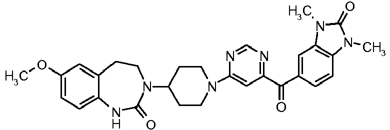 |
| (81) | 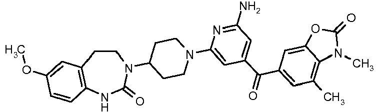 |
| (82) | 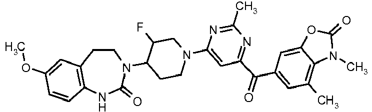 |
| (83) | 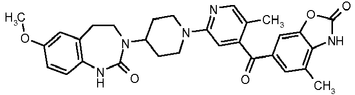 |
| (84) | 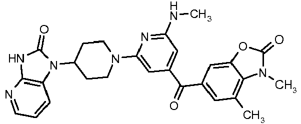 |
| (85) | 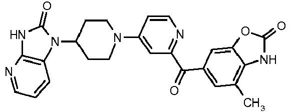 |
the enantiomers, the diastereomers, the hydrates, the mixtures thereof and the salts thereof as well as the hydrates of the salts, particularly the physiologically acceptable salts thereof with inorganic or organic acids or bases.

13. Physiologically acceptable salts of the compounds according to one of claims 1 to 12 with inorganic or organic acids or bases.

14. Pharmaceutical compositions, containing a compound according to one of claims 1 to 12 or a physiologically acceptable salt according to claim 13 optionally together with one or more inert carriers and/or diluents.

15. Use of a compound according to one of claims 1 to 13 for preparing a pharmaceutical composition for the acute and prophylactic treatment of headaches, particularly migraine or cluster headaches.

16. Use of a compound according to one of claims 1 to 13 for preparing a pharmaceutical composition for treating non-insulin-dependent diabetes mellitus ("NIDDM"), complex regional pain syndrome (CRPS1), cardiovascular diseases, morphine tolerance, diarrhoea caused by clostridium toxin, skin diseases, particularly thermal and radiation-induced damage including sunburn, inflammatory diseases, e.g. inflammatory diseases of the joints (arthritis), neurogenic inflammation of the oral mucosa, inflammatory lung diseases, allergic rhinitis, asthma, diseases accompanied by excessive vasodilatation and resultant reduced vascular blood flow, e.g. shock and sepsis, for relieving pain, or for preventive or acute therapeutic treatment of the symptoms of menopausal hot flushes caused by vasodilatation and increased blood flow in oestrogen-deficient women and hormone-treated patients with prostate carcinoma.

17. Process for preparing a pharmaceutical composition according to claim 14, **characterised in that** a compound according to one of claims 1 to 13 is incorporated in one or more inert carriers and/or diluents by a non-chemical method.

## Revendications

1. Composés de formule générale I 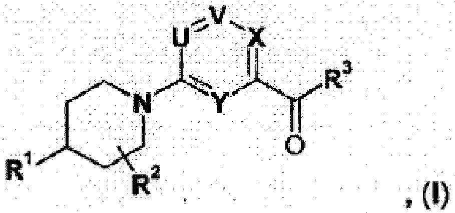 dans laquelle
R¹ représente un groupe de formule générale 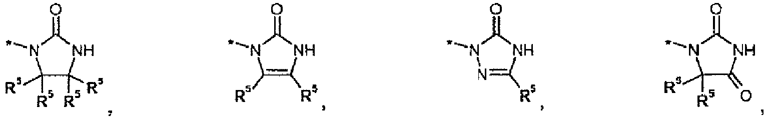 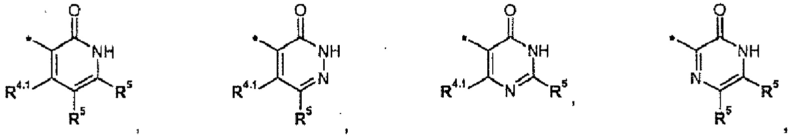 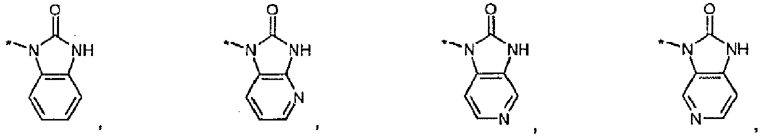 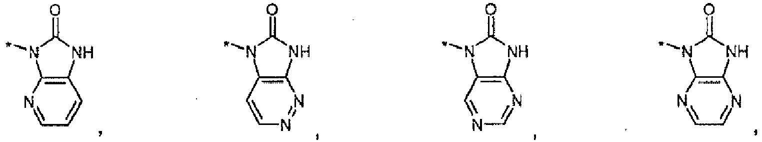 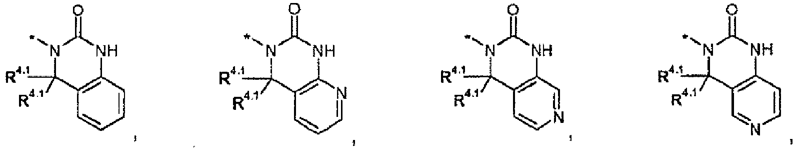 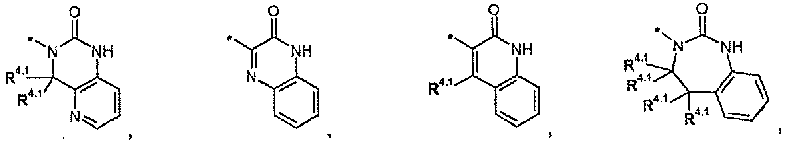 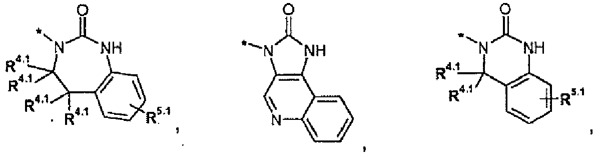
R² est
(a) H,
(b) F, -CN, un groupe alkyle en C₁ à C₃, -CO₂-R^{2.1} ou
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
R^{2.1} est H ou un groupe alkyle en C₁ à C₆
R³ est un groupe aryle de 6 ou 10 chaînons substitué par les radicaux R^{3.1}, R^{3.2} et R^{3.3} ou, un groupe hétéroaryle de 6 chaînons substitué par les radicaux R^{3.1}, R^{3.2} et R^{3.3} qui est lié par un atome de carbone,
R^{3.1} est
(a) H
(b) un atome d'halogène, -NH₂, un groupe (alkyle en C₁ à C₄) -NH-, (alkyle en C₁ à C₄) ₂N-, (alkyle en C₁ à C₃)-C(O)-NH-, (alkyle en C₁ à C₃) -S(O₂)-NH-, -CN, -OH, -O-C(O)-NH-(alkyle en C₁ à C₃),
(c) un groupe alkyle en C₁ à C₄, R^{3.1.1}-(alkylène en C₁ à C₃), alcényle en C₂ à C₄, alcinyle en C₂ à C₄, (alkyle en C₁ à C₃)-O-, (alkyle en C₁ à C₃)-S(O)ₘ-, cyclopropyle,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
(e) -C(O)-R^{3.1.2},
(f) -S(O)₂-R^{3.1.3},
R^{3.1.1} est
(a) H,
(b) un groupe cycloalkyle en C₃ à C₆, cycloalcényle en C₅ à C₆,
(c) (R^{3.1.1.1})₂N-,
(d) un hétérocycle de 5 ou 6 chaînons, saturé, mono- ou bi-insaturé qui est substitué sur un atome d'azote par un radical R^{3.1.1.1} et sur un atome de carbone, par un ou deux radicaux R^{3.1.1.2} ou
(e) un groupe hétéroaryle qui est substitué sur un atome de carbone par un radical R^{3.1.1.2},
R^{3.1.1.1} indépendamment les uns des autres, sont
(a) H, un groupe alkyle en C₁ à C₄, cycloalkyle en C₃ à C₆,
(b) un groupe hétérocyclyle,
(c) un groupe aryl-alkylène en C₀ à C₃ ou hétéroaryl-alkylène en C₀ à C₃,
R^{3.1.1.2} indépendamment les uns des autres, sont
(a) H, F, un groupe alkyle en C₁ à C₃, -CN, -OH,-O-(alkyle en C₁ à C₃), -CO(O)R^{3.1.1.2.1}, H₂N-, (alkyle en C₁ à C₄)-NH-, (alkyle en C₁ à C₄)₂N-,
(b) un groupe phényle ou phényl-CH₂-,
(c) un groupe alkyle en C₁ à C₃ ou -O-(alkyle en C₁ à C₃), dans lequel chaque groupe méthylène est substitué par jusqu'à deux atomes de fluor et chaque groupe méthyle est substitué par jusqu'à trois atomes de fluor, ou
R^{3.1.1.2.1} est H, un groupe alkyle en C₁ à C₆, benzyle,
R^{3.1.2} est un groupe -O-(alkyle en C₁ à C₃), -OH, NR^{3.1. 2.1}R^{3.1.2.2},
R^{3.1.2.1} est H, un groupe alkyle en C₁ à C₃,
R^{3.1.2.2} est H, un groupe alkyle en C₁ à C₃,
R^{3.1.2.1} et R^{3.1.2.1} peuvent également former ensemble un cycle qui est choisi dans le groupe constitué des groupes azétidinyle, pyrrolidinyle, pipéridinyle, pipérazinyle et morpholinyle,
R^{3.1.3} est un groupe -O-(alkyle en C₁ à C₃), - NR^{3.1.3.1}R^{3.1.3.2},
R^{3.1.3.1} est H, un groupe alkyle en C₁ à C₃,
R^{3.1.3.2} est H, un groupe alkyle en C₁ à C₃,
R^{3.1.3.1} et R^{3.1.3.1} peuvent également former ensemble un cycle qui est choisi dans le groupe constitué des groupes azétidinyle, pyrrolidinyle, pipéridinyle, pipérazinyle et morpholinyle,
R^{3.2} est
(a) H
(b) un atome d'halogène, -NH₂, un groupe (alkyle en C₁ à C₄)-NH-, (alkyle en C₁ à C₄)₂N-, (alkyle en C₁ à C₃)-C(O)-NH-, (alkyle en C₁ à C₃)-S(O₂)-NH-, -CN, -OH, -O-C(O)-NH-(alkyle en C₁ à C₃),
(c) un groupe alkyle en C₁ à C₄, alcényle en C₂ à C₄, alcinyle en C₂ à C₄, (alkyle en C₁ à C₃)-O-, (alkyle en C₁ à C₃)-S(O)ₘ-, cyclopropyle,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
(e) -C(O)-R^{3.2.1},
(f) -S(0) ₂-R^{3.2.2},
R^{3.2.1} est un groupe -O-(alkyle en C₁ à C₃), -OH, - NR^{3.2.1.1}R^{3.2.1.2},
R^{3.2.1.1} est H, un groupe alkyle en C₁ à C₃,
R^{3.2.1.2} est H, un groupe alkyle en C₁ à C₃,
R^{3.2.1.1} et R^{3.2.1.2} peuvent également former ensemble un cycle qui est choisi dans le groupe constitué des groupes azétidinyle, pyrrolidinyle, pipéridinyle, pipérazinyle et morpholinyle,
R^{3.2.2} est -NR^{3.2.2.1}R^{3.2.2.2},
R^{3.2.2.1} est H, un groupe alkyle en C₁ à C₃,
R^{3.2.2.2} est H, un groupe alkyle en C₁ à C₃,
R^{3.2.2.1} et R^{3.2.2.2} peuvent également former ensemble un cycle qui est choisi dans le groupe constitué des groupes azétidinyle, pyrrolidinyle, pipéridinyle, pipérazinyle et morpholinyle,
R^{3.3} est
(a) H
(b) un atome d'halogène, -NH₂, un groupe (alkyle en C₁ à C₄)-NH-, (alkyle en C₁ à C₄)₂N-, (alkyle en C₁ à C₃)-C(O)-NH-, (alkyle en C₁ à C₃)-S(O₂)-NH-, -CN, -OH, -O-C(O)-NH-(alkyle en C₁ à C₃),
(c) un groupe alkyle en C₁ à C₄, alcényle en C₂ à C₄, alcinyle en C₂ à C₄, (alkyle en C₁ à C₃)-O-, (alkyle en C₁ à C₃)-S(O)ₘ-, cyclopropyle,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
(e) -C (O) -R^{3.3.1},
(f) -S(O)₂-R^{3.3.2},
R^{3.3.1} est un groupe -O-(alkyle en C₁ à C₃), -OH, - NR^{3.3.1.1}R^{3.3.1.2},
R^{3.3.1.1} est H, un groupe alkyle en C₁ à C₃,
R^{3.3.1.2} est H, un groupe alkyle en C₁ à C₃,
R^{3.3.1.1} et R^{3.3.1.2} peuvent également former ensemble un cycle qui est choisi dans le groupe constitué des groupes azétidinyle, pyrrolidinyle, pipéridinyle, pipérazinyle et morpholinyle,
R^{3.3.2} est un groupe -O-(alkyle en C₁ à C₃),-NR^{3.3.2.1}R^{3.3.2.2},
R^{3.3.2.1} est H, un groupe alkyle en C₁ à C₃,
R^{3.3.2.2} est H, un groupe alkyle en C₁ à C₃,
R^{3.3.2.1} et R^{3.3.2.2} peuvent également former ensemble un cycle qui est choisi dans le groupe constitué des groupes azétidinyle, pyrrolidinyle, pipéridinyle, pipérazinyle et morpholinyle, ou
R^{3.2} et R^{3.3} forment conjointement avec les atomes de carbone auxquels ils sont liés, un hétérocycle à 5 chaînons mono-insaturé ou à 6 chaînons mono- ou bi-insaturé ou un groupe hétéroaryle de 5 à 6 chaînons, dans lequel
les hétérocycles mentionnés précédemment peuvent contenir un groupe carbonyle, thiocarbonyle ou cyanimino voisins d'un atome d'azote, et
éventuellement en outre, peuvent être substitués sur un ou deux atomes d'azote, respectivement par un radical R^{3.3.3} et
éventuellement en outre, peuvent être substitués sur un ou deux atomes de carbone, respectivement par un ou deux radicaux R^{3.3.4},
R^{3.3.3} indépendamment les uns des autres, sont
(a) un groupe alkyle en C₁ à C₄ ou
(b) un groupe cycloalkyle en C₃ à C₆,
R^{3.3.4} indépendamment les uns des autres, sont
(a) un groupe alkyle en C₁ à C₄ ou
(b) un groupe cycloalkyle en C₃ à C₆,
(c) un atome d'halogène, CN, un groupe -O-(alkyle en C₁ à C₃), -NH₂,
(d) un groupe alkyle en C₁ à C₃ ou (alkyle en C₁ à C₃)-O- dans lequel chaque groupe méthylène est substitué par jusqu'à deux, et chaque groupe méthyle, par jusqu'à trois atomes de fluor,
R^{4.1} indépendamment les uns des autres, sont
(a) H
(b) un groupe alkyle en C₁ à C₃, -OH, -O-(alkyle en C₁ à C₃),
(c) un groupe alkyle en C₁ à C₃ ou (alkyle en C₁ à C₃)-O dans lequel chaque groupe méthylène est substitué par jusqu'à deux atomes de fluor et chaque groupe méthyle est substitué par jusqu'à trois atomes de fluor,
R⁵ indépendamment les uns des autres, sont
(a) H, un groupe alkyle en C₁ à C₆, cycloalkyle en C₃ à C₆,
(b) un groupe phényle éventuellement substitué par 1, 2 ou 3 substituants R^{5.2}, les substituants R^{5.2} pouvant être identiques ou différents,
(c) un groupe hétéroaryle substitué éventuellement par 1, 2 ou 3 substituants R^{5.2} qui est choisi dans le groupe constitué des groupes benzimidazol, benzothiophène, furane, imidazol, indol, ixoxazol, oxazol, pyrazin, pyrazol, pyridazine, pyridine, pyrimidine, pyrrol, thiazol, thiophène et triazol, les substituants R^{5.2} pouvant être identiques ou différents,
(d) un hétérocycle éventuellement substitué par 1, 2 ou 3 substituants R^{5.2}, les substituants R^{5.2} pouvant être identiques ou différents,
R^{5.1} est
(a) H, un atome d'halogène, un groupe alkyle en C₁ à C₆, cycloalkyle en C₃ à C₆,
(b) -O-(alkylène en C₁ à C₆)-NR⁷R⁸, -O-R⁶, -CO₂-R⁶, -C (O) -NR⁷R⁸, -SO₂-NR⁷R⁸, -NR⁷-SO₂-R⁸, -S(O)ₘ-R⁷, -CN, -NR⁷R⁸, O-C(O)-R⁶,
(c) est un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
R^{5.2} est
(a) un atome d'halogène, un groupe alkyle en C₁ à C₆, cycloalkyle en C₃ à C₆,
(b) -O-R⁶, -O-(CH₂)ₛ-O-R⁶, -CO₂R⁶, -S(O)ₘ-R⁷, -CN, - O-C-(O)-R⁶ ou
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
R⁶ est
(a) H,
(b) un groupe alkyle en C₁ à C₆, cycloalkyle en C₃ à C₆, aryle, hétéroaryle, benzyle qui peut être substitué par un radical R^{6.1}, ou
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
R^{6.1} est HO- ou un groupe alkyle en C₁ à C₆-O-,
R⁷ est
(a) H,
(b) un groupe alkyle en C₁ à C₃, phényle ou benzyle, les radicaux pouvant être non substitués ou substitués par un atome d'halogène, HO- ou H₃C-O-,
R⁸ est
(a) H,
(b) un groupe alkyle en C₁ à C₃, phényle ou benzyle, les radicaux pouvant être non substitués ou substitués par un atome d'halogène, HO- ou H₃C-O-, ou
R⁷ et R⁸ peuvent former ensemble également un cycle qui est choisi dans le groupe constitué des groupes azétidinyle, pyrrolidinyle, pipéridinyle, pipérazinyle et morpholinyle, le cycle pouvant être non substitué ou substitué par un substituant R⁶,
m est l'un des chiffres 0, 1 ou 2,
s est l'un des chiffres 1, 2 ou 3,
U est N, un groupe N-oxyde ou C-R⁹,
V est N, un groupe N-oxyde ou C-R¹⁰,
X est N, un groupe N-oxyde ou C-R¹¹,
Y est N ou C-R¹²,
où au plus trois des radicaux U, V, X ou Y mentionnés précédemment représentent en même temps un atome d'azote,
R⁹ est
(a) H,
(b) un groupe alkyle en C₁ à C₆ ou alkyle en C₁ à C₃-O- qui est substitué respectivement par un radical R^{9.1},
(c) R^{9.2}R^{9.3}N-, R^{9.2}R^{9.3}N-(alkylène en C₁ à C₃),
(d) un atome d'halogène, -CN, -OH, -COOH, un groupe (alkyle en C₁ à C₃)-O-(alkylène en C₁ à C₃), cycloalkyle en C₃ à C₆, (cycloalkyle en C₃ à C₆)-(alkylène en C₁ à C₄), (alkyle en C₁ à C₃)-C(O)-O-(alkylène en C₁ à C₃),
(e) un groupe alkyle en C₁ à C₃ ou alkyle en C₁ à C₃-O- dans lequel chaque groupe méthylène est substitué par jusqu'à deux atomes de fluor et chaque groupe méthyle est substitué par jusqu'à trois atomes de fluor,
R^{9.1} est H, OH ou -O-CH₃,
R^{9.2} est H ou un groupe alkyle en C₁ à C₃,
R^{9.3} est H ou un groupe alkyle en C₁ à C₃, ou
R^{9.2} et R^{9.3} conjointement avec l'atome d'azote auquel ils sont liés sont un hétérocycle de 3 à 6 chaînons,
R¹⁰ est
(a) H
(b) un groupe alkyle en C₁ à C₆ ou (alkyle en C₁ à C₃)-O- qui est substitué respectivement par un radical R^{10.1},
(c) -NR^{10.2}R^{10.3}, NR^{10.2}R^{10.3}-(alkylène en C₁ à C₃),
(d) un atome d'halogène, -CN, -OH, un groupe (alkyle en C₁ à C₃)-O-(alkylène en C₁ à C₃), cycloalkyle en C₃ à C₆, (cycloalkyle en C₃ à C₆)-(alkylène en C₁ à C₄), (alkyle en C₁ à C₃)-C(O)-O-(alkylène en C₁ à C₃),
(e) un groupe aryl-(alkylène en C₀ à C₃)-O-
(f) un groupe alkyle en C₁ à C₃ ou (alkyle en C₁ à C₃)-O-, dans lequel chaque groupe méthylène est substitué par jusqu'à deux atomes de fluor et chaque groupe méthyle est substitué par jusqu'à trois atomes de fluor,
R^{10.1} est H, OH ou -O-CH₃,
R^{10.2} est H ou un groupe alkyle en C₁ à C₆,
R^{10.3} est H, un groupe alkyle en C₁ à C₆ ou -SO₂-(alkyle en C₁ à C₃), ou
R^{10.2} et R^{10.3} conjointement avec l'atome d'azote auquel ils sont liés sont un hétérocycle de 3 à 6 chaînons,
R¹¹ est
(a) H,
(b) un groupe alkyle en C₁ à C₆ ou (alkyle en C₁ à C₃)-O- qui est substitué respectivement par un radical R^{11.1},
(c) R^{11.2}R^{11.3}N-, R^{11.2}R^{11.3}N- (alkylène en C₁ à C₃),
(d) un atome d'halogène, -CN, -OH, -COOH, un groupe (alkyle en C₁ à C₃)-O-(alkylène en C₁ à C₃), cycloalkyle en C₃ à C₆, (cycloalkyle en C₃ à C₆)-(alkylène en C₁ à C₄), (alkyle en C₁ à C₃)-C(O)-O-(alkylène en C₁ à C₃),
(e) un groupe alkyle en C₁ à C₃ ou (alkyle en C₁ à C₃)-O-, dans lequel chaque groupe méthylène est substitué par jusqu'à deux atomes de fluor et chaque groupe méthyle est substitué par jusqu'à trois atomes de fluor,
R^{11.1} est H, OH ou -O-CH₃,
R^{11.2} est H ou un groupe alkyle en C₁ à C₃,
R^{11.3} est H, un groupe alkyle en C₁ à C₃ ou
R^{11.2} et R^{11.3} conjointement avec l'atome d'azote auquel ils sont liés sont un hétérocycle de 3 à 6 chaînons, et
R¹² est H, un atome d'halogène ou un groupe alkyle en C₁ à C₃,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

2. Composés de formule générale I selon la revendication 1, dans laquelle U, V, X, Y, R² et R³ sont tels que définis dans la revendication 1 et
R¹ est un groupe choisi parmi 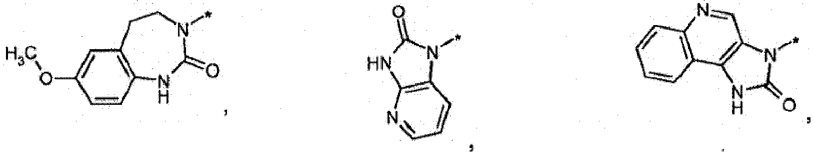 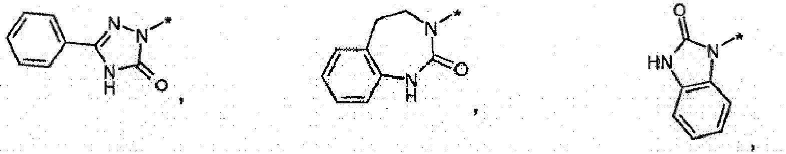 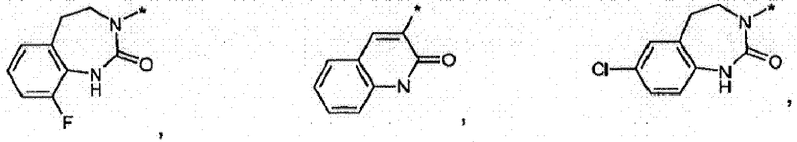
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

3. Composés de formule générale I selon la revendication 1, dans laquelle U, V, X, Y, R¹ et R³ sont tels que définis dans la revendication 1 ou 2 et R² est un atome d'hydrogène,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

4. Composés de formule générale I selon la revendication 1, dans laquelle U, V, X, Y, R¹ et R² sont tels que définis dans la revendication 1, 2 ou 3 et
R³ est un groupe de formule générale III 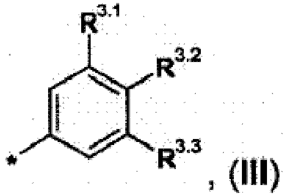
R^{3.1} est
(a) H
(b) F, Cl, Br, -NH₂, un groupe (alkyle en C₁ à C₃)-NH-, (alkyle en C₁ à C₃)₂N-, (alkyle en C₁ à C₃)-C(O)-NH-, -CN, -OH,
(c) un groupe alkyle en C₁ à C₄, alcényle en C₂ à C₄, alcinyle en C₂ à C₄, (alkyle en C₁ à C₃)-O-, (alkyle en C₁ à C₃)-S-,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
R^{3.2} est
(a) H
(b) F, Cl, Br, H₂N, un groupe (alkyle en C₁ à C₄)-NH-, (alkyle en C₁ à C₄)₂N-, (alkyle en C₁ à C₃)-C(O)-NH-, -OH,
(c) un groupe alkyle en C₁ à C₄,
(d) un groupe alkyle en C₁ à C₃ ou (alkyle en C₁ à C₃)-O-, dans lequel chaque groupe méthylène est substitué par jusqu'à deux atomes de fluor et chaque groupe méthyle est substitué par jusqu'à trois atomes de fluor,
R^{3.3} est
(a) H
(b) F, Cl, Br, H₂N, un groupe (alkyle en C₁ à C₄)-NH-, (alkyle en C₁ à C₄)₂N-, (alkyle en C₁ à C₃)-C(O)-NH-, -OH,
(c) un groupe alkyle en C₁ à C₄,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor, ou
R^{3.2} et R^{3.3} forment conjointement avec les atomes de carbone auxquels ils sont liés, un hétérocycle à 5 chaînons mono-insaturé ou à 6 chaînons mono- ou bi-insaturé ou un groupe hétéroaryle de 5 à 6 chaînons, dans lequel
les hétérocycles mentionnés précédemment peuvent contenir un groupe carbonyle, thiocarbonyle ou cyanimino voisins d'un atome d'azote, et éventuellement en outre, peuvent être substitués sur un ou deux atomes d'azote, respectivement par un radical R^{3.3.3} et
éventuellement en outre, peuvent être substitués sur un ou deux atomes de carbone, respectivement par un ou deux radicaux R^{3.3.4},
R^{3.3.3} indépendamment les uns des autres, sont
(a) un groupe alkyle en C₁ à C₄ ou
(b) un groupe cycloalkyle en C₃ à C₆, et
R^{3.3.4} indépendamment les uns des autres, sont
(a) un groupe alkyle en C₁ à C₄, cycloalkyle en C₃ à C₆,
(b) un atome d'halogène, CN, un groupe (alkyle en C₁ à C₃)-O-, -NH₂,
(c) un groupe alkyle en C₁ à C₃ ou (alkyle en C₁ à C₃)-O- dans lequel chaque groupe méthylène est substitué par jusqu'à deux atomes de fluor, et chaque groupe méthyle, par jusqu'à trois atomes de fluor,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

5. Composés de formule générale I selon la revendication 1, dans laquelle U, V, X, Y, R¹ et R² sont tels que définis dans la revendication 1, 2 ou 3 et
R³ est un groupe choisi parmi 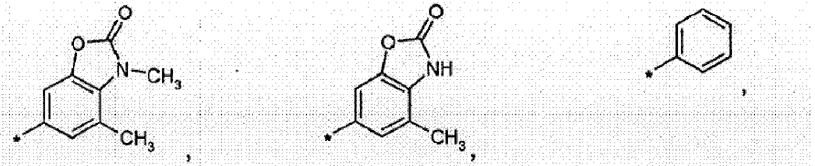 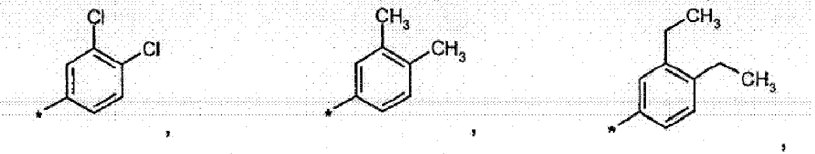 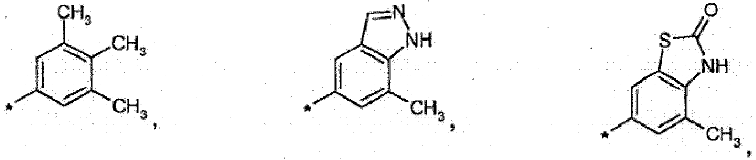 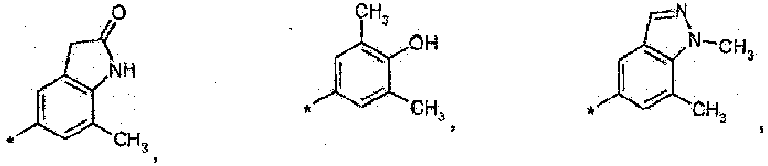 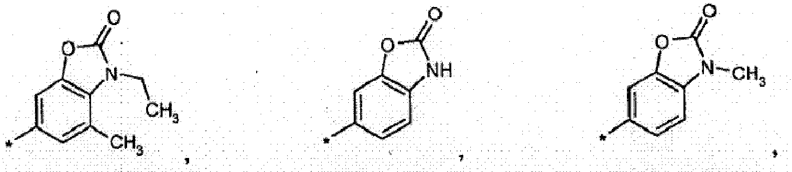 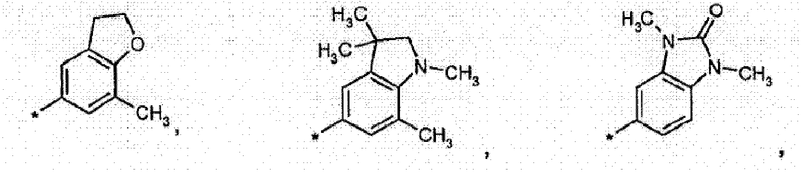 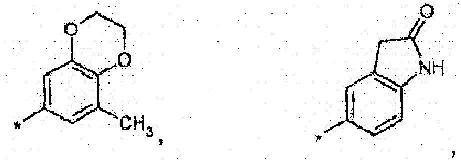
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

6. Composés de formule générale I selon la revendication 1, dans laquelle U, V, X, Y, R¹ et R² sont tels que définis dans la revendication 1, 2 ou 3 et
R³ est un groupe de formule générale III 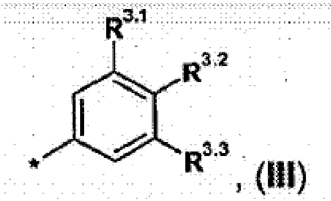
R^{3.1} est
(a) H
(b) F, Cl, Br, -NH₂, un groupe (alkyle en C₁ à C₃)-NH-, (alkyle en C₁ à C₃)₂N-, (alkyle en C₁ à C₃)-C(O)-NH-, -CN, -OH,
(c) un groupe alkyle en C₁ à C₄, alcényle en C₂ à C₄, alcinyle en C₂ à C₄, (alkyle en C₁ à C₃)-O-, (alkyle en C₁ à C₃)-S-,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
R^{3.2} et R^{3.3} forment conjointement avec les atomes de carbone auxquels ils sont liés, un hétérocycle à 5 chaînons mono-insaturé ou à 6 chaînons mono- ou bi-insaturé ou un groupe hétéroaryle de 5 à 6 chaînons, dans lequel
les hétérocycles mentionnés précédemment peuvent contenir un groupe carbonyle, thiocarbonyle ou cyanimino voisins d'un atome d'azote, et
éventuellement en outre, peuvent être substitués sur un ou deux atomes d'azote, respectivement par un radical R^{3.3.3} et
éventuellement en outre, peuvent être substitués sur un ou deux atomes de carbone, respectivement par un ou deux radicaux R^{3.3.4},
R^{3.3.3} indépendamment les uns des autres, sont
(a) un groupe alkyle en C₁ à C₄ ou
(b) un groupe cycloalkyle en C₃ à C₆, et
R^{3.3.4} indépendamment les uns des autres, sont
(a) un groupe alkyle en C₁ à C₄, cycloalkyle en C₃ à C₆,
(b) un atome d'halogène, -CN, un groupe -O-(alkyle en C₁ à C₃), -NH₂,
(c) un groupe alkyle en C₁ à C₃ ou (alkyle en C₁ à C₃)-O- dans lequel chaque groupe méthylène est substitué par jusqu'à deux atomes de fluor, et chaque groupe méthyle, par jusqu'à trois atomes de fluor,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

7. Composés de formule générale I selon la revendication 1, dans laquelle U, V, X, Y, R¹ et R² sont tels que définis dans la revendication 1, 2 ou 3 et
R³ est un groupe de formule générale III 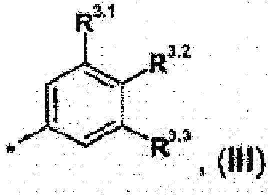
R^{3.1} est
(a) H
(b) F, Cl, Br, -NH₂, un groupe (alkyle en C₁ à C₃)-NH-, (alkyle en C₁ à C₃)₂N-, (alkyle en C₁ à C₃)-C(O)-NH-, -CN, -OH,
(c) un groupe alkyle en C₁ à C₄, alcényle en C₂ à C₄, alcinyle en C₂ à C₄, (alkyle en C₁ à C₃)-O-, (alkyle en C₁ à C₃)-S-,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
R^{3.2} et R^{3.3} forment conjointement avec les atomes de carbone auxquels ils sont liés, un hétérocycle à 5 chaînons mono-insaturé ou un groupe hétéroaryle à 5 chaînons, dans lequel
les hétérocycles mentionnés précédemment peuvent contenir un groupe carbonyle, thiocarbonyle ou cyanimino voisins d'un atome d'azote, et éventuellement en outre, peuvent être substitués sur un ou deux atomes d'azote, respectivement par un radical R^{3.3.3} et
éventuellement en outre, peuvent être substitués sur un ou deux atomes de carbone, respectivement par un ou deux radicaux R^{3.3.4},
R^{3.3.3} indépendamment les uns des autres, sont
(a) un groupe alkyle en C₁ à C₄ ou
(b) un groupe cycloalkyle en C₃ à C₆, et
R^{3.3.4} indépendamment les uns des autres, sont
(a) un groupe alkyle en C₁ à C₄, cycloalkyle en C₃ à C₆,
(b) un atome d'halogène, -CN, un groupe -O-(alkyle en C₁ à C₃), -NH₂,
(c) un groupe alkyle en C₁ à C₃ ou (alkyle en C₁ à C₃)-O- dans lequel chaque groupe méthylène est substitué par jusqu'à deux atomes de fluor et chaque groupe méthyle par jusqu'à trois atomes de fluor,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

8. Composés de formule générale I selon la revendication 1, dans laquelle U, V, X, Y, R¹ et R² sont tels que définis dans la revendication 1, 2 ou 3 et
R³ est un groupe de formule générale IIIa 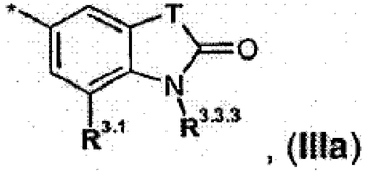
T est O, S, CH₂, NH ou N-R^{3.3.3},
R^{3.1} est
(a) H
(b) F, Cl, Br, -NH₂, un groupe (alkyle en C₁ à C₃)-NH-, (alkyle en C₁ à C₃)₂N-, (alkyle en C₁ à C₃)-C(O)-NH-, -CN, -OH,
(c) un groupe alkyle en C₁ à C₄, alcényle en C₂ à C₄, alcinyle en C₂ à C₄, (alkyle en C₁ à C₃)-O-, (alkyle en C₁ à C₃)-S-,
(d) un groupe alkyle en C₁ à C₃ ou (alkyle en C₁ à C₃)-O- dans lequel chaque groupe méthylène est substitué par jusqu'à deux atomes de fluor et chaque groupe méthyle est substitué par jusqu'à trois atomes de fluor,
R^{3.3.3} indépendamment les uns des autres, sont
(a) un groupe alkyle en C₁ à C₄ ou
(b) un groupe cycloalkyle en C₃ à C₆,
leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

9. Composés de formule générale I selon la revendication 1, dans laquelle U, V, X, Y, R¹ et R² sont tels que définis dans la revendication 1, 2 ou 3 et
R³ est un groupe choisi parmi 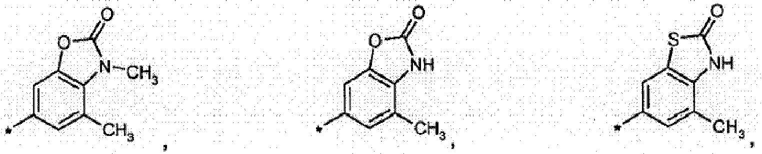 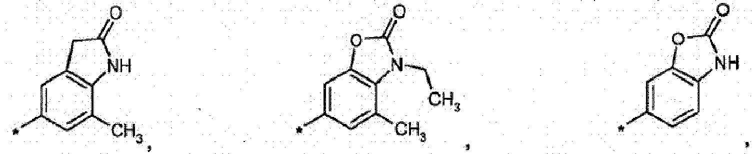 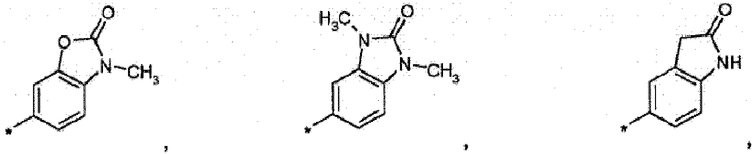 leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

10. Composés de formule générale I selon la revendication 1, dans laquelle Y, R¹, R² et R³ sont tels que définis dans la revendication 1, 2, 3, 4, 5, 6, 7, 8 ou 9 et le cycle 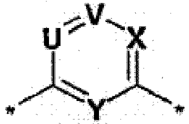 est un groupe choisi parmi 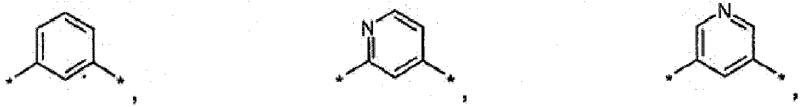 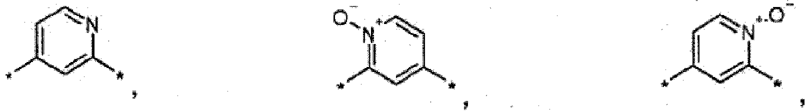 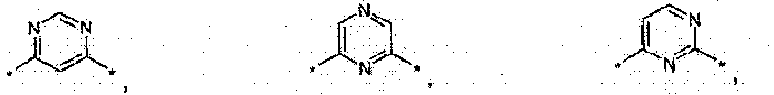 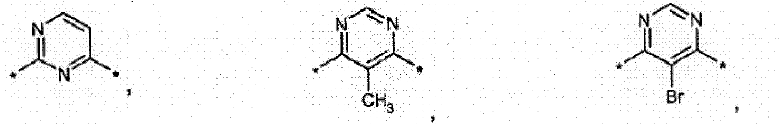 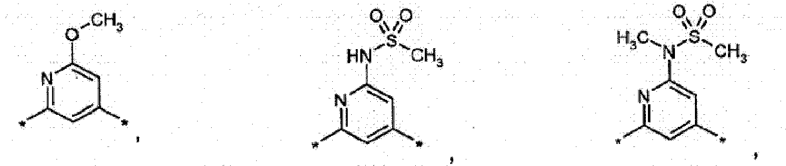 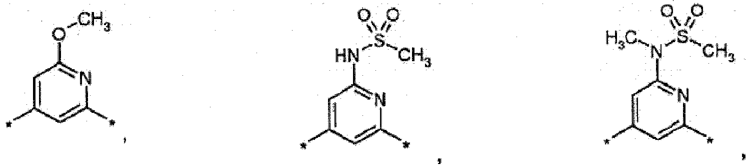 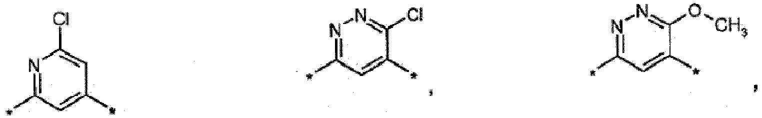 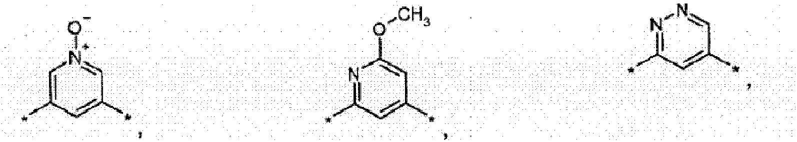 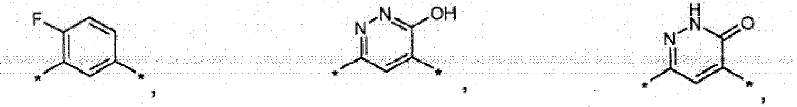 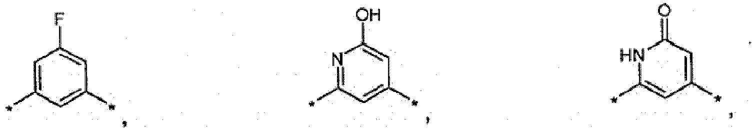 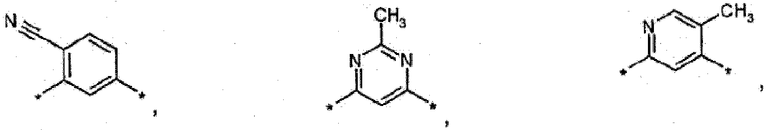 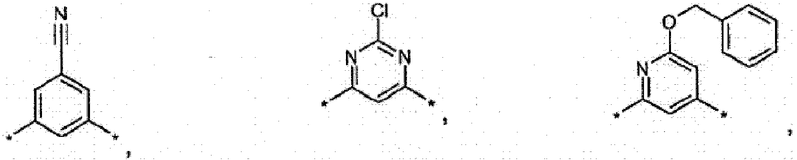 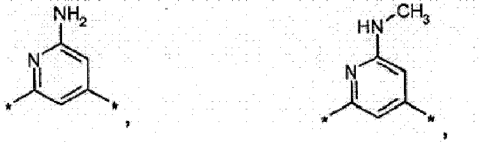 leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

11. Composés de formule générale I selon la revendication 1, dans laquelle
R¹ est un groupe choisi parmi 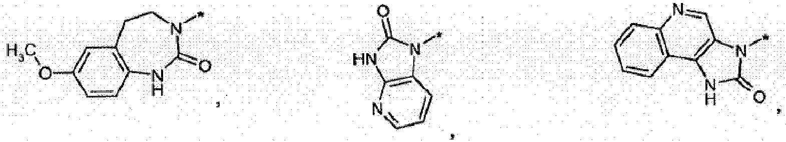 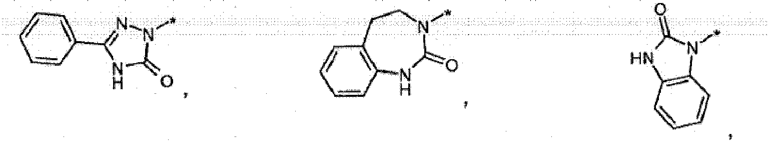 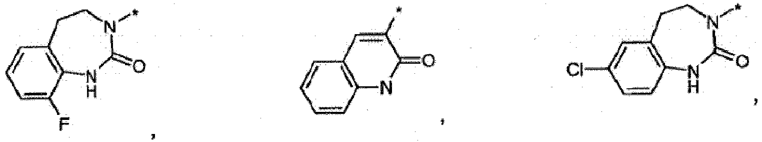
R² est H et
R³ est un groupe choisi parmi 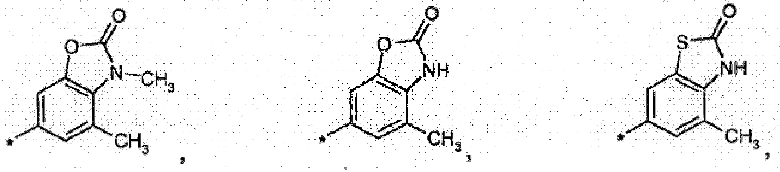 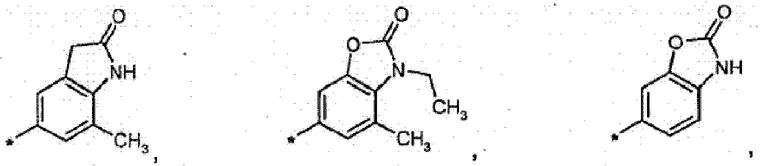 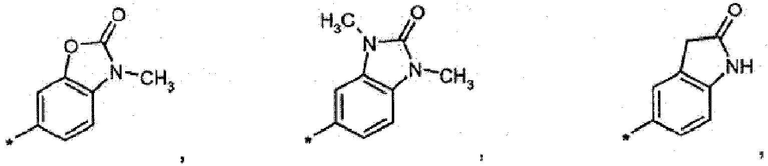 et le cycle 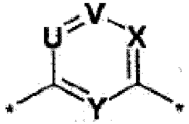 est un groupe choisi parmi 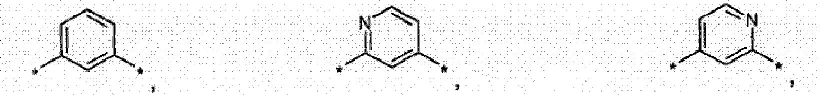 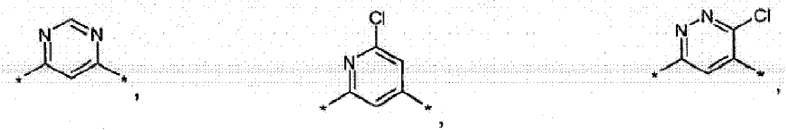 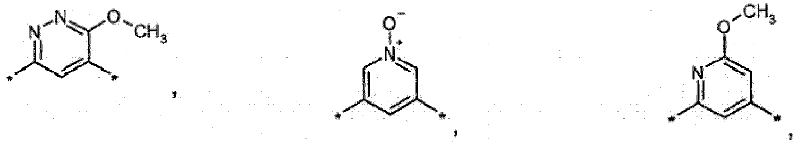 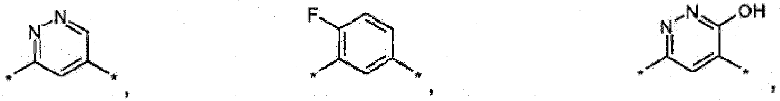 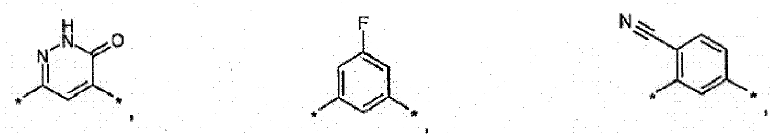 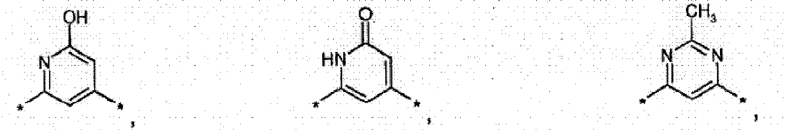 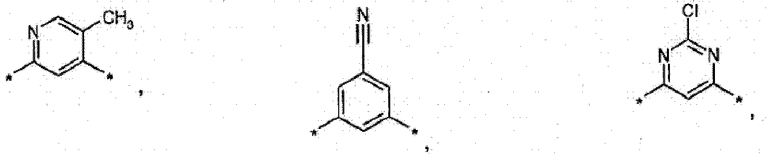 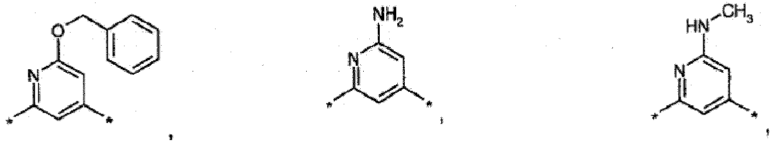 leurs tautomères, leurs diastéréomères, leurs énantiomères, leurs hydrates, leurs mélanges et leurs sels et les hydrates des sels, en particulier, leurs sels physiologiquement acceptables avec des acides ou des bases inorganiques ou organiques.

12. Composés de formule générale I selon la revendication 1 : 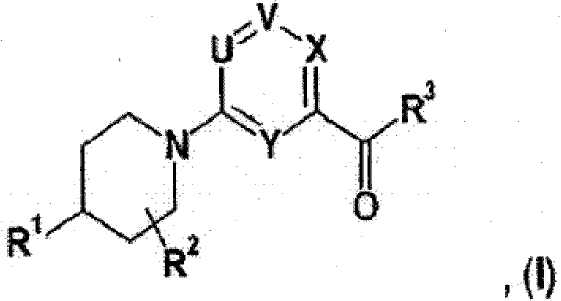
| N° | structure |
|---|---|
| (1) | 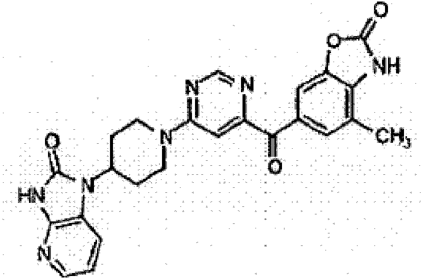 |
| (2) | 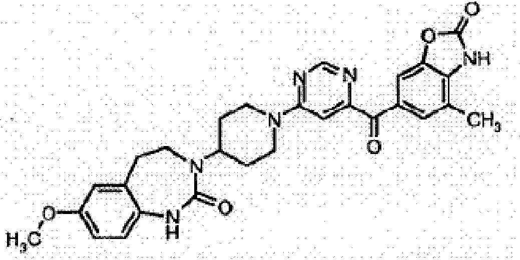 |
| (3) | 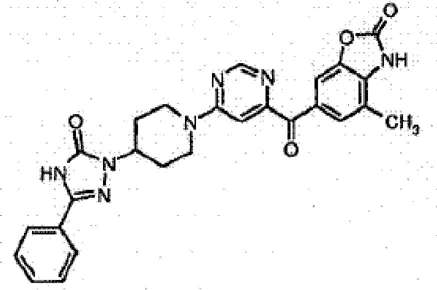 |
| (4) | 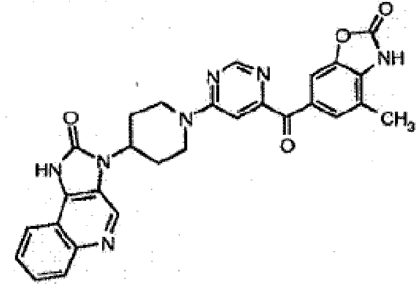 |
| (5) | 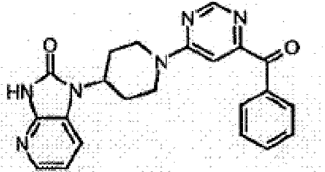 |
| (6) | 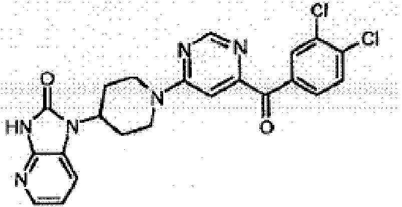 |
| (7) | 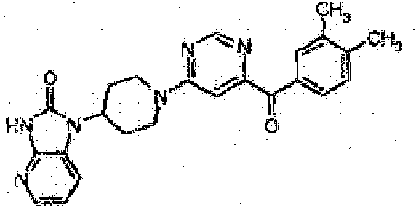 |
| (8) | 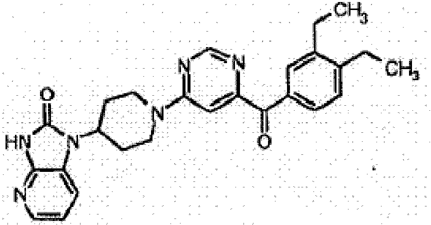 |
| (9) | 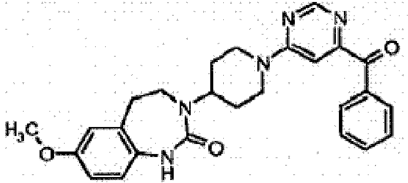 |
| (10) | 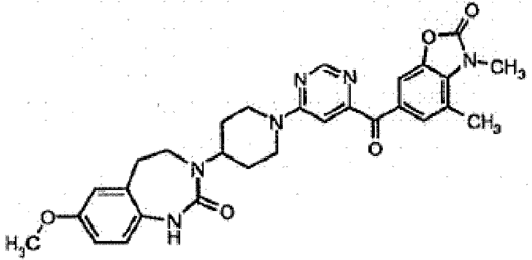 |
| (11) | 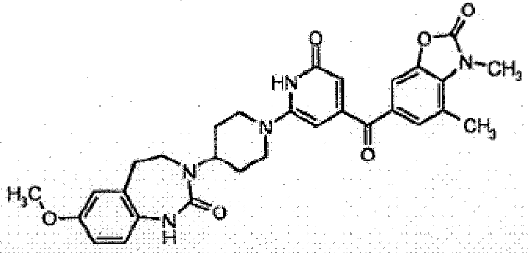 |
| (12) | 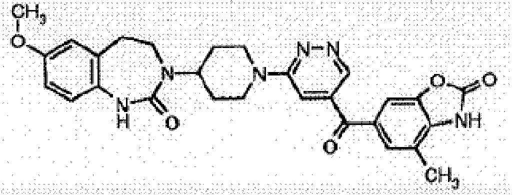 |
| (13) | 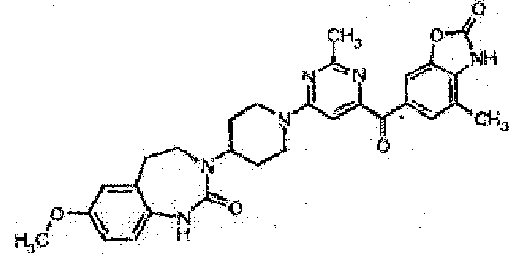 |
| (14) | 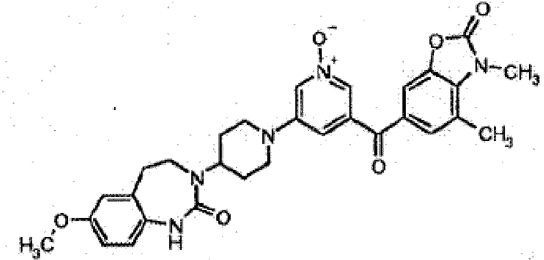 |
| (15) | 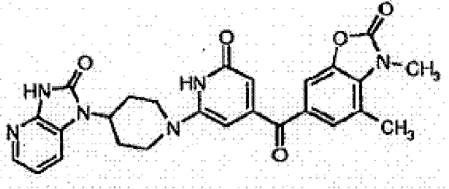 |
| (16) | 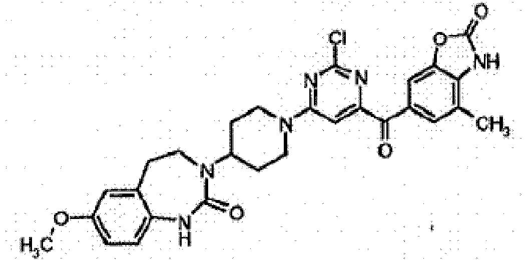 |
| (17) | 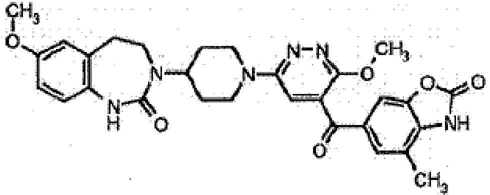 |
| (18) | 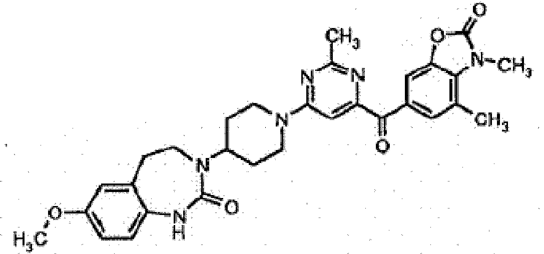 |
| (19) | 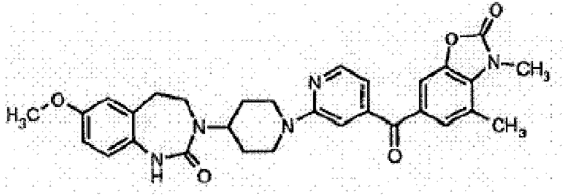 |
| (20) | 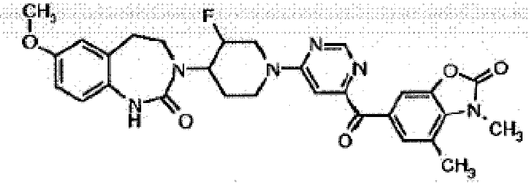 |
| (21) | 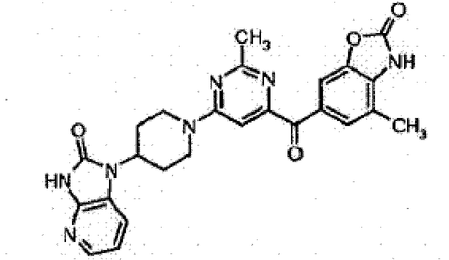 |
| (22) | 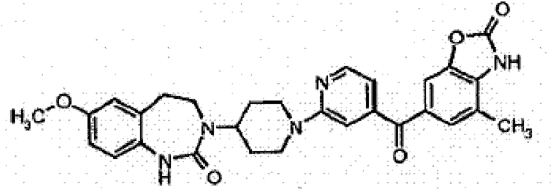 |
| (23) | 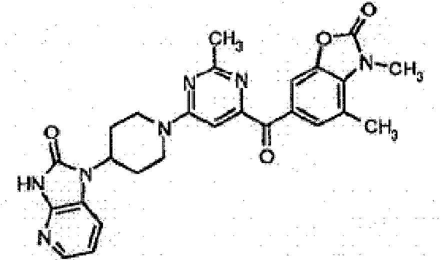 |
| (24) | 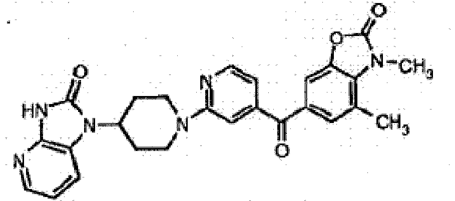 |
| (25) | 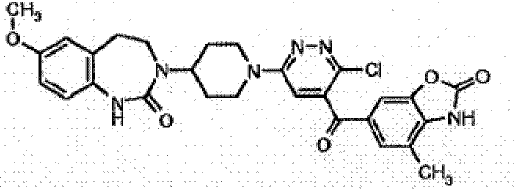 |
| (26) | 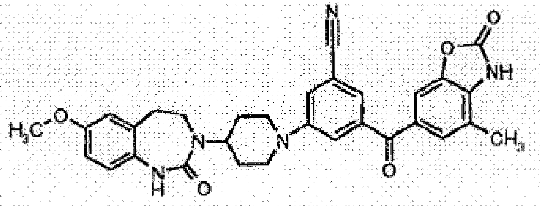 |
| (27) | 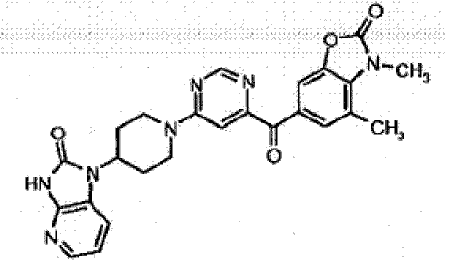 |
| (28) | 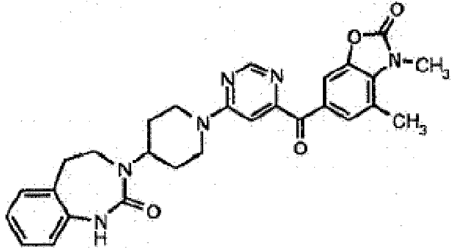 |
| (29) | 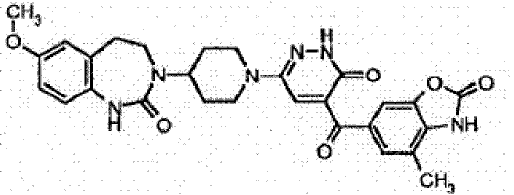 |
| (30) | 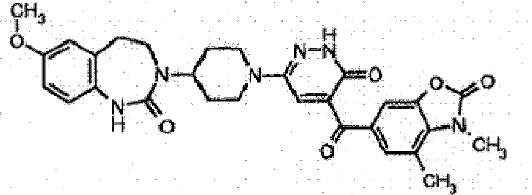 |
| (31) | 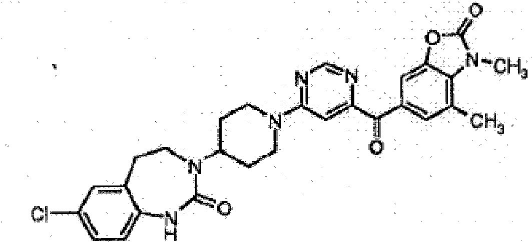 |
| (32) | 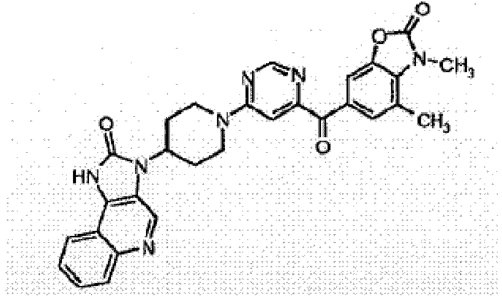 |
| (33) | 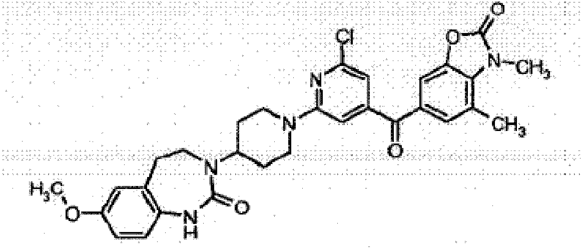 |
| (34) | 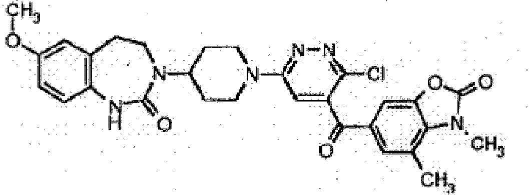 |
| (35) | 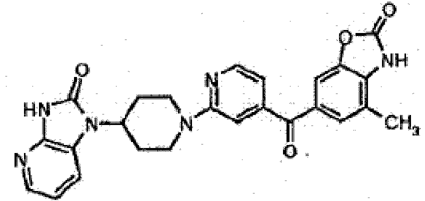 |
| (36) | 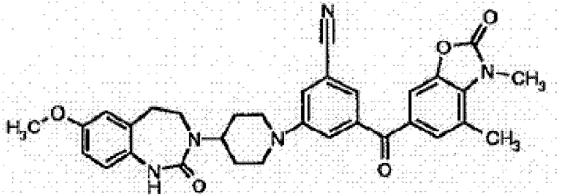 |
| (37) | 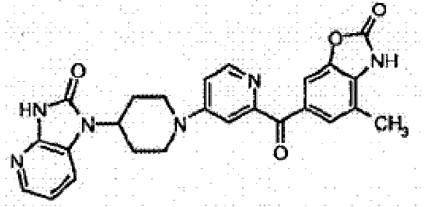 |
| (38) | 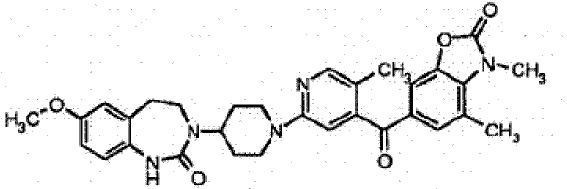 |
| (39) | 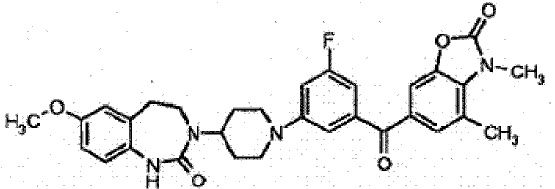 |
| (40) | 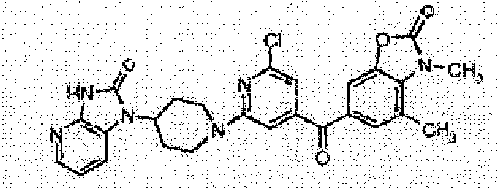 |
| (41) | 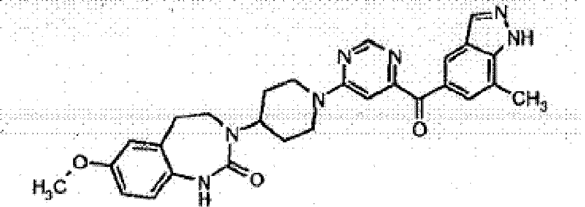 |
| (42) | 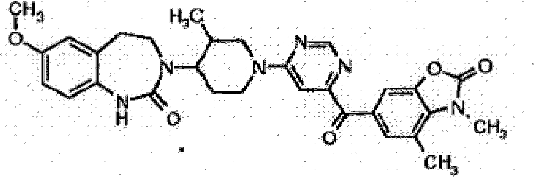 |
| (43) | 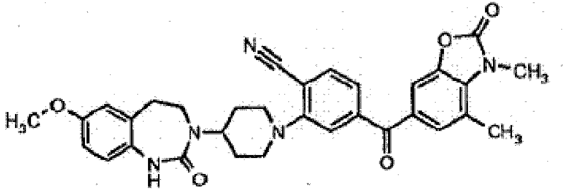 |
| (44) | 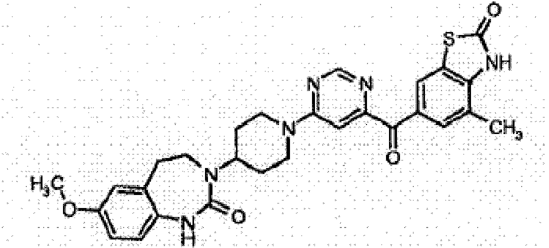 |
| (45) | 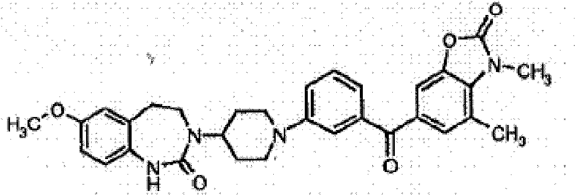 |
| (46) | 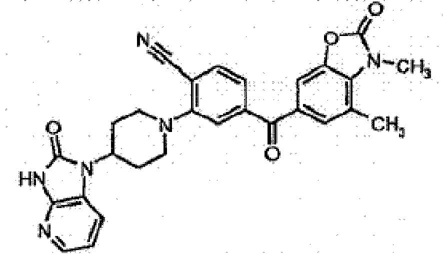 |
| (47) | 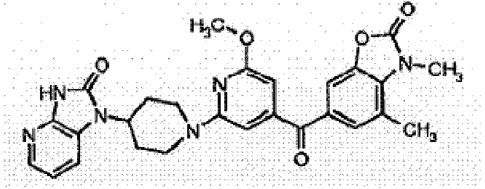 |
| (48) | 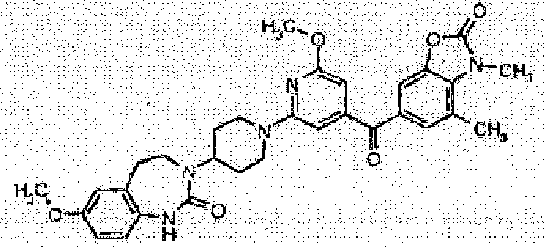 |
| (49) | 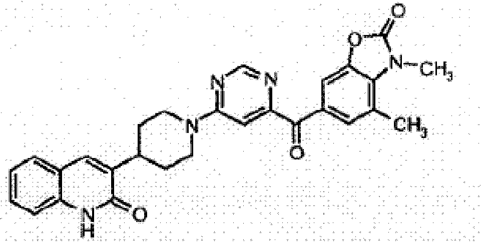 |
| (50) | 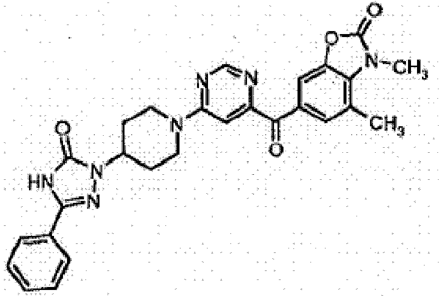 |
| (51) | 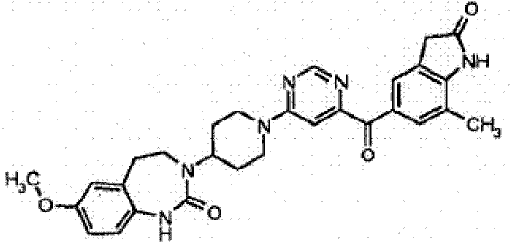 |
| (52) | 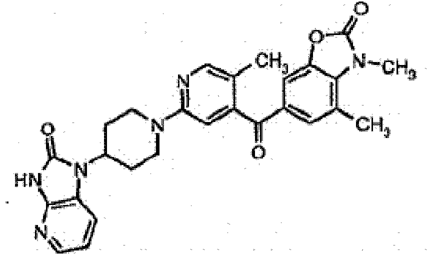 |
| (53) | 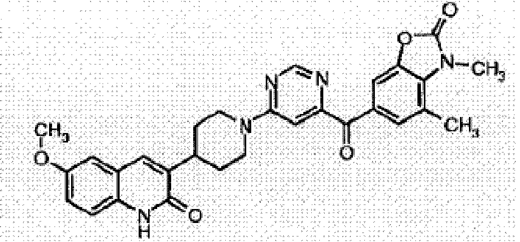 |
| (54) | 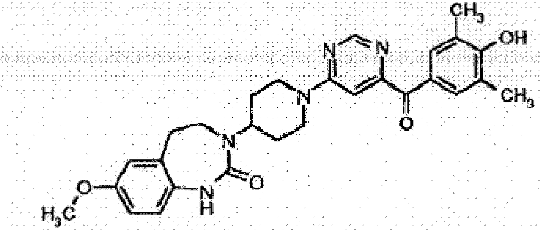 |
| (55) | 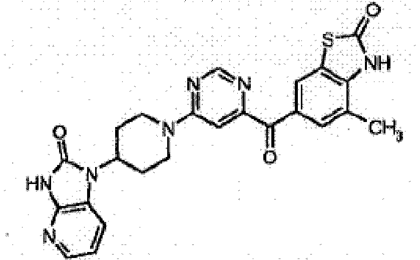 |
| (56) | 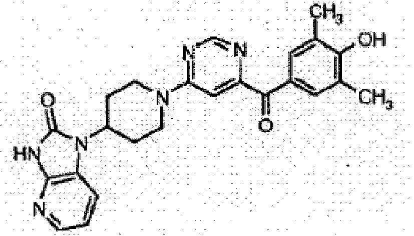 |
| (57) | 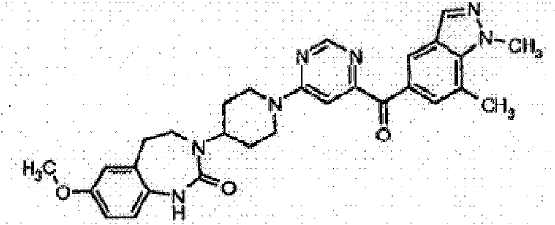 |
| (58) | 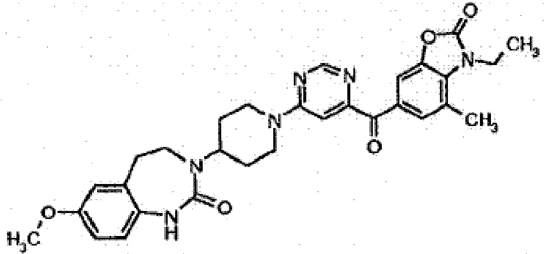 |
| (59) | 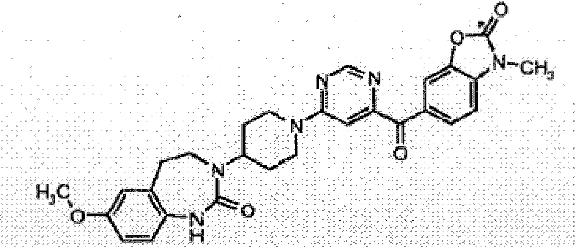 |
| (60) | 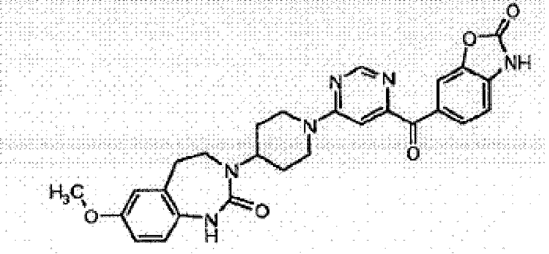 |
| (61) | 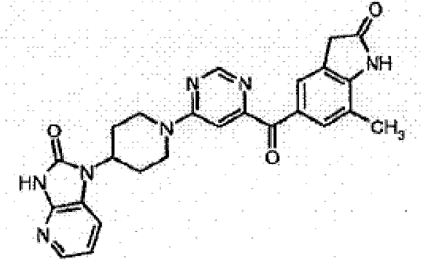 |
| (62) | 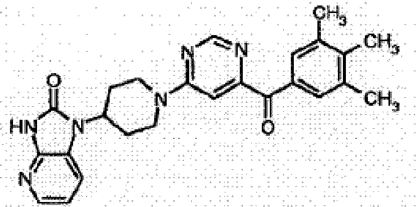 |
| (63) | 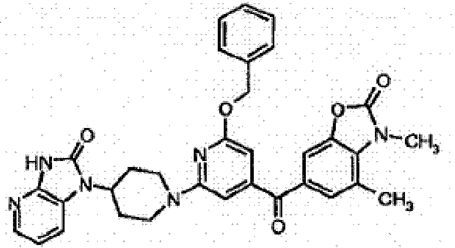 |
| (64) | 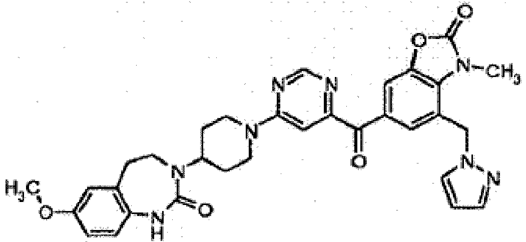 |
| (65) | 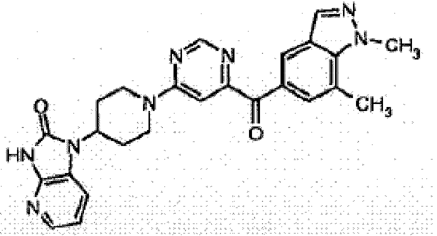 |
| (66) | 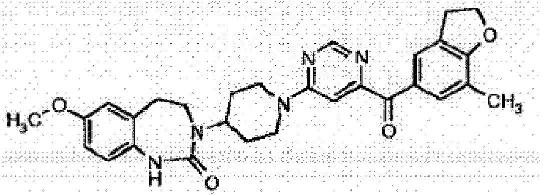 |
| (67) | 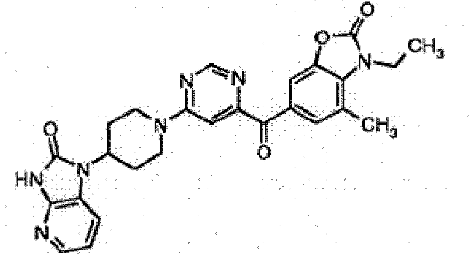 |
| (68) | 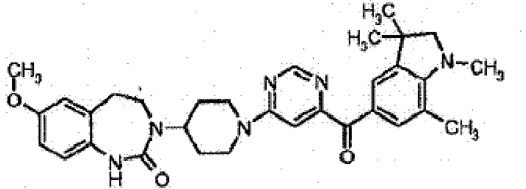 |
| (69) | 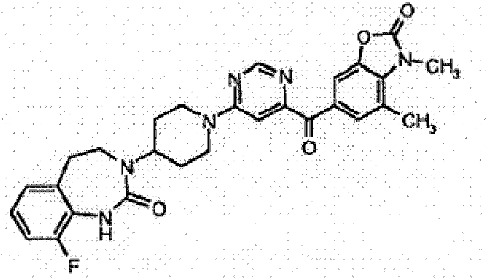 |
| (70) | 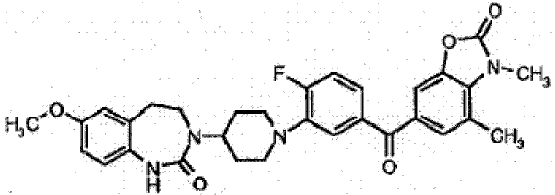 |
| (71) | 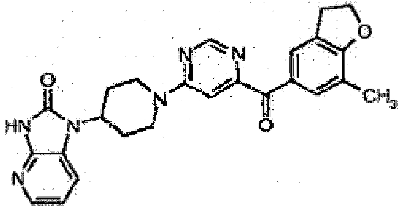 |
| (72) | 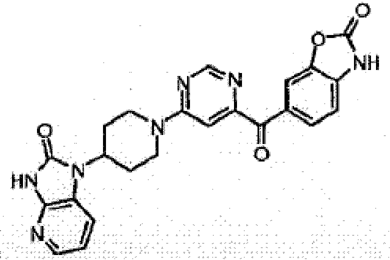 |
| (73) | 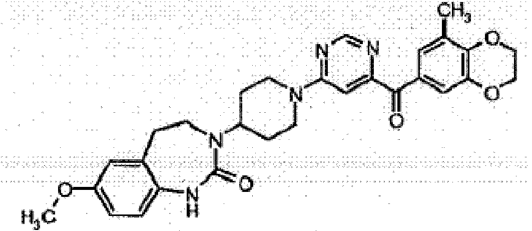 |
| (74) | 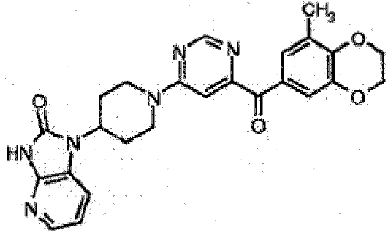 |
| (75) | 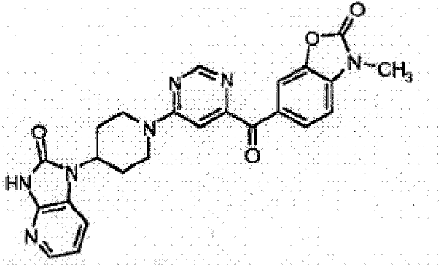 |
| (76) | 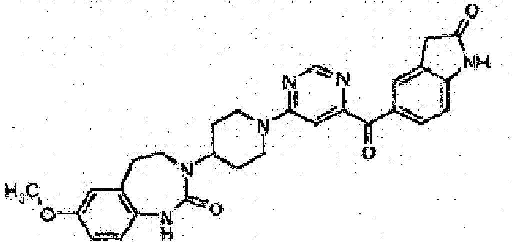 |
| (77) | 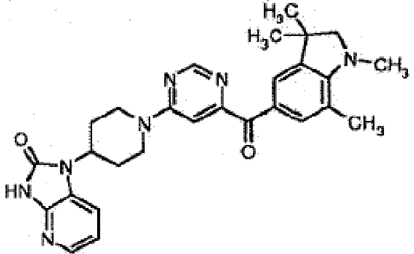 |
| (78) | 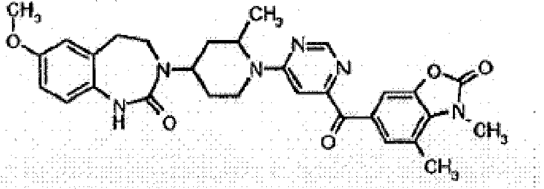 |
| (79) | 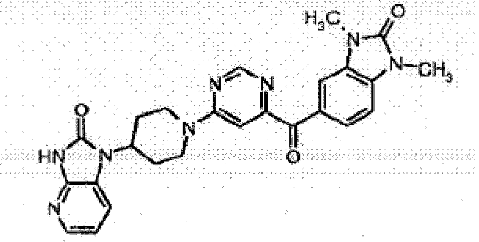 |
| (80) | 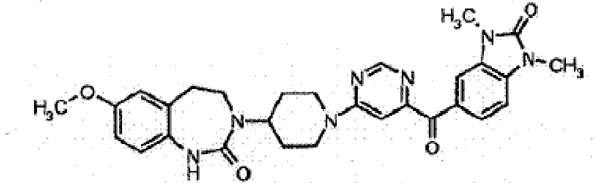 |
| (81) | 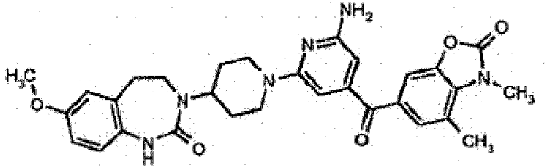 |
| (82) | 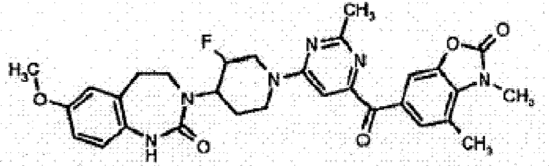 |
| (83) | 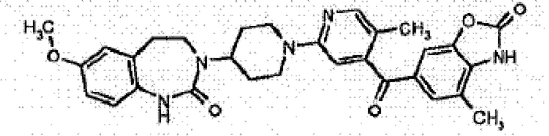 |
| (84) | 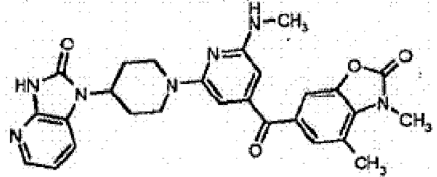 |
| (85) | 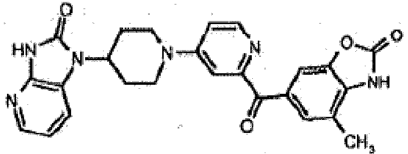 |
leurs énantiomères, leurs diastéréomères, leurs hydrates, leurs mélanges et leurs sels et les hydrates des sels, en particulier, leurs sels physiologiquement acceptables avec des acides ou des bases inorganiques ou organiques.

13. Sels physiologiquement acceptables des composés selon l'une des revendications 1 à 12, avec des acides ou des bases inorganiques ou organiques.

14. Médicament contenant un composé selon l'une des revendications 1 à 12 ou un sel physiologiquement acceptable selon la revendication 13, outre éventuellement un ou plusieurs adjuvants et/ou diluants inertes.

15. Utilisation d'un composé selon l'une des revendications 1 à 13, pour la production d'un médicament pour le traitement aigu ou prophylactique des céphalées, en particulier, des migraines et des céphalées de Horton (cluster).

16. Utilisation d'un composé selon l'une des revendications 1 à 13, pour la production d'un médicament pour le traitement du diabète sucré non insulinodépendant ("NIDDM"), du syndrome douloureux régional complexe (CRPS1), des maladies cardiovasculaires, de la tolérance à la morphine, des diarrhées dues à la toxine Clostridium, des maladies de peau, en particulier, des lésions cutanées thermiques et dues aux rayonnements, y compris des coups de soleil, des maladies inflammatoires, par exemple, les maladies articulaires inflammatoires (arthrite), des inflammations neurogènes de la muqueuse buccale, des maladies pulmonaires inflammatoires, de la rhinite allergique, de l'asthme, des maladies qui s'accompagnent d'une vasodilatation excessive et de la réduction de l'irrigation sanguine des tissus due à celle-ci, par exemple, d'un choc et d'une septicémie, pour l'atténuation des douleurs ou pour la prévention ou le traitement thérapeutique aigu des symptômes des bouffées de chaleur ménopausiques provoquées par une vasodilatation et une augmentation du débit sanguin des femmes présentant un déficit d'oestrogènes et des patients souffrant d'un cancer de la prostate soumis à un traitement hormonal.

17. Procédé de production d'un médicament selon la revendication 14, **caractérisé en ce que**, de façon non chimique, un composé selon l'une des revendications 1 à 13 est intégré dans un ou plusieurs adjuvants et/ou solvants inertes.
